# EUROPEAN PATENT SPECIFICATION

(11) **EP 1 155 304 B1**
(45) Date of publication and mention of the grant of the patent: **07.05.2003**
(21) Application number: 00914701.8
(22) Date of filing: 25.02.2000
(51) Int. Cl.: G01N 15/14

(54) **A SYSTEM FOR CELL-BASED SCREENING**
EIN SYSTEM FÜR ZELLBASIERTE REIHENUNTERSUCHUNGEN
SYSTEME DE CRIBLAGE CELLULAIRE

(30) Priority: 26.02.1999 US 122152 P; 08.03.1999 US 123399 P; 12.07.1999 US 352171
(43) Date of publication of application: 21.11.2001
(60) Divisional application: 03004224.6
(73) Proprietor: Cellomics, Inc., Pittsburgh, PA 15238 (US)
(72) Inventor: GIULIANO, Kenneth, A., Pittsburgh, PA 15209 (US); KAPUR, Ravi, Gibsonia, PA 15044 (US)
(74) Representative: Grund, Martin, Dr.
(86) International application number: US0004794
(87) International publication number: WO00050872

(56) References cited:
- WO-A-97/38127
- WO-A-98/38490
- WO-A-98/45704
- GIULIANO K A ET AL: "Fluorescent-protein biosensors: new tools for drug discovery" TRENDS IN BIOTECHNOLOGY,GB,ELSEVIER PUBLICATIONS, CAMBRIDGE, vol. 16, no. 3, 1 March 1998 (1998-03-01), pages 135-140, XP004108592 ISSN: 0167-7799

### SEQUENCE LISTING

<110> Giuliano, Kenneth A.
   Kapur, Ravi
<120> A System for Cell Based Screening
<130> 97-022-L
<140> To Be Assigned
<141> Filed Herewith
<160> 180
<170> PatentIn Ver. 2.0
<210> 1
<211> 1770
<212> DNA
<213> Artificial Sequence
<220>
<221> CDS
<222> (1)..(882)
<220>
<223> Description of Artificial Sequence:
   GFP-DEVD-Annexin II construct
<400> 1
   atg gtg agc aag ggc gag gag ctg ttc acc ggg gtg gtg ccc atc ctg   48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc   96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc   144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc   192
   Cys Thr Thr Gly Lys Leu Pro Val Pro Trp Pro Thr Leu Val Thr Thr
       50                  55                  60
   ctg acc tac ggc gtg cag tgc ttc agc cgc tac ccc gac cac atg aag   240
   Leu Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag   288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag   336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc   384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac    432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac    480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc    528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc    576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg    624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc    672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag tcc    720
   Val Thr Ala Ala Gly Ile Thr Leu Gly Met Asp Glu Leu Tyr Lys Ser
   225                 230                 235                 240
   gga ctc aga tct ggc gcc ggc gct gga gcc gga gct ggc gcc gga gcc    768
   Gly Leu Arg Ser Gly Ala Gly Ala Gly Ala Gly Ala Gly Ala Gly Ala
                   245                 250                 255
   gac gag gtg gac ggc gcc ggc gcc gat gaa gta gat ggc gcc atg tct    816
   Asp Glu Val Asp Gly Ala Gly Ala Asp Glu Val Asp Gly Ala Met Ser
               260                 265                 270
   act gtc cac gaa atc ctg tgc aag ctc agc ttg gag ggt gat cat tct    864
   Thr Val His Glu Ile Leu Cys Lys Leu Ser Leu Glu Gly Asp His Ser
           275                 280                 285
   aca ccc cca agt gcc tat tgaatggtga gcaagggcga ggagctgttc          912
   Thr Pro Pro Ser Ala Tyr
       290
   accggggtgg tgcccatcct ggtcgagctg gacggcgacg taaacggcca caagttcagc 972
   gtgtccggcg agggcgaggg cgatgccacc tacggcaagc tgaccctgaa gttcatctgc 1032
   accaccggca agctgcccgt gccctggccc accctcgtga ccaccctgac ctacggcgtg 1092
   cagtgcttca gccgctaccc cgaccacatg aagcagcacg acttcttcaa gtccgccatg 1152
   cccgaaggct acgtccagga gcgcaccatc ttcttcaagg acgacggcaa ctacaagacc 1212
   cgcgccgagg tgaagttcga gggcgacacc ctggtgaacc gcatcgagct gaagggcatc 1272
   gacttcaagg aggacggcaa catcctgggg cacaagctgg agtacaacta caacagccac 1332
   aacgtctata tcatggccga caagcagaag aacggcatca aggtgaactt caagatccgc 1392
   cacaacatcg aggacggcag cgtgcagctc gccgaccact accagcagaa cacccccatc 1452
   ggcgacggcc ccgtgctgct gcccgacaac cactacctga gcacccagtc cgccctgagc 1512
   aaagacccca acgagaagcg cgatcacatg gtcctgctgg agttcgtgac cgccgccggg 1572
   atcactctcg gcatggacga gctgtacaag tccggactca gatctggcgc cggcgctgga 1632
   gccggagctg gcgccggagc cgacgaggtg gacggcgccg gcgccgatga agtagatggc 1692
   gccatgtcta ctgtccacga aatcctgtgc aagctcagct tggagggtga tcattctaca 1752
   cccccaagtg cctattga 1770
<210> 2
<211> 294
<212> PRT
<213> Artificial Sequence
<220>
<223> Description of Artificial Sequence:
   GFP-DEVD-Annexin II construct
<400> 2
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Ser
   225                 230                 235                 240
   Gly Leu Arg Ser Gly Ala Gly Ala Gly Ala Gly Ala Gly Ala Gly Ala
                   245                 250                 255
   Asp Glu Val Asp Gly Ala Gly Ala Asp Glu Val Asp Gly Ala Met Ser
               260                 265                 270
   Thr Val His Glu Ile Leu Cys Lys Leu Ser Leu Glu Gly Asp His Ser
           275                 280                 285
   Thr Pro Pro Ser Ala Tyr
       290
<210> 3
<211> 2439
<212> DNA
<213> Artificial Sequence
<220>
<221> CDS
<222> (1)..(2436)
<220>
<223> Description of Artificial Sequence:
   EYFP-DEVD-MAPKDM construct
<400> 3
   atg gtg agc aag ggc gag gag ctg ttc acc ggg gtg gtg ccc atc ctg     48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc     96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc    144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc    192
   Cys Thr Thr Gly Lys Leu Pro Val Pro Trp Pro Thr Leu Val Thr Thr
       50                  55                  60
   ttc ggc tac ggc ctg cag tgc ttc gcc cgc tac ccc gac cac atg aag    240
   Phe Gly Tyr Gly Leu Gln Cys Phe Ala Arg Tyr Pro Asp His Met Lys
   65                  70                  75                  80
   cag cac gac ttc ttc aag tcc gcc atg ccc gaa ggc tac gtc cag gag    288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag    336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg     624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag aag     720
   Val Thr Ala Ala Gly Ile Thr Leu Gly Met Asp Glu Leu Tyr Lys Lys
   225                 230                 235                 240
   gga gac gaa gtg gac gga gcc gac ctc agt ctt gtg gat gcg ttg aca     768
   Gly Asp Glu Val Asp Gly Ala Asp Leu Ser Leu Val Asp Ala Leu Thr
                   245                 250                 255
   gaa cca cct cca gaa att gag gga gaa ata aag cga gac ttc atg gct     816
   Glu Pro Pro Pro Glu Ile Glu Gly Glu Ile Lys Arg Asp Phe Met Ala
               260                 265                 270
   gcg ctg gag gca gag ccc tat gat gac atc gtg gga gaa act gtg gag     864
   Ala Leu Glu Ala Glu Pro Tyr Asp Asp Ile Val Gly Glu Thr Val Glu
           275                 280                 285
   aaa act gag ttt att cct ctc ctg gat ggt gat gag aaa acc ggg aac     912
   Lys Thr Glu Phe Ile Pro Leu Leu Asp Gly Asp Glu Lys Thr Gly Asn
       290                 295                 300
   tca gag tcc aaa aag aaa ccc tgc tta gac act agc cag gtt gaa ggt     960
   Ser Glu Ser Lys Lys Lys Pro Cys Leu Asp Thr Ser Gln Val Glu Gly
   305                 310                 315                 320
   atc cca tct tct aaa cca aca ctc cta gcc aat ggt gat cat gga atg    1008
   Ile Pro Ser Ser Lys Pro Thr Leu Leu Ala Asn Gly Asp His Gly Met
                   325                 330                 335
   gag ggg aat aac act gca ggg tct cca act gac ttc ctt gaa gag aga    1056
   Glu Gly Asn Asn Thr Ala Gly Ser Pro Thr Asp Phe Leu Glu Glu Arg
   340 345 350
   gtg gac tat ccg gat tat cag agc agc cag aac tgg cca gaa gat gca 1104
   Val Asp Tyr Pro Asp Tyr Gln Ser Ser Gln Asn Trp Pro Glu Asp Ala
   355 360 365
   agc ttt tgt ttc cag cct cag caa gtg tta gat act gac cag gct gag 1152
   Ser Phe Cys Phe Gln Pro Gln Gln Val Leu Asp Thr Asp Gln Ala Glu
   370 375 380
   ccc ttt aac gag cac cgt gat gat ggt ttg gca gat ctg ctc ttt gtc 1200
   Pro Phe Asn Glu His Arg Asp Asp Gly Leu Ala Asp Leu Leu Phe Val
   385 390 395 400
   tcc agt gga ccc acg aac gct tct gca ttt aca gag cga gac aat cct 1248
   Ser Ser Gly Pro Thr Asn Ala Ser Ala Phe Thr Glu Arg Asp Asn Pro
   405 410 415
   tca gaa gac agt tac ggt atg ctt ccc tgt gac tca ttt gct tcc acg 1296
   Ser Glu Asp Ser Tyr Gly Met Leu Pro Cys Asp Ser Phe Ala Ser Thr
   420 425 430
   gct gtt gta tct cag gag tgg tct gtg gga gcc cca aac tct cca tgt 1344
   Ala Val Val Ser Gln Glu Trp Ser Val Gly Ala Pro Asn Ser Pro Cys
   435 440 445
   tca gag tcc tgt gtc tcc cca gag gtt act ata gaa acc cta cag cca 1392
   Ser Glu Ser Cys Val Ser Pro Glu Val Thr Ile Glu Thr Leu Gln Pro
   450 455 460
   gca aca gag ctc tcc aag gca gca gaa gtg gaa tca gtg aaa gag cag 1440
   Ala Thr Glu Leu Ser Lys Ala Ala Glu Val Glu Ser Val Lys Glu Gln
   465 470 475 480
   ctg cca gct aaa gca ttg gaa acg atg gca gag cag acc act gat gtg 1488
   Leu Pro Ala Lys Ala Leu Glu Thr Met Ala Glu Gln Thr Thr Asp Val
   485 490 495
   gtg cac tct cca tcc aca gac aca aca cca ggc cca gac aca gag gca 1536
   Val His Ser Pro Ser Thr Asp Thr Thr Pro Gly Pro Asp Thr Glu Ala
   500 505 510
   gca ctg gct aaa gac ata gaa gag atc acc aag cca gat gtg ata ttg 1584
   Ala Leu Ala Lys Asp Ile Glu Glu Ile Thr Lys Pro Asp Val Ile Leu
   515 520 525
   gca aat gtc acg cag cca tct act gaa tcg gat atg ttc ctg gcc cag 1632
   Ala Asn Val Thr Gln Pro Ser Thr Glu Ser Asp Met Phe Leu Ala Gln
   530 535 540
   gac atg gaa cta ctc aca gga aca gag gca gcc cac gct aac aat atc 1680
   Asp Met Glu Leu Leu Thr Gly Thr Glu Ala Ala His Ala Asn Asn Ile
   545 550 555 560
   ata ttg cct aca gaa cca gac gaa tct tca acc aag gat gta gca cca 1728
   Ile Leu Pro Thr Glu Pro Asp Glu Ser Ser Thr Lys Asp Val Ala Pro
   565 570 575
   cct atg gaa gaa gaa att gtc cca ggc aat gat acg aca tcc ccc aaa 1776
   Pro Met Glu Glu Glu Ile Val Pro Gly Asn Asp Thr Thr Ser Pro Lys
   580 585 590
   gaa aca gag aca aca ctt cca ata aaa atg gac ttg gca cca cct gag   1824
   Glu Thr Glu Thr Thr Leu Pro Ile Lys Met Asp Leu Ala Pro Pro Glu
           595                 600                 605
   gat gtg tta ctt acc aaa gaa aca gaa cta gcc cca gcc aag ggc atg   1872
   Asp Val Leu Leu Thr Lys Glu Thr Glu Leu Ala Pro Ala Lys Gly Met
       610                 615                 620
   gtt tca ctc tca gaa ata gaa gag gct ctg gca aag aat gat gtt cgc   1920
   Val Ser Leu Ser Glu Ile Glu Glu Ala Leu Ala Lys Asn Asp Val Arg
   625                 630                 635                 640
   tct gca gaa ata cct gtg gct cag gag aca gtg gtc tca gaa aca gag   1968
   Ser Ala Glu Ile Pro Val Ala Gln Glu Thr Val Val Ser Glu Thr Glu
                   645                 650                 655
   gtg gtc ctg gca aca gaa gtg gta ctg ccc tca gat ccc ata aca aca   2016
   Val Val Leu Ala Thr Glu Val Val Leu Pro Ser Asp Pro Ile Thr Thr
               660                 665                 670
   ttg aca aag gat gtg aca ctc ccc tta gaa gca gag aga ccg ttg gtg   2064
   Leu Thr Lys Asp Val Thr Leu Pro Leu Glu Ala Glu Arg Pro Leu Val
           675                 680                 685
   acg gac atg act cca tct ctg gaa aca gaa atg acc cta ggc aaa gag   2112
   Thr Asp Met Thr Pro Ser Leu Glu Thr Glu Met Thr Leu Gly Lys Glu
       690                 695                 700
   aca gct cca ccc aca gaa aca aat ttg ggc atg gcc aaa gac atg tct   2160
   Thr Ala Pro Pro Thr Glu Thr Asn Leu Gly Met Ala Lys Asp Met Ser
   705                 710                 715                 720
   cca ctc cca gaa tca gaa gtg act ctg ggc aag gac gtg gtt ata ctt   2208
   Pro Leu Pro Glu Ser Glu Val Thr Leu Gly Lys Asp Val Val Ile Leu
                   725                 730                 735
   cca gaa aca aag gtg gct gag ttt aac aat gtg act cca ctt tca gaa   2256
   Pro Glu Thr Lys Val Ala Glu Phe Asn Asn Val Thr Pro Leu Ser Glu
               740                 745                 750
   gaa gag gta acc tca gtc aag gac atg tct ccg tct gca gaa aca gag   2304
   Glu Glu Val Thr Ser Val Lys Asp Met Ser Pro Ser Ala Glu Thr Glu
           755                 760                 765
   gct ccc ctg gct aag aat gct gat ctg cac tca gga aca gag ctg att   2352
   Ala Pro Leu Ala Lys Asn Ala Asp Leu His Ser Gly Thr Glu Leu Ile
       770                 775                 780
   gtg gac aac agc atg gct cca gcc tcc gat ctt gca ctg ccc ttg gaa   2400
   Val Asp Asn Ser Met Ala Pro Ala Ser Asp Leu Ala Leu Pro Leu Glu
   785                 790                 795                 800
   aca aaa gta gca aca gtt cca att aaa gac aaa gga tga               2439
   Thr Lys Val Ala Thr Val Pro Ile Lys Asp Lys Gly
                   805                 810
<210> 4
<211> 812
<212> PRT
<213> Artificial Sequence
<220>
<223> Description of Artificial Sequence:
   EYFP-DEVD-MAPKDM construct
<400> 4
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Lys
   225                 230                 235                 240
   Gly Asp Glu Val Asp Gly Ala Asp Leu Ser Leu Val Asp Ala Leu Thr
                   245                 250                 255
   Glu Pro Pro Pro Glu Ile Glu Gly Glu Ile Lys Arg Asp Phe Met Ala
               260                 265                 270
   Ala Leu Glu Ala Glu Pro Tyr Asp Asp Ile Val Gly Glu Thr Val Glu
           275                 280                 285
   Lys Thr Glu Phe Ile Pro Leu Leu Asp Gly Asp Glu Lys Thr Gly Asn
   290 295 300
   Ser Glu Ser Lys Lys Lys Pro Cys Leu Asp Thr Ser Gln Val Glu Gly
   305 310 315 320
   Ile Pro Ser Ser Lys Pro Thr Leu Leu Ala Asn Gly Asp His Gly Met
   325 330 335
   Glu Gly Asn Asn Thr Ala Gly Ser Pro Thr Asp Phe Leu Glu Glu Arg
   340 345 350
   Val Asp Tyr Pro Asp Tyr Gln Ser Ser Gln Asn Trp Pro Glu Asp Ala
   355 360 365
   Ser Phe Cys Phe Gln Pro Gln Gln Val Leu Asp Thr Asp Gln Ala Glu
   370 375 380
   Pro Phe Asn Glu His Arg Asp Asp Gly Leu Ala Asp Leu Leu Phe Val
   385 390 395 400
   Ser Ser Gly Pro Thr Asn Ala Ser Ala Phe Thr Glu Arg Asp Asn Pro
   405 410 415
   Ser Glu Asp Ser Tyr Gly Met Leu Pro Cys Asp Ser Phe Ala Ser Thr
   420 425 430
   Ala Val Val Ser Gln Glu Trp Ser Val Gly Ala Pro Asn Ser Pro Cys
   435 440 445
   Ser Glu Ser Cys Val Ser Pro Glu Val Thr Ile Glu Thr Leu Gln Pro
   450 455 460
   Ala Thr Glu Leu Ser Lys Ala Ala Glu Val Glu Ser Val Lys Glu Gln
   465 470 475 480
   Leu Pro Ala Lys Ala Leu Glu Thr Met Ala Glu Gln Thr Thr Asp Val
   485 490 495
   Val His Ser Pro Ser Thr Asp Thr Thr Pro Gly Pro Asp Thr Glu Ala
   500 505 510
   Ala Leu Ala Lys Asp Ile Glu Glu Ile Thr Lys Pro Asp Val Ile Leu
   515 520 525
   Ala Asn Val Thr Gln Pro Ser Thr Glu Ser Asp Met Phe Leu Ala Gln
   530 535 540
   Asp Met Glu Leu Leu Thr Gly Thr Glu Ala Ala His Ala Asn Asn Ile
   545 550 555 560
   Ile Leu Pro Thr Glu Pro Asp Glu Ser Ser Thr Lys Asp Val Ala Pro
   565 570 575
   Pro Met Glu Glu Glu Ile Val Pro Gly Asn Asp Thr Thr Ser Pro Lys
   580 585 590
   Glu Thr Glu Thr Thr Leu Pro Ile Lys Met Asp Leu Ala Pro Pro Glu
   595 600 605
   Asp Val Leu Leu Thr Lys Glu Thr Glu Leu Ala Pro Ala Lys Gly Met
   610 615 620
   Val Ser Leu Ser Glu Ile Glu Glu Ala Leu Ala Lys Asn Asp Val Arg
   625 630 635 640
   Ser Ala Glu Ile Pro Val Ala Gln Glu Thr Val Val Ser Glu Thr Glu
   645 650 655
   Val Val Leu Ala Thr Glu Val Val Leu Pro Ser Asp Pro Ile Thr Thr
   660 665 670
   Leu Thr Lys Asp Val Thr Leu Pro Leu Glu Ala Glu Arg Pro Leu Val
   675 680 685
   Thr Asp Met Thr Pro Ser Leu Glu Thr Glu Met Thr Leu Gly Lys Glu
   690 695 700
   Thr Ala Pro Pro Thr Glu Thr Asn Leu Gly Met Ala Lys Asp Met Ser
   705 710 715 720
   Pro Leu Pro Glu Ser Glu Val Thr Leu Gly Lys Asp Val Val Ile Leu
   725 730 735
   Pro Glu Thr Lys Val Ala Glu Phe Asn Asn Val Thr Pro Leu Ser Glu
   740 745 750
   Glu Glu Val Thr Ser Val Lys Asp Met Ser Pro Ser Ala Glu Thr Glu
   755 760 765
   Ala Pro Leu Ala Lys Asn Ala Asp Leu His Ser Gly Thr Glu Leu Ile
   770 775 780
   Val Asp Asn Ser Met Ala Pro Ala Ser Asp Leu Ala Leu Pro Leu Glu
   785 790 795 800
   Thr Lys Val Ala Thr Val Pro Ile Lys Asp Lys Gly
   805 810
<210> 5
<211> 2439
<212> DNA
<213> Artificial Sequence
<220>
<221> CDS
<222> (1)..(2436)
<220>
<223> Description of Artificial Sequence:
   EYFP-DEAD-MAPKDM construct
<400> 5
   atg gtg agc aag ggc gag gag ctg ttc acc ggg gtg gtg ccc atc ctg 48
   Met Val Ser Lys Gly Glu Glu Leu Phe Thr Gly Val Val Pro Ile Leu
   1 5 10 15
   gtc gag ctg gac ggc gac gta aac ggc cac aag ttc agc gtg tcc ggc 96
   Val Glu Leu Asp Gly Asp Val Asn Gly His Lys Phe Ser Val Ser Gly
   20 25 30
   gag ggc gag ggc gat gcc acc tac ggc aag ctg acc ctg aag ttc atc     144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ttc ggc tac ggc ctg cag tgc ttc gcc cgc tac ccc gac cac atg aag     240
   Phe Gly Tyr Gly Leu Gln Cys Phe Ala Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg     624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag ccc     720
   Val Thr Ala Ala Gly Ile Thr Leu Gly Met Asp Glu Leu Tyr Lys Pro
   225                 230                 235                 240
   aga gac gaa gcc gac agc gcc gac ctc agt ctt gtg gat gcg ttg aca     768
   Arg Asp Glu Ala Asp Ser Ala Asp Leu Ser Leu Val Asp Ala Leu Thr
                   245                 250                 255
   gaa cca cct cca gaa att gag gga gaa ata aag cga gac ttc atg gct     816
   Glu Pro Pro Pro Glu Ile Glu Gly Glu Ile Lys Arg Asp Phe Met Ala
               260                 265                 270
   gcg ctg gag gca gag ccc tat gat gac atc gtg gga gaa act gtg gag     864
   Ala Leu Glu Ala Glu Pro Tyr Asp Asp Ile Val Gly Glu Thr Val Glu
           275                 280                 285
   aaa act gag ttt att cct ctc ctg gat ggt gat gag aaa acc ggg aac      912
   Lys Thr Glu Phe Ile Pro Leu Leu Asp Gly Asp Glu Lys Thr Gly Asn
       290                 295                 300
   tca gag tcc aaa aag aaa ccc tgc tta gac act agc cag gtt gaa ggt      960
   Ser Glu Ser Lys Lys Lys Pro Cys Leu Asp Thr Ser Gln Val Glu Gly
   305                 310                 315                 320
   atc cca tct tct aaa cca aca ctc cta gcc aat ggt gat cat gga atg     1008
   Ile Pro Ser Ser Lys Pro Thr Leu Leu Ala Asn Gly Asp His Gly Met
                   325                 330                 335
   gag ggg aat aac act gca ggg tct cca act gac ttc ctt gaa gag aga     1056
   Glu Gly Asn Asn Thr Ala Gly Ser Pro Thr Asp Phe Leu Glu Glu Arg
               340                 345                 350
   gtg gac tat ccg gat tat cag agc agc cag aac tgg cca gaa gat gca     1104
   Val Asp Tyr Pro Asp Tyr Gln Ser Ser Gln Asn Trp Pro Glu Asp Ala
           355                 360                 365
   agc ttt tgt ttc cag cct cag caa gtg tta gat act gac cag gct gag     1152
   Ser Phe Cys Phe Gln Pro Gln Gln Val Leu Asp Thr Asp Gln Ala Glu
       370                 375                 380
   ccc ttt aac gag cac cgt gat gat ggt ttg gca gat ctg ctc ttt gtc     1200
   Pro Phe Asn Glu His Arg Asp Asp Gly Leu Ala Asp Leu Leu Phe Val
   385                 390                 395                 400
   tcc agt gga ccc acg aac gct tct gca ttt aca gag cga gac aat cct     1248
   Ser Ser Gly Pro Thr Asn Ala Ser Ala Phe Thr Glu Arg Asp Asn Pro
                   405                 410                 415
   tca gaa gac agt tac ggt atg ctt ccc tgt gac tca ttt gct tcc acg     1296
   Ser Glu Asp Ser Tyr Gly Met Leu Pro Cys Asp Ser Phe Ala Ser Thr
               420                 425                 430
   gct gtt gta tct cag gag tgg tct gtg gga gcc cca aac tct cca tgt     1344
   Ala Val Val Ser Gln Glu Trp Ser Val Gly Ala Pro Asn Ser Pro Cys
           435                 440                 445
   tca gag tcc tgt gtc tcc cca gag gtt act ata gaa acc cta cag cca     1392
   Ser Glu Ser Cys Val Ser Pro Glu Val Thr Ile Glu Thr Leu Gln Pro
       450                 455                 460
   gca aca gag ctc tcc aag gca gca gaa gtg gaa tca gtg aaa gag cag     1440
   Ala Thr Glu Leu Ser Lys Ala Ala Glu Val Glu Ser Val Lys Glu Gln
   465                 470                 475                 480
   ctg cca gct aaa gca ttg gaa acg atg gca gag cag acc act gat gtg     1488
   Leu Pro Ala Lys Ala Leu Glu Thr Met Ala Glu Gln Thr Thr Asp Val
                   485                 490                 495
   gtg cac tct cca tcc aca gac aca aca cca ggc cca gac aca gag gca     1536
   Val His Ser Pro Ser Thr Asp Thr Thr Pro Gly Pro Asp Thr Glu Ala
               500                 505                 510
   gca ctg gct aaa gac ata gaa gag atc acc aag cca gat gtg ata ttg     1584
   Ala Leu Ala Lys Asp Ile Glu Glu Ile Thr Lys Pro Asp Val Ile Leu
   515 520 525
   gca aat gtc acg cag cca tct act gaa tcg gat atg ttc ctg gcc cag 1632
   Ala Asn Val Thr Gln Pro Ser Thr Glu Ser Asp Met Phe Leu Ala Gln
   530 535 540
   gac atg gaa cta ctc aca gga aca gag gca gcc cac gct aac aat atc 1680
   Asp Met Glu Leu Leu Thr Gly Thr Glu Ala Ala His Ala Asn Asn Ile
   545 550 555 560
   ata ttg cct aca gaa cca gac gaa tct tca acc aag gat gta gca cca 1728
   Ile Leu Pro Thr Glu Pro Asp Glu Ser Ser Thr Lys Asp Val Ala Pro
   565 570 575
   cct atg gaa gaa gaa att gtc cca ggc aat gat acg aca tcc ccc aaa 1776
   Pro Met Glu Glu Glu Ile Val Pro Gly Asn Asp Thr Thr Ser Pro Lys
   580 585 590
   gaa aca gag aca aca ctt cca ata aaa atg gac ttg gca cca cct gag 1824
   Glu Thr Glu Thr Thr Leu Pro Ile Lys Met Asp Leu Ala Pro Pro Glu
   595 600 605
   gat gtg tta ctt acc aaa gaa aca gaa cta gcc cca gcc aag ggc atg 1872
   Asp Val Leu Leu Thr Lys Glu Thr Glu Leu Ala Pro Ala Lys Gly Met
   610 615 620
   gtt tca ctc tca gaa ata gaa gag gct ctg gca aag aat gat gtt cgc 1920
   Val Ser Leu Ser Glu Ile Glu Glu Ala Leu Ala Lys Asn Asp Val Arg
   625 630 635 640
   tct gca gaa ata cct gtg gct cag gag aca gtg gtc tca gaa aca gag 1968
   Ser Ala Glu Ile Pro Val Ala Gln Glu Thr Val Val Ser Glu Thr Glu
   645 650 655
   gtg gtc ctg gca aca gaa gtg gta ctg ccc tca gat ccc ata aca aca 2016
   Val Val Leu Ala Thr Glu Val Val Leu Pro Ser Asp Pro Ile Thr Thr
   660 665 670
   ttg aca aag gat gtg aca ctc ccc tta gaa gca gag aga ccg ttg gtg 2064
   Leu Thr Lys Asp Val Thr Leu Pro Leu Glu Ala Glu Arg Pro Leu Val
   675 680 685
   acg gac atg act cca tct ctg gaa aca gaa atg acc cta ggc aaa gag 2112
   Thr Asp Met Thr Pro Ser Leu Glu Thr Glu Met Thr Leu Gly Lys Glu
   690 695 700
   aca gct cca ccc aca gaa aca aat ttg ggc atg gcc aaa gac atg tct 2160
   Thr Ala Pro Pro Thr Glu Thr Asn Leu Gly Met Ala Lys Asp Met Ser
   705 710 715 720
   cca ctc cca gaa tca gaa gtg act ctg ggc aag gac gtg gtt ata ctt 2208
   Pro Leu Pro Glu Ser Glu Val Thr Leu Gly Lys Asp Val Val Ile Leu
   725 730 735
   cca gaa aca aag gtg gct gag ttt aac aat gtg act cca ctt tca gaa 2256
   Pro Glu Thr Lys Val Ala Glu Phe Asn Asn Val Thr Pro Leu Ser Glu
   740 745 750
   gaa gag gta acc tca gtc aag gac atg tct ccg tct gca gaa aca gag 2304
   Glu Glu Val Thr Ser Val Lys Asp Met Ser Pro Ser Ala Glu Thr Glu
   755 760 765
   gct ccc ctg gct aag aat gct gat ctg cac tca gga aca gag ctg att     2352
   Ala Pro Leu Ala Lys Asn Ala Asp Leu His Ser Gly Thr Glu Leu Ile
       770                 775                 780
   gtg gac aac agc atg gct cca gcc tcc gat ctt gca ctg ccc ttg gaa     2400
   Val Asp Asn Ser Met Ala Pro Ala Ser Asp Leu Ala Leu Pro Leu Glu
   785                 790                 795                 800
   aca aaa gta gca aca gtt cca att aaa gac aaa gga tga                 2439
   Thr Lys Val Ala Thr Val Pro Ile Lys Asp Lys Gly
                   805                 810
<210> 6
<211> 812
<212> PRT
<213> Artificial Sequence
<220>
<223> Description of Artificial Sequence:
   EYFP-DEAD-MAPKDM construct
<400> 6
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
               195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Pro
   225                 230                 235                 240
   Arg Asp Glu Ala Asp Ser Ala Asp Leu Ser Leu Val Asp Ala Leu Thr
                   245                 250                 255
   Glu Pro Pro Pro Glu Ile Glu Gly Glu Ile Lys Arg Asp Phe Met Ala
               260                 265                 270
   Ala Leu Glu Ala Glu Pro Tyr Asp Asp Ile Val Gly Glu Thr Val Glu
           275                 280                 285
   Lys Thr Glu Phe Ile Pro Leu Leu Asp Gly Asp Glu Lys Thr Gly Asn
       290                 295                 300
   Ser Glu Ser Lys Lys Lys Pro Cys Leu Asp Thr Ser Gln Val Glu Gly
   305                 310                 315                 320
   Ile Pro Ser Ser Lys Pro Thr Leu Leu Ala Asn Gly Asp His Gly Met
                   325                 330                 335
   Glu Gly Asn Asn Thr Ala Gly Ser Pro Thr Asp Phe Leu Glu Glu Arg
               340                 345                 350
   Val Asp Tyr Pro Asp Tyr Gln Ser Ser Gln Asn Trp Pro Glu Asp Ala
           355                 360                 365
   Ser Phe Cys Phe Gln Pro Gln Gln Val Leu Asp Thr Asp Gln Ala Glu
       370                 375                 380
   Pro Phe Asn Glu His Arg Asp Asp Gly Leu Ala Asp Leu Leu Phe Val
   385                 390                 395                 400
   Ser Ser Gly Pro Thr Asn Ala Ser Ala Phe Thr Glu Arg Asp Asn Pro
                   405                 410                 415
   Ser Glu Asp Ser Tyr Gly Met Leu Pro Cys Asp Ser Phe Ala Ser Thr
               420                 425                 430
   Ala Val Val Ser Gln Glu Trp Ser Val Gly Ala Pro Asn Ser Pro Cys
           435                 440                 445
   Ser Glu Ser Cys Val Ser Pro Glu Val Thr Ile Glu Thr Leu Gln Pro
       450                 455                 460
   Ala Thr Glu Leu Ser Lys Ala Ala Glu Val Glu Ser Val Lys Glu Gln
   465                 470                 475                 480
   Leu Pro Ala Lys Ala Leu Glu Thr Met Ala Glu Gln Thr Thr Asp Val
                   485                 490                 495
   Val His Ser Pro Ser Thr Asp Thr Thr Pro Gly Pro Asp Thr Glu Ala
               500                 505                 510
   Ala Leu Ala Lys Asp Ile Glu Glu Ile Thr Lys Pro Asp Val Ile Leu
           515                 520                 525
   Ala Asn Val Thr Gln Pro Ser Thr Glu Ser Asp Met Phe Leu Ala Gln
       530                 535                 540
   Asp Met Glu Leu Leu Thr Gly Thr Glu Ala Ala His Ala Asn Asn Ile
   545                 550                 555                 560
   Ile Leu Pro Thr Glu Pro Asp Glu Ser Ser Thr Lys Asp Val Ala Pro
                   565                 570                 575
   Pro Met Glu Glu Glu Ile Val Pro Gly Asn Asp Thr Thr Ser Pro Lys
               580                 585                 590
   Glu Thr Glu Thr Thr Leu Pro Ile Lys Met Asp Leu Ala Pro Pro Glu
           595                 600                 605
   Asp Val Leu Leu Thr Lys Glu Thr Glu Leu Ala Pro Ala Lys Gly Met
       610                 615                 620
   Val Ser Leu Ser Glu Ile Glu Glu Ala Leu Ala Lys Asn Asp Val Arg
   625                 630                 635                 640
   Ser Ala Glu Ile Pro Val Ala Gln Glu Thr Val Val Ser Glu Thr Glu
                   645                 650                 655
   Val Val Leu Ala Thr Glu Val Val Leu Pro Ser Asp Pro Ile Thr Thr
               660                 665                 670
   Leu Thr Lys Asp Val Thr Leu Pro Leu Glu Ala Glu Arg Pro Leu Val
           675                 680                 685
   Thr Asp Met Thr Pro Ser Leu Glu Thr Glu Met Thr Leu Gly Lys Glu
       690                 695                 700
   Thr Ala Pro Pro Thr Glu Thr Asn Leu Gly Met Ala Lys Asp Met Ser
   705                 710                 715                 720
   Pro Leu Pro Glu Ser Glu Val Thr Leu Gly Lys Asp Val Val Ile Leu
                   725                 730                 735
   Pro Glu Thr Lys Val Ala Glu Phe Asn Asn Val Thr Pro Leu Ser Glu
               740                 745                 750
   Glu Glu Val Thr Ser Val Lys Asp Met Ser Pro Ser Ala Glu Thr Glu
           755                 760                 765
   Ala Pro Leu Ala Lys Asn Ala Asp Leu His Ser Gly Thr Glu Leu Ile
       770                 775                 780
   Val Asp Asn Ser Met Ala Pro Ala Ser Asp Leu Ala Leu Pro Leu Glu
   785                 790                 795                 800
   Thr Lys Val Ala Thr Val Pro Ile Lys Asp Lys Gly
                   805                 810
<210> 7
<211> 864
<212> DNA
<213> Artificial Sequence
<220>
<221> CDS
<222> (1)..(861)
<220>
<223> Description of Artificial Sequence: F25-MEK1
   construct
<400> 7
   atg gct agc aaa gga gaa gaa ctc ttc act gga gtt gtc cca att ctt     48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga     96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc    144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act    192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa    240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa    288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa    336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt    384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac    432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat    480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc    528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc    576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt    624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt    672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac acc    720
   Val Thr Ala Ala Gly Ile Thr His Gly Met Asp Glu Leu Tyr Asn Thr
   225                 230                 235                 240
   ggt atg ccc aag aag aag ccg acg ccc atc cag ctg aac ccg gcc ccc    768
   Gly Met Pro Lys Lys Lys Pro Thr Pro Ile Gln Leu Asn Pro Ala Pro
                   245                 250                 255
   gac ggc tct gca gtt aac ggg acc agc tct gcg gag acc aac ttg gag    816
   Asp Gly Ser Ala Val Asn Gly Thr Ser Ser Ala Glu Thr Asn Leu Glu
               260                 265                 270
   gcc ttg cag aag aag ctg gag gag cta gag ctt gat gag cag cag tga    864
   Ala Leu Gln Lys Lys Leu Glu Glu Leu Glu Leu Asp Glu Gln Gln
           275                 280                 285
<210> 8
<211> 287
<212> PRT
<213> Artificial Sequence
<220>
<223> Description of Artificial Sequence: F25-MEK1
   construct
<400> 8
   Met Ala Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Thr
   225                 230                 235                 240
   Gly Met Pro Lys Lys Lys Pro Thr Pro Ile Gln Leu Asn Pro Ala Pro
                   245                 250                 255
   Asp Gly Ser Ala Val Asn Gly Thr Ser Ser Ala Glu Thr Asn Leu Glu
               260                 265                 270
   Ala Leu Gln Lys Lys Leu Glu Glu Leu Glu Leu Asp Glu Gln Gln
           275                 280                 285
<210> 9
<211> 876
<212> DNA
<213> Artificial Sequence
<220>
<221> CDS
<222> (1)..(873)
<220>
<223> Description of Artificial Sequence: F25-MEK2
   construct
<400> 9
   atg gct agc aaa gga gaa gaa ctc ttc act gga gtt gtc cca att ctt      48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga      96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc     144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act     192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa     240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa     288
   Arg His Asp Phe Phe Lys Ser Ala Met Pro Glu Gly Tyr Val Gln Glu
                   85                      90                      95
   agg acc atc ttc ttc aaa gat gac ggc aac tac aag aca cgt gct gaa   336
   Arg Thr Ile Phe Phe Lys Asp Asp Gly Asn Tyr Lys Thr Arg Ala Glu
               100                     105                     110
   gtc aag ttt gaa ggt gat acc ctt gtt aat aga atc gag tta aaa ggt   384
   Val Lys Phe Glu Gly Asp Thr Leu Val Asn Arg Ile Glu Leu Lys Gly
           115                     120                     125
   att gac ttc aag gaa gat ggc aac att ctg gga cac aaa ttg gaa tac   432
   Ile Asp Phe Lys Glu Asp Gly Asn Ile Leu Gly His Lys Leu Glu Tyr
       130                     135                     140
   aac tat aac tca cac aat gta tac atc atg gca gac aaa caa aag aat   480
   Asn Tyr Asn Ser His Asn Val Tyr Ile Met Ala Asp Lys Gln Lys Asn
   145                     150                     155                     160
   gga atc aaa gtg aac ttc aag acc cgc cac aac att gaa gat gga agc   528
   Gly Ile Lys Val Asn Phe Lys Thr Arg His Asn Ile Glu Asp Gly Ser
                   165                     170                     175
   gtt caa cta gca gac cat tat caa caa aat act cca att ggc gat ggc   576
   Val Gln Leu Ala Asp His Tyr Gln Gln Asn Thr Pro Ile Gly Asp Gly
               180                     185                     190
   cct gtc ctt tta cca gac aac cat tac ctg tcc aca caa tct gcc ctt   624
   Pro Val Leu Leu Pro Asp Asn His Tyr Leu Ser Thr Gln Ser Ala Leu
           195                     200                     205
   tcg aaa gat ccc aac gaa aag aga gac cac atg gtc ctt ctt gag ttt   672
   Ser Lys Asp Pro Asn Glu Lys Arg Asp His Met Val Leu Leu Glu Phe
       210                     215                     220
   gta aca gct gct ggg att aca cat ggc atg gat gaa ctg tac aac acc   720
   Val Thr Ala Ala Gly Ile Thr His Gly Met Asp Glu Leu Tyr Asn Thr
   225                     230                     235                     240
   ggt atg ctg gcc cgg agg aag ccg gtg ctg ccg gcg ctc acc atc aac   768
   Gly Met Leu Ala Arg Arg Lys Pro Val Leu Pro Ala Leu Thr Ile Asn
                   245                     250                     255
   cct acc atc gcc gag ggc cca tcc cct acc agc gag ggc gcc tcc gag   816
   Pro Thr Ile Ala Glu Gly Pro Ser Pro Thr Ser Glu Gly Ala Ser Glu
               260                     265                     270
   gca aac ctg gtg gac ctg cag aag aag ctg gag gag ctg gaa ctt gac   864
   Ala Asn Leu Val Asp Leu Gln Lys Lys Leu Glu Glu Leu Glu Leu Asp
           275                     280                     285
   gag cag cag taa                                                   876
   Glu Gln Gln
       290
<210> 10
<211> 291
<212> PRT
<213> Artificial Sequence
<220>
<223> Description of Artificial Sequence: F25-MEK2
   construct
<400> 10
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Thr
   225                 230                 235                 240
   Gly Met Leu Ala Arg Arg Lys Pro Val Leu Pro Ala Leu Thr Ile Asn
                   245                 250                 255
   Pro Thr Ile Ala Glu Gly Pro Ser Pro Thr Ser Glu Gly Ala Ser Glu
               260                 265                 270
   Ala Asn Leu Val Asp Leu Gln Lys Lys Leu Glu Glu Leu Glu Leu Asp
           275                 280                 285
   Glu Gln Gln
       290
<210> 11
<211> 889
<212> DNA
<213> Artificial Sequence
<220>
<221> CDS
<222> (1)..(888)
<220>
<223> Description of Artificial Sequence: Caspase
   3-DEVD-substrate construct
<400> 11
   atg gct agc aaa gga gaa gaa ctc ttc act gga gtt gtc cca att ctt     48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga     96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc    144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act    192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa    240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa    288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa    336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt    384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac    432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat    480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc    528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc    576
   Val Gln Leu Ala Asp His Tyr Gln Gln Asn Thr Pro Ile Gly Asp Gly
               180                     185                     190
   cct gtc ctt tta cca gac aac cat tac ctg tcc aca caa tct gcc ctt     624
   Pro Val Leu Leu Pro Asp Asn His Tyr Leu Ser Thr Gln Ser Ala Leu
           195                 200                     205
   tcg aaa gat ccc aac gaa aag aga gac cac atg gtc ctt ctt gag ttt     672
   Ser Lys Asp Pro Asn Glu Lys Arg Asp His Met Val Leu Leu Glu Phe
       210                 215                     220
   gta aca gct gct ggg att aca cat ggc atg gat gaa ctg tac aac tcc     720
   Val Thr Ala Ala Gly Ile Thr His Gly Met Asp Glu Leu Tyr Asn Ser
   225                 230                     235                 240
   gga aga agg aaa cga caa aag cga tcg gct gtt aaa tct gaa gga aag     768
   Gly Arg Arg Lys Arg Gln Lys Arg Ser Ala Val Lys Ser Glu Gly Lys
                   245                     250                     255
   aga aag tgt gac gaa gtt gat gga att gat gaa gta gca agt act atg     816
   Arg Lys Cys Asp Glu Val Asp Gly Ile Asp Glu Val Ala Ser Thr Met
               260                     265                     270
   tct act gtc cac gaa atc ctg tgc aag ctc agc ttg gag ggt gtt cat     864
   Ser Thr Val His Glu Ile Leu Cys Lys Leu Ser Leu Glu Gly Val His
           275                     280                     285
   tct aca ccc cca agt acc cgg atc c                                   889
   Ser Thr Pro Pro Ser Thr Arg Ile
       290                 295
<210> 12
<211> 296
<212> PRT
<213> Artificial Sequence
<220>
<223> Description of Artificial Sequence: Caspase
   3-DEVD-substrate construct
<400> 12
   Met Ala Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Ser
   225                 230                 235                 240
   Gly Arg Arg Lys Arg Gln Lys Arg Ser Ala Val Lys Ser Glu Gly Lys
                   245                 250                 255
   Arg Lys Cys Asp Glu Val Asp Gly Ile Asp Glu Val Ala Ser Thr Met
               260                 265                 270
   Ser Thr Val His Glu Ile Leu Cys Lys Leu Ser Leu Glu Gly Val His
           275                 280                 285
   Ser Thr Pro Pro Ser Thr Arg Ile
       290                 295
<210> 13
<211> 846
<212> DNA
<213> Artificial Sequence
<220>
<221> CDS
<222> (1)..(B46)
<220>
<223> Description of Artificial Sequence: Caspase
   6-VEID-substrate construct
<400> 13
   atg gct agc aaa gga gaa gaa ctc ttc act gga gtt gtc cca att ctt    48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga    96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc    144
   Glu Gly Glu Gly Asp Ala Thr Tyr Gly Lys Leu Thr Leu Lys Phe Ile
           35                  40                  45
   tgc act act ggc aaa ctg cct gtt cca tgg cca aca cta gtc act act     192
   Cys Thr Thr Gly Lys Leu Pro Val Pro Trp Pro Thr Leu Val Thr Thr
       50                  55                  60
   ctg tgc tat ggt gtt caa tgc ttt tca aga tac ccg gat cat atg aaa     240
   Leu Cys Tyr Gly Val Gln Cys Phe Ser Arg Tyr Pro Asp His Met Lys
   65                  70                  75                  80
   cgg cat gac ttt ttc aag agt gcc atg ccc gaa ggt tat gta cag gaa     288
   Arg His Asp Phe Phe Lys Ser Ala Met Pro Glu Gly Tyr Val Gln Glu
                   85                  90                  95
   agg acc atc ttc ttc aaa gat gac ggc aac tac aag aca cgt gct gaa     336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt     384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac     432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat     480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc     528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc     576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt     624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt     672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tcc     720
   Val Thr Ala Ala Gly Ile Thr His Gly Met Asp Glu Leu Tyr Asn Ser
   225                 230                 235                 240
   gga aga agg aaa cga caa aag cga tcg aca aga ctt gtt gaa att gac     768
   Gly Arg Arg Lys Arg Gln Lys Arg Ser Thr Arg Leu Val Glu Ile Asp
                   245                 250                 255
   aac agt act atg agc aca gta cac gaa att tta tgt aaa tta agc tta     816
   Asn Ser Thr Met Ser Thr Val His Glu Ile Leu Cys Lys Leu Ser Leu
               260                 265                 270
   gaa gga gta cac agt aca cca cca agc gca                             846
   Glu Gly Val His Ser Thr Pro Pro Ser Ala
   275 280
<210> 14
<211> 282
<212> PRT
<213> Artificial Sequence
<220>
<223> Description of Artificial Sequence: Caspase
   6-VEID-substrate construct
<400> 14
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Ser
   225                 230                 235                 240
   Gly Arg Arg Lys Arg Gln Lys Arg Ser Thr Arg Leu Val Glu Ile Asp
                   245                 250                 255
   Asn Ser Thr Met Ser Thr Val His Glu Ile Leu Cys Lys Leu Ser Leu
               260                 265                 270
   Glu Gly Val His Ser Thr Pro Pro Ser Ala
           275                 280
<210> 15
<211> 876
<212> DNA
<213> Artificial Sequence
<220>
<221> CDS
<222> (1)..(876)
<220>
<223> Description of Artificial Sequence: Caspase 8-VETD
   construct
<400> 15
   atg gct agc aaa gga gaa gaa ctc ttc act gga gtt gtc cca att ctt      48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga      96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc     144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act     192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa     240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa     288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa     336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt     384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac     432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat     480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc     528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc     576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt     624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt     672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tcc     720
   Val Thr Ala Ala Gly Ile Thr His Gly Met Asp Glu Leu Tyr Asn Ser
   225                 230                 235                 240
   gga aga agc aaa cga caa aag cga tcg tat gaa aaa gga ata cca gtt     768
   Gly Arg Ser Lys Arg Gln Lys Arg Ser Tyr Glu Lys Gly Ile Pro Val
                   245                 250                 255
   gaa aca gac agc gaa gag caa gct tat agt act atg tct act gtc cac     816
   Glu Thr Asp Ser Glu Glu Gln Ala Tyr Ser Thr Met Ser Thr Val His
               260                 265                 270
   gaa atc ctg tgc aag ctc agc ttg gag ggt gtt cat tct aca ccc cca     864
   Glu Ile Leu Cys Lys Leu Ser Leu Glu Gly Val His Ser Thr Pro Pro
           275                 280                 285
   agt gcc gga tcc                                                     876
   Ser Ala Gly Ser
       290
<210> 16
<211> 292
<212> PRT
<213> Artificial Sequence
<220>
<223> Description of Artificial Sequence: Caspase B-VETD
   construct
<400> 16
   Met Ala Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Ser
   225                 230                 235                 240
   Gly Arg Ser Lys Arg Gln Lys Arg Ser Tyr Glu Lys Gly Ile Pro Val
                   245                 250                 255
   Glu Thr Asp Ser Glu Glu Gln Ala Tyr Ser Thr Met Ser Thr Val His
               260                 265                 270
   Glu Ile Leu Cys Lys Leu Ser Leu Glu Gly Val His Ser Thr Pro Pro
           275                 280                 285
   Ser Ala Gly Ser
       290
<210> 17
<211> 906
<212> DNA
<213> Artificial Sequence
<220>
<221> CDS
<222> (1)..(906)
<220>
<223> Description of Artificial Sequence: Cas 3-multiple
   DEVD construct
<400> 17
   atg gct agc aaa gga gaa gaa ctc ttc act gga gtt gtc cca att ctt   48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga 96
   Val Glu Leu Asp Gly Asp Val Asn Gly His Lys Phe Ser Val Ser Gly
   20 25 30
   gag ggt gaa ggt gat gca aca tac gga aaa ctt acc ctg aag ttc atc 144
   Glu Gly Glu Gly Asp Ala Thr Tyr Gly Lys Leu Thr Leu Lys Phe Ile
   35 40 45
   tgc act act ggc aaa ctg cct gtt cca tgg cca aca cta gtc act act 192
   Cys Thr Thr Gly Lys Leu Pro Val Pro Trp Pro Thr Leu Val Thr Thr
   50 55 60
   ctg tgc tat ggt gtt caa tgc ttt tca aga tac ccg gat cat atg aaa 240
   Leu Cys Tyr Gly Val Gln Cys Phe Ser Arg Tyr Pro Asp His Met Lys
   65 70 75 80
   cgg cat gac ttt ttc aag agt gcc atg ccc gaa ggt tat gta cag gaa 288
   Arg His Asp Phe Phe Lys Ser Ala Met Pro Glu Gly Tyr Val Gln Glu
   85 90 95
   agg acc atc ttc ttc aaa gat gac ggc aac tac aag aca cgt gct gaa 336
   Arg Thr Ile Phe Phe Lys Asp Asp Gly Asn Tyr Lys Thr Arg Ala Glu
   100 105 110
   gtc aag ttt gaa ggt gat acc ctt gtt aat aga atc gag tta aaa ggt 384
   Val Lys Phe Glu Gly Asp Thr Leu Val Asn Arg Ile Glu Leu Lys Gly
   115 120 125
   att gac ttc aag gaa gat ggc aac att ctg gga cac aaa ttg gaa tac 432
   Ile Asp Phe Lys Glu Asp Gly Asn Ile Leu Gly His Lys Leu Glu Tyr
   130 135 140
   aac tat aac tca cac aat gta tac atc atg gca gac aaa caa aag aat 480
   Asn Tyr Asn Ser His Asn Val Tyr Ile Met Ala Asp Lys Gln Lys Asn
   145 150 155 160
   gga atc aaa gtg aac ttc aag acc cgc cac aac att gaa gat gga agc 528
   Gly Ile Lys Val Asn Phe Lys Thr Arg His Asn Ile Glu Asp Gly Ser
   165 170 175
   gtt caa cta gca gac cat tat caa caa aat act cca att ggc gat ggc 576
   Val Gln Leu Ala Asp His Tyr Gln Gln Asn Thr Pro Ile Gly Asp Gly
   180 185 190
   cct gtc ctt tta cca gac aac cat tac ctg tcc aca caa tct gcc ctt 624
   Pro Val Leu Leu Pro Asp Asn His Tyr Leu Ser Thr Gln Ser Ala Leu
   195 200 205
   tcg aaa gat ccc aac gaa aag aga gac cac atg gtc ctt ctt gag ttt 672
   Ser Lys Asp Pro Asn Glu Lys Arg Asp His Met Val Leu Leu Glu Phe
   210 215 220
   gta aca gct gct ggg att aca cat ggc atg gat gaa ctg tac aac tcc 720
   Val Thr Ala Ala Gly Ile Thr His Gly Met Asp Glu Leu Tyr Asn Ser
   225 230 235 240
   gga aga agg aaa cga caa aag cga tcg gca ggt gac gaa gtt gat gca 768
   Gly Arg Arg Lys Arg Gln Lys Arg Ser Ala Gly Asp Glu Val Asp Ala
   245 250 255
   ggt gac gaa gtt gat gca ggt gac gaa gtt gat gca ggt gac gaa gtt    816
   Gly Asp Glu Val Asp Ala Gly Asp Glu Val Asp Ala Gly Asp Glu Val
               260                 265                     270
   gac gca ggt agt act atg tct act gtc cac gaa atc ctg tgc aag ctc    864
   Asp Ala Gly Ser Thr Met Ser Thr Val His Glu Ile Leu Cys Lys Leu
           275                 280                 285
   agc ttg gag ggt gtt cat tct aca ccc cca agt gcc gga tcc            906
   Ser Leu Glu Gly Val His Ser Thr Pro Pro Ser Ala Gly Ser
       290                 295                 300
<210> 18
<211> 302
<212> PRT
<213> Artificial Sequence
<220>
<223> Description of Artificial Sequence: Cas 3-multiple
   DEVD construct
<400> 18
   Met Ala Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Cys Tyr Gly Val Gln Cys Phe Ser Arg Tyr Pro Asp His Met Lys
   65 70 75 80
   Arg His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Thr Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr His Gly Met Asp Glu Leu Tyr Asn Ser
   225 230 235 240
   Gly Arg Arg Lys Arg Gln Lys Arg Ser Ala Gly Asp Glu Val Asp Ala
   245 250 255
   Gly Asp Glu Val Asp Ala Gly Asp Glu Val Asp Ala Gly Asp Glu Val
   260 265 270
   Asp Ala Gly Ser Thr Met Ser Thr Val His Glu Ile Leu Cys Lys Leu
   275 280 285
   Ser Leu Glu Gly Val His Ser Thr Pro Pro Ser Ala Gly Ser
   290 295 300
<210> 19
<211> 906
<212> DNA
<213> Artificial Sequence
<220>
<221> CDS
<222> (1)..(885)
<220>
<223> Description of Artificial Sequence: Caspase
   8-multiple VETD construct
<400> 19
   atg gct agc aaa gga gaa gaa ctc ttc act gga gtt gtc cca att ctt      48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga      96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc      144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act      192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa      240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa      288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa      336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt    384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac    432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat    480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc    528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc    576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt    624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt    672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tcc    720
   Val Thr Ala Ala Gly Ile Thr His Gly Met Asp Glu Leu Tyr Asn Ser
   225                 230                 235                 240
   gga aga agg aaa cga caa aag cga tcg gca ggt gtt gaa aca gac gca    768
   Gly Arg Arg Lys Arg Gln Lys Arg Ser Ala Gly Val Glu Thr Asp Ala
                   245                 250                 255
   ggt gtt gaa aca gac gca ggt gtt gaa aca gac gca ggt gtt gaa aca    816
   Gly Val Glu Thr Asp Ala Gly Val Glu Thr Asp Ala Gly Val Glu Thr
               260                 265                 270
   gac gca ggt agt act atg tct act gtc cac gaa atc ctg tgc aag ctc    864
   Asp Ala Gly Ser Thr Met Ser Thr Val His Glu Ile Leu Cys Lys Leu
           275                 280                 285
   agc ttg gag ggt gtt cat tct acacccccaa gtgccggatc c                906
   Ser Leu Glu Gly Val His Ser
       290                 295
<210> 20
<211> 295
<212> PRT
<213> Artificial Sequence
<220>
<223> Description of Artificial Sequence: Caspase
   8-multiple VETD construct
<400> 20
   Met Ala Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn Ser
   225                 230                 235                 240
   Gly Arg Arg Lys Arg Gln Lys Arg Ser Ala Gly Val Glu Thr Asp Ala
                   245                 250                 255
   Gly Val Glu Thr Asp Ala Gly Val Glu Thr Asp Ala Gly Val Glu Thr
               260                 265                 270
   Asp Ala Gly Ser Thr Met Ser Thr Val His Glu Ile Leu Cys Lys Leu
           275                 280                 285
   Ser Leu Glu Gly Val His Ser
       290                 295
<210> 21
<211> 4833
<212> DNA
<213> Artificial Sequence
<220>
<221> CDS
<222> (1)..(4830)
<220>
<223> Description of Artificial Sequence:
   EYFP-DEVD-MAP4-EBFP construct
<400> 21
   atg gtg agc aag ggc gag gag ctg ttc acc ggg gtg gtg ccc atc ctg     48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc     96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc    144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc    192
   Cys Thr Thr Gly Lys Leu Pro Val Pro Trp Pro Thr Leu Val Thr Thr
       50                  55                  60
   ttc ggc tac ggc ctg cag tgc ttc gcc cgc tac ccc gac cac atg aag    240
   Phe Gly Tyr Gly Leu Gln Cys Phe Ala Arg Tyr Pro Asp His Met Lys
   65                  70                  75                  80
   cag cac gac ttc ttc aag tcc gcc atg ccc gaa ggc tac gtc cag gag    288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag    336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc    384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac    432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac    480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc    528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc    576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg    624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc    672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag aag    720
   Val Thr Ala Ala Gly Ile Thr Leu Gly Met Asp Glu Leu Tyr Lys Lys
   225                 230                 235                 240
   gga gac gaa gtg gac gga atg gcc gac ctc agt ctt gtg gat gcg ttg    768
   Gly Asp Glu Val Asp Gly Met Ala Asp Leu Ser Leu Val Asp Ala Leu
                   245                 250                 255
   aca gaa cca cct cca gaa att gag gga gaa ata aag cga gac ttc atg    816
   Thr Glu Pro Pro Pro Glu Ile Glu Gly Glu Ile Lys Arg Asp Phe Met
               260                 265                 270
   gct gcg ctg gag gca gag ccc tat gat gac atc gtg gga gaa act gtg    864
   Ala Ala Leu Glu Ala Glu Pro Tyr Asp Asp Ile Val Gly Glu Thr Val
           275                 280                 285
   gag aaa act gag ttt att cct ctc ctg gat ggt gat gag aaa acc ggg    912
   Glu Lys Thr Glu Phe Ile Pro Leu Leu Asp Gly Asp Glu Lys Thr Gly
       290                 295                 300
   aac tca gag tcc aaa aag aaa ccc tgc tta gac act agc cag gtt gaa    960
   Asn Ser Glu Ser Lys Lys Lys Pro Cys Leu Asp Thr Ser Gln Val Glu
   305                 310                 315                 320
   ggt atc cca tct tct aaa cca aca ctc cta gcc aat ggt gat cat gga    1008
   Gly Ile Pro Ser Ser Lys Pro Thr Leu Leu Ala Asn Gly Asp His Gly
                   325                 330                 335
   atg gag ggg aat aac act gca ggg tct cca act gac ttc ctt gaa gag    1056
   Met Glu Gly Asn Asn Thr Ala Gly Ser Pro Thr Asp Phe Leu Glu Glu
               340                 345                 350
   aga gtg gac tat ccg gat tat cag agc agc cag aac tgg cca gaa gat    1104
   Arg Val Asp Tyr Pro Asp Tyr Gln Ser Ser Gln Asn Trp Pro Glu Asp
           355                 360                 365
   gca agc ttt tgt ttc cag cct cag caa gtg tta gat act gac cag gct    1152
   Ala Ser Phe Cys Phe Gln Pro Gln Gln Val Leu Asp Thr Asp Gln Ala
       370                 375                 380
   gag ccc ttt aac gag cac cgt gat gat ggt ttg gca gat ctg ctc ttt    1200
   Glu Pro Phe Asn Glu His Arg Asp Asp Gly Leu Ala Asp Leu Leu Phe
   385                 390                 395                 400
   gtc tcc agt gga ccc acg aac gct tct gca ttt aca gag cga gac aat    1248
   Val Ser Ser Gly Pro Thr Asn Ala Ser Ala Phe Thr Glu Arg Asp Asn
                   405                 410                 415
   cct tca gaa gac agt tac ggt atg ctt ccc tgt gac tca ttt gct tcc    1296
   Pro Ser Glu Asp Ser Tyr Gly Met Leu Pro Cys Asp Ser Phe Ala Ser
               420                 425                 430
   acg gct gtt gta tct cag gag tgg tct gtg gga gcc cca aac tct cca    1344
   Thr Ala Val Val Ser Gln Glu Trp Ser Val Gly Ala Pro Asn Ser Pro
           435                 440                 445
   tgt tca gag tcc tgt gtc tcc cca gag gtt act ata gaa acc cta cag    1392
   Cys Ser Glu Ser Cys Val Ser Pro Glu Val Thr Ile Glu Thr Leu Gln
       450                 455                 460
   cca gca aca gag ctc tcc aag gca gca gaa gtg gaa tca gtg aaa gag    1440
   Pro Ala Thr Glu Leu Ser Lys Ala Ala Glu Val Glu Ser Val Lys Glu
   465                 470                 475                 480
   cag ctg cca gct aaa gca ttg gaa acg atg gca gag cag acc act gat    1488
   Gln Leu Pro Ala Lys Ala Leu Glu Thr Met Ala Glu Gln Thr Thr Asp
                   485                 490                 495
   gtg gtg cac tct cca tcc aca gac aca aca cca ggc cca gac aca gag    1536
   Val Val His Ser Pro Ser Thr Asp Thr Thr Pro Gly Pro Asp Thr Glu
               500                 505                 510
   gca gca ctg gct aaa gac ata gaa gag atc acc aag cca gat gtg ata    1584
   Ala Ala Leu Ala Lys Asp Ile Glu Glu Ile Thr Lys Pro Asp Val Ile
           515                 520                 525
   ttg gca aat gtc acg cag cca tct act gaa tcg gat atg ttc ctg gcc    1632
   Leu Ala Asn Val Thr Gln Pro Ser Thr Glu Ser Asp Met Phe Leu Ala
       530                 535                 540
   cag gac atg gaa cta ctc aca gga aca gag gca gcc cac gct aac aat    1680
   Gln Asp Met Glu Leu Leu Thr Gly Thr Glu Ala Ala His Ala Asn Asn
   545                 550                 555                 560
   atc ata ttg cct aca gaa cca gac gaa tct tca acc aag gat gta gca    1728
   Ile Ile Leu Pro Thr Glu Pro Asp Glu Ser Ser Thr Lys Asp Val Ala
                   565                 570                 575
   cca cct atg gaa gaa gaa att gtc cca ggc aat gat acg aca tcc ccc    1776
   Pro Pro Met Glu Glu Glu Ile Val Pro Gly Asn Asp Thr Thr Ser Pro
               580                 585                 590
   aaa gaa aca gag aca aca ctt cca ata aaa atg gac ttg gca cca cct    1824
   Lys Glu Thr Glu Thr Thr Leu Pro Ile Lys Met Asp Leu Ala Pro Pro
           595                 600                 605
   gag gat gtg tta ctt acc aaa gaa aca gaa cta gcc cca gcc aag ggc    1872
   Glu Asp Val Leu Leu Thr Lys Glu Thr Glu Leu Ala Pro Ala Lys Gly
       610                 615                 620
   atg gtt tca ctc tca gaa ata gaa gag gct ctg gca aag aat gat gtt    1920
   Met Val Ser Leu Ser Glu Ile Glu Glu Ala Leu Ala Lys Asn Asp Val
   625                 630                 635                 640
   cgc tct gca gaa ata cct gtg gct cag gag aca gtg gtc tca gaa aca    1968
   Arg Ser Ala Glu Ile Pro Val Ala Gln Glu Thr Val Val Ser Glu Thr
                   645                 650                 655
   gag gtg gtc ctg gca aca gaa gtg gta ctg ccc tca gat ccc ata aca    2016
   Glu Val Val Leu Ala Thr Glu Val Val Leu Pro Ser Asp Pro Ile Thr
               660                 665                 670
   aca ttg aca aag gat gtg aca ctc ccc tta gaa gca gag aga ccg ttg    2064
   Thr Leu Thr Lys Asp Val Thr Leu Pro Leu Glu Ala Glu Arg Pro Leu
           675                 680                 685
   gtg acg gac atg act cca tct ctg gaa aca gaa atg acc cta ggc aaa    2112
   Val Thr Asp Met Thr Pro Ser Leu Glu Thr Glu Met Thr Leu Gly Lys
       690                 695                 700
   gag aca gct cca ccc aca gaa aca aat ttg ggc atg gcc aaa gac atg    2160
   Glu Thr Ala Pro Pro Thr Glu Thr Asn Leu Gly Met Ala Lys Asp Met
   705                 710                 715                 720
   tct cca ctc cca gaa tca gaa gtg act ctg ggc aag gac gtg gtt ata    2208
   Ser Pro Leu Pro Glu Ser Glu Val Thr Leu Gly Lys Asp Val Val Ile
                   725                 730                 735
   ctt cca gaa aca aag gtg gct gag ttt aac aat gtg act cca ctt tca    2256
   Leu Pro Glu Thr Lys Val Ala Glu Phe Asn Asn Val Thr Pro Leu Ser
               740                 745                 750
   gaa gaa gag gta acc tca gtc aag gac atg tct ccg tct gca gaa aca    2304
   Glu Glu Glu Val Thr Ser Val Lys Asp Met Ser Pro Ser Ala Glu Thr
           755                 760                 765
   gag gct ccc ctg gct aag aat gct gat ctg cac tca gga aca gag ctg    2352
   Glu Ala Pro Leu Ala Lys Asn Ala Asp Leu His Ser Gly Thr Glu Leu
       770                 775                 780
   att gtg gac aac agc atg gct cca gcc tcc gat ctt gca ctg ccc ttg    2400
   Ile Val Asp Asn Ser Met Ala Pro Ala Ser Asp Leu Ala Leu Pro Leu
   785                 790                 795                 800
   gaa aca aaa gta gca aca gtt cca att aaa gac aaa gga act gta cag    2448
   Glu Thr Lys Val Ala Thr Val Pro Ile Lys Asp Lys Gly Thr Val Gln
                   805                 810                 815
   act gaa gaa aaa cca cgt gaa gac tcc cag tta gca tct atg cag cac    2496
   Thr Glu Glu Lys Pro Arg Glu Asp Ser Gln Leu Ala Ser Met Gln His
               820                 825                 830
   aag gga cag tca aca gta cct cct tgc acg gct tca cca gaa cca gtc    2544
   Lys Gly Gln Ser Thr Val Pro Pro Cys Thr Ala Ser Pro Glu Pro Val
           835                 840                 845
   aaa gct gca gaa caa atg tct acc tta cca ata gat gca cct tct cca    2592
   Lys Ala Ala Glu Gln Met Ser Thr Leu Pro Ile Asp Ala Pro Ser Pro
       850                 855                 860
   tta gag aac tta gag cag aag gaa acg cct ggc agc cag cct tct gag    2640
   Leu Glu Asn Leu Glu Gln Lys Glu Thr Pro Gly Ser Gln Pro Ser Glu
   865                 870                 875                 880
   cct tgc tca gga gta tcc cgg caa gaa gaa gca aag gct gct gta ggt    2688
   Pro Cys Ser Gly Val Ser Arg Gln Glu Glu Ala Lys Ala Ala Val Gly
                   885                 890                 895
   gtg act gga aat gac atc act acc ccg cca aac aag gag cca cca cca    2736
   Val Thr Gly Asn Asp Ile Thr Thr Pro Pro Asn Lys Glu Pro Pro Pro
               900                 905                 910
   agc cca gaa aag aaa gca aag cct ttg gcc acc act caa cct gca aag    2784
   Ser Pro Glu Lys Lys Ala Lys Pro Leu Ala Thr Thr Gln Pro Ala Lys
           915                 920                 925
   act tca aca tcg aaa gcc aaa aca cag ccc act tct ctc cct aag caa    2832
   Thr Ser Thr Ser Lys Ala Lys Thr Gln Pro Thr Ser Leu Pro Lys Gln
           930                 935                 940
   cca gct ccc acc acc tct ggt ggg ttg aat aaa aaa ccc atg agc ctc     2880
   Pro Ala Pro Thr Thr Ser Gly Gly Leu Asn Lys Lys Pro Met Ser Leu
   945                 950                 955                 960
   gcc tca ggc tca gtg cca gct gcc cca cac aaa cgc cct gct gct gcc     2928
   Ala Ser Gly Ser Val Pro Ala Ala Pro His Lys Arg Pro Ala Ala Ala
                   965                 970                 975
   act gct act gcc agg cct tcc acc cta cct gcc aga gac gtg aag cca     2976
   Thr Ala Thr Ala Arg Pro Ser Thr Leu Pro Ala Arg Asp Val Lys Pro
               980                 985                 990
   aag cca att aca gaa gct aag gtt gcc gaa aag cgg acc tct cca tcc     3024
   Lys Pro Ile Thr Glu Ala Lys Val Ala Glu Lys Arg Thr Ser Pro Ser
           995                 1000                1005
   aag cct tca tct gcc cca gcc ctc aaa cct gga cct aaa acc acc cca     3072
   Lys Pro Ser Ser Ala Pro Ala Leu Lys Pro Gly Pro Lys Thr Thr Pro
       1010                1015                1020
   acc gtt tca aaa gcc aca tct ccc tca act ctt gtt tcc act gga cca     3120
   Thr Val Ser Lys Ala Thr Ser Pro Ser Thr Leu Val Ser Thr Gly Pro
   1025                1030                1035                1040
   agt agt aga agt cca gct aca act ctg cct aag agg cca acc agc atc     3168
   Ser Ser Arg Ser Pro Ala Thr Thr Leu Pro Lys Arg Pro Thr Ser Ile
                   1045                1050                1055
   aag act gag ggg aaa cct gct gat gtc aaa agg atg act gct aag tct     3216
   Lys Thr Glu Gly Lys Pro Ala Asp Val Lys Arg Met Thr Ala Lys Ser
               1060                1065                1070
   gcc tca gct gac ttg agt cgc tca aag acc acc tct gcc agt tct gtg     3264
   Ala Ser Ala Asp Leu Ser Arg Ser Lys Thr Thr Ser Ala Ser Ser Val
           1075                1080                1085
   aag aga aac acc act ccc act ggg gca gca ccc cca gca ggg atg act     3312
   Lys Arg Asn Thr Thr Pro Thr Gly Ala Ala Pro Pro Ala Gly Met Thr
       1090                1095                1100
   tcc act cga gtc aag ccc atg tct gca cct agc cgc tct tct ggg gct     3360
   Ser Thr Arg Val Lys Pro Met Ser Ala Pro Ser Arg Ser Ser Gly Ala
   1105                1110                1115                1120
   ctt tct gtg gac aag aag ccc act tcc act aag cct agc tcc tct gct     3408
   Leu Ser Val Asp Lys Lys Pro Thr Ser Thr Lys Pro Ser Ser Ser Ala
                   1125                1130                1135
   ccc agg gtg agc cgc ctg gcc aca act gtt tct gcc cct gac ctg aag     3456
   Pro Arg Val Ser Arg Leu Ala Thr Thr Val Ser Ala Pro Asp Leu Lys
               1140                1145                1150
   agt gtt cgc tcc aag gtc ggc tct aca gaa aac atc aaa cac cag cct     3504
   Ser Val Arg Ser Lys Val Gly Ser Thr Glu Asn Ile Lys His Gln Pro
           1155                1160                1165
   gga gga ggc cgg gcc aaa gta gag aaa aaa aca gag gca gct acc aca     3552
   Gly Gly Gly Arg Ala Lys Val Glu Lys Lys Thr Glu Ala Ala Thr Thr
       1170                1175                1180
   gct ggg aag cct gaa cct aat gca gtc act aaa gca gcc ggc tcc att    3600
   Ala Gly Lys Pro Glu Pro Asn Ala Val Thr Lys Ala Ala Gly Ser Ile
   1185                1190                1195                1200
   gcg agt gca cag aaa ccg cct gct ggg aaa gtc cag ata gta tcc aaa    3648
   Ala Ser Ala Gln Lys Pro Pro Ala Gly Lys Val Gln Ile Val Ser Lys
                   1205                1210                1215
   aaa gtg agc tac agt cat att caa tcc aag tgt gtt tcc aag gac aat    3696
   Lys Val Ser Tyr Ser His Ile Gln Ser Lys Cys Val Ser Lys Asp Asn
               1220                1225                1230
   att aag cat gtc cct gga tgt ggc aat gtt cag att cag aac aag aaa    3744
   Ile Lys His Val Pro Gly Cys Gly Asn Val Gln Ile Gln Asn Lys Lys
           1235                1240                1245
   gtg gac ata tcc aag gtc tcc tcc aag tgt ggg tcc aaa gct aat atc    3792
   Val Asp Ile Ser Lys Val Ser Ser Lys Cys Gly Ser Lys Ala Asn Ile
       1250                1255                1260
   aag cac aag cct ggt gga gga gat gtc aag att gaa agt cag aag ttg    3840
   Lys His Lys Pro Gly Gly Gly Asp Val Lys Ile Glu Ser Gln Lys Leu
   1265                1270                1275                1280
   aac ttc aag gag aag gcc caa gcc aaa gtg gga tcc ctt gat aac gtt    3888
   Asn Phe Lys Glu Lys Ala Gln Ala Lys Val Gly Ser Leu Asp Asn Val
                   1285                1290                1295
   ggc cac ttt cct gca gga ggt gcc gtg aag act gag ggc ggt ggc agt    3936
   Gly His Phe Pro Ala Gly Gly Ala Val Lys Thr Glu Gly Gly Gly Ser
               1300                1305                1310
   gag gcc ctt ccg tgt cca ggc ccc ccc gct ggg gag gag cca gtc atc    3984
   Glu Ala Leu Pro Cys Pro Gly Pro Pro Ala Gly Glu Glu Pro Val Ile
           1315                1320                1325
   cct gag gct gcg cct gac cgt ggc gcc cct act tca gcc agt ggc ctc    4032
   Pro Glu Ala Ala Pro Asp Arg Gly Ala Pro Thr Ser Ala Ser Gly Leu
       1330                1335                1340
   agt ggc cac acc acc ctg tca ggg ggt ggt gac caa agg gag ccc cag    4080
   Ser Gly His Thr Thr Leu Ser Gly Gly Gly Asp Gln Arg Glu Pro Gln
   1345                1350                1355                1360
   acc ttg gac agc cag atc cag gag aca agc atc atg gtg agc aag ggc    4128
   Thr Leu Asp Ser Gln Ile Gln Glu Thr Ser Ile Met Val Ser Lys Gly
                   1365                1370                1375
   gag gag ctg ttc acc ggg gtg gtg ccc atc ctg gtc gag ctg gac ggc    4176
   Glu Glu Leu Phe Thr Gly Val Val Pro Ile Leu Val Glu Leu Asp Gly
               1380                1385                1390
   gac gta aac ggc cac aag ttc agc gtg tcc ggc gag ggc gag ggc gat    4224
   Asp Val Asn Gly His Lys Phe Ser Val Ser Gly Glu Gly Glu Gly Asp
           1395                1400                1405
   gcc acc tac ggc aag ctg acc ctg aag ttc atc tgc acc acc ggc aag    4272
   Ala Thr Tyr Gly Lys Leu Thr Leu Lys Phe Ile Cys Thr Thr Gly Lys
       1410                1415                1420
   ctg ccc gtg ccc tgg ccc acc ctc gtg acc acc ctg acc cac ggc gtg    4320
   Leu Pro Val Pro Trp Pro Thr Leu Val Thr Thr Leu Thr His Gly Val
   1425                1430                1435                1440
   cag tgc ttc agc cgc tac ccc gac cac atg aag cag cac gac ttc ttc    4368
   Gln Cys Phe Ser Arg Tyr Pro Asp His Met Lys Gln His Asp Phe Phe
                   1445                1450                1455
   aag tcc gcc atg ccc gaa ggc tac gtc cag gag cgc acc atc ttc ttc    4416
   Lys Ser Ala Met Pro Glu Gly Tyr Val Gln Glu Arg Thr Ile Phe Phe
               1460                1465                1470
   aag gac gac ggc aac tac aag acc cgc gcc gag gtg aag ttc gag ggc    4464
   Lys Asp Asp Gly Asn Tyr Lys Thr Arg Ala Glu Val Lys Phe Glu Gly
           1475                1480                1485
   gac acc ctg gtg aac cgc atc gag ctg aag ggc atc gac ttc aag gag    4512
   Asp Thr Leu Val Asn Arg Ile Glu Leu Lys Gly Ile Asp Phe Lys Glu
       1490                1495                1500
   gac ggc aac atc ctg ggg cac aag ctg gag tac aac ttc aac agc cac    4560
   Asp Gly Asn Ile Leu Gly His Lys Leu Glu Tyr Asn Phe Asn Ser His
   1505                1510                1515                1520
   aac gtc tat atc atg gcc gac aag cag aag aac ggc atc aag gtg aac    4608
   Asn Val Tyr Ile Met Ala Asp Lys Gln Lys Asn Gly Ile Lys Val Asn
                   1525                1530                1535
   ttc aag atc cgc cac aac atc gag gac ggc agc gtg cag ctc gcc gac    4656
   Phe Lys Ile Arg His Asn Ile Glu Asp Gly Ser Val Gln Leu Ala Asp
               1540                1545                1550
   cac tac cag cag aac acc ccc atc ggc gac ggc ccc gtg ctg ctg ccc    4704
   His Tyr Gln Gln Asn Thr Pro Ile Gly Asp Gly Pro Val Leu Leu Pro
           1555                1560                1565
   gac aac cac tac ctg agc acc cag tcc gcc ctg agc aaa gac ccc aac    4752
   Asp Asn His Tyr Leu Ser Thr Gln Ser Ala Leu Ser Lys Asp Pro Asn
       1570                1575                1580
   gag aag cgc gat cac atg gtc ctg ctg gag ttc gtg acc gcc gcc ggg    4800
   Glu Lys Arg Asp His Met Val Leu Leu Glu Phe Val Thr Ala Ala Gly
   1585                1590                1595                1600
   atc act ctc ggc atg gac gag ctg tac aag tag                        4833
   Ile Thr Leu Gly Met Asp Glu Leu Tyr Lys
                   1605                1610
<210> 22
<211> 1610
<212> PRT
<213> Artificial Sequence
<220>
<223> Description of Artificial Sequence:
   EYFP-DEVD-MAP4-EBFP construct
<400> 22
   Met Val Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Phe Gly Tyr Gly Leu Gln Cys Phe Ala Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Tyr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys Lys
   225 230 235 240
   Gly Asp Glu Val Asp Gly Met Ala Asp Leu Ser Leu Val Asp Ala Leu
   245 250 255
   Thr Glu Pro Pro Pro Glu Ile Glu Gly Glu Ile Lys Arg Asp Phe Met
   260 265 270
   Ala Ala Leu Glu Ala Glu Pro Tyr Asp Asp Ile Val Gly Glu Thr Val
   275 280 285
   Glu Lys Thr Glu Phe Ile Pro Leu Leu Asp Gly Asp Glu Lys Thr Gly
   290 295 300
   Asn Ser Glu Ser Lys Lys Lys Pro Cys Leu Asp Thr Ser Gln Val Glu
   305 310 315 320
   Gly Ile Pro Ser Ser Lys Pro Thr Leu Leu Ala Asn Gly Asp His Gly
   325 330 335
   Met Glu Gly Asn Asn Thr Ala Gly Ser Pro Thr Asp Phe Leu Glu Glu
                   340                 345                 350
   Arg Val Asp Tyr Pro Asp Tyr Gln Ser Ser Gln Asn Trp Pro Glu Asp
               355                 360                 365
   Ala Ser Phe Cys Phe Gln Pro Gln Gln Val Leu Asp Thr Asp Gln Ala
       370                 375                 380
   Glu Pro Phe Asn Glu His Arg Asp Asp Gly Leu Ala Asp Leu Leu Phe
   385                 390                 395                 400
   Val Ser Ser Gly Pro Thr Asn Ala Ser Ala Phe Thr Glu Arg Asp Asn
                   405                 410                 415
   Pro Ser Glu Asp Ser Tyr Gly Met Leu Pro Cys Asp Ser Phe Ala Ser
               420                 425                 430
   Thr Ala Val Val Ser Gln Glu Trp Ser Val Gly Ala Pro Asn Ser Pro
           435                 440                 445
   Cys Ser Glu Ser Cys Val Ser Pro Glu Val Thr Ile Glu Thr Leu Gln
       450                 455                 460
   Pro Ala Thr Glu Leu Ser Lys Ala Ala Glu Val Glu Ser Val Lys Glu
   465                 470                 475                 480
   Gln Leu Pro Ala Lys Ala Leu Glu Thr Met Ala Glu Gln Thr Thr Asp
                   485                 490                 495
   Val Val His Ser Pro Ser Thr Asp Thr Thr Pro Gly Pro Asp Thr Glu
               500                 505                 510
   Ala Ala Leu Ala Lys Asp Ile Glu Glu Ile Thr Lys Pro Asp Val Ile
           515                 520                 525
   Leu Ala Asn Val Thr Gln Pro Ser Thr Glu Ser Asp Met Phe Leu Ala
       530                 535                 540
   Gln Asp Met Glu Leu Leu Thr Gly Thr Glu Ala Ala His Ala Asn Asn
   545                 550                 555                 560
   Ile Ile Leu Pro Thr Glu Pro Asp Glu Ser Ser Thr Lys Asp Val Ala
                   565                 570                 575
   Pro Pro Met Glu Glu Glu Ile Val Pro Gly Asn Asp Thr Thr Ser Pro
               580                 585                 590
   Lys Glu Thr Glu Thr Thr Leu Pro Ile Lys Met Asp Leu Ala Pro Pro
           595                 600                 605
   Glu Asp Val Leu Leu Thr Lys Glu Thr Glu Leu Ala Pro Ala Lys Gly
       610                 615                 620
   Met Val Ser Leu Ser Glu Ile Glu Glu Ala Leu Ala Lys Asn Asp Val
   625                 630                 635                 640
   Arg Ser Ala Glu Ile Pro Val Ala Gln Glu Thr Val Val Ser Glu Thr
                   645                 650                 655
   Glu Val Val Leu Ala Thr Glu Val Val Leu Pro Ser Asp Pro Ile Thr
   660 665 670
   Thr Leu Thr Lys Asp Val Thr Leu Pro Leu Glu Ala Glu Arg Pro Leu
   675 680 685
   Val Thr Asp Met Thr Pro Ser Leu Glu Thr Glu Met Thr Leu Gly Lys
   690 695 700
   Glu Thr Ala Pro Pro Thr Glu Thr Asn Leu Gly Met Ala Lys Asp Met
   705 710 715 720
   Ser Pro Leu Pro Glu Ser Glu Val Thr Leu Gly Lys Asp Val Val Ile
   725 730 735
   Leu Pro Glu Thr Lys Val Ala Glu Phe Asn Asn Val Thr Pro Leu Ser
   740 745 750
   Glu Glu Glu Val Thr Ser Val Lys Asp Met Ser Pro Ser Ala Glu Thr
   755 760 765
   Glu Ala Pro Leu Ala Lys Asn Ala Asp Leu His Ser Gly Thr Glu Leu
   770 775 780
   Ile Val Asp Asn Ser Met Ala Pro Ala Ser Asp Leu Ala Leu Pro Leu
   785 790 795 800
   Glu Thr Lys Val Ala Thr Val Pro Ile Lys Asp Lys Gly Thr Val Gln
   805 810 815
   Thr Glu Glu Lys Pro Arg Glu Asp Ser Gln Leu Ala Ser Met Gln His
   820 825 830
   Lys Gly Gln Ser Thr Val Pro Pro Cys Thr Ala Ser Pro Glu Pro Val
   835 840 845
   Lys Ala Ala Glu Gln Met Ser Thr Leu Pro Ile Asp Ala Pro Ser Pro
   850 855 860
   Leu Glu Asn Leu Glu Gln Lys Glu Thr Pro Gly Ser Gln Pro Ser Glu
   865 870 875 880
   Pro Cys Ser Gly Val Ser Arg Gln Glu Glu Ala Lys Ala Ala Val Gly
   885 890 895
   Val Thr Gly Asn Asp Ile Thr Thr Pro Pro Asn Lys Glu Pro Pro Pro
   900 905 910
   Ser Pro Glu Lys Lys Ala Lys Pro Leu Ala Thr Thr Gln Pro Ala Lys
   915 920 925
   Thr Ser Thr Ser Lys Ala Lys Thr Gln Pro Thr Ser Leu Pro Lys Gln
   930 935 940
   Pro Ala Pro Thr Thr Ser Gly Gly Leu Asn Lys Lys Pro Met Ser Leu
   945 950 955 960
   Ala Ser Gly Ser Val Pro Ala Ala Pro His Lys Arg Pro Ala Ala Ala
   965 970 975
   Thr Ala Thr Ala Arg Pro Ser Thr Leu Pro Ala Arg Asp Val Lys Pro
   980 985 990
   Lys Pro Ile Thr Glu Ala Lys Val Ala Glu Lys Arg Thr Ser Pro Ser
   995 1000 1005
   Lys Pro Ser Ser Ala Pro Ala Leu Lys Pro Gly Pro Lys Thr Thr Pro
   1010 1015 1020
   Thr Val Ser Lys Ala Thr Ser Pro Ser Thr Leu Val Ser Thr Gly Pro
   1025 1030 1035 1040
   Ser Ser Arg Ser Pro Ala Thr Thr Leu Pro Lys Arg Pro Thr Ser Ile
   1045 1050 1055
   Lys Thr Glu Gly Lys Pro Ala Asp Val Lys Arg Met Thr Ala Lys Ser
   1060 1065 1070
   Ala Ser Ala Asp Leu Ser Arg Ser Lys Thr Thr Ser Ala Ser Ser Val
   1075 1080 1085
   Lys Arg Asn Thr Thr Pro Thr Gly Ala Ala Pro Pro Ala Gly Met Thr
   1090 1095 1100
   Ser Thr Arg Val Lys Pro Met Ser Ala Pro Ser Arg Ser Ser Gly Ala
   1105 1110 1115 1120
   Leu Ser Val Asp Lys Lys Pro Thr Ser Thr Lys Pro Ser Ser Ser Ala
   1125 1130 1135
   Pro Arg Val Ser Arg Leu Ala Thr Thr Val Ser Ala Pro Asp Leu Lys
   1140 1145 1150
   Ser Val Arg Ser Lys Val Gly Ser Thr Glu Asn Ile Lys His Gln Pro
   1155 1160 1165
   Gly Gly Gly Arg Ala Lys Val Glu Lys Lys Thr Glu Ala Ala Thr Thr
   1170 1175 1180
   Ala Gly Lys Pro Glu Pro Asn Ala Val Thr Lys Ala Ala Gly Ser Ile
   1185 1190 1195 1200
   Ala Ser Ala Gln Lys Pro Pro Ala Gly Lys Val Gln Ile Val Ser Lys
   1205 1210 1215
   Lys Val Ser Tyr Ser His Ile Gln Ser Lys Cys Val Ser Lys Asp Asn
   1220 1225 1230
   Ile Lys His Val Pro Gly Cys Gly Asn Val Gln Ile Gln Asn Lys Lys
   1235 1240 1245
   Val Asp Ile Ser Lys Val Ser Ser Lys Cys Gly Ser Lys Ala Asn Ile
   1250 1255 1260
   Lys His Lys Pro Gly Gly Gly Asp Val Lys Ile Glu Ser Gln Lys Leu
   1265 1270 1275 1280
   Asn Phe Lys Glu Lys Ala Gln Ala Lys Val Gly Ser Leu Asp Asn Val
   1285 1290 1295
   Gly His Phe Pro Ala Gly Gly Ala Val Lys Thr Glu Gly Gly Gly Ser
   1300 1305 1310
   Glu Ala Leu Pro Cys Pro Gly Pro Pro Ala Gly Glu Glu Pro Val Ile
   1315 1320 1325
   Pro Glu Ala Ala Pro Asp Arg Gly Ala Pro Thr Ser Ala Ser Gly Leu
   1330 1335 1340
   Ser Gly His Thr Thr Leu Ser Gly Gly Gly Asp Gln Arg Glu Pro Gln
   1345 1350 1355 1360
   Thr Leu Asp Ser Gln Ile Gln Glu Thr Ser Ile Met Val Ser Lys Gly
   1365 1370 1375
   Glu Glu Leu Phe Thr Gly Val Val Pro Ile Leu Val Glu Leu Asp Gly
   1380 1385 1390
   Asp Val Asn Gly His Lys Phe Ser Val Ser Gly Glu Gly Glu Gly Asp
   1395 1400 1405
   Ala Thr Tyr Gly Lys Leu Thr Leu Lys Phe Ile Cys Thr Thr Gly Lys
   1410 1415 1420
   Leu Pro Val Pro Trp Pro Thr Leu Val Thr Thr Leu Thr His Gly Val
   1425 1430 1435 1440
   Gln Cys Phe Ser Arg Tyr Pro Asp His Met Lys Gln His Asp Phe Phe
   1445 1450 1455
   Lys Ser Ala Met Pro Glu Gly Tyr Val Gln Glu Arg Thr Ile Phe Phe
   1460 1465 1470
   Lys Asp Asp Gly Asn Tyr Lys Thr Arg Ala Glu Val Lys Phe Glu Gly
   1475 1480 1485
   Asp Thr Leu Val Asn Arg Ile Glu Leu Lys Gly Ile Asp Phe Lys Glu
   1490 1495 1500
   Asp Gly Asn Ile Leu Gly His Lys Leu Glu Tyr Asn Phe Asn Ser His
   1505 1510 1515 1520
   Asn Val Tyr Ile Met Ala Asp Lys Gln Lys Asn Gly Ile Lys Val Asn
   1525 1530 1535
   Phe Lys Ile Arg His Asn Ile Glu Asp Gly Ser Val Gln Leu Ala Asp
   1540 1545 1550
   His Tyr Gln Gln Asn Thr Pro Ile Gly Asp Gly Pro Val Leu Leu Pro
   1555 1560 1565
   Asp Asn His Tyr Leu Ser Thr Gln Ser Ala Leu Ser Lys Asp Pro Asn
   1570 1575 1580
   Glu Lys Arg Asp His Met Val Leu Leu Glu Phe Val Thr Ala Ala Gly
   1585 1590 1595 1600
   Ile Thr Leu Gly Met Asp Glu Leu Tyr Lys
   1605 1610
<210> 23
<211> 978
<212> DNA
<213> Artificial Sequence
<220>
<221> CDS
<222> (1)..(978)
<220>
<223> Description of Artificial Sequence:
   GFP-nucleolus-Caspase 8-annexin II construct
<400> 23
   atg gct agc aaa gga gaa gaa ctc ttc act gga gtt gtc cca att ctt    48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga    96
   Val Glu Leu Asp Gly Asp Val Asn Gly His Lys Phe Ser Val Ser Gly
                20                  25                  30
   gag ggt gaa ggt gat gca aca tac gga aaa ctt acc ctg aag ttc atc   144
   Glu Gly Glu Gly Asp Ala Thr Tyr Gly Lys Leu Thr Leu Lys Phe Ile
            35                  40                  45
   tgc act act ggc aaa ctg cct gtt cca tgg cca aca cta gtc act act   192
   Cys Thr Thr Gly Lys Leu Pro Val Pro Trp Pro Thr Leu Val Thr Thr
        50                  55                  60
   ctg tgc tat ggt gtt caa tgc ttt tca aga tac ccg gat cat atg aaa   240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa   288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa   336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt   384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac   432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat   480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc   528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc   576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt   624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt    672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tcc    720
   Val Thr Ala Ala Gly Ile Thr His Gly Met Asp Glu Leu Tyr Asn Ser
   225                 230                 235                 240
   gga aga aaa cgt ata cgt act tac ctc aag tcc tgc agg cgg atg aaa    768
   Gly Arg Lys Arg Ile Arg Thr Tyr Leu Lys Ser Cys Arg Arg Met Lys
                   245                 250                 255
   aga agt ggt ttt gag atg tct cga cct att cct tcc cac ctt act cga    816
   Arg Ser Gly Phe Glu Met Ser Arg Pro Ile Pro Ser His Leu Thr Arg
               260                 265                 270
   tcg gca ggt gtt gaa aca gac gca ggt gtt gaa aca gac gca ggt gtt    864
   Ser Ala Gly Val Glu Thr Asp Ala Gly Val Glu Thr Asp Ala Gly Val
           275                 280                 285
   gaa aca gac gca ggt gtt gaa aca gac gca ggt agt act atg tct act    912
   Glu Thr Asp Ala Gly Val Glu Thr Asp Ala Gly Ser Thr Met Ser Thr
       290                 295                 300
   gtc cac gaa atc ctg tgc aag ctc agc ttg gag ggt gtt cat tct aca    960
   Val His Glu Ile Leu Cys Lys Leu Ser Leu Glu Gly Val His Ser Thr
   305                 310                 315                 320
   ccc cca agt gcc gga tcc                                            978
   Pro Pro Ser Ala Gly Ser
                   325
<210> 24
<211> 326
<212> PRT
<213> Artificial Sequence
<220>
<223> Description of Artificial Sequence:
   GFP-nucleolus-Caspase 8-annexin II construct
<400> 24
   Met Ala Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Thr Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr His Gly Met Asp Glu Leu Tyr Asn Ser
   225 230 235 240
   Gly Arg Lys Arg Ile Arg Thr Tyr Leu Lys Ser Cys Arg Arg Met Lys
   245 250 255
   Arg Ser Gly Phe Glu Met Ser Arg Pro Ile Pro Ser His Leu Thr Arg
   260 265 270
   Ser Ala Gly Val Glu Thr Asp Ala Gly Val Glu Thr Asp Ala Gly Val
   275 280 285
   Glu Thr Asp Ala Gly Val Glu Thr Asp Ala Gly Ser Thr Met Ser Thr
   290 295 300
   Val His Glu Ile Leu Cys Lys Leu Ser Leu Glu Gly Val His Ser Thr
   305 310 315 320
   Pro Pro Ser Ala Gly Ser
   325
<210> 25
<211> 948
<212> DNA
<213> Artificial Sequence
<220>
<221> CDS
<222> (1)..(948)
<220>
<223> Description of Artificial Sequence:
   GFP-nucleolus-Caspase 3-annexin II construct
<400> 25
   atg gct agc aaa gga gaa gaa ctc ttc act gga gtt gtc cca att ctt      48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga      96
   Val Glu Leu Asp Gly Asp Val Asn Gly His Lys Phe Ser Val Ser Gly
               20                  25                  30
   gag ggt gaa ggt gat gca aca tac gga aaa ctt acc ctg aag ttc atc     144
   Glu Gly Glu Gly Asp Ala Thr Tyr Gly Lys Leu Thr Leu Lys Phe Ile
           35                  40                  45
   tgc act act ggc aaa ctg cct gtt cca tgg cca aca cta gtc act act     192
   Cys Thr Thr Gly Lys Leu Pro Val Pro Trp Pro Thr Leu Val Thr Thr
       50                  55                  60
   ctg tgc tat ggt gtt caa tgc ttt tca aga tac ccg gat cat atg aaa     240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa     288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa     336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt     384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac     432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat     480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc     528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc     576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt     624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt     672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tcc     720
   Val Thr Ala Ala Gly Ile Thr His Gly Met Asp Glu Leu Tyr Asn Ser
   225                 230                 235                 240
   gga aga aaa cgt ata cgt act tac ctc aag tcc tgc agg cgg atg aaa     768
   Gly Arg Lys Arg Ile Arg Thr Tyr Leu Lys Ser Cys Arg Arg Met Lys
                   245                 250                 255
   aga agt ggt ttt gag atg tct cga cct att cct tcc cac ctt act cga     816
   Arg Ser Gly Phe Glu Met Ser Arg Pro Ile Pro Ser His Leu Thr Arg
               260                 265                 270
   tcg tat gaa aaa gga ata cca gtt gaa aca gac agc gaa gag caa gct     864
   Ser Tyr Glu Lys Gly Ile Pro Val Glu Thr Asp Ser Glu Glu Gln Ala
           275                 280                 285
   tat agt act atg tct act gtc cac gaa atc ctg tgc aag ctc agc ttg     912
   Tyr Ser Thr Met Ser Thr Val His Glu Ile Leu Cys Lys Leu Ser Leu
       290                 295                 300
   gag ggt gtt cat tct aca ccc cca agt gcc gga tcc                     948
   Glu Gly Val His Ser Thr Pro Pro Ser Ala Gly Ser
   305                 310                 315
<210> 26
<211> 316
<212> PRT
<213> Artificial Sequence
<220>
<223> Description of Artificial Sequence:
   GFP-nucleolus-Caspase 3-annexin II construct
<400> 26
   Met Ala Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Cys Tyr Gly Val Gln Cys Phe Ser Arg Tyr Pro Asp His Met Lys
   65 70 75 80
   Arg His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Thr Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr His Gly Met Asp Glu Leu Tyr Asn Ser
   225 230 235 240
   Gly Arg Lys Arg Ile Arg Thr Tyr Leu Lys Ser Cys Arg Arg Met Lys
   245 250 255
   Arg Ser Gly Phe Glu Met Ser Arg Pro Ile Pro Ser His Leu Thr Arg
   260 265 270
   Ser Tyr Glu Lys Gly Ile Pro Val Glu Thr Asp Ser Glu Glu Gln Ala
   275 280 285
   Tyr Ser Thr Met Ser Thr Val His Glu Ile Leu Cys Lys Leu Ser Leu
   290 295 300
   Glu Gly Val His Ser Thr Pro Pro Ser Ala Gly Ser
   305 310 315
<210> 27
<211> 2088
<212> DNA
<213> Artificial Sequence
<220>
<221> CDS
<222> (1)..(1041)
<220>
<223> Description of Artificial Sequence:
   NLS-Pred25-synaptobrevin construct
<400> 27
   atg aga aga aaa cga caa aag gct agc aaa gga gaa gaa ctc ttc act 48
   Met Arg Arg Lys Arg Gln Lys Ala Ser Lys Gly Glu Glu Leu Phe Thr
   1 5 10 15
   gga gtt gtc cca att ctt gtt gaa tta gat ggt gat gtt aac ggc cac 96
   Gly Val Val Pro Ile Leu Val Glu Leu Asp Gly Asp Val Asn Gly His
   20 25 30
   aag ttc tct gtc agt gga gag ggt gaa ggt gat gca aca tac gga aaa 144
   Lys Phe Ser Val Ser Gly Glu Gly Glu Gly Asp Ala Thr Tyr Gly Lys
   35 40 45
   ctt acc ctg aag ttc atc tgc act act ggc aaa ctg cct gtt cca tgg 192
   Leu Thr Leu Lys Phe Ile Cys Thr Thr Gly Lys Leu Pro Val Pro Trp
   50 55 60
   cca aca cta gtc act act ctg tgc tat ggt gtt caa tgc ttt tca aga    240
   Pro Thr Leu Val Thr Thr Leu Cys Tyr Gly Val Gln Cys Phe Ser Arg
    65                 70                  75                  80
   tac ccg gat cat atg aaa cgg cat gac ttt ttc aag agt gcc atg ccc    288
   Tyr Pro Asp His Met Lys Arg His Asp Phe Phe Lys Ser Ala Met Pro
                   85                  90                  95
   gaa ggt tat gta cag gaa agg acc atc ttc ttc aaa gat gac ggc aac    336
   Glu Gly Tyr Val Gln Glu Arg Thr Ile Phe Phe Lys Asp Asp Gly Asn
               100                 105                 110
   tac aag aca cgt gct gaa gtc aag ttt gaa ggt gat acc ctt gtt aat    384
   Tyr Lys Thr Arg Ala Glu Val Lys Phe Glu Gly Asp Thr Leu Val Asn
           115                 120                 125
   aga atc gag tta aaa ggt att gac ttc aag gaa gat ggc aac att ctg    432
   Arg Ile Glu Leu Lys Gly Ile Asp Phe Lys Glu Asp Gly Asn Ile Leu
       130                 135                 140
   gga cac aaa ttg gaa tac aac tat aac tca cac aat gta tac atc atg    480
   Gly His Lys Leu Glu Tyr Asn Tyr Asn Ser His Asn Val Tyr Ile Met
   145                 150                 155                 160
   gca gac aaa caa aag aat gga atc aaa gtg aac ttc aag acc cgc cac    528
   Ala Asp Lys Gln Lys Asn Gly Ile Lys Val Asn Phe Lys Thr Arg His
                   165                 170                 175
   aac att gaa gat gga agc gtt caa cta gca gac cat tat caa caa aat    576
   Asn Ile Glu Asp Gly Ser Val Gln Leu Ala Asp His Tyr Gln Gln Asn
               180                 185                 190
   act cca att ggc gat ggc cct gtc ctt tta cca gac aac cat tac ctg    624
   Thr Pro Ile Gly Asp Gly Pro Val Leu Leu Pro Asp Asn His Tyr Leu
           195                 200                 205
   tcc aca caa tct gcc ctt tcg aaa gat ccc aac gaa aag aga gac cac    672
   Ser Thr Gln Ser Ala Leu Ser Lys Asp Pro Asn Glu Lys Arg Asp His
       210                 215                 220
   atg gtc ctt ctt gag ttt gta aca gct gct ggg att aca cat ggc atg    720
   Met Val Leu Leu Glu Phe Val Thr Ala Ala Gly Ile Thr His Gly Met
   225                 230                 235                 240
   gat gaa ctg tac aac acc ggt atg tct aca ggt cca act gct gcc act    768
   Asp Glu Leu Tyr Asn Thr Gly Met Ser Thr Gly Pro Thr Ala Ala Thr
                   245                 250                 255
   ggc agt aat cga aga ctt cag cag aca caa aat caa gta gat gag gtg    816
   Gly Ser Asn Arg Arg Leu Gln Gln Thr Gln Asn Gln Val Asp Glu Val
               260                 265                 270
   gtg gac ata atg cga gtt aac gtg gac aag gtt ctg gaa aga gac cag    864
   Val Asp Ile Met Arg Val Asn Val Asp Lys Val Leu Glu Arg Asp Gln
           275                 280                 285
   aag ctc tct gag tta gac gac cgt gca gac gca ctg cag gca ggc gct    912
   Lys Leu Ser Glu Leu Asp Asp Arg Ala Asp Ala Leu Gln Ala Gly Ala
       290                 295                 300
   tct caa ttt gaa acg agc gca gcc aag ttg aag agg aaa tat tgg tgg    960
   Ser Gln Phe Glu Thr Ser Ala Ala Lys Leu Lys Arg Lys Tyr Trp Trp
   305                 310                 315                 320
   aag aat tgc aag atg tgg gca atc ggg att act gtt ctg gtt atc ttc   1008
   Lys Asn Cys Lys Met Trp Ala Ile Gly Ile Thr Val Leu Val Ile Phe
                   325                 330                 335
   atc atc atc atc atc gtg tgg gtt gtc tct tca tgaatgagaa gaaaacgaca 1061
   Ile Ile Ile Ile Ile Val Trp Val Val Ser Ser
               340                 345
   aaaggctagc aaaggagaag aactcttcac tggagttgtc ccaattcttg ttgaattaga 1121
   tggtgatgtt aacggccaca agttctctgt cagtggagag ggtgaaggtg atgcaacata 1181
   cggaaaactt accctgaagt tcatctgcac tactggcaaa ctgcctgttc catggccaac 1241
   actagtcact actctgtgct atggtgttca atgcttttca agatacccgg atcatatgaa 1301
   acggcatgac tttttcaaga gtgccatgcc cgaaggttat gtacaggaaa ggaccatctt 1361
   cttcaaagat gacggcaact acaagacacg tgctgaagtc aagtttgaag gtgataccct 1421
   tgttaataga atcgagttaa aaggtattga cttcaaggaa gatggcaaca ttctgggaca 1481
   caaattggaa tacaactata actcacacaa tgtatacatc atggcagaca aacaaaagaa 1541
   tggaatcaaa gtgaacttca agacccgcca caacattgaa gatggaagcg ttcaactagc 1601
   agaccattat caacaaaata ctccaattgg cgatggccct gtccttttac cagacaacca 1661
   ttacctgtcc acacaatctg ccctttcgaa agatcccaac gaaaagagag accacatggt 1721
   ccttcttgag tttgtaacag ctgctgggat tacacatggc atggatgaac tgtacaacac 1781
   cggtatgtct acaggtccaa ctgctgccac tggcagtaat cgaagacttc agcagacaca 1841
   aaatcaagta gatgaggtgg tggacataat gcgagttaac gtggacaagg ttctggaaag 1901
   agaccagaag ctctctgagt tagacgaccg tgcagacgca ctgcaggcag gcgcttctca 1961
   atttgaaacg agcgcagcca agttgaagag gaaatattgg tggaagaatt gcaagatgtg 2021
   ggcaatcggg attactgttc tggttatctt catcatcatc atcatcgtgt gggttgtctc 2081
   ttcatga                                                            2088
<210> 28
<211> 347
<212> PRT
<213> Artificial Sequence
<220>
<223> Description of Artificial Sequence:
   NLS-Fred25-synaptobrevin construct
<400> 28
   Met Arg Arg Lys Arg Gln Lys Ala Ser Lys Gly Glu Glu Leu Phe Thr
     1               5                  10                  15
   Gly Val Val Pro Ile Leu Val Glu Leu Asp Gly Asp Val Asn Gly His
   20 25 30
   Lys Phe Ser Val Ser Gly Glu Gly Glu Gly Asp Ala Thr Tyr Gly Lys
   35 40 45
   Leu Thr Leu Lys Phe Ile Cys Thr Thr Gly Lys Leu Pro Val Pro Trp
   50 55 60
   Pro Thr Leu Val Thr Thr Leu Cys Tyr Gly Val Gln Cys Phe Ser Arg
   65 70 75 80
   Tyr Pro Asp His Met Lys Arg His Asp Phe Phe Lys Ser Ala Met Pro
   85 90 95
   Glu Gly Tyr Val Gln Glu Arg Thr Ile Phe Phe Lys Asp Asp Gly Asn
   100 105 110
   Tyr Lys Thr Arg Ala Glu Val Lys Phe Glu Gly Asp Thr Leu Val Asn
   115 120 125
   Arg Ile Glu Leu Lys Gly Ile Asp Phe Lys Glu Asp Gly Asn Ile Leu
   130 135 140
   Gly His Lys Leu Glu Tyr Asn Tyr Asn Ser His Asn Val Tyr Ile Met
   145 150 155 160
   Ala Asp Lys Gln Lys Asn Gly Ile Lys Val Asn Phe Lys Thr Arg His
   165 170 175
   Asn Ile Glu Asp Gly Ser Val Gln Leu Ala Asp His Tyr Gln Gln Asn
   180 185 190
   Thr Pro Ile Gly Asp Gly Pro Val Leu Leu Pro Asp Asn His Tyr Leu
   195 200 205
   Ser Thr Gln Ser Ala Leu Ser Lys Asp Pro Asn Glu Lys Arg Asp His
   210 215 220
   Met Val Leu Leu Glu Phe Val Thr Ala Ala Gly Ile Thr His Gly Met
   225 230 235 240
   Asp Glu Leu Tyr Asn Thr Gly Met Ser Thr Gly Pro Thr Ala Ala Thr
   245 250 255
   Gly Ser Asn Arg Arg Leu Gln Gln Thr Gln Asn Gln Val Asp Glu Val
   260 265 270
   Val Asp Ile Met Arg Val Asn Val Asp Lys Val Leu Glu Arg Asp Gln
   275 280 285
   Lys Leu Ser Glu Leu Asp Asp Arg Ala Asp Ala Leu Gln Ala Gly Ala
   290 295 300
   Ser Gln Phe Glu Thr Ser Ala Ala Lys Leu Lys Arg Lys Tyr Trp Trp
   305 310 315 320
   Lys Asn Cys Lys Met Trp Ala Ile Gly Ile Thr Val Leu Val Ile Phe
   325 330 335
   Ile Ile Ile Ile Ile Val Trp Val Val Ser Ser
   340 345
<210> 29
<211> 2106
<212> DNA
<213> Artificial Sequence
<220>
<221> CDS
<222> (1)..(1050)
<220>
<223> Description of Artificial Sequence:
   NLS-Fred25-cellubrevin construct
<400> 29
   atg aga aga aaa cga caa aag gct agc aaa gga gaa gaa ctc ttc act     48
   Met Arg Arg Lys Arg Gln Lys Ala Ser Lys Gly Glu Glu Leu Phe Thr
    1               5                  10                  15
   gga gtt gtc cca att ctt gtt gaa tta gat ggt gat gtt aac ggc cac     96
   Gly Val Val Pro Ile Leu Val Glu Leu Asp Gly Asp Val Asn Gly His
               20                  25                  30
   aag ttc tct gtc agt gga gag ggt gaa ggt gat gca aca tac gga aaa    144
   Lys Phe Ser Val Ser Gly Glu Gly Glu Gly Asp Ala Thr Tyr Gly Lys
           35                  40                  45
   ctt acc ctg aag ttc atc tgc act act ggc aaa ctg cct gtt cca tgg    192
   Leu Thr Leu Lys Phe Ile Cys Thr Thr Gly Lys Leu Pro Val Pro Trp
       50                  55                  60
   cca aca cta gtc act act ctg tgc tat ggt gtt caa tgc ttt tca aga    240
   Pro Thr Leu Val Thr Thr Leu Cys Tyr Gly Val Gln Cys Phe Ser Arg
   65                  70                  75                  80
   tac ccg gat cat atg aaa cgg cat gac ttt ttc aag agt gcc atg ccc    288
   Tyr Pro Asp His Met Lys Arg His Asp Phe Phe Lys Ser Ala Met Pro
                   85                  90                  95
   gaa ggt tat gta cag gaa agg acc atc ttc ttc aaa gat gac ggc aac    336
   Glu Gly Tyr Val Gln Glu Arg Thr Ile Phe Phe Lys Asp Asp Gly Asn
               100                 105                 110
   tac aag aca cgt gct gaa gtc aag ttt gaa ggt gat acc ctt gtt aat    384
   Tyr Lys Thr Arg Ala Glu Val Lys Phe Glu Gly Asp Thr Leu Val Asn
           115                 120                 125
   aga atc gag tta aaa ggt att gac ttc aag gaa gat ggc aac att ctg    432
   Arg Ile Glu Leu Lys Gly Ile Asp Phe Lys Glu Asp Gly Asn Ile Leu
       130                 135                 140
   gga cac aaa ttg gaa tac aac tat aac tca cac aat gta tac atc atg    480
   Gly His Lys Leu Glu Tyr Asn Tyr Asn Ser His Asn Val Tyr Ile Met
   145                 150                 155                 160
   gca gac aaa caa aag aat gga atc aaa gtg aac ttc aag acc cgc cac    528
   Ala Asp Lys Gln Lys Asn Gly Ile Lys Val Asn Phe Lys Thr Arg His
                   165                 170                 175
   aac att gaa gat gga agc gtt caa cta gca gac cat tat caa caa aat    576
   Asn Ile Glu Asp Gly Ser Val Gln Leu Ala Asp His Tyr Gln Gln Asn
               180                 185                 190
   act cca att ggc gat ggc cct gtc ctt tta cca gac aac cat tac ctg    624
   Thr Pro Ile Gly Asp Gly Pro Val Leu Leu Pro Asp Asn His Tyr Leu
           195                 200                 205
   tcc aca caa tct gcc ctt tcg aaa gat ccc aac gaa aag aga gac cac    672
   Ser Thr Gln Ser Ala Leu Ser Lys Asp Pro Asn Glu Lys Arg Asp His
       210                 215                 220
   atg gtc ctt ctt gag ttt gta aca gct gct ggg att aca cat ggc atg    720
   Met Val Leu Leu Glu Phe Val Thr Ala Ala Gly Ile Thr His Gly Met
   225                 230                 235                 240
   gat gaa ctg tac aac acc ggt atg tct aca ggt gtg cct tcg ggg tca    768
   Asp Glu Leu Tyr Asn Thr Gly Met Ser Thr Gly Val Pro Ser Gly Ser
                   245                 250                 255
   agt gct gcc act ggc agt aat cga aga ctc cag cag aca caa aat caa    816
   Ser Ala Ala Thr Gly Ser Asn Arg Arg Leu Gln Gln Thr Gln Asn Gln
               260                 265                 270
   gta gat gag gtg gtt gac atc atg aga gtc aat gtg gat aag gtg tta    864
   Val Asp Glu Val Val Asp Ile Met Arg Val Asn Val Asp Lys Val Leu
           275                 280                 285
   gaa aga gac cag aag ctc tcg gag cta gat gac cgc gca gat gca ctg    912
   Glu Arg Asp Gln Lys Leu Ser Glu Leu Asp Asp Arg Ala Asp Ala Leu
       290                 295                 300
   cag gca ggt gcc tcg cag ttt gaa aca agt gct gcc aag ttg aag aga    960
   Gln Ala Gly Ala Ser Gln Phe Glu Thr Ser Ala Ala Lys Leu Lys Arg
   305                 310                 315                 320
   aag tat tgg tgg aag aac tgc aag atg tgg gcg ata ggg atc agt gtc   1008
   Lys Tyr Trp Trp Lys Asn Cys Lys Met Trp Ala Ile Gly Ile Ser Val
                   325                 330                 335
   ctg gtg atc att gtc atc atc atc atc gtg tgg tgt gtc tct           1050
   Leu Val Ile Ile Val Ile Ile Ile Ile Val Trp Cys Val Ser
               340                 345                 350
   taaatgagaa gaaaacgaca aaaggctagc aaaggagaag aactcttcac tggagttgtc 1110
   ccaattcttg ttgaattaga tggtgatgtt aacggccaca agttctctgt cagtggagag 1170
   ggtgaaggtg atgcaacata cggaaaactt accctgaagt tcatctgcac tactggcaaa 1230
   ctgcctgttc catggccaac actagtcact actctgtgct atggtgttca atgcttttca 1290
   agatacccgg atcatatgaa acggcatgac tttttcaaga gtgccatgcc cgaaggttat 1350
   gtacaggaaa ggaccatctt cttcaaagat gacggcaact acaagacacg tgctgaagtc 1410
   aagtttgaag gtgataccct tgttaataga atcgagttaa aaggtattga cttcaaggaa 1470
   gatggcaaca ttctgggaca caaattggaa tacaactata actcacacaa tgtatacatc 1530
   atggcagaca aacaaaagaa tggaatcaaa gtgaacttca agacccgcca caacattgaa 1590
   gatggaagcg ttcaactagc agaccattat caacaaaata ctccaattgg cgatggccct 1650
   gtccttttac cagacaacca ttacctgtcc acacaatctg ccctttcgaa agatcccaac 1710
   gaaaagagag accacatggt ccttcttgag tttgtaacag ctgctgggat tacacatggc 1770
   atggatgaac tgtacaacac cggtatgtct acaggtgtgc cttcggggtc aagtgctgcc 1830
   actggcagta atcgaagact ccagcagaca caaaatcaag tagatgaggt ggttgacatc 1890
   atgagagtca atgtggataa ggtgttagaa agagaccaga agctctcgga gctagatgac 1950
   cgcgcagatg cactgcaggc aggtgcctcg cagtttgaaa caagtgctgc caagttgaag 2010
   agaaagtatt ggtggaagaa ctgcaagatg tgggcgatag ggatcagtgt cctggtgatc 2070
   attgtcatca tcatcatcgt gtggtgtgtc tcttaa 2106
<210> 30
<211> 350
<212> PRT
<213> Artificial Sequence
<220>
<223> Description of Artificial Sequence:
   NLS-Fred25-cellubrevin construct
<400> 30
   Met Arg Arg Lys Arg Gln Lys Ala Ser Lys Gly Glu Glu Leu Phe Thr
    1               5                  10                  15
   Gly Val Val Pro Ile Leu Val Glu Leu Asp Gly Asp Val Asn Gly His
               20                  25                  30
   Lys Phe Ser Val Ser Gly Glu Gly Glu Gly Asp Ala Thr Tyr Gly Lys
           35                  40                  45
   Leu Thr Leu Lys Phe Ile Cys Thr Thr Gly Lys Leu Pro Val Pro Trp
       50                  55                  60
   Pro Thr Leu Val Thr Thr Leu Cys Tyr Gly Val Gln Cys Phe Ser Arg
   65                  70                  75                  80
   Tyr Pro Asp His Met Lys Arg His Asp Phe Phe Lys Ser Ala Met Pro
                   85                  90                  95
   Glu Gly Tyr Val Gln Glu Arg Thr Ile Phe Phe Lys Asp Asp Gly Asn
               100                 105                 110
   Tyr Lys Thr Arg Ala Glu Val Lys Phe Glu Gly Asp Thr Leu Val Asn
           115                 120                 125
   Arg Ile Glu Leu Lys Gly Ile Asp Phe Lys Glu Asp Gly Asn Ile Leu
       130                 135                 140
   Gly His Lys Leu Glu Tyr Asn Tyr Asn Ser His Asn Val Tyr Ile Met
   145                 150                 155                 160
   Ala Asp Lys Gln Lys Asn Gly Ile Lys Val Asn Phe Lys Thr Arg His
   165 170 175
   Asn Ile Glu Asp Gly Ser Val Gln Leu Ala Asp His Tyr Gln Gln Asn
   180 185 190
   Thr Pro Ile Gly Asp Gly Pro Val Leu Leu Pro Asp Asn His Tyr Leu
   195 200 205
   Ser Thr Gln Ser Ala Leu Ser Lys Asp Pro Asn Glu Lys Arg Asp His
   210 215 220
   Met Val Leu Leu Glu Phe Val Thr Ala Ala Gly Ile Thr His Gly Met
   225 230 235 240
   Asp Glu Leu Tyr Asn Thr Gly Met Ser Thr Gly Val Pro Ser Gly Ser
   245 250 255
   Ser Ala Ala Thr Gly Ser Asn Arg Arg Leu Gln Gln Thr Gln Asn Gln
   260 265 270
   Val Asp Glu Val Val Asp Ile Met Arg Val Asn Val Asp Lys Val Leu
   275 280 285
   Glu Arg Asp Gln Lys Leu Ser Glu Leu Asp Asp Arg Ala Asp Ala Leu
   290 295 300
   Gln Ala Gly Ala Ser Gln Phe Glu Thr Ser Ala Ala Lys Leu Lys Arg
   305 310 315 320
   Lys Tyr Trp Trp Lys Asn Cys Lys Met Trp Ala Ile Gly Ile Ser Val
   325 330 335
   Leu Val Ile Ile Val Ile Ile Ile Ile Val Trp Cys Val Ser
   340 345 350
<210> 31
<211> 3171
<212> DNA
<213> Artificial Sequence
<220>
<221> CDS
<222> (1)..(3168)
<220>
<223> Description of Artificial Sequence:
   NLS-EYFP-MAPKDM-EBFP construct
<400> 31
   atg agg ccc aga aga aag gtg agc aag ggc gag gag ctg ttc acc ggg    48
   Met Arg Pro Arg Arg Lys Val Ser Lys Gly Glu Glu Leu Phe Thr Gly
    1               5                  10                  15
   gtg gtg ccc atc ctg gtc gag ctg gac ggc gac gta aac ggc cac aag    96
   Val Val Pro Ile Leu Val Glu Leu Asp Gly Asp Val Asn Gly His Lys
               20                  25                  30
   ttc agc gtg tcc ggc gag ggc gag ggc gat gcc acc tac ggc aag ctg   144
   Phe Ser Val Ser Gly Glu Gly Glu Gly Asp Ala Thr Tyr Gly Lys Leu
           35                  40                  45
   acc ctg aag ttc atc tgc acc acc ggc aag ctg ccc gtg ccc tgg ccc     192
   Thr Leu Lys Phe Ile Cys Thr Thr Gly Lys Leu Pro Val Pro Trp Pro
        50                  55                  60
   acc ctc gtg acc acc ttc ggc tac ggc ctg cag tgc ttc gcc cgc tac     240
   Thr Leu Val Thr Thr Phe Gly Tyr Gly Leu Gln Cys Phe Ala Arg Tyr
    65                  70                  75                  80
   ccc gac cac atg aag cag cac gac ttc ttc aag tcc gcc atg ccc gaa     288
   Pro Asp His Met Lys Gln His Asp Phe Phe Lys Ser Ala Met Pro Glu
                    85                  90                  95
   ggc tac gtc cag gag cgc acc atc ttc ttc aag gac gac ggc aac tac     336
   Gly Tyr Val Gln Glu Arg Thr Ile Phe Phe Lys Asp Asp Gly Asn Tyr
               100                 105                 110
   aag acc cgc gcc gag gtg aag ttc gag ggc gac acc ctg gtg aac cgc     384
   Lys Thr Arg Ala Glu Val Lys Phe Glu Gly Asp Thr Leu Val Asn Arg
           115                 120                 125
   atc gag ctg aag ggc atc gac ttc aag gag gac ggc aac atc ctg ggg     432
   Ile Glu Leu Lys Gly Ile Asp Phe Lys Glu Asp Gly Asn Ile Leu Gly
       130                 135                 140
   cac aag ctg gag tac aac tac aac agc cac aac gtc tat atc atg gcc     480
   His Lys Leu Glu Tyr Asn Tyr Asn Ser His Asn Val Tyr Ile Met Ala
   145                 150                 155                 160
   gac aag cag aag aac ggc atc aag gtg aac ttc aag atc cgc cac aac     528
   Asp Lys Gln Lys Asn Gly Ile Lys Val Asn Phe Lys Ile Arg His Asn
                   165                 170                 175
   atc gag gac ggc agc gtg cag ctc gcc gac cac tac cag cag aac acc     576
   Ile Glu Asp Gly Ser Val Gln Leu Ala Asp His Tyr Gln Gln Asn Thr
               180                 185                 190
   ccc atc ggc gac ggc ccc gtg ctg ctg ccc gac aac cac tac ctg agc     624
   Pro Ile Gly Asp Gly Pro Val Leu Leu Pro Asp Asn His Tyr Leu Ser
           195                 200                 205
   tac cag tcc gcc ctg agc aaa gac ccc aac gag aag cgc gat cac atg     672
   Tyr Gln Ser Ala Leu Ser Lys Asp Pro Asn Glu Lys Arg Asp His Met
       210                 215                 220
   gtc ctg ctg gag ttc gtg acc gcc gcc ggg atc act ctc ggc atg gac     720
   Val Leu Leu Glu Phe Val Thr Ala Ala Gly Ile Thr Leu Gly Met Asp
   225                 230                 235                 240
   gag ctg tac aag aag gga gac gaa gtg gac gga gcc gac ctc agt ctt     768
   Glu Leu Tyr Lys Lys Gly Asp Glu Val Asp Gly Ala Asp Leu Ser Leu
                   245                 250                 255
   gtg gat gcg ttg aca gaa cca cct cca gaa att gag gga gaa ata aag     816
   Val Asp Ala Leu Thr Glu Pro Pro Pro Glu Ile Glu Gly Glu Ile Lys
               260                 265                 270
   cga gac ttc atg gct gcg ctg gag gca gag ccc tat gat gac atc gtg     864
   Arg Asp Phe Met Ala Ala Leu Glu Ala Glu Pro Tyr Asp Asp Ile Val
           275                 280                 285
   gga gaa act gtg gag aaa act gag ttt att cct ctc ctg gat ggt gat    912
   Gly Glu Thr Val Glu Lys Thr Glu Phe Ile Pro Leu Leu Asp Gly Asp
       290                 295                 300
   gag aaa acc ggg aac tca gag tcc aaa aag aaa ccc tgc tta gac act    960
   Glu Lys Thr Gly Asn Ser Glu Ser Lys Lys Lys Pro Cys Leu Asp Thr
   305                 310                 315                 320
   agc cag gtt gaa ggt atc cca tct tct aaa cca aca ctc cta gcc aat   1008
   Ser Gln Val Glu Gly Ile Pro Ser Ser Lys Pro Thr Leu Leu Ala Asn
                   325                 330                 335
   ggt gat cat gga atg gag ggg aat aac act gca ggg tct cca act gac   1056
   Gly Asp His Gly Met Glu Gly Asn Asn Thr Ala Gly Ser Pro Thr Asp
               340                 345                 350
   ttc ctt gaa gag aga gtg gac tat ccg gat tat cag agc agc cag aac   1104
   Phe Leu Glu Glu Arg Val Asp Tyr Pro Asp Tyr Gln Ser Ser Gln Asn
           355                 360                 365
   tgg cca gaa gat gca agc ttt tgt ttc cag cct cag caa gtg tta gat   1152
   Trp Pro Glu Asp Ala Ser Phe Cys Phe Gln Pro Gln Gln Val Leu Asp
       370                 375                 380
   act gac cag gct gag ccc ttt aac gag cac cgt gat gat ggt ttg gca   1200
   Thr Asp Gln Ala Glu Pro Phe Asn Glu His Arg Asp Asp Gly Leu Ala
   385                 390                 395                 400
   gat ctg ctc ttt gtc tcc agt gga ccc acg aac gct tct gca ttt aca   1248
   Asp Leu Leu Phe Val Ser Ser Gly Pro Thr Asn Ala Ser Ala Phe Thr
                   405                 410                 415
   gag cga gac aat cct tca gaa gac agt tac ggt atg ctt ccc tgt gac   1296
   Glu Arg Asp Asn Pro Ser Glu Asp Ser Tyr Gly Met Leu Pro Cys Asp
               420                 425                 430
   tca ttt gct tcc acg gct gtt gta tct cag gag tgg tct gtg gga gcc   1344
   Ser Phe Ala Ser Thr Ala Val Val Ser Gln Glu Trp Ser Val Gly Ala
           435                 440                 445
   cca aac tct cca tgt tca gag tcc tgt gtc tcc cca gag gtt act ata   1392
   Pro Asn Ser Pro Cys Ser Glu Ser Cys Val Ser Pro Glu Val Thr Ile
       450                 455                 460
   gaa acc cta cag cca gca aca gag ctc tcc aag gca gca gaa gtg gaa   1440
   Glu Thr Leu Gln Pro Ala Thr Glu Leu Ser Lys Ala Ala Glu Val Glu
   465                 470                 475                 480
   tca gtg aaa gag cag ctg cca gct aaa gca ttg gaa acg atg gca gag   1488
   Ser Val Lys Glu Gln Leu Pro Ala Lys Ala Leu Glu Thr Met Ala Glu
                   485                 490                 495
   cag acc act gat gtg gtg cac tct cca tcc aca gac aca aca cca ggc   1536
   Gln Thr Thr Asp Val Val His Ser Pro Ser Thr Asp Thr Thr Pro Gly
               500                 505                 510
   cca gac aca gag gca gca ctg gct aaa gac ata gaa gag atc acc aag   1584
   Pro Asp Thr Glu Ala Ala Leu Ala Lys Asp Ile Glu Glu Ile Thr Lys
           515                 520                 525
   cca gat gtg ata ttg gca aat gtc acg cag cca tct act gaa tcg gat   1632
   Pro Asp Val Ile Leu Ala Asn Val Thr Gln Pro Ser Thr Glu Ser Asp
       530                 535                 540
   atg ttc ctg gcc cag gac atg gaa cta ctc aca gga aca gag gca gcc    1680
   Met Phe Leu Ala Gln Asp Met Glu Leu Leu Thr Gly Thr Glu Ala Ala
   545                 550                 555                 560
   cac gct aac aat atc ata ttg cct aca gaa cca gac gaa tct tca acc    1728
   His Ala Asn Asn Ile Ile Leu Pro Thr Glu Pro Asp Glu Ser Ser Thr
                   565                 570                 575
   aag gat gta gca cca cct atg gaa gaa gaa att gtc cca ggc aat gat    1776
   Lys Asp Val Ala Pro Pro Met Glu Glu Glu Ile Val Pro Gly Asn Asp
               580                 585                 590
   acg aca tcc ccc aaa gaa aca gag aca aca ctt cca ata aaa atg gac    1824
   Thr Thr Ser Pro Lys Glu Thr Glu Thr Thr Leu Pro Ile Lys Met Asp
           595                 600                 605
   ttg gca cca cct gag gat gtg tta ctt acc aaa gaa aca gaa cta gcc    1872
   Leu Ala Pro Pro Glu Asp Val Leu Leu Thr Lys Glu Thr Glu Leu Ala
       610                 615                 620
   cca gcc aag ggc atg gtt tca ctc tca gaa ata gaa gag gct ctg gca    1920
   Pro Ala Lys Gly Met Val Ser Leu Ser Glu Ile Glu Glu Ala Leu Ala
   625                 630                 635                 640
   aag aat gat gtt cgc tct gca gaa ata cct gtg gct cag gag aca gtg    1968
   Lys Asn Asp Val Arg Ser Ala Glu Ile Pro Val Ala Gln Glu Thr Val
                   645                 650                 655
   gtc tca gaa aca gag gtg gtc ctg gca aca gaa gtg gta ctg ccc tca    2016
   Val Ser Glu Thr Glu Val Val Leu Ala Thr Glu Val Val Leu Pro Ser
               660                 665                 670
   gat ccc ata aca aca ttg aca aag gat gtg aca ctc ccc tta gaa gca    2064
   Asp Pro Ile Thr Thr Leu Thr Lys Asp Val Thr Leu Pro Leu Glu Ala
           675                 680                 685
   gag aga ccg ttg gtg acg gac atg act cca tct ctg gaa aca gaa atg    2112
   Glu Arg Pro Leu Val Thr Asp Met Thr Pro Ser Leu Glu Thr Glu Met
       690                 695                 700
   acc cta ggc aaa gag aca gct cca ccc aca gaa aca aat ttg ggc atg    2160
   Thr Leu Gly Lys Glu Thr Ala Pro Pro Thr Glu Thr Asn Leu Gly Met
   705                 710                 715                 720
   gcc aaa gac atg tct cca ctc cca gaa tca gaa gtg act ctg ggc aag    2208
   Ala Lys Asp Met Ser Pro Leu Pro Glu Ser Glu Val Thr Leu Gly Lys
                   725                 730                 735
   gac gtg gtt ata ctt cca gaa aca aag gtg gct gag ttt aac aat gtg    2256
   Asp Val Val Ile Leu Pro Glu Thr Lys Val Ala Glu Phe Asn Asn Val
               740                 745                 750
   act cca ctt tca gaa gaa gag gta acc tca gtc aag gac atg tct ccg    2304
   Thr Pro Leu Ser Glu Glu Glu Val Thr Ser Val Lys Asp Met Ser Pro
           755                 760                 765
   tct gca gaa aca gag gct ccc ctg gct aag aat gct gat ctg cac tca    2352
   Ser Ala Glu Thr Glu Ala Pro Leu Ala Lys Asn Ala Asp Leu His Ser
               770                 775                 780
   gga aca gag ctg att gtg gac aac agc atg gct cca gcc tcc gat ctt     2400
   Gly Thr Glu Leu Ile Val Asp Asn Ser Met Ala Pro Ala Ser Asp Leu
   785                 790                 795                 800
   gca ctg ccc ttg gaa aca aaa gta gca aca gtt cca att aaa gac aaa     2448
   Ala Leu Pro Leu Glu Thr Lys Val Ala Thr Val Pro Ile Lys Asp Lys
                   805                 810                 815
   gga atg gtg agc aag ggc gag gag ctg ttc acc ggg gtg gtg ccc atc     2496
   Gly Met Val Ser Lys Gly Glu Glu Leu Phe Thr Gly Val Val Pro Ile
               820                 825                 830
   ctg gtc gag ctg gac ggc gac gta aac ggc cac aag ttc agc gtg tcc     2544
   Leu Val Glu Leu Asp Gly Asp Val Asn Gly His Lys Phe Ser Val Ser
           835                 840                 845
   ggc gag ggc gag ggc gat gcc acc tac ggc aag ctg acc ctg aag ttc     2592
   Gly Glu Gly Glu Gly Asp Ala Thr Tyr Gly Lys Leu Thr Leu Lys Phe
       850                 855                 860
   atc tgc acc acc ggc aag ctg ccc gtg ccc tgg ccc acc ctc gtg acc     2640
   Ile Cys Thr Thr Gly Lys Leu Pro Val Pro Trp Pro Thr Leu Val Thr
   865                 870                 875                 880
   acc ctg acc cac ggc gtg cag tgc ttc agc cgc tac ccc gac cac atg     2688
   Thr Leu Thr His Gly Val Gln Cys Phe Ser Arg Tyr Pro Asp His Met
                   885                 890                 895
   aag cag cac gac ttc ttc aag tcc gcc atg ccc gaa ggc tac gtc cag     2736
   Lys Gln His Asp Phe Phe Lys Ser Ala Met Pro Glu Gly Tyr Val Gln
               900                 905                 910
   gag cgc acc atc ttc ttc aag gac gac ggc aac tac aag acc cgc gcc     2784
   Glu Arg Thr Ile Phe Phe Lys Asp Asp Gly Asn Tyr Lys Thr Arg Ala
           915                 920                 925
   gag gtg aag ttc gag ggc gac acc ctg gtg aac cgc atc gag ctg aag     2832
   Glu Val Lys Phe Glu Gly Asp Thr Leu Val Asn Arg Ile Glu Leu Lys
       930                 935                 940
   ggc atc gac ttc aag gag gac ggc aac atc ctg ggg cac aag ctg gag     2880
   Gly Ile Asp Phe Lys Glu Asp Gly Asn Ile Leu Gly His Lys Leu Glu
   945                 950                 955                 960
   tac aac ttc aac agc cac aac gtc tat atc atg gcc gac aag cag aag     2928
   Tyr Asn Phe Asn Ser His Asn Val Tyr Ile Met Ala Asp Lys Gln Lys
                   965                 970                 975
   aac ggc atc aag gtg aac ttc aag atc cgc cac aac atc gag gac ggc     2976
   Asn Gly Ile Lys Val Asn Phe Lys Ile Arg His Asn Ile Glu Asp Gly
               980                 985                 990
   agc gtg cag ctc gcc gac cac tac cag cag aac acc ccc atc ggc gac     3024
   Ser Val Gln Leu Ala Asp His Tyr Gln Gln Asn Thr Pro Ile Gly Asp
           995                 1000                1005
   ggc ccc gtg ctg ctg ccc gac aac cac tac ctg agc acc cag tcc gcc     3072
   Gly Pro Val Leu Leu Pro Asp Asn His Tyr Leu Ser Thr Gln Ser Ala
       1010                1015                1020
   ctg agc aaa gac ccc aac gag aag cgc gat cac atg gtc ctg ctg gag    3120
   Leu Ser Lys Asp Pro Asn Glu Lys Arg Asp His Met Val Leu Leu Glu
   1025                1030                1035                1040
   ttc gtg acc gcc gcc ggg atc act ctc ggc atg gac gag ctg tac aag    3168
   Phe Val Thr Ala Ala Gly Ile Thr Leu Gly Met Asp Glu Leu Tyr Lys
                   1045                1050                1055
   tag                                                                3171
<210> 32
<211> 1056
<212> PRT
<213> Artificial Sequence
<220>
<223> Description of Artificial Sequence:
   NLS-EYFP-MAPKDM-EBFP construct
<400> 32
   Met Arg Pro Arg Arg Lys Val Ser Lys Gly Glu Glu Leu Phe Thr Gly
   1 5 10 15
   Val Val Pro Ile Leu Val Glu Leu Asp Gly Asp Val Asn Gly His Lys
   20 25 30
   Phe Ser Val Ser Gly Glu Gly Glu Gly Asp Ala Thr Tyr Gly Lys Leu
   35 40 45
   Thr Leu Lys Phe Ile Cys Thr Thr Gly Lys Leu Pro Val Pro Trp Pro
   50 55 60
   Thr Leu Val Thr Thr Phe Gly Tyr Gly Leu Gln Cys Phe Ala Arg Tyr
   65 70 75 80
   Pro Asp His Met Lys Gln His Asp Phe Phe Lys Ser Ala Met Pro Glu
   85 90 95
   Gly Tyr Val Gln Glu Arg Thr Ile Phe Phe Lys Asp Asp Gly Asn Tyr
   100 105 110
   Lys Thr Arg Ala Glu Val Lys Phe Glu Gly Asp Thr Leu Val Asn Arg
   115 120 125
   Ile Glu Leu Lys Gly Ile Asp Phe Lys Glu Asp Gly Asn Ile Leu Gly
   130 135 140
   His Lys Leu Glu Tyr Asn Tyr Asn Ser His Asn Val Tyr Ile Met Ala
   145 150 155 160
   Asp Lys Gln Lys Asn Gly Ile Lys Val Asn Phe Lys Ile Arg His Asn
   165 170 175
   Ile Glu Asp Gly Ser Val Gln Leu Ala Asp His Tyr Gln Gln Asn Thr
   180 185 190
   Pro Ile Gly Asp Gly Pro Val Leu Leu Pro Asp Asn His Tyr Leu Ser
   195 200 205
   Tyr Gln Ser Ala Leu Ser Lys Asp Pro Asn Glu Lys Arg Asp His Met
       210                 215                 220
   Val Leu Leu Glu Phe Val Thr Ala Ala Gly Ile Thr Leu Gly Met Asp
   225                 230                 235                 240
   Glu Leu Tyr Lys Lys Gly Asp Glu Val Asp Gly Ala Asp Leu Ser Leu
                   245                 250                 255
   Val Asp Ala Leu Thr Glu Pro Pro Pro Glu Ile Glu Gly Glu Ile Lys
               260                 265                 270
   Arg Asp Phe Met Ala Ala Leu Glu Ala Glu Pro Tyr Asp Asp Ile Val
           275                 280                 285
   Gly Glu Thr Val Glu Lys Thr Glu Phe Ile Pro Leu Leu Asp Gly Asp
       290                 295                 300
   Glu Lys Thr Gly Asn Ser Glu Ser Lys Lys Lys Pro Cys Leu Asp Thr
   305                 310                 315                 320
   Ser Gln Val Glu Gly Ile Pro Ser Ser Lys Pro Thr Leu Leu Ala Asn
                   325                 330                 335
   Gly Asp His Gly Met Glu Gly Asn Asn Thr Ala Gly Ser Pro Thr Asp
               340                 345                 350
   Phe Leu Glu Glu Arg Val Asp Tyr Pro Asp Tyr Gln Ser Ser Gln Asn
           355                 360                 365
   Trp Pro Glu Asp Ala Ser Phe Cys Phe Gln Pro Gln Gln Val Leu Asp
       370                 375                 380
   Thr Asp Gln Ala Glu Pro Phe Asn Glu His Arg Asp Asp Gly Leu Ala
   385                 390                 395                 400
   Asp Leu Leu Phe Val Ser Ser Gly Pro Thr Asn Ala Ser Ala Phe Thr
                   405                 410                 415
   Glu Arg Asp Asn Pro Ser Glu Asp Ser Tyr Gly Met Leu Pro Cys Asp
               420                 425                 430
   Ser Phe Ala Ser Thr Ala Val Val Ser Gln Glu Trp Ser Val Gly Ala
           435                 440                 445
   Pro Asn Ser Pro Cys Ser Glu Ser Cys Val Ser Pro Glu Val Thr Ile
       450                 455                 460
   Glu Thr Leu Gln Pro Ala Thr Glu Leu Ser Lys Ala Ala Glu Val Glu
   465                 470                 475                 480
   Ser Val Lys Glu Gln Leu Pro Ala Lys Ala Leu Glu Thr Met Ala Glu
                   485                 490                 495
   Gln Thr Thr Asp Val Val His Ser Pro Ser Thr Asp Thr Thr Pro Gly
               500                 505                 510
   Pro Asp Thr Glu Ala Ala Leu Ala Lys Asp Ile Glu Glu Ile Thr Lys
           515                 520                 525
   Pro Asp Val Ile Leu Ala Asn Val Thr Gln Pro Ser Thr Glu Ser Asp
   530 535 540
   Met Phe Leu Ala Gln Asp Met Glu Leu Leu Thr Gly Thr Glu Ala Ala
   545 550 555 560
   His Ala Asn Asn Ile Ile Leu Pro Thr Glu Pro Asp Glu Ser Ser Thr
   565 570 575
   Lys Asp Val Ala Pro Pro Met Glu Glu Glu Ile Val Pro Gly Asn Asp
   580 585 590
   Thr Thr Ser Pro Lys Glu Thr Glu Thr Thr Leu Pro Ile Lys Met Asp
   595 600 605
   Leu Ala Pro Pro Glu Asp Val Leu Leu Thr Lys Glu Thr Glu Leu Ala
   610 615 620
   Pro Ala Lys Gly Met Val Ser Leu Ser Glu Ile Glu Glu Ala Leu Ala
   625 630 635 640
   Lys Asn Asp Val Arg Ser Ala Glu Ile Pro Val Ala Gln Glu Thr Val
   645 650 655
   Val Ser Glu Thr Glu Val Val Leu Ala Thr Glu Val Val Leu Pro Ser
   660 665 670
   Asp Pro Ile Thr Thr Leu Thr Lys Asp Val Thr Leu Pro Leu Glu Ala
   675 680 685
   Glu Arg Pro Leu Val Thr Asp Met Thr Pro Ser Leu Glu Thr Glu Met
   690 695 700
   Thr Leu Gly Lys Glu Thr Ala Pro Pro Thr Glu Thr Asn Leu Gly Met
   705 710 715 720
   Ala Lys Asp Met Ser Pro Leu Pro Glu Ser Glu Val Thr Leu Gly Lys
   725 730 735
   Asp Val Val Ile Leu Pro Glu Thr Lys Val Ala Glu Phe Asn Asn Val
   740 745 750
   Thr Pro Leu Ser Glu Glu Glu Val Thr Ser Val Lys Asp Met Ser Pro
   755 760 765
   Ser Ala Glu Thr Glu Ala Pro Leu Ala Lys Asn Ala Asp Leu His Ser
   770 775 780
   Gly Thr Glu Leu Ile Val Asp Asn Ser Met Ala Pro Ala Ser Asp Leu
   785 790 795 800
   Ala Leu Pro Leu Glu Thr Lys Val Ala Thr Val Pro Ile Lys Asp Lys
   805 810 815
   Gly Met Val Ser Lys Gly Glu Glu Leu Phe Thr Gly Val Val Pro Ile
   820 825 830
   Leu Val Glu Leu Asp Gly Asp Val Asn Gly His Lys Phe Ser Val Ser
   835 840 845
   Gly Glu Gly Glu Gly Asp Ala Thr Tyr Gly Lys Leu Thr Leu Lys Phe
   850 855 860
   Ile Cys Thr Thr Gly Lys Leu Pro Val Pro Trp Pro Thr Leu Val Thr
   865                 870                 875                 880
   Thr Leu Thr His Gly Val Gln Cys Phe Ser Arg Tyr Pro Asp His Met
                   885                 890                 895
   Lys Gln His Asp Phe Phe Lys Ser Ala Met Pro Glu Gly Tyr Val Gln
               900                 905                 910
   Glu Arg Thr Ile Phe Phe Lys Asp Asp Gly Asn Tyr Lys Thr Arg Ala
           915                 920                 925
   Glu Val Lys Phe Glu Gly Asp Thr Leu Val Asn Arg Ile Glu Leu Lys
       930                 935                 940
   Gly Ile Asp Phe Lys Glu Asp Gly Asn Ile Leu Gly His Lys Leu Glu
   945                 950                 955                 960
   Tyr Asn Phe Asn Ser His Asn Val Tyr Ile Met Ala Asp Lys Gln Lys
                   965                 970                 975
   Asn Gly Ile Lys Val Asn Phe Lys Ile Arg His Asn Ile Glu Asp Gly
               980                 985                 990
   Ser Val Gln Leu Ala Asp His Tyr Gln Gln Asn Thr Pro Ile Gly Asp
           995                 1000                1005
   Gly Pro Val Leu Leu Pro Asp Asn His Tyr Leu Ser Thr Gln Ser Ala
       1010                1015                1020
   Leu Ser Lys Asp Pro Asn Glu Lys Arg Asp His Met Val Leu Leu Glu
   1025                1030                1035                1040
   Phe Val Thr Ala Ala Gly Ile Thr Leu Gly Met Asp Glu Leu Tyr Lys
                   1045                1050                1055
<210> 33
<211> 1623
<212> DNA
<213> Artificial Sequence
<220>
<221> CDS
<222> (1)..(1623)
<220>
<223> Description of Artificial Sequence:
   YFP-NLS-CP3-multiple DEVD-CFP-Annexin II construct
<400> 33
   atg gtg agc aag ggc gag gag ctg ttc acc ggg gtg gtg ccc atc ctg    48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc    96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc   144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ttc ggc tac ggc ctg cag tgc ttc gcc cgc tac ccc gac cac atg aag     240
   Phe Gly Tyr Gly Leu Gln Cys Phe Ala Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg     624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag tcc     720
   Val Thr Ala Ala Gly Ile Thr Leu Gly Met Asp Glu Leu Tyr Lys Ser
   225                 230                 235                 240
   gga aga agg aaa cga caa aag cga tcg gca ggt gac gaa gtt gat gca     768
   Gly Arg Arg Lys Arg Gln Lys Arg Ser Ala Gly Asp Glu Val Asp Ala
                   245                 250                 255
   ggt gac gaa gtt gat gca ggt gac gaa gtt gat gca ggt gac gaa gtt     816
   Gly Asp Glu Val Asp Ala Gly Asp Glu Val Asp Ala Gly Asp Glu Val
               260                 265                 270
   gac gca ggt agt act atg gtg agc aag ggc gag gag ctg ttc acc ggg     864
   Asp Ala Gly Ser Thr Met Val Ser Lys Gly Glu Glu Leu Phe Thr Gly
           275                 280                 285
   gtg gtg ccc atc ctg gtc gag ctg gac ggc gac gta aac ggc cac aag    912
   Val Val Pro Ile Leu Val Glu Leu Asp Gly Asp Val Asn Gly His Lys
       290                 295                 300
   ttc agc gtg tcc ggc gag ggc gag ggc gat gcc acc tac ggc aag ctg    960
   Phe Ser Val Ser Gly Glu Gly Glu Gly Asp Ala Thr Tyr Gly Lys Leu
   305                 310                 315                 320
   acc ctg aag ttc atc tgc acc acc ggc aag ctg ccc gtg ccc tgg ccc   1008
   Thr Leu Lys Phe Ile Cys Thr Thr Gly Lys Leu Pro Val Pro Trp Pro
                   325                 330                 335
   acc ctc gtg acc acc ctg acc tgg ggc gtg cag tgc ttc agc cgc tac   1056
   Thr Leu Val Thr Thr Leu Thr Trp Gly Val Gln Cys Phe Ser Arg Tyr
               340                 345                 350
   ccc gac cac atg aag cag cac gac ttc ttc aag tcc gcc atg ccc gaa   1104
   Pro Asp His Met Lys Gln His Asp Phe Phe Lys Ser Ala Met Pro Glu
           355                 360                 365
   ggc tac gtc cag gag cgc acc atc ttc ttc aag gac gac ggc aac tac   1152
   Gly Tyr Val Gln Glu Arg Thr Ile Phe Phe Lys Asp Asp Gly Asn Tyr
       370                 375                 380
   aag acc cgc gcc gag gtg aag ttc gag ggc gac acc ctg gtg aac cgc   1200
   Lys Thr Arg Ala Glu Val Lys Phe Glu Gly Asp Thr Leu Val Asn Arg
   385                 390                 395                 400
   atc gag ctg aag ggc atc gac ttc aag gag gac ggc aac atc ctg ggg   1248
   Ile Glu Leu Lys Gly Ile Asp Phe Lys Glu Asp Gly Asn Ile Leu Gly
                   405                 410                 415
   cac aag ctg gag tac aac tac atc agc cac aac gtc tat atc acc gcc   1296
   His Lys Leu Glu Tyr Asn Tyr Ile Ser His Asn Val Tyr Ile Thr Ala
               420                 425                 430
   gac aag cag aag aac ggc atc aag gcc aac ttc aag atc cgc cac aac   1344
   Asp Lys Gln Lys Asn Gly Ile Lys Ala Asn Phe Lys Ile Arg His Asn
           435                 440                 445
   atc gag gac ggc agc gtg cag ctc gcc gac cac tac cag cag aac acc   1392
   Ile Glu Asp Gly Ser Val Gln Leu Ala Asp His Tyr Gln Gln Asn Thr
       450                 455                 460
   ccc atc ggc gac ggc ccc gtg ctg ctg ccc gac aac cac tac ctg agc   1440
   Pro Ile Gly Asp Gly Pro Val Leu Leu Pro Asp Asn His Tyr Leu Ser
   465                 470                 475                 480
   acc cag tcc gcc ctg agc aaa gac ccc aac gag aag cgc gat cac atg   1488
   Thr Gln Ser Ala Leu Ser Lys Asp Pro Asn Glu Lys Arg Asp His Met
                   485                 490                 495
   gtc ctg ctg gag ttc gtg acc gcc gcc ggg atc act ctc ggc atg gac   1536
   Val Leu Leu Glu Phe Val Thr Ala Ala Gly Ile Thr Leu Gly Met Asp
               500                 505                 510
   gag ctg tac aag atg tct act gtc cac gaa atc ctg tgc aag ctc agc   1584
   Glu Leu Tyr Lys Met Ser Thr Val His Glu Ile Leu Cys Lys Leu Ser
           515                 520                 525
   ttg gag ggt gtt cat tct aca ccc cca agt gcc gga tcc
   Leu Glu Gly Val His Ser Thr Pro Pro Ser Ala Gly Ser
       530                 535                 540
<210> 34
<211> 541
<212> PRT
<213> Artificial Sequence
<220>
<223> Description of Artificial Sequence:
   YFP-NLS-CP3-multiple DEVD-CFP-Annexin II construct
<400> 34
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Phe Gly Tyr Gly Leu Gln Cys Phe Ala Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Tyr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Ser
   225                 230                 235                 240
   Gly Arg Arg Lys Arg Gln Lys Arg Ser Ala Gly Asp Glu Val Asp Ala
                   245                 250                 255
   Gly Asp Glu Val Asp Ala Gly Asp Glu Val Asp Ala Gly Asp Glu Val
               260                 265                 270
   Asp Ala Gly Ser Thr Met Val Ser Lys Gly Glu Glu Leu Phe Thr Gly
           275                 280                 285
   Val Val Pro Ile Leu Val Glu Leu Asp Gly Asp Val Asn Gly His Lys
       290                 295                 300
   Phe Ser Val Ser Gly Glu Gly Glu Gly Asp Ala Thr Tyr Gly Lys Leu
   305                 310                 315                 320
   Thr Leu Lys Phe Ile Cys Thr Thr Gly Lys Leu Pro Val Pro Trp Pro
                   325                 330                 335
   Thr Leu Val Thr Thr Leu Thr Trp Gly Val Gln Cys Phe Ser Arg Tyr
               340                 345                 350
   Pro Asp His Met Lys Gln His Asp Phe Phe Lys Ser Ala Met Pro Glu
           355                 360                 365
   Gly Tyr Val Gln Glu Arg Thr Ile Phe Phe Lys Asp Asp Gly Asn Tyr
       370                 375                 380
   Lys Thr Arg Ala Glu Val Lys Phe Glu Gly Asp Thr Leu Val Asn Arg
   385                 390                 395                 400
   Ile Glu Leu Lys Gly Ile Asp Phe Lys Glu Asp Gly Asn Ile Leu Gly
                   405                 410                 415
   His Lys Leu Glu Tyr Asn Tyr Ile Ser His Asn Val Tyr Ile Thr Ala
               420                 425                 430
   Asp Lys Gln Lys Asn Gly Ile Lys Ala Asn Phe Lys Ile Arg His Asn
           435                 440                 445
   Ile Glu Asp Gly Ser Val Gln Leu Ala Asp His Tyr Gln Gln Asn Thr
       450                 455                 460
   Pro Ile Gly Asp Gly Pro Val Leu Leu Pro Asp Asn His Tyr Leu Ser
   465                 470                 475                 480
   Thr Gln Ser Ala Leu Ser Lys Asp Pro Asn Glu Lys Arg Asp His Met
                   485                 490                 495
   Val Leu Leu Glu Phe Val Thr Ala Ala Gly Ile Thr Leu Gly Met Asp
               500                 505                 510
   Glu Leu Tyr Lys Met Ser Thr Val His Glu Ile Leu Cys Lys Leu Ser
           515                 520                 525
   Leu Glu Gly Val His Ser Thr Pro Pro Ser Ala Gly Ser
       530                 535                 540
<210> 35
<211> 24
<212> DNA
<213> Artificial Sequence
<220>
<223> Description of Artificial Sequence: FLAG epitope
<400> 35
   gactacaaag acgacgacga caaa 24
<210> 36
<211> 8
<212> PRT
<213> Artificial Sequence
<220>
<223> Description of Artificial Sequence: FLAG epitope
<400> 36
   Asp Tyr Lys Asp Asp Asp Asp Lys
   1 5
<210> 37
<211> 27
<212> DNA
<213> Artificial Sequence
<220>
<223> Description of Artificial Sequence: HA epitope
<400> 37
   tacccatacg acgtaccaga ctacgca 27
<210> 38
<211> 9
<212> PRT
<213> Artificial Sequence
<220>
<223> Description of Artificial Sequence: HA epitope
<400> 38
   Tyr Pro Tyr Asp Val Pro Asp Tyr Ala
   1 5
<210> 39
<211> 18
<212> DNA
<213> Artificial Sequence
<220>
<223> Description of Artificial Sequence: KT3 epitope
<400> 39
   ccaccagaac cagaaaca 18
<210> 40
<211> 6
<212> PRT
<213> Artificial Sequence
<220>
<223> Description of Artificial Sequence: KT3 epitope
<400> 40
   Pro Pro Glu Pro Glu Thr
     1               5
<210> 41
<211> 36
<212> DNA
<213> Artificial Sequence
<220>
<223> Description of Artificial Sequence: Myc epitope
<400> 41
   gcagaagaac aaaaattaat aagcgaagaa gactta                                36
<210> 42
<211> 12
<212> PRT
<213> Artificial Sequence
<220>
<223> Description of Artificial Sequence: Myc epitope
<400> 42
   Ala Glu Glu Gln Lys Leu Ile Ser Glu Glu Asp Leu
     1               5                  10
<210> 43
<211> 717
<212> DNA
<213> Artificial Sequence
<220>
<221> CDS
<222> (1)..(717)
<220>
<223> Description of Artificial Sequence: EYFP
<400> 43
   atg gtg agc aag ggc gag gag ctg ttc acc ggg gtg gtg ccc atc ctg   48
   Met Val Ser Lys Gly Glu Glu Leu Phe Thr Gly Val Val Pro Ile Leu
     1               5                  10                  15
   gtc gag ctg gac ggc gac gta aac ggc cac aag ttc agc gtg tcc ggc   96
   Val Glu Leu Asp Gly Asp Val Asn Gly His Lys Phe Ser Val Ser Gly
                20                  25                  30
   gag ggc gag ggc gat gcc acc tac ggc aag ctg acc ctg aag ttc atc   144
   Glu Gly Glu Gly Asp Ala Thr Tyr Gly Lys Leu Thr Leu Lys Phe Ile
            35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
        50                  55                  60
   ttc ggc tac ggc ctg cag tgc ttc gcc cgc tac ccc gac cac atg aag     240
   Phe Gly Tyr Gly Leu Gln Cys Phe Ala Arg Tyr Pro Asp His Met Lys
    65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                    85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc tac cag tcc gcc ctg     624
   Pro Val Leu Leu Pro Asp Asn His Tyr Leu Ser Tyr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag         717
   Val Thr Ala Ala Gly Ile Thr Leu Gly Met Asp Glu Leu Tyr Lys
   225                 230                 235
<210> 44
<211> 239
<212> PRT
<213> Artificial Sequence
<220>
<223> Description of Artificial Sequence: EYFP
<400> 44
   Met Val Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Phe Gly Tyr Gly Leu Gln Cys Phe Ala Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Tyr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys
   225 230 235
<210> 45
<211> 717
<212> DNA
<213> Artificial Sequence
<220>
<221> CDS
<222> (1)..(717)
<220>
<223> Description of Artificial Sequence: EGFP
<400> 45
   atg gtg agc aag ggc gag gag ctg ttc acc ggg gtg gtg ccc atc ctg    48
   Met Val Ser Lys Gly Glu Glu Leu Phe Thr Gly Val Val Pro Ile Leu
     1               5                  10                  15
   gtc gag ctg gac ggc gac gta aac ggc cac aag ttc agc gtg tcc ggc    96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc     144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc     192
   Cys Thr Thr Gly Lys Leu Pro Val Pro Trp Pro Thr Leu Val Thr Thr
       50                  55                  60
   ctg acc tac ggc gtg cag tgc ttc agc cgc tac ccc gac cac atg aag     240
   Leu Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag     288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag     336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc     384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac     432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac     480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc     528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc     576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg     624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc     672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag         717
   Val Thr Ala Ala Gly Ile Thr Leu Gly Met Asp Glu Leu Tyr Lys
   225                 230                 235
<210> 46
<211> 239
<212> PRT
<213> Artificial Sequence
<220>
<223> Description of Artificial Sequence: EGFP
<400> 46
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
    65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 47
<211> 717
<212> DNA
<213> Artificial Sequence
<220>
<221> CDS
<222> (1)..(717)
<220>
<223> Description of Artificial Sequence: EBFP
<400> 47
   atg gtg agc aag ggc gag gag ctg ttc acc ggg gtg gtg ccc atc ctg     48
   Met Val Ser Lys Gly Glu Glu Leu Phe Thr Gly Val Val Pro Ile Leu
    1               5                  10                  15
   gtc gag ctg gac ggc gac gta aac ggc cac aag ttc agc gtg tcc ggc     96
   Val Glu Leu Asp Gly Asp Val Asn Gly His Lys Phe Ser Val Ser Gly
               20                  25                  30
   gag ggc gag ggc gat gcc acc tac ggc aag ctg acc ctg aag ttc atc    144
   Glu Gly Glu Gly Asp Ala Thr Tyr Gly Lys Leu Thr Leu Lys Phe Ile
           35                  40                  45
   tgc acc acc ggc aag ctg ccc gtg ccc tgg ccc acc ctc gtg acc acc    192
   Cys Thr Thr Gly Lys Leu Pro Val Pro Trp Pro Thr Leu Val Thr Thr
       50                  55                  60
   ctg acc cac ggc gtg cag tgc ttc agc cgc tac ccc gac cac atg aag    240
   Leu Thr His Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag    288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag    336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc    384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac    432
   Ile Asp Phe Lys Glu Asp Gly Asn Ile Leu Gly His Lys Leu Glu Tyr
       130                 135                 140
   aac ttc aac agc cac aac gtc tat atc atg gcc gac aag cag aag aac    480
   Asn Phe Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc    528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc    576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg    624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc    672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag        717
   Val Thr Ala Ala Gly Ile Thr Leu Gly Met Asp Glu Leu Tyr Lys
   225                 230                 235
<210> 48
<211> 239
<212> PRT
<213> Artificial Sequence
<220>
<223> Description of Artificial Sequence: EBFP
<400> 48
   Met Val Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr His Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Phe Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys
   225                 230                 235
<210> 49
<211> 717
<212> DNA
<213> Artificial Sequence
<220>
<221> CDS
<222> (1)..(717)
<220>
<223> Description of Artificial Sequence: ECFP
<400> 49
   atg gtg agc aag ggc gag gag ctg ttc acc ggg gtg gtg ccc atc ctg     48
   Met Val Ser Lys Gly Glu Glu Leu Phe Thr Gly Val Val Pro Ile Leu
     1               5                  10                  15
   gtc gag ctg gac ggc gac gta aac ggc cac aag ttc agc gtg tcc ggc     96
   Val Glu Leu Asp Gly Asp Val Asn Gly His Lys Phe Ser Val Ser Gly
                20                  25                  30
   gag ggc gag ggc gat gcc acc tac ggc aag ctg acc ctg aag ttc atc    144
   Glu Gly Glu Gly Asp Ala Thr Tyr Gly Lys Leu Thr Leu Lys Phe Ile
            35                  40                  45
   tgc acc acc ggc aag ctg ccc gtg ccc tgg ccc acc ctc gtg acc acc    192
   Cys Thr Thr Gly Lys Leu Pro Val Pro Trp Pro Thr Leu Val Thr Thr
        50                  55                  60
   ctg acc tgg ggc gtg cag tgc ttc agc cgc tac ccc gac cac atg aag    240
   Leu Thr Trp Gly Val Gln Cys Phe Ser Arg Tyr Pro Asp His Met Lys
    65                  70                  75                  80
   cag cac gac ttc ttc aag tcc gcc atg ccc gaa ggc tac gtc cag gag    288
   Gln His Asp Phe Phe Lys Ser Ala Met Pro Glu Gly Tyr Val Gln Glu
                    85                  90                  95
   cgc acc atc ttc ttc aag gac gac ggc aac tac aag acc cgc gcc gag    336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc    384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac    432
   Ile Asp Phe Lys Glu Asp Gly Asn Ile Leu Gly His Lys Leu Glu Tyr
       130                 135                 140
   aac tac atc agc cac aac gtc tat atc acc gcc gac aag cag aag aac    480
   Asn Tyr Ile Ser His Asn Val Tyr Ile Thr Ala Asp Lys Gln Lys Asn
   145                 150                 155                 160
   ggc atc aag gcc aac ttc aag atc cgc cac aac atc gag gac ggc agc    528
   Gly Ile Lys Ala Asn Phe Lys Ile Arg His Asn Ile Glu Asp Gly Ser
                   165                 170                 175
   gtg cag ctc gcc gac cac tac cag cag aac acc ccc atc ggc gac ggc    576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg    624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc   672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag       717
   Val Thr Ala Ala Gly Ile Thr Leu Gly Met Asp Glu Leu Tyr Lys
   225                 230                 235
<210> 50
<211> 239
<212> PRT
<213> Artificial Sequence
<220>
<223> Description of Artificial Sequence: ECFP
<400> 50
   Met Val Ser Lys Gly Glu Glu Leu Phe Thr Gly Val Val Pro Ile Leu
   1 5 10 15
   Val Glu Leu Asp Gly Asp Val Asn Gly His Lys Phe Ser Val Ser Gly
   20 25 30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Thr Trp Gly Val Gln Cys Phe Ser Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Ile Ser His Asn Val Tyr Ile Thr Ala Asp Lys Gln Lys Asn
   145 150 155 160
   Gly Ile Lys Ala Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys
   225 230 235
<210> 51
<211> 720
<212> DNA
<213> Artificial Sequence
<220>
<221> CDS
<222> (1)..(717)
<220>
<223> Description of Artificial Sequence: Fred25
<400> 51
   atg gct agc aaa gga gaa gaa ctc ttc act gga gtt gtc cca att ctt     48
   Met Ala Ser Lys Gly Glu Glu Leu Phe Thr Gly Val Val Pro Ile Leu
     1               5                  10                  15
   gtt gaa tta gat ggt gat gtt aac ggc cac aag ttc tct gtc agt gga     96
   Val Glu Leu Asp Gly Asp Val Asn Gly His Lys Phe Ser Val Ser Gly
                    20                  25                  30
   gag ggt gaa ggt gat gca aca tac gga aaa ctt acc ctg aag ttc atc    144
   Glu Gly Glu Gly Asp Ala Thr Tyr Gly Lys Leu Thr Leu Lys Phe Ile
                35                  40                  45
   tgc act act ggc aaa ctg cct gtt cca tgg cca aca cta gtc act act    192
   Cys Thr Thr Gly Lys Leu Pro Val Pro Trp Pro Thr Leu Val Thr Thr
            50                  55                  60
   ctg tgc tat ggt gtt caa tgc ttt tca aga tac ccg gat cat atg aaa    240
   Leu Cys Tyr Gly Val Gln Cys Phe Ser Arg Tyr Pro Asp His Met Lys
    65                  70                  75                  80
   cgg cat gac ttt ttc aag agt gcc atg ccc gaa ggt tat gta cag gaa    288
   Arg His Asp Phe Phe Lys Ser Ala Met Pro Glu Gly Tyr Val Gln Glu
                    85                  90                  95
   agg acc atc ttc ttc aaa gat gac ggc aac tac aag aca cgt gct gaa    336
   Arg Thr Ile Phe Phe Lys Asp Asp Gly Asn Tyr Lys Thr Arg Ala Glu
               100                 105                 110
   gtc aag ttt gaa ggt gat acc ctt gtt aat aga atc gag tta aaa ggt    384
   Val Lys Phe Glu Gly Asp Thr Leu Val Asn Arg Ile Glu Leu Lys Gly
           115                 120                 125
   att gac ttc aag gaa gat ggc aac att ctg gga cac aaa ttg gaa tac    432
   Ile Asp Phe Lys Glu Asp Gly Asn Ile Leu Gly His Lys Leu Glu Tyr
       130                 135                 140
   aac tat aac tca cac aat gta tac atc atg gca gac aaa caa aag aat    480
   Asn Tyr Asn Ser His Asn Val Tyr Ile Met Ala Asp Lys Gln Lys Asn
   145                 150                 155                 160
   gga atc aaa gtg aac ttc aag acc cgc cac aac att gaa gat gga agc    528
   Gly Ile Lys Val Asn Phe Lys Thr Arg His Asn Ile Glu Asp Gly Ser
                   165                 170                 175
   gtt caa cta gca gac cat tat caa caa aat act cca att ggc gat ggc    576
   Val Gln Leu Ala Asp His Tyr Gln Gln Asn Thr Pro Ile Gly Asp Gly
               180                 185                 190
   cct gtc ctt tta cca gac aac cat tac ctg tcc aca caa tct gcc ctt    624
   Pro Val Leu Leu Pro Asp Asn His Tyr Leu Ser Thr Gln Ser Ala Leu
           195                 200                 205
   tcg aaa gat ccc aac gaa aag aga gac cac atg gtc ctt ctt gag ttt    672
   Ser Lys Asp Pro Asn Glu Lys Arg Asp His Met Val Leu Leu Glu Phe
       210                 215                 220
   gta aca gct gct ggg att aca cat ggc atg gat gaa ctg tac aac tag    720
   Val Thr Ala Ala Gly Ile Thr His Gly Met Asp Glu Leu Tyr Asn
   225                 230                 235
<210> 52
<211> 239
<212> PRT
<213> Artificial Sequence
<220>
<223> Description of Artificial Sequence: Fred25
<400> 52
   Met Ala Ser Lys Gly Glu Glu Leu Phe Thr Gly Val Val Pro Ile Leu
    1               5                  10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Cys Tyr Gly Val Gln Cys Phe Ser Arg Tyr Pro Asp His Met Lys
   65                  70                  75                  80
   Arg His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Thr Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr His Gly Met Asp Glu Leu Tyr Asn
   225                 230                 235
<210> 53
<211> 14
<212> DNA
<213> Artificial Sequence
<220>
<223> Description of Artificial Sequence: Caspase-1,4,5
   substrate recognition sequence
<400> 53
   tgggaacatg acaa                                                    14
<210> 54
<211> 4
<212> PRT
<213> Artificial Sequence
<220>
<223> Description of Artificial Sequence: Caspase-1,4,5
   substrate recognition sequence
<400> 54
   Trp Glu His Asp
   1
<210> 55
<211> 12
<212> DNA
<213> Artificial Sequence
<220>
<223> Description of Artificial Sequence: proCaspase-1
   substrate recognition sequence
<400> 55
   tggtttaaag ac 12
<210> 56
<211> 4
<212> PRT
<213> Artificial Sequence
<220>
<223> Description of Artificial Sequence: proCaspase-1
   substrate recognition sequence
<400> 56
   Trp Phe Lys Asp
   1
<210> 57
<211> 12
<212> DNA
<213> Artificial Sequence
<220>
<223> Description of Artificial Sequence: Caspase-2
   substrate recognition sequence
<400> 57
   gacgaacacg ac 12
<210> 58
<211> 4
<212> PRT
<213> Artificial Sequence
<220>
<223> Description of Artificial Sequence: Caspase-2
   substrate recognition sequence
<400> 58
   Asp Glu His Asp
   1
<210> 59
<211> 12
<212> DNA
<213> Artificial Sequence
<220>
<223> Description of Artificial Sequence: Caspase-3,7
   substrate recognition sequence
<400> 59
   gacgaagttg ac 12
<210> 60
<211> 4
<212> PRT
<213> Artificial Sequence
<220>
<223> Description of Artificial Sequence: Caspase-3,7
   substrate recognition sequence
<400> 60
   Asp Glu Val Asp
   1
<210> 61
<211> 12
<212> DNA
<213> Artificial Sequence
<220>
<223> Description of Artificial Sequence: proCaspase-3
   substrate recognition sequence
<400> 61
   atagaaacag ac 12
<210> 62
<211> 4
<212> PRT
<213> Artificial Sequence
<220>
<223> Description of Artificial Sequence: proCaspase-3
   substrate recognition sequence
<400> 62
   Ile Glu Thr Asp
   1
<210> 63
<211> 12
<212> DNA
<213> Artificial Sequence
<220>
<223> Description of Artificial Sequence: proCaspase-4,5
   substrate recognition sequence
<400> 63
   tgggtaagag ac 12
<210> 64
<211> 4
<212> PRT
<213> Artificial Sequence
<220>
<223> Description of Artificial Sequence: proCaspase-4,5
   substrate recognition sequence
<400> 64
   Trp Val Arg Asp
   1
<210> 65
<211> 12
<212> DNA
<213> Artificial Sequence
<220>
<223> Description of Artificial Sequence: Caspase-6
   substrate recognition sequence
<400> 65
   gtagaaatag ac 12
<210> 66
<211> 4
<212> PRT
<213> Artificial Sequence
<220>
<223> Description of Artificial Sequence: Caspase-6
   substrate recognition sequence
<400> 66
   Val Glu Ile Asp
   1
<210> 67
<211> 12
<212> DNA
<213> Artificial Sequence
<220>
<223> Description of Artificial Sequence: Caspase-6
   substrate recognition sequence
<400> 67
   gtagaacacg ac 12
<210> 68
<211> 4
<212> PRT
<213> Artificial Sequence
<220>
<223> Description of Artificial Sequence: Caspase-6
   substrate recognition sequence
<400> 68
   Val Glu His Asp
   1
<210> 69
<211> 12
<212> DNA
<213> Artificial Sequence
<220>
<223> Description of Artificial Sequence: proCaspase-6
   substrate recognition sequence
<400> 69
   acagaagtag ac 12
<210> 70
<211> 4
<212> PRT
<213> Artificial Sequence
<220>
<223> Description of Artificial Sequence: proCaspase-6
   substrate recognition sequence
<400> 70
   Thr Glu Val Asp
   1
<210> 71
<211> 12
<212> DNA
<213> Artificial Sequence
<220>
<223> Description of Artificial Sequence: proCaspase-7
   substrate recognition sequence
<400> 71
   atacaagcag ac 12
<210> 72
<211> 4
<212> PRT
<213> Artificial Sequence
<220>
<223> Description of Artificial Sequence: proCaspase-7
   substrate recognition sequence
<400> 72
   Ile Gln Ala Asp
   1
<210> 73
<211> 12
<212> DNA
<213> Artificial Sequence
<220>
<223> Description of Artificial Sequence: Caspase-8
   substrate recognition sequence
<400> 73
   gtagaaacag ac 12
<210> 74
<211> 4
<212> PRT
<213> Artificial Sequence
<220>
<223> Description of Artificial Sequence: Caspase-8
   substrate recognition sequence
<400> 74
   Val Glu Thr Asp
   1
<210> 75
<211> 12
<212> DNA
<213> Artificial Sequence
<220>
<223> Description of Artificial Sequence: proCaspase-8
   substrate recognition sequence
<400> 75
   ttagaaacag ac 12
<210> 76
<211> 4
<212> PRT
<213> Artificial Sequence
<220>
<223> Description of Artificial Sequence: proCaspase-8
   substrate recognition sequence
<400> 76
   Leu Glu Thr Asp
   1
<210> 77
<211> 12
<212> DNA
<213> Artificial Sequence
<220>
<223> Description of Artificial Sequence: Caspase-9
   substrate recognition sequence
<400> 77
   ttagaacacg ac 12
<210> 78
<211> 4
<212> PRT
<213> Artificial Sequence
<220>
<223> Description of Artificial Sequence: Caspase-9
   substrate recognition sequence
<400> 78
   Leu Glu His Asp
   1
<210> 79
<211> 12
<212> DNA
<213> Artificial Sequence
<220>
<223> Description of Artificial Sequence: proCaspase-9
   substrate recognition sequence
<400> 79
   ttagaacacg ac 12
<210> 80
<211> 4
<212> PRT
<213> Artificial Sequence
<220>
<223> Description of Artificial Sequence: proCaspase-9
   substrate recognition sequence
<400> 80
   Leu Glu His Asp
   1
<210> 81
<211> 12
<212> DNA
<213> Artificial Sequence
<220>
<223> Description of Artificial Sequence: HIV protease
   substrate recognition sequence
<400> 81
   agccaaaatt ac 12
<210> 82
<211> 4
<212> PRT
<213> Artificial Sequence
<220>
<223> Description of Artificial Sequence: HIV protease
   substrate recognition sequence
<400> 82
   Ser Gln Asn Tyr
   1
<210> 83
<211> 12
<212> DNA
<213> Artificial Sequence
<220>
<223> Description of Artificial Sequence: HIV protease
   substrate recognition sequence
<400> 83
   ccaatagtac aa 12
<213> Artificial Sequence
<220>
<223> Description of Artificial Sequence: Caspase-2
   substrate recognition sequence
<400> 57
<210> 58
<211> 4
<212> PRT
<213> Artificial Sequence
<220>
<223> Description of Artificial Sequence: Caspase-2
   substrate recognition sequence
<400> 58
   Asp Glu His Asp
   1
<210> 59
<211> 12
<212> DNA
<213> Artificial Sequence
<220>
<223> Description of Artificial Sequence: Caspase-3,7
   substrate recognition sequence
<400> 59
   gacgaagttg ac 12
<210> 60
<211> 4
<212> PRT
<213> Artificial Sequence
<220>
<223> Description of Artificial Sequence: Caspase-3,7
   substrate recognition sequence
<400> 60
   Asp Glu Val Asp
   1
<210> 61
<211> 12
<212> DNA
<213> Artificial Sequence
<220>
<223> Description of Artificial Sequence: proCaspase-3
   substrate recognition sequence
<400> 61
   atagaaacag ac 12
<210> 62
<211> 4
<212> PRT
<213> Artificial Sequence
<220>
<223> Description of Artificial Sequence: proCaspase-3
   substrate recognition sequence
<400> 62
   Ile Glu Thr Asp
   1
<210> 63
<211> 12
<212> DNA
<213> Artificial Sequence
<220>
<223> Description of Artificial Sequence: proCaspase-4,5
   substrate recognition sequence
<400> 63
   tgggtaagag ac 12
<210> 64
<211> 4
<212> PRT
<213> Artificial Sequence
<220>
<223> Description of Artificial Sequence: proCaspase-4,5
   substrate recognition sequence
<400> 64
   Trp Val Arg Asp
   1
<210> 65
<211> 12
<212> DNA
<213> Artificial Sequence
<220>
<223> Description of Artificial Sequence: Caspase-6
   substrate recognition sequence
<400> 65
   gtagaaatag ac 12
<210> 66
<211> 4
<212> PRT
<213> Artificial Sequence
<220>
<223> Description of Artificial Sequence: Caspase-6
   substrate recognition sequence
<400> 66
   Val Glu Ile Asp
   1
<210> 67
<211> 12
<212> DNA
<213> Artificial Sequence
<220>
<223> Description of Artificial Sequence: Caspase-6
   substrate recognition sequence
<400> 67
   gtagaacacg ac 12
<210> 68
<211> 4
<212> PRT
<213> Artificial Sequence
<220>
<223> Description of Artificial Sequence: Caspase-6
   substrate recognition sequence
<400> 68
   Val Glu His Asp
   1
<210> 69
<211> 12
<212> DNA
<213> Artificial Sequence
<220>
<223> Description of Artificial Sequence: proCaspase-6
   substrate recognition sequence
<400> 69
   acagaagtag ac 12
<210> 70
<211> 4
<212> PRT
<213> Artificial Sequence
<220>
<223> Description of Artificial Sequence: proCaspase-6
   substrate recognition sequence
<400> 70
   Thr Glu Val Asp
   1
<210> 71
<211> 12
<212> DNA
<213> Artificial Sequence
<220>
<223> Description of Artificial Sequence: proCaspase-7
   substrate recognition sequence
<400> 71
   atacaagcag ac 12
<210> 72
<211> 4
<212> PRT
<213> Artificial Sequence
<220>
<223> Description of Artificial Sequence: proCaspase-7
   substrate recognition sequence
<400> 72
   Ile Gln Ala Asp
   1
<210> 73
<211> 12
<212> DNA
<213> Artificial Sequence
<220>
<223> Description of Artificial Sequence: Caspase-8
   substrate recognition sequence
<400> 73
   gtagaaacag ac 12
<210> 74
<211> 4
<212> PRT
<213> Artificial Sequence
<220>
<223> Description of Artificial Sequence: Caspase-8
   substrate recognition sequence
<400> 74
   Val Glu Thr Asp
   1
<210> 75
<211> 12
<212> DNA
<213> Artificial Sequence
<220>
<223> Description of Artificial Sequence: proCaspase-8
   substrate recognition sequence
<400> 75
   ttagaaacag ac 12
<210> 76
<211> 4
<212> PRT
<213> Artificial Sequence
<220>
<223> Description of Artificial Sequence: proCaspase-8
   substrate recognition sequence
<400> 76
   Leu Glu Thr Asp
   1
<210> 77
<211> 12
<212> DNA
<213> Artificial Sequence
<220>
<223> Description of Artificial Sequence: Caspase-9
   substrate recognition sequence
<400> 77
   ttagaacacg ac 12
<210> 78
<211> 4
<212> PRT
<213> Artificial Sequence
<220>
<223> Description of Artificial Sequence: Caspase-9
   substrate recognition sequence
<400> 78
   Leu Glu His Asp
   1
<210> 79
<211> 12
<212> DNA
<213> Artificial Sequence
<220>
<223> Description of Artificial Sequence: proCaspase-9
   substrate recognition sequence
<400> 79
   ttagaacacg ac 12
<210> 80
<211> 4
<212> PRT
<213> Artificial Sequence
<220>
<223> Description of Artificial Sequence: proCaspase-9
   substrate recognition sequence
<400> 80
   Leu Glu His Asp
   1
<210> 81
<211> 12
<212> DNA
<213> Artificial Sequence
<220>
<223> Description of Artificial Sequence: HIV protease
   substrate recognition sequence
<400> 81
   agccaaaatt ac 12
<210> 82
<211> 4
<212> PRT
<213> Artificial Sequence
<220>
<223> Description of Artificial Sequence: HIV protease
   substrate recognition sequence
<400> 82
   Ser Gln Asn Tyr
   1
<210> 83
<211> 12
<212> DNA
<213> Artificial Sequence
<220>
<223> Description of Artificial Sequence: HIV protease
   substrate recognition sequence
<400> 83
   ccaatagtac aa 12
<210> 84
<211> 4
<212> PRT
<213> Artificial Sequence
<220>
<223> Description of Artificial Sequence: HIV protease
   substrate recognition sequence
<400> 84
   Pro Ile Val Gln
   1
<210> 85
<211> 12
<212> DNA
<213> Artificial Sequence
<220>
<223> Description of Artificial Sequence: Adenovirus
   endopeptidase substrate recognition sequence
<400> 85
   atgtttggag ga 12
<210> 86
<211> 4
<212> PRT
<213> Artificial Sequence
<220>
<223> Description of Artificial Sequence: Adenovirus
   endopeptidase substrate recognition sequence
<400> 86
   Met Phe Gly Gly
   1
<210> 87
<211> 12
<212> DNA
<213> Artificial Sequence
<220>
<223> Description of Artificial Sequence: Adenovirus
   endopeptidase substrate recognition sequence
<400> 87
   gcaaaaaaaa ga 12
<210> 88
<211> 4
<212> PRT
<213> Artificial Sequence
<220>
<223> Description of Artificial Sequence: Adenovirus
   endopeptidase substrate recognition sequence
<400> 88
   Ala Lys Lys Arg
   1
<210> 89
<211> 9
<212> DNA
<213> Artificial Sequence
<220>
<223> Description of Artificial Sequence: b-Secretase
   substrate recognition sequence
<400> 89
   gtgaaaatg 9
<210> 90
<211> 3
<212> PRT
<213> Artificial Sequence
<220>
<223> Description of Artificial Sequence: b-Secretase
   substrate recognition sequence
<400> 90
   Val Lys Met
   1
<210> 91
<211> 12
<212> DNA
<213> Artificial Sequence
<220>
<223> Description of Artificial Sequence: b-Secretase
   substrate recognition sequence
<400> 91
   gacgcagaat tc 12
<210> 92
<211> 4
<212> PRT
<213> Artificial Sequence
<220>
<223> Description of Artificial Sequence: b-Secretase
   substrate recognition sequence
<400> 92
   Asp Ala Glu Phe
   1
<210> 93
<211> 15
<212> DNA
<213> Artificial Sequence
<220>
<223> Description of Artificial Sequence: Cathepsin D
   substrate recognition sequence
<400> 93
   aaaccagcat tattc 15
<210> 94
<211> 5
<212> PRT
<213> Artificial Sequence
<220>
<223> Description of Artificial Sequence: Cathepsin D
   substrate recognition sequence
<400> 94
   Lys Pro Ala Leu Phe
   1 5
<210> 95
<211> 9
<212> DNA
<213> Artificial Sequence
<220>
<223> Description of Artificial Sequence: Cathepsin D
   substrate recognition sequence
<400> 95
   ttcagatta 9
<210> 96
<211> 3
<212> PRT
<213> Artificial Sequence
<220>
<223> Description of Artificial Sequence: Cathepsin D
   substrate recognition sequence
<400> 96
   Phe Arg Leu
   1
<210> 97
<211> 15
<212> DNA
<213> Artificial Sequence
<220>
<223> Description of Artificial Sequence: Matrix
   Metalloprotease substrate recognition sequence
<400> 97
   ggaccattag gacca 15
<210> 98
<211> 5
<212> PRT
<213> Artificial Sequence
<220>
<223> Description of Artificial Sequence: Matrix
   Metalloprotease substrate recognition sequence
<400> 98
   Gly Pro Leu Gly Pro
   1 5
<210> 99
<211> 12
<212> DNA
<213> Artificial Sequence
<220>
<223> Description of Artificial Sequence: Granzyme B
   substrate recognition sequence
<400> 99
   atagaaccag ac 12
<210> 100
<211> 4
<212> PRT
<213> Artificial Sequence
<220>
<223> Description of Artificial Sequence: Granzyme B
   substrate recognition sequence
<400> 100
   Ile Glu Pro Asp
   1
<210> 101
<211> 36
<212> DNA
<213> Artificial Sequence
<220>
<223> Description of Artificial Sequence: Anthrax
   protease substrate recognition sequence
<400> 101
   atgcccaaga agaagccgac gcccatccag ctgaac 36
<210> 102
<211> 12
<212> PRT
<213> Artificial Sequence
<220>
<223> Description of Artificial Sequence: Anthrax
   protease substrate recognition sequence
<400> 102
   Met Pro Lys Lys Lys Pro Thr Pro Ile Gln Leu Asn
   1 5 10
<210> 103
<211> 45
<212> DNA
<213> Artificial Sequence
<220>
<223> Description of Artificial Sequence: Anthrax
   protease substrate recognition sequence
<400> 103
   atgctggccc ggaggaagcc ggtgctgccg gcgctcacca tcaac 45
<210> 104
<211> 15
<212> PRT
<213> Artificial Sequence
<220>
<223> Description of Artificial Sequence: Anthrax
   protease substrate recognition sequence
<400> 104
   Met Leu Ala Arg Arg Lys Pro Val Leu Pro Ala Leu Thr Ile Asn
   1 5 10 15
<210> 105
<211> 18
<212> DNA
<213> Artificial Sequence
<220>
<223> Description of Artificial Sequence:
   tetanus/botulium substrate recognition sequence
<400> 105
   gcctcgcagt ttgaaaca 18
<210> 106
<211> 6
<212> PRT
<213> Artificial Sequence
<220>
<223> Description of Artificial Sequence:
   tetanus/botulium substrate recognition sequence
<400> 106
   Ala Ser Gln Phe Glu Thr
   1 5
<210> 107
<211> 18
<212> DNA
<213> Artificial Sequence
<220>
<223> Description of Artificial Sequence:
   tetanus/botulium substrate recognition sequence
<400> 107
   gcttctcaat ttgaaacg 18
<210> 108
<211> 6
<212> PRT
<213> Artificial Sequence
<220>
<223> Description of Artificial Sequence:
   tetanus/botulium substrate recognition sequence
<400> 108
   Ala Ser Gln Phe Glu Thr
   1 5
<210> 109
<211> 18
<212> DNA
<213> Artificial Sequence
<220>
<223> Description of Artificial Sequence: Botulinum
   neurotoxin A substrate recognition sequence
<400> 109
   gccaaccaac gtgcaaca 18
<210> 110
<211> 6
<212> PRT
<213> Artificial Sequence
<220>
<223> Description of Artificial Sequence: Botulinum
   neurotoxin A substrate recognition sequence
<400> 110
   Ala Asn Gln Arg Ala Thr
   1 5
<210> 111
<211> 18
<212> DNA
<213> Artificial Sequence
<220>
<223> Description of Artificial Sequence: Botulinum
   neurotoxin B substrate recognition sequence
<400> 111
   gcttctcaat ttgaaacg 18
<210> 112
<211> 6
<212> PRT
<213> Artificial Sequence
<220>
<223> Description of Artificial Sequence: Botulinum
   neurotoxin B substrate recognition sequence
<400> 112
   Ala Ser Gln Phe Glu Thr
     1               5
<210> 113
<211> 18
<212> DNA
<213> Artificial Sequence
<220>
<223> Description of Artificial Sequence: Botulinum
   neurotoxin C substrate recognition sequence
<400> 113
   acgaaaaaag ctgtgaaa                                          18
<210> 114
<211> 6
<212> PRT
<213> Artificial Sequence
<220>
<223> Description of Artificial Sequence: Botulinum
   neurotoxin C substrate recognition sequence
<400> 114
   Thr Lys Lys Ala Val Lys
     1               5
<210> 115
<211> 18
<212> DNA
<213> Artificial Sequence
<220>
<223> Description of Artificial Sequence: Botulinum
   neurotoxin D substrate recognition sequence
<400> 115
   gaccagaagc tctctgag                                          18
<210> 116
<211> 6
<212> PRT
<213> Artificial Sequence
<220>
<223> Description of Artificial Sequence: Botulinum
   neurotoxin D substrate recognition sequence
<400> 116
   Asp Gln Lys Leu Ser Glu
   1 5
<210> 117
<211> 18
<212> DNA
<213> Artificial Sequence
<220>
<223> Description of Artificial Sequence: Botulinum
   neurotoxin E substrate recognition sequence
<400> 117
   atcgacagga tcatggag 18
<210> 118
<211> 6
<212> PRT
<213> Artificial Sequence
<220>
<223> Description of Artificial Sequence: Botulinum
   neurotoxin E substrate recognition sequence
<400> 118
   Ile Asp Arg Ile Met Glu
   1 5
<210> 119
<211> 18
<212> DNA
<213> Artificial Sequence
<220>
<223> Description of Artificial Sequence: Botulinum
   neurotoxin F substrate recognition sequence
<400> 119
   agagaccaga agctctct 18
<210> 120
<211> 6
<212> PRT
<213> Artificial Sequence
<220>
<223> Description of Artificial Sequence: Botulinum
   neurotoxin F substrate recognition sequence
<400> 120
   Arg Asp Gln Lys Leu Ser
   1 5
<210> 121
<211> 18
<212> DNA
<213> Artificial Sequence
<220>
<223> Description of Artificial Sequence: Botulinum
   neurotoxin G substrate recognition sequence
<400> 121
   acgagcgcag ccaagttg 18
<210> 122
<211> 6
<212> PRT
<213> Artificial Sequence
<220>
<223> Description of Artificial Sequence: Botulinum
   neurotoxin G substrate recognition sequence
<400> 122
   Thr Ser Ala Ala Lys Leu
   1 5
<210> 123
<211> 69
<212> DNA
<213> Artificial Sequence
<220>
<223> Description of Artificial Sequence:
   Cytoplasm/cytoskeleton target sequence
<400> 123
   atgtctactg tccacgaaat cctgtgcaag ctcagcttgg agggtgttca ttctacaccc 60
   ccaagtgcc 69
<210> 124
<211> 23
<212> PRT
<213> Artificial Sequence
<220>
<223> Description of Artificial Sequence:
   Cytoplasm/cytoskeleton target sequence
<400> 124
   Met Ser Thr Val His Glu Ile Leu Cys Lys Leu Ser Leu Glu Gly Val
   1 5 10 15
   His Ser Thr Pro Pro Ser Ala
   20
<210> 125
<211> 96
<212> DNA
<213> Artificial Sequence
<220>
<223> Description of Artificial Sequence: Inner surface
   of plasma membrane target sequence
<400> 125
   atgggatgta cattaagcgc agaagacaaa gcagcagtag aaagaagcaa aatgatagac 60
   agaaacttaa gagaagacgg agaaaaagct gctaga 96
<210> 126
<211> 32
<212> PRT
<213> Artificial Sequence
<220>
<223> Description of Artificial Sequence: Inner surface
   of plasma membrane target sequence
<400> 126
   Met Gly Cys Thr Leu Ser Ala Glu Asp Lys Ala Ala Val Glu Arg Ser
   1 5 10 15
   Lys Met Ile Asp Arg Asn Leu Arg Glu Asp Gly Glu Lys Ala Ala Arg
   20 25 30
<210> 127
<211> 18
<212> DNA
<213> Artificial Sequence
<220>
<223> Description of Artificial Sequence: Nucleus target
   sequence
<400> 127
   agaaggaaac gacaaaag 18
<210> 128
<211> 6
<212> PRT
<213> Artificial Sequence
<220>
<223> Description of Artificial Sequence: Nucleus target
   sequence
<400> 128
   Arg Arg Lys Arg Gln Lys
   1 5
<210> 129
<211> 90
<212> DNA
<213> Artificial Sequence
<220>
<223> Description of Artificial Sequence: Nucleolus
   target sequence
<400> 129
   agaaaacgta tacgtactta cctcaagtcc tgcaggcgga tgaaaagaag tggttttgag 60
   atgtctcgac ctattccttc ccaccttact 90
<210> 130
<211> 30
<212> PRT
<213> Artificial Sequence
<220>
<223> Description of Artificial Sequence: Nucleolus
   target sequence
<400> 130
   Arg Lys Arg Ile Arg Thr Tyr Leu Lys Ser Cys Arg Arg Met Lys Arg
   1 5 10 15
   Ser Gly Phe Glu Met Ser Arg Pro Ile Pro Ser His Leu Thr
   20 25 30
<210> 131
<211> 87
<212> DNA
<213> Artificial Sequence
<220>
<223> Description of Artificial Sequence: Mitochondria
   target sequence
<400> 131
   atgtccgtcc tgacgccgct gctgctgcgg ggcttgacag gctcggcccg gcggctccca 60
   gtgccgcgcg ccaagatcca ttcgttg 87
<210> 132
<211> 29
<212> PRT
<213> Artificial Sequence
<220>
<223> Description of Artificial Sequence: Mitochondria
   target sequence
<400> 132
   Met Ser Val Leu Thr Pro Leu Leu Leu Arg Gly Leu Thr Gly Ser Ala
   1 5 10 15
   Arg Arg Leu Pro Val Pro Arg Ala Leu Ile His Ser Leu
   20 25
<210> 133
<211> 99
<212> DNA
<213> Artificial Sequence
<220>
<223> Description of Artificial Sequence: Nuclear
   Envelope target sequence
<400> 133
   atgagcattg ttttaataat tgttattgtg gtgatttttt taatatgttt tttatattta 60
   agcaacagca aagatcccag agtaccagtt gaattaatg 99
<210> 134
<211> 33
<212> PRT
<213> Artificial Sequence
<220>
<223> Description of Artificial Sequence: Nuclear
   Envelope target sequence
<400> 134
   Met Ser Ile Val Leu Ile Ile Val Ile Val Val Ile Phe Leu Ile Cys
   1 5 10 15
   Phe Leu Tyr Leu Ser Asn Ser Lys Asp Pro Arg Val Pro Val Glu Leu
   20 25 30
   Met
<210> 135
<211> 246
<212> DNA
<213> Artificial Sequence
<220>
<223> Description of Artificial Sequence: Golgi target
   sequence
<400> 135
   atgaggcttc gggagccgct cctgagcggc agcgccgcga tgccaggcgc gtccctacag 60
   cgggcctgcc gcctgctcgt ggccgtctgc gctctgcacc ttggcgtcac cctcgtttac 120
   tacctggctg gccgcgacct gagccgcctg ccccaactgg tcggagtctc cacaccgctg 180
   cagggcggct cgaacagtgc cgccgccatc gggcagtcct ccggggagct ccggaccgga 240
   ggggcc 246
<210> 136
<211> 82
<212> PRT
<213> Artificial Sequence
<220>
<223> Description of Artificial Sequence: Golgi target sequence
<400> 136
   Met Arg Leu Arg Glu Pro Leu Leu Ser Gly Ser Ala Ala Met Pro Gly
   1               5                   10                  15
   Ala Ser Leu Gln Arg Ala Cys Arg Leu Leu Val Ala Val Cys Ala Leu
               20                  25                  30
   His Leu Gly Val Thr Leu Val Tyr Tyr Leu Ala Gly Arg Asp Leu Ser
           35                  40                  45
   Arg Leu Pro Gln Leu Val Gly Val Ser Thr Pro Leu Gln Gly Gly Ser
       50                  55                  60
   Asn Ser Ala Ala Ala Ile Gly Gln Ser Ser Gly Glu Leu Arg Thr Gly
   65                  70                  75                  80
   Gly Ala
<210> 137
<211> 150
<212> DNA
<213> Artificial Sequence
<220>
<223> Description of Artificial Sequence: Endoplasmic
   reticulum target sequence
<400> 137
   gaaacaataa gacctataag aataagaaga tgttcttatt ttacatctac agacagcaaa 60
   atggcaattc aattaagatc tccctttcca ttagcattac caggaatgtt agctttatta 120
   ggatggtggt ggtttttcag tagaaaaaaa 150
<210> 138
<211> 50
<212> PRT
<213> Artificial Sequence
<220>
<223> Description of Artificial Sequence: Endoplasmic
   reticulum target sequence
<400> 138
   Glu Thr Ile Arg Pro Ile Arg Ile Arg Arg Cys Ser Tyr Phe Thr Ser
   1               5                   10                  15
   Thr Asp Ser Lys Met Ala Ile Gln Leu Arg Ser Pro Phe Pro Leu Ala
               20                  25                  30
   Leu Pro Gly Met Leu Ala Leu Leu Gly Trp Trp Trp Phe Phe Ser Arg
           35                  40                  45
   Lys Lys
       50
<210> 139
<211> 39
<212> DNA
<213> Artificial Sequence
<220>
<223> Description of Artificial Sequence: Nuclear Export
   target sequence
<400> 139
   gccttgcaga agaagctgga ggagctagag cttgatgag 39
<210> 140
<211> 13
<212> PRT
<213> Artificial Sequence
<220>
<223> Description of Artificial Sequence: Nuclear Export
   target sequence
<400> 140
   Ala Leu Gln Lys Lys Leu Glu Glu Leu Glu Leu Asp Glu
   1 5 10
<210> 141
<211> 1024
<212> DNA
<213> Artificial Sequence
<220>
<223> Description of Artificial Sequence: Size exclusion
   target sequence
<400> 141
   gccgacctca gtcttgtgga tgcgttgaca gaaccacctc cagaaattga gggagaaata 60
   aagcgagact tcatggctgc gctggaggca gagccctatg atgacatcgt gggagaaact 120
   gtggagaaaa ctgagtttat tcctctcctg gatggtgatg agaaaaccgg gaactcagag 180
   tccaaaaaga aaccctgctt agacactagc caggttgaag gtatcccatc ttctaaacca 240
   acactcctag ccaatggtga tcatggaatg gaggggaata acactgcagg gtctccaact 300
   gacttccttg aagagagagt ggactatccg gattatcaga gcagccagaa ctggccagaa 360
   gatgcaagct tttgtttcca gcctcagcaa gtgttagata ctgaccaggc tgagcccttt 420
   aacgagcacc gtgatgatgg tttggcagat ctgctctttg tctccagtgg acccacgaac 480
   gcttctgcat ttacagagcg agacaatcct tcagaagaca gttacggtat gcttccctgt 540
   gactcatttg cttccacggc tgttgtatct caggagtggt ctgtgggagc cccaaactct 600
   ccatgttcag agtcctgtgt ctccccagag gttactatag aaaccctaca gccagcaaca 660
   gagctctcca aggcagcaga agtggaatca gtgaaagagc agctgccagc taaagcattg 720
   gaaacgatgg cagagcagac cactgatgtg gtgcactctc catccacaga cacaacacca 780
   ggcccagaca cagaggcagc actggctaaa gacatagaag agatcaccaa gccagatgtg 840
   atattggcaa atgtcacgca gccatctact gaatcggata tgttcctggc ccaggacatg 900
   gaactactca caggaacaga ggcagcccac gctaacaata tcatattgcc tacagaacca 960
   gacgaatctt caaccaagga tgtagcacca cctatggaag aagaaattgt cccaggcaat 1020
   gata 1024
<210> 142
<211> 566
<212> PRT
<213> Artificial Sequence
<220>
<223> Description of Artificial Sequence: Size exclusion target sequence
<400> 142
   Ala Asp Leu Ser Leu Val Asp Ala Leu Thr Glu Pro Pro Pro Glu Ile
   1               5                   10                  15
   Glu Gly Glu Ile Lys Arg Asp Phe Met Ala Ala Leu Glu Ala Glu Pro
               20                  25                  30
   Tyr Asp Asp Ile Val Gly Glu Thr Val Glu Lys Thr Glu Phe Ile Pro
           35                  40                  45
   Leu Leu Asp Gly Asp Glu Lys Thr Gly Asn Ser Glu Ser Lys Lys Lys
       50                  55                  60
   Pro Cys Leu Asp Thr Ser Gln Val Glu Gly Ile Pro Ser Ser Lys Pro
   65                  70                  75                  80
   Thr Leu Leu Ala Asn Gly Asp His Gly Met Glu Gly Asn Asn Thr Ala
                   85                  90                  95
   Gly Ser Pro Thr Asp Phe Leu Glu Glu Arg Val Asp Tyr Pro Asp Tyr
               100                 105                 110
   Gln Ser Ser Gln Asn Trp Pro Glu Asp Ala Ser Phe Cys Phe Gln Pro
           115                 120                 125
   Gln Gln Val Leu Asp Thr Asp Gln Ala Glu Pro Phe Asn Glu His Arg
       130                 135                 140
   Asp Asp Gly Leu Ala Asp Leu Leu Phe Val Ser Ser Gly Pro Thr Asn
   145                 150                 155                 160
   Ala Ser Ala Phe Thr Glu Arg Asp Asn Pro Ser Glu Asp Ser Tyr Gly
                   165                 170                 175
   Met Leu Pro Cys Asp Ser Phe Ala Ser Thr Ala Val Val Ser Gln Glu
               180                 185                 190
   Trp Ser Val Gly Ala Pro Asn Ser Pro Cys Ser Glu Ser Cys Val Ser
           195                 200                 205
   Pro Glu Val Thr Ile Glu Thr Leu Gln Pro Ala Thr Glu Leu Ser Lys
       210                 215                 220
   Ala Ala Glu Val Glu Ser Val Lys Glu Gln Leu Pro Ala Lys Ala Leu
   225                 230                 235                 240
   Glu Thr Met Ala Glu Gln Thr Thr Asp Val Val His Ser Pro Ser Thr
                   245                 250                 255
   Asp Thr Thr Pro Gly Pro Asp Thr Glu Ala Ala Leu Ala Lys Asp Ile
               260                 265                 270
   Glu Glu Ile Thr Lys Pro Asp Val Ile Leu Ala Asn Val Thr Gln Pro
           275                 280                 285
   Ser Thr Glu Ser Asp Met Phe Leu Ala Gln Asp Met Glu Leu Leu Thr
       290                 295                 300
   Gly Thr Glu Ala Ala His Ala Asn Asn Ile Ile Leu Pro Thr Glu Pro
   305                 310                 315                 320
   Asp Glu Ser Ser Thr Lys Asp Val Ala Pro Pro Met Glu Glu Glu Ile
                   325                 330                 335
   Val Pro Gly Asn Asp Thr Thr Ser Pro Lys Glu Thr Glu Thr Thr Leu
               340                 345                 350
   Pro Ile Lys Met Asp Leu Ala Pro Pro Glu Asp Val Leu Leu Thr Lys
           355                 360                 365
   Glu Thr Glu Leu Ala Pro Ala Lys Gly Met Val Ser Leu Ser Glu Ile
       370                 375                 380
   Glu Glu Ala Leu Ala Lys Asn Asp Val Arg Ser Ala Glu Ile Pro Val
   385                 390                 395                 400
   Ala Gln Glu Thr Val Val Ser Glu Thr Glu Val Val Leu Ala Thr Glu
                   405                 410                 415
   Val Val Leu Pro Ser Asp Pro Ile Thr Thr Leu Thr Lys Asp Val Thr
               420                 425                 430
   Leu Pro Leu Glu Ala Glu Arg Pro Leu Val Thr Asp Met Thr Pro Ser
           435                 440                 445
   Leu Glu Thr Glu Met Thr Leu Gly Lys Glu Thr Ala Pro Pro Thr Glu
       450                 455                 460
   Thr Asn Leu Gly Met Ala Lys Asp Met Ser Pro Leu Pro Glu Ser Glu
   465                 470                 475                 480
   Val Thr Leu Gly Lys Asp Val Val Ile Leu Pro Glu Thr Lys Val Ala
                   485                 490                 495
   Glu Phe Asn Asn Val Thr Pro Leu Ser Glu Glu Glu Val Thr Ser Val
               500                 505                 510
   Lys Asp Met Ser Pro Ser Ala Glu Thr Glu Ala Pro Leu Ala Lys Asn
           515                 520                 525
   Ala Asp Leu His Ser Gly Thr Glu Leu Ile Val Asp Asn Ser Met Ala
       530                 535                 540
   Pro Ala Ser Asp Leu Ala Leu Pro Leu Glu Thr Lys Val Ala Thr Val
   545                 550                 555                 560
   Pro Ile Lys Asp Lys Gly
                   565
<210> 143
<211> 63
<212> DNA
<213> Artificial Sequence
<220>
<223> Description of Artificial Sequence: Vesicle
   membrane target sequence
<400> 143
   atgtgggcaa tcgggattac tgttctggtt atcttcatca tcatcatcat cgtgtgggtt 60
   gtc 63
<210> 144
<211> 21
<212> PRT
<213> Artificial Sequence
<220>
<223> Description of Artificial Sequence: Vesicle
   membrane target sequence
<400> 144
   Met Trp Ala Ile Gly Ile Thr Val Leu Val Ile Phe Ile Ile Ile Ile
   1 5 10 15
   Ile Val Trp Val Val
   20
<210> 145
<211> 61
<212> DNA
<213> Artificial Sequence
<220>
<223> Description of Artificial Sequence: Vesicle
   membrane target sequence
<400> 145
   atgtgggcga tagggatcag tgtcctggtg atcattgtca tcatcatcat cgtgtggtgt 60
   g 61
<210> 146
<211> 20
<212> PRT
<213> Artificial Sequence
<220>
<223> Description of Artificial Sequence: Vesicle
   membrane target sequence
<400> 146
   Met Trp Ala Ile Gly Ile Ser Val Leu Val Ile Ile Val Ile Ile Ile
   1 5 10 15
   Ile Val Trp Cys
<210> 147
<211> 39
<212> DNA
<213> Artificial Sequence
<220>
<223> Description of Artificial Sequence: Nuclear Export
   target sequence
<400> 147
   gacctgcaga agaagctgga ggagctggaa cttgacgag 39
<210> 148
<211> 13
<212> PRT
<213> Artificial Sequence
<220>
<223> Description of Artificial Sequence: Nuclear Export
   target sequence
<400> 148
   Asp Leu Gln Lys Lys Leu Glu Glu Leu Glu Leu Asp Glu
   1 5 10
<210> 149
<211> 9
<212> DNA
<213> Artificial Sequence
<220>
<223> Description of Artificial Sequence: Peroxisome
   target sequence
<400> 149
   tctaaactg 9
<210> 150
<211> 3
<212> PRT
<213> Artificial Sequence
<220>
<223> Description of Artificial Sequence: Peroxisome
   target sequence
<400> 150
   Ser Lys Leu
   1
<210> 151
<211> 3378
<212> DNA
<213> Mus musculus
<220>
<221> CDS
<222> (1)..(3375)
<400> 151
   atg gcc gac ctc agt ctt gtg gat gcg ttg aca gaa cca cct cca gaa    48
   Met Ala Asp Leu Ser Leu Val Asp Ala Leu Thr Glu Pro Pro Pro Glu
    1               5                  10                  15
   att gag gga gaa ata aag cga gac ttc atg gct gcg ctg gag gca gag    96
   Ile Glu Gly Glu Ile Lys Arg Asp Phe Met Ala Ala Leu Glu Ala Glu
               20                  25                  30
   ccc tat gat gac atc gtg gga gaa act gtg gag aaa act gag ttt att   144
   Pro Tyr Asp Asp Ile Val Gly Glu Thr Val Glu Lys Thr Glu Phe Ile
           35                  40                  45
   cct ctc ctg gat ggt gat gag aaa acc ggg aac tca gag tcc aaa aag   192
   Pro Leu Leu Asp Gly Asp Glu Lys Thr Gly Asn Ser Glu Ser Lys Lys
       50                  55                  60
   aaa ccc tgc tta gac act agc cag gtt gaa ggt atc cca tct tct aaa   240
   Lys Pro Cys Leu Asp Thr Ser Gln Val Glu Gly Ile Pro Ser Ser Lys
   65                  70                  75                  80
   cca aca ctc cta gcc aat ggt gat cat gga atg gag ggg aat aac act   288
   Pro Thr Leu Leu Ala Asn Gly Asp His Gly Met Glu Gly Asn Asn Thr
                   85                  90                  95
   gca ggg tct cca act gac ttc ctt gaa gag aga gtg gac tat ccg gat   336
   Ala Gly Ser Pro Thr Asp Phe Leu Glu Glu Arg Val Asp Tyr Pro Asp
               100                 105                 110
   tat cag agc agc cag aac tgg cca gaa gat gca agc ttt tgt ttc cag   384
   Tyr Gln Ser Ser Gln Asn Trp Pro Glu Asp Ala Ser Phe Cys Phe Gln
           115                 120                 125
   cct cag caa gtg tta gat act gac cag gct gag ccc ttt aac gag cac   432
   Pro Gln Gln Val Leu Asp Thr Asp Gln Ala Glu Pro Phe Asn Glu His
       130                 135                 140
   cgt gat gat ggt ttg gca gat ctg ctc ttt gtc tcc agt gga ccc acg   480
   Arg Asp Asp Gly Leu Ala Asp Leu Leu Phe Val Ser Ser Gly Pro Thr
   145                 150                 155                 160
   aac gct tct gca ttt aca gag cga gac aat cct tca gaa gac agt tac   528
   Asn Ala Ser Ala Phe Thr Glu Arg Asp Asn Pro Ser Glu Asp Ser Tyr
                   165                 170                 175
   ggt atg ctt ccc tgt gac tca ttt gct tcc acg gct gtt gta tct cag   576
   Gly Met Leu Pro Cys Asp Ser Phe Ala Ser Thr Ala Val Val Ser Gln
               180                 185                 190
   gag tgg tct gtg gga gcc cca aac tct cca tgt tca gag tcc tgt gtc    624
   Glu Trp Ser Val Gly Ala Pro Asn Ser Pro Cys Ser Glu Ser Cys Val
           195                 200                 205
   tcc cca gag gtt act ata gaa acc cta cag cca gca aca gag ctc tcc    672
   Ser Pro Glu Val Thr Ile Glu Thr Leu Gln Pro Ala Thr Glu Leu Ser
       210                 215                 220
   aag gca gca gaa gtg gaa tca gtg aaa gag cag ctg cca gct aaa gca    720
   Lys Ala Ala Glu Val Glu Ser Val Lys Glu Gln Leu Pro Ala Lys Ala
   225                 230                 235                 240
   ttg gaa acg atg gca gag cag acc act gat gtg gtg cac tct cca tcc    768
   Leu Glu Thr Met Ala Glu Gln Thr Thr Asp Val Val His Ser Pro Ser
                   245                 250                 255
   aca gac aca aca cca ggc cca gac aca gag gca gca ctg gct aaa gac    816
   Thr Asp Thr Thr Pro Gly Pro Asp Thr Glu Ala Ala Leu Ala Lys Asp
               260                 265                 270
   ata gaa gag atc acc aag cca gat gtg ata ttg gca aat gtc acg cag    864
   Ile Glu Glu Ile Thr Lys Pro Asp Val Ile Leu Ala Asn Val Thr Gln
           275                 280                 285
   cca tct act gaa tcg gat atg ttc ctg gcc cag gac atg gaa cta ctc    912
   Pro Ser Thr Glu Ser Asp Met Phe Leu Ala Gln Asp Met Glu Leu Leu
       290                 295                 300
   aca gga aca gag gca gcc cac gct aac aat atc ata ttg cct aca gaa    960
   Thr Gly Thr Glu Ala Ala His Ala Asn Asn Ile Ile Leu Pro Thr Glu
   305                 310                 315                 320
   cca gac gaa tct tca acc aag gat gta gca cca cct atg gaa gaa gaa    1008
   Pro Asp Glu Ser Ser Thr Lys Asp Val Ala Pro Pro Met Glu Glu Glu
                   325                 330                 335
   att gtc cca ggc aat gat acg aca tcc ccc aaa gaa aca gag aca aca    1056
   Ile Val Pro Gly Asn Asp Thr Thr Ser Pro Lys Glu Thr Glu Thr Thr
               340                 345                 350
   ctt cca ata aaa atg gac ttg gca cca cct gag gat gtg tta ctt acc    1104
   Leu Pro Ile Lys Met Asp Leu Ala Pro Pro Glu Asp Val Leu Leu Thr
           355                 360                 365
   aaa gaa aca gaa cta gcc cca gcc aag ggc atg gtt tca ctc tca gaa    1152
   Lys Glu Thr Glu Leu Ala Pro Ala Lys Gly Met Val Ser Leu Ser Glu
       370                 375                 380
   ata gaa gag gct ctg gca aag aat gat gtt cgc tct gca gaa ata cct    1200
   Ile Glu Glu Ala Leu Ala Lys Asn Asp Val Arg Ser Ala Glu Ile Pro
   385                 390                 395                 400
   gtg gct cag gag aca gtg gtc tca gaa aca gag gtg gtc ctg gca aca    1248
   Val Ala Gln Glu Thr Val Val Ser Glu Thr Glu Val Val Leu Ala Thr
                   405                 410                 415
   gaa gtg gta ctg ccc tca gat ccc ata aca aca ttg aca aag gat gtg    1296
   Glu Val Val Leu Pro Ser Asp Pro Ile Thr Thr Leu Thr Lys Asp Val
               420                 425                 430
   aca ctc ccc tta gaa gca gag aga ccg ttg gtg acg gac atg act cca    1344
   Thr Leu Pro Leu Glu Ala Glu Arg Pro Leu Val Thr Asp Met Thr Pro
           435                 440                 445
   tct ctg gaa aca gaa atg acc cta ggc aaa gag aca gct cca ccc aca    1392
   Ser Leu Glu Thr Glu Met Thr Leu Gly Lys Glu Thr Ala Pro Pro Thr
       450                 455                 460
   gaa aca aat ttg ggc atg gcc aaa gac atg tct cca ctc cca gaa tca    1440
   Glu Thr Asn Leu Gly Met Ala Lys Asp Met Ser Pro Leu Pro Glu Ser
   465                 470                 475                 480
   gaa gtg act ctg ggc aag gac gtg gtt ata ctt cca gaa aca aag gtg    1488
   Glu Val Thr Leu Gly Lys Asp Val Val Ile Leu Pro Glu Thr Lys Val
                   485                 490                 495
   gct gag ttt aac aat gtg act cca ctt tca gaa gaa gag gta acc tca    1536
   Ala Glu Phe Asn Asn Val Thr Pro Leu Ser Glu Glu Glu Val Thr Ser
               500                 505                 510
   gtc aag gac atg tct ccg tct gca gaa aca gag gct ccc ctg gct aag    1584
   Val Lys Asp Met Ser Pro Ser Ala Glu Thr Glu Ala Pro Leu Ala Lys
           515                 520                 525
   aat gct gat ctg cac tca gga aca gag ctg att gtg gac aac agc atg    1632
   Asn Ala Asp Leu His Ser Gly Thr Glu Leu Ile Val Asp Asn Ser Met
       530                 535                 540
   gct cca gcc tcc gat ctt gca ctg ccc ttg gaa aca aaa gta gca aca    1680
   Ala Pro Ala Ser Asp Leu Ala Leu Pro Leu Glu Thr Lys Val Ala Thr
   545                 550                 555                 560
   gtt cca att aaa gac aaa gga act gta cag act gaa gaa aaa cca cgt    1728
   Val Pro Ile Lys Asp Lys Gly Thr Val Gln Thr Glu Glu Lys Pro Arg
                   565                 570                 575
   gaa gac tcc cag tta gca tct atg cag cac aag gga cag tca aca gta    1776
   Glu Asp Ser Gln Leu Ala Ser Met Gln His Lys Gly Gln Ser Thr Val
               580                 585                 590
   cct cct tgc acg gct tca cca gaa cca gtc aaa gct gca gaa caa atg    1824
   Pro Pro Cys Thr Ala Ser Pro Glu Pro Val Lys Ala Ala Glu Gln Met
           595                 600                 605
   tct acc tta cca ata gat gca cct tct cca tta gag aac tta gag cag    1872
   Ser Thr Leu Pro Ile Asp Ala Pro Ser Pro Leu Glu Asn Leu Glu Gln
       610                 615                 620
   aag gaa acg cct ggc agc cag cct tct gag cct tgc tca gga gta tcc    1920
   Lys Glu Thr Pro Gly Ser Gln Pro Ser Glu Pro Cys Ser Gly Val Ser
   625                 630                 635                 640
   cgg caa gaa gaa gca aag gct gct gta ggt gtg act gga aat gac atc    1968
   Arg Gln Glu Glu Ala Lys Ala Ala Val Gly Val Thr Gly Asn Asp Ile
   645 650 655
   act acc ccg cca aac aag gag cca cca cca agc cca gaa aag aaa gca 2016
   Thr Thr Pro Pro Asn Lys Glu Pro Pro Pro Ser Pro Glu Lys Lys Ala
   660 665 670
   aag cct ttg gcc acc act caa cct gca aag act tca aca tcg aaa gcc 2064
   Lys Pro Leu Ala Thr Thr Gln Pro Ala Lys Thr Ser Thr Ser Lys Ala
   675 680 685
   aaa aca cag ccc act tct ctc cct aag caa cca gct ccc acc acc tct 2112
   Lys Thr Gln Pro Thr Ser Leu Pro Lys Gln Pro Ala Pro Thr Thr Ser
   690 695 700
   ggt ggg ttg aat aaa aaa ccc atg agc ctc gcc tca ggc tca gtg cca 2160
   Gly Gly Leu Asn Lys Lys Pro Met Ser Leu Ala Ser Gly Ser Val Pro
   705 710 715 720
   gct gcc cca cac aaa cgc cct gct gct gcc act gct act gcc agg cct 2208
   Ala Ala Pro His Lys Arg Pro Ala Ala Ala Thr Ala Thr Ala Arg Pro
   725 730 735
   tcc acc cta cct gcc aga gac gtg aag cca aag cca att aca gaa gct 2256
   Ser Thr Leu Pro Ala Arg Asp Val Lys Pro Lys Pro Ile Thr Glu Ala
   740 745 750
   aag gtt gcc gaa aag cgg acc tct cca tcc aag cct tca tct gcc cca 2304
   Lys Val Ala Glu Lys Arg Thr Ser Pro Ser Lys Pro Ser Ser Ala Pro
   755 760 765
   gcc ctc aaa cct gga cct aaa acc acc cca acc gtt tca aaa gcc aca 2352
   Ala Leu Lys Pro Gly Pro Lys Thr Thr Pro Thr Val Ser Lys Ala Thr
   770 775 780
   tct ccc tca act ctt gtt tcc act gga cca agt agt aga agt cca gct 2400
   Ser Pro Ser Thr Leu Val Ser Thr Gly Pro Ser Ser Arg Ser Pro Ala
   785 790 795 800
   aca act ctg cct aag agg cca acc agc atc aag act gag ggg aaa cct 2448
   Thr Thr Leu Pro Lys Arg Pro Thr Ser Ile Lys Thr Glu Gly Lys Pro
   805 810 815
   gct gat gtc aaa agg atg act gct aag tct gcc tca gct gac ttg agt 2496
   Ala Asp Val Lys Arg Met Thr Ala Lys Ser Ala Ser Ala Asp Leu Ser
   820 825 830
   cgc tca aag acc acc tct gcc agt tct gtg aag aga aac acc act ccc 2544
   Arg Ser Lys Thr Thr Ser Ala Ser Ser Val Lys Arg Asn Thr Thr Pro
   835 840 845
   act ggg gca gca ccc cca gca ggg atg act tcc act cga gtc aag ccc 2592
   Thr Gly Ala Ala Pro Pro Ala Gly Met Thr Ser Thr Arg Val Lys Pro
   850 855 860
   atg tct gca cct agc cgc tct tct ggg gct ctt tct gtg gac aag aag 2640
   Met Ser Ala Pro Ser Arg Ser Ser Gly Ala Leu Ser Val Asp Lys Lys
   865                 870                 875                 880
   ccc act tcc act aag cct agc tcc tct gct ccc agg gtg agc cgc ctg     2688
   Pro Thr Ser Thr Lys Pro Ser Ser Ser Ala Pro Arg Val Ser Arg Leu
                   885                 890                 895
   gcc aca act gtt tct gcc cct gac ctg aag agt gtt cgc tcc aag gtc     2736
   Ala Thr Thr Val Ser Ala Pro Asp Leu Lys Ser Val Arg Ser Lys Val
               900                 905                 910
   ggc tct aca gaa aac atc aaa cac cag cct gga gga ggc cgg gcc aaa     2784
   Gly Ser Thr Glu Asn Ile Lys His Gln Pro Gly Gly Gly Arg Ala Lys
           915                 920                 925
   gta gag aaa aaa aca gag gca gct acc aca gct ggg aag cct gaa cct     2832
   Val Glu Lys Lys Thr Glu Ala Ala Thr Thr Ala Gly Lys Pro Glu Pro
       930                 935                 940
   aat gca gtc act aaa gca gcc ggc tcc att gcg agt gca cag aaa ccg     2880
   Asn Ala Val Thr Lys Ala Ala Gly Ser Ile Ala Ser Ala Gln Lys Pro
   945                 950                 955                 960
   cct gct ggg aaa gtc cag ata gta tcc aaa aaa gtg agc tac agt cat     2928
   Pro Ala Gly Lys Val Gln Ile Val Ser Lys Lys Val Ser Tyr Ser His
                   965                 970                 975
   att caa tcc aag tgt gtt tcc aag gac aat att aag cat gtc cct gga     2976
   Ile Gln Ser Lys Cys Val Ser Lys Asp Asn Ile Lys His Val Pro Gly
               980                 985                 990
   tgt ggc aat gtt cag att cag aac aag aaa gtg gac ata tcc aag gtc     3024
   Cys Gly Asn Val Gln Ile Gln Asn Lys Lys Val Asp Ile Ser Lys Val
           995                 1000                1005
   tcc tcc aag tgt ggg tcc aaa gct aat atc aag cac aag cct ggt gga     3072
   Ser Ser Lys Cys Gly Ser Lys Ala Asn Ile Lys His Lys Pro Gly Gly
       1010                1015                1020
   gga gat gtc aag att gaa agt cag aag ttg aac ttc aag gag aag gcc     3120
   Gly Asp Val Lys Ile Glu Ser Gln Lys Leu Asn Phe Lys Glu Lys Ala
   1025                1030                1035                1040
   caa gcc aaa gtg gga tcc ctt gat aac gtt ggc cac ttt cct gca gga     3168
   Gln Ala Lys Val Gly Ser Leu Asp Asn Val Gly His Phe Pro Ala Gly
                   1045                1050                1055
   ggt gcc gtg aag act gag ggc ggt ggc agt gag gcc ctt ccg tgt cca     3216
   Gly Ala Val Lys Thr Glu Gly Gly Gly Ser Glu Ala Leu Pro Cys Pro
               1060                1065                1070
   ggc ccc ccc gct ggg gag gag cca gtc atc cct gag gct gcg cct gac     3264
   Gly Pro Pro Ala Gly Glu Glu Pro Val Ile Pro Glu Ala Ala Pro Asp
           1075                1080                1085
   cgt ggc gcc cct act tca gcc agt ggc ctc agt ggc cac acc acc ctg     3312
   Arg Gly Ala Pro Thr Ser Ala Ser Gly Leu Ser Gly His Thr Thr Leu
       1090                1095                1100
   tca ggg ggt ggt gac caa agg gag ccc cag acc ttg gac agc cag atc    3360
   Ser Gly Gly Gly Asp Gln Arg Glu Pro Gln Thr Leu Asp Ser Gln Ile
   1105                1110                1115                1120
   cag gag aca agc atc taa                                            3378
   Gln Glu Thr Ser Ile
                   1125
<210> 152
<211> 1125
<212> PRT
<213> Mus musculus
<400> 152
   Met Ala Asp Leu Ser Leu Val Asp Ala Leu Thr Glu Pro Pro Pro Glu
   1               5                   10                  15
   Ile Glu Gly Glu Ile Lys Arg Asp Phe Met Ala Ala Leu Glu Ala Glu
               20                  25                  30
   Pro Tyr Asp Asp Ile Val Gly Glu Thr Val Glu Lys Thr Glu Phe Ile
           35                  40                  45
   Pro Leu Leu Asp Gly Asp Glu Lys Thr Gly Asn Ser Glu Ser Lys Lys
       50                  55                  60
   Lys Pro Cys Leu Asp Thr Ser Gln Val Glu Gly Ile Pro Ser Ser Lys
   65                  70                  75                  80
   Pro Thr Leu Leu Ala Asn Gly Asp His Gly Met Glu Gly Asn Asn Thr
                   85                  90                  95
   Ala Gly Ser Pro Thr Asp Phe Leu Glu Glu Arg Val Asp Tyr Pro Asp
               100                 105                 110
   Tyr Gln Ser Ser Gln Asn Trp Pro Glu Asp Ala Ser Phe Cys Phe Gln
           115                 120                 125
   Pro Gln Gln Val Leu Asp Thr Asp Gln Ala Glu Pro Phe Asn Glu His
       130                 135                 140
   Arg Asp Asp Gly Leu Ala Asp Leu Leu Phe Val Ser Ser Gly Pro Thr
   145                 150                 155                 160
   Asn Ala Ser Ala Phe Thr Glu Arg Asp Asn Pro Ser Glu Asp Ser Tyr
                   165                 170                 175
   Gly Met Leu Pro Cys Asp Ser Phe Ala Ser Thr Ala Val Val Ser Gln
               180                 185                 190
   Glu Trp Ser Val Gly Ala Pro Asn Ser Pro Cys Ser Glu Ser Cys Val
           195                 200                 205
   Ser Pro Glu Val Thr Ile Glu Thr Leu Gln Pro Ala Thr Glu Leu Ser
       210                 215                 220
   Lys Ala Ala Glu Val Glu Ser Val Lys Glu Gln Leu Pro Ala Lys Ala
   225                 230                 235                 240
   Leu Glu Thr Met Ala Glu Gln Thr Thr Asp Val Val His Ser Pro Ser
                   245                 250                 255
   Thr Asp Thr Thr Pro Gly Pro Asp Thr Glu Ala Ala Leu Ala Lys Asp
               260                 265                 270
   Ile Glu Glu Ile Thr Lys Pro Asp Val Ile Leu Ala Asn Val Thr Gln
           275                 280                 285
   Pro Ser Thr Glu Ser Asp Met Phe Leu Ala Gln Asp Met Glu Leu Leu
       290                 295                 300
   Thr Gly Thr Glu Ala Ala His Ala Asn Asn Ile Ile Leu Pro Thr Glu
   305                 310                 315                 320
   Pro Asp Glu Ser Ser Thr Lys Asp Val Ala Pro Pro Met Glu Glu Glu
                   325                 330                 335
   Ile Val Pro Gly Asn Asp Thr Thr Ser Pro Lys Glu Thr Glu Thr Thr
               340                 345                 350
   Leu Pro Ile Lys Met Asp Leu Ala Pro Pro Glu Asp Val Leu Leu Thr
           355                 360                 365
   Lys Glu Thr Glu Leu Ala Pro Ala Lys Gly Met Val Ser Leu Ser Glu
       370                 375                 380
   Ile Glu Glu Ala Leu Ala Lys Asn Asp Val Arg Ser Ala Glu Ile Pro
   385                 390                 395                 400
   Val Ala Gln Glu Thr Val Val Ser Glu Thr Glu Val Val Leu Ala Thr
                   405                 410                 415
   Glu Val Val Leu Pro Ser Asp Pro Ile Thr Thr Leu Thr Lys Asp Val
               420                 425                 430
   Thr Leu Pro Leu Glu Ala Glu Arg Pro Leu Val Thr Asp Met Thr Pro
           435                 440                 445
   Ser Leu Glu Thr Glu Met Thr Leu Gly Lys Glu Thr Ala Pro Pro Thr
       450                 455                 460
   Glu Thr Asn Leu Gly Met Ala Lys Asp Met Ser Pro Leu Pro Glu Ser
   465                 470                 475                 480
   Glu Val Thr Leu Gly Lys Asp Val Val Ile Leu Pro Glu Thr Lys Val
                   485                 490                 495
   Ala Glu Phe Asn Asn Val Thr Pro Leu Ser Glu Glu Glu Val Thr Ser
               500                 505                 510
   Val Lys Asp Met Ser Pro Ser Ala Glu Thr Glu Ala Pro Leu Ala Lys
           515                 520                 525
   Asn Ala Asp Leu His Ser Gly Thr Glu Leu Ile Val Asp Asn Ser Met
       530                 535                 540
   Ala Pro Ala Ser Asp Leu Ala Leu Pro Leu Glu Thr Lys Val Ala Thr
   545                 550                 555                 560
   Val Pro Ile Lys Asp Lys Gly Thr Val Gln Thr Glu Glu Lys Pro Arg
                   565                 570                 575
   Glu Asp Ser Gln Leu Ala Ser Met Gln His Lys Gly Gln Ser Thr Val
               580                 585                 590
   Pro Pro Cys Thr Ala Ser Pro Glu Pro Val Lys Ala Ala Glu Gln Met
           595                 600                 605
   Ser Thr Leu Pro Ile Asp Ala Pro Ser Pro Leu Glu Asn Leu Glu Gln
       610                 615                 620
   Lys Glu Thr Pro Gly Ser Gln Pro Ser Glu Pro Cys Ser Gly Val Ser
   625                 630                 635                 640
   Arg Gln Glu Glu Ala Lys Ala Ala Val Gly Val Thr Gly Asn Asp Ile
                   645                 650                 655
   Thr Thr Pro Pro Asn Lys Glu Pro Pro Pro Ser Pro Glu Lys Lys Ala
               660                 665                 670
   Lys Pro Leu Ala Thr Thr Gln Pro Ala Lys Thr Ser Thr Ser Lys Ala
           675                 680                 685
   Lys Thr Gln Pro Thr Ser Leu Pro Lys Gln Pro Ala Pro Thr Thr Ser
       690                 695                 700
   Gly Gly Leu Asn Lys Lys Pro Met Ser Leu Ala Ser Gly Ser Val Pro
   705                 710                 715                 720
   Ala Ala Pro His Lys Arg Pro Ala Ala Ala Thr Ala Thr Ala Arg Pro
                   725                 730                 735
   Ser Thr Leu Pro Ala Arg Asp Val Lys Pro Lys Pro Ile Thr Glu Ala
               740                 745                 750
   Lys Val Ala Glu Lys Arg Thr Ser Pro Ser Lys Pro Ser Ser Ala Pro
           755                 760                 765
   Ala Leu Lys Pro Gly Pro Lys Thr Thr Pro Thr Val Ser Lys Ala Thr
       770                 775                 780
   Ser Pro Ser Thr Leu Val Ser Thr Gly Pro Ser Ser Arg Ser Pro Ala
   785                 790                 795                 800
   Thr Thr Leu Pro Lys Arg Pro Thr Ser Ile Lys Thr Glu Gly Lys Pro
                   805                 810                 815
   Ala Asp Val Lys Arg Met Thr Ala Lys Ser Ala Ser Ala Asp Leu Ser
               820                 825                 830
   Arg Ser Lys Thr Thr Ser Ala Ser Ser Val Lys Arg Asn Thr Thr Pro
   835 840 845
   Thr Gly Ala Ala Pro Pro Ala Gly Met Thr Ser Thr Arg Val Lys Pro
   850 855 860
   Met Ser Ala Pro Ser Arg Ser Ser Gly Ala Leu Ser Val Asp Lys Lys
   865 870 875 880
   Pro Thr Ser Thr Lys Pro Ser Ser Ser Ala Pro Arg Val Ser Arg Leu
   885 890 895
   Ala Thr Thr Val Ser Ala Pro Asp Leu Lys Ser Val Arg Ser Lys Val
   900 905 910
   Gly Ser Thr Glu Asn Ile Lys His Gln Pro Gly Gly Gly Arg Ala Lys
   915 920 925
   Val Glu Lys Lys Thr Glu Ala Ala Thr Thr Ala Gly Lys Pro Glu Pro
   930 935 940
   Asn Ala Val Thr Lys Ala Ala Gly Ser Ile Ala Ser Ala Gln Lys Pro
   945 950 955 960
   Pro Ala Gly Lys Val Gln Ile Val Ser Lys Lys Val Ser Tyr Ser His
   965 970 975
   Ile Gln Ser Lys Cys Val Ser Lys Asp Asn Ile Lys His Val Pro Gly
   980 985 990
   Cys Gly Asn Val Gln Ile Gln Asn Lys Lys Val Asp Ile Ser Lys Val
   995 1000 1005
   Ser Ser Lys Cys Gly Ser Lys Ala Asn Ile Lys His Lys Pro Gly Gly
   1010 1015 1020
   Gly Asp Val Lys Ile Glu Ser Gln Lys Leu Asn Phe Lys Glu Lys Ala
   1025 1030 1035 1040
   Gln Ala Lys Val Gly Ser Leu Asp Asn Val Gly His Phe Pro Ala Gly
   1045 1050 1055
   Gly Ala Val Lys Thr Glu Gly Gly Gly Ser Glu Ala Leu Pro Cys Pro
   1060 1065 1070
   Gly Pro Pro Ala Gly Glu Glu Pro Val Ile Pro Glu Ala Ala Pro Asp
   1075 1080 1085
   Arg Gly Ala Pro Thr Ser Ala Ser Gly Leu Ser Gly His Thr Thr Leu
   1090 1095 1100
   Ser Gly Gly Gly Asp Gln Arg Glu Pro Gln Thr Leu Asp Ser Gln Ile
   1105 1110 1115 1120
   Gln Glu Thr Ser Ile
   1125
<210> 153
<211> 96
<212> DNA
<213> Artificial Sequence
<220>
<223> Description of Artificial Sequence:
   oligonucleotide
<400> 153
   tcatcatccg gagctggagc cggagctggc cgatcggctg ttaaatctga aggaaagaga 60
   aagtgtgacg aagttgatgg aattgatgaa gtagca 96
<210> 154
<211> 99
<212> DNA
<213> Artificial Sequence
<220>
<223> Description of Artificial Sequence:
   oligonucleotide
<400> 154
   gaagaaggat ccggcacttg ggggtgtaga atgaacaccc tccaagctga gcttgcacag 60
   gatttcgtgg acagtagaca tagtacttgc tacttcatc 99
<210> 155
<211> 18
<212> DNA
<213> Artificial Sequence
<220>
<223> Description of Artificial Sequence:
   oligonucleotide
<400> 155
   tcatcatccg gagctgga 18
<210> 156
<211> 18
<212> DNA
<213> Artificial Sequence
<220>
<223> Description of Artificial Sequence:
   oligonucleotide
<400> 156
   gaagaaggat ccggcact 18
<210> 157
<211> 96
<212> DNA
<213> Artificial Sequence
<220>
<223> Description of Artificial Sequence:
   oligonucleotide
<400> 157
   tcatcatccg gaagaaggaa acgacaaaag cgatcggctg ttaaatctga aggaaagaga 60
   aagtgtgacg aagttgatgg aattgatgaa gtagca 96
<210> 158
<211> 18
<212> DNA
<213> Artificial Sequence
<220>
<223> Description of Artificial Sequence:
   oligonucleotide
<400> 158
   tcatcatccg gaagaagg 18
<210> 159
<211> 60
<212> DNA
<213> Artificial Sequence
<220>
<223> Description of Artificial Sequence:
   oligonucleotide
<400> 159
   tcatcatccg gaagaaggaa acgacaaaag cgatcgacaa gacttgttga aattgacaac 60
<210> 160
<211> 99
<212> DNA
<213> Artificial Sequence
<220>
<223> Description of Artificial Sequence:
   oligonucleotide
<400> 160
   gaagaaggat ccggcacttg ggggtgtaga atgaacaccc tccaagctga gcttgcacag 60
   gatttcgtgg acagtagaca tagtactgtt gtcaatttc 99
<210> 161
<211> 84
<212> DNA
<213> Artificial Sequence
<220>
<223> Description of Artificial Sequence:
   oligonucleotide
<400> 161
   tcatcatccg gaagaaggaa acgacaaaag cgatcgtatc aaaaaggaat accagttgaa 60
   acagacagcg aagagcaacc ttat 84
<210> 162
<211> 99
<212> DNA
<213> Artificial Sequence
<220>
<223> Description of Artificial Sequence:
   oligonucleotide
<400> 162
   gaagaaggat ccggcacttg ggggtgtaga atgaacaccc tccaagctga gcttgcacag 60
   gatttcgtgg acagtagaca tagtactata aggttgctc 99
<210> 163
<211> 60
<212> DNA
<213> Artificial Sequence
<220>
<223> Description of Artificial Sequence:
   oligonucleotide
<400> 163
   tcatcatccg gaagaaaacg tatacgtact tacctcaagt cctgcaggcg gatgaaaaga 60
<210> 164
<211> 63
<212> DNA
<213> Artificial Sequence
<220>
<223> Description of Artificial Sequence:
   oligonucleotide
<400> 164
   gaagaacgat cgagtaaggt gggaaggaat aggtcgagac atctcaaaac cacttctttt 60
   cat 63
<210> 165
<211> 18
<212> DNA
<213> Artificial Sequence
<220>
<223> Description of Artificial Sequence:
   oligonucleotide
<400> 165
   tcatcatccg gaagaaaa 18
<210> 166
<211> 18
<212> DNA
<213> Artificial Sequence
<220>
<223> Description of Artificial Sequence:
   oligonucleotide
<400> 166
   gaagaacgat cgagtaag 18
<210> 167
<211> 14
<212> DNA
<213> Artificial Sequence
<220>
<223> Description of Artificial Sequence: Caspase-1,4,5
   substrate recognition sequence
<400> 167
   ttagaacatg acaa 14
<210> 168
<211> 4
<212> PRT
<213> Artificial Sequence
<220>
<223> Description of Artificial Sequence: Caspase-1,4,5
   substrate recognition sequence
<400> 168
   Leu Glu His Asp
   1
<210> 169
<211> 1380
<212> DNA
<213> Artificial Sequence
<220>
<223> Description of Artificial Sequence: GFP-HSP27
<220>
<221> CDS
<222> (1)..(1380)
<400> 169
   atg gtg agc aag ggc gag gag ctg ttc acc ggg gtg gtg ccc atc ctg      48
   gtc gag ctg gac ggc gac gta aac ggc cac aag ttc agc gtg tcc ggc      96
   gag ggc gag ggc gat gcc acc tac ggc aag ctg acc ctg aag ttc atc     144
   tgc acc acc ggc aag ctg ccc gtg ccc tgg ccc acc ctc gtg acc acc     192
   ctg acc tac ggc gtg cag tgc ttc agc cgc tac ccc gac cac atg aag     240
   cag cac gac ttc ttc aag tcc gcc atg ccc gaa ggc tac gtc cag gag     288
   cgc acc atc ttc ttc aag gac gac ggc aac tac aag acc cgc gcc gag     336
   gtg aag ttc gag ggc gac acc ctg gtg aac cgc atc gag ctg aag ggc     384
   atc gac ttc aag gag gac ggc aac atc ctg ggg cac aag ctg gag tac     432
   aac tac aac agc cac aac gtc tat atc atg gcc gac aag cag aag aac     480
   ggc atc aag gtg aac ttc aag atc cgc cac aac atc gag gac ggc agc     528
   gtg cag ctc gcc gac cac tac cag cag aac acc ccc atc ggc gac ggc     576
   ccc gtg ctg ctg ccc gac aac cac tac ctg agc acc cag tcc gcc ctg    624
   agc aaa gac ccc aac gag aag cgc gat cac atg gtc ctg ctg gag ttc    672
   gtg acc gcc gcc ggg atc act ctc ggc atg gac gag ctg tac aag tcc    720
   gga ctc aga tct cga gcg gcg tcc aga gca gag tca gcc agc atg acc    768
   gag cgc cgc gtc ccc ttc tcg ctc ctg cgg ggc ccc agc tgg gac ccc    816
   ttc cgc gac tgg tac ccg cat agc cgc ctc ttc gac cag gcc ttc ggg    864
   ctg ccc cgg ctg ccg gag gag tgg tcg cag tgg tta ggc ggc agc agc    912
   tgg cca ggc tac gtg cgc ccc ctg ccc ccc gcc gcc atc gag agc ccc    960
   gca gtg gcc gcg ccc gcc tac agc cgc gcg ctc agc cgg caa ctc agc   1008
   agc ggg gtc tcg gag atc cgg cac act gcg gac cgc tgg cgc gtg tcc   1056
   ctg gat gtc aac cac ttc gcc ccg gac gag ctg acg gtc aag acc aag   1104
   gat ggc gtg gtg gag atc acc ggc aag cac gag gag cgg cag gac gag   1152
   cat ggc tac atc tcc cgg tgc ttc acg cgg aaa tac acg ctg ccc ccc   1200
   ggt gtg gac ccc acc caa gtt tcc tcc tcc ctg tcc cct gag ggc aca   1248
   ctg acc gtg gag gcc ccc atg ccc aag cta gcc acg cag tcc aac gag   1296
   atc acc atc cca gtc acc ttc gag tcg cgg gcc cag ctt ggg ggc cca      1344
   gaa gct gca aaa tcc gat gag act gcc gcc aag taa                      1380
<210> 170
<211> 459
<212> PRT
<213> Artificial Sequence
<220>
<223> Description of Artificial Sequence: GFP-HSP27
<400> 170
<210> 171
<211> 2823
<212> DNA
<213> Artificial Sequence
<220>
<223> Description of Artificial Sequence: GFP-HSP70
<220>
<221> CDS
<222> (1)..(2823)
<400> 171
   atg gtg agc aag ggc gag gag ctg ttc acc ggg gtg gtg ccc atc ctg     48
   gtc gag ctg gac ggc gac gta aac ggc cac aag ttc agc gtg tcc ggc     96
   gag ggc gag ggc gat gcc acc tac ggc aag ctg acc ctg aag ttc atc     144
   tgc acc acc ggc aag ctg ccc gtg ccc tgg ccc acc ctc gtg acc acc     192
   ctg acc tac ggc gtg cag tgc ttc agc cgc tac ccc gac cac atg aag     240
   cag cac gac ttc ttc aag tcc gcc atg ccc gaa ggc tac gtc cag gag     288
   cgc acc atc ttc ttc aag gac gac ggc aac tac aag acc cgc gcc gag     336
   gtg aag ttc gag ggc gac acc ctg gtg aac cgc atc gag ctg aag ggc     384
   atc gac ttc aag gag gac ggc aac atc ctg ggg cac aag ctg gag tac     432
   aac tac aac agc cac aac gtc tat atc atg gcc gac aag cag aag aac     480
   ggc atc aag gtg aac ttc aag atc cgc cac aac atc gag gac ggc agc     528
   gtg cag ctc gcc gac cac tac cag cag aac acc ccc atc ggc gac ggc     576
   ccc gtg ctg ctg ccc gac aac cac tac ctg agc acc cag tcc gcc ctg     624
   agc aaa gac ccc aac gag aag cgc gat cac atg gtc ctg ctg gag ttc    672
   gtg acc gcc gcc ggg atc act ctc ggc atg gac gag ctg tac aag tcc    720
   gga atg tcg gtg gtg ggc ata gac ctg ggc ttc cag agc tgc tac gtc    768
   gct gtg gcc cgc gcc ggc ggc atc gag act atc gct aat gag tat agc    816
   gac cgc tgc acg ccg gct tgc att tct ttt ggt cct aag aat cgt tca    864
   att gga gca gca gct aaa agc cag gta att tct aat gca aag aac aca    912
   gtc caa gga ttt aaa aga ttc cat ggc cga gca ttc tct gat cca ttt    960
   gtg gag gca gaa aaa tct aac ctt gca tat gat att gtg cag tgg cct    1008
   aca gga tta aca ggt ata aag gtg aca tat atg gag gaa gag cga aat    1056
   ttt acc act gag caa gtg act gcc atg ctt ttg tcc aaa ctg aag gag    1104
   aca gcc gaa agt gtt ctt aag aag cct gta gtt gac tgt gtt gtt tcg    1152
   gtt cct tgt ttc tat act gat gca gaa aga cga tca gtg atg gat gca    1200
   aca cag att gct ggt ctt aat tgc ttg cga tta atg aat gaa acc act    1248
   gca gtt gct ctt gca tat gga atc tat aag cag gat ctt cct cgc tta    1296
   gaa gag aaa cca aga aat gta gtt ttt gta gac atg ggc cac tct gct     1344
   tat caa gtt tct gta tgt gca ttt aat aga gga aaa ctg aaa gtt ctg     1392
   gcc act gca ttt gac acg aca ttg gga ggt aga aaa ttt gat gaa gtg     1440
   tta gta aat cac ttc tgt gaa gaa ttt ggg aag aaa tac aag cta gac     1488
   att aag tcc aaa atc cgt gca tta tta cga ctc tct cag gag tgt gag     1536
   aaa ctc aag aaa ttg atg agt gca aat gct tca gat ctc cct ttg agc     1584
   att gaa tgt ttt atg aat gat gtt gat gta tct gga act atg aat aga     1632
   ggc aaa ttt ctg gag atg tgc aat gat ctc tta gct aga gtg gag cca     1680
   cca ctt cgt agt gtt ttg gaa caa acc aag tta aag aaa gaa gat att     1728
   tat gca gtg gag ata gtt ggt ggt gct aca cga atc cct gcg gta aaa     1776
   gag aag atc agc aaa ttt ttc ggt aaa gaa ctt agt aca aca tta aat     1824
   gct gat gaa gct gtc act cga ggc tgt gca ttg cag tgt gcc atc tta     1872
   tcg cct gct ttc aaa gtc aga gaa ttt tct atc act gat gta gta cca     1920
   tat cca ata tct ctg aga tgg aat tct cca gct gaa gaa ggg tca agt     1968
   gac tgt gaa gtc ttt tcc aaa aat cat gct gct cct ttc tct aaa gtt 2016
   ctt aca ttt tat aga aag gaa cct ttc act ctt gag gcc tac tac agc 2064
   tct cct cag gat ttg ccc tat cca gat cct gct ata gct cag ttt tca 2112
   gtt cag aaa gtc act cct cag tct gat ggc tcc agt tca aaa gtg aaa 2160
   gtc aaa gtt cga gta aat gtc cat ggc att ttc agt gtg tcc agt gca 2208
   tct tta gtg gag gtt cac aag tct gag gaa aat gag gag cca atg gaa 2256
   aca gat cag aat gca aag gag gaa gag aag atg caa gtg gac cag gag 2304
   gaa cca cat gtt gaa gag caa cag cag cag aca cca gca gaa aat aag 2352
   gca gag tct gaa gaa atg gag acc tct caa gct gga tcc aag gat aaa 2400
   aag atg gac caa cca ccc caa tgc caa gaa ggc aaa agt gaa gac cag 2448
   tac tgt gga cct gcc aat cga gaa tca gct ata tgg cag ata gac aga 2496
   gag atg ctc aac ttg tac att gaa aat gag ggt aag atg atc atg cag 2544
   gat aaa ctg gag aag gag cgg aat gat gct aag aac gca gtg gag gaa 2592
   tat gtg tat gaa atg aga gac aag ctt agt ggt gaa tat gag aag ttt 2640
   gtg agt gaa gat gat cgt aac agt ttt act ttg aaa ctg gaa gat act     2688
   gaa aat tgg ttg tat gag gat gga gaa gac cag cca aag caa gtt tat     2736
   gtt gat aag ttg gct gaa tta aaa aat cta ggt caa cct att aag ata     2784
   cgt ttc cag gaa tct gaa gaa cga cca aat tat ttg aag                 2823
<210> 172
<211> 941
<212> PRT
<213> Artificial Sequence
<220>
<223> Description of Artificial Sequence: GFP-HSP70
<400> 172
<210> 173
<211> 2674
<212> DNA
<213> Artificial Sequence
<220>
<223> Description of Artificial Sequence: GFP-HSC70
<220>
<221> CDS
<222> (1)..(2673)
<400> 173
   atg gtg agc aag ggc gag gag ctg ttc acc ggg gtg gtg ccc atc ctg 48
   Met Val Ser Lys Gly Glu Glu Leu Phe Thr Gly Val Val Pro Ile Leu
   1 5 10 15
   gtc gag ctg gac ggc gac gta aac ggc cac aag ttc agc gtg tcc ggc 96
   Val Glu Leu Asp Gly Asp Val Asn Gly His Lys Phe Ser Val Ser Gly
   20 25 30
   gag ggc gag ggc gat gcc acc tac ggc aag ctg acc ctg aag ttc atc 144
   Glu Gly Glu Gly Asp Ala Thr Tyr Gly Lys Leu Thr Leu Lys Phe Ile
   35 40 45
   tgc acc acc ggc aag ctg ccc gtg ccc tgg ccc acc ctc gtg acc acc    192
   Cys Thr Thr Gly Lys Leu Pro Val Pro Trp Pro Thr Leu Val Thr Thr
        50                  55                  60
   ctg acc tac ggc gtg cag tgc ttc agc cgc tac ccc gac cac atg aag    240
   Leu Thr Tyr Gly Val Gln Cys Phe Ser Arg Tyr Pro Asp His Met Lys
   65                  70                  75                  80
   cag cac gac ttc ttc aag tcc gcc atg ccc gaa ggc tac gtc cag gag    288
   Gln His Asp Phe Phe Lys Ser Ala Met Pro Glu Gly Tyr Val Gln Glu
                   85                  90                  95
   cgc acc atc ttc ttc aag gac gac ggc aac tac aag acc cgc gcc gag    336
   Arg Thr Ile Phe Phe Lys Asp Asp Gly Asn Tyr Lys Thr Arg Ala Glu
               100                 105                 110
   gtg aag ttc gag ggc gac acc ctg gtg aac cgc atc gag ctg aag ggc    384
   Val Lys Phe Glu Gly Asp Thr Leu Val Asn Arg Ile Glu Leu Lys Gly
           115                 120                 125
   atc gac ttc aag gag gac ggc aac atc ctg ggg cac aag ctg gag tac    432
   Ile Asp Phe Lys Glu Asp Gly Asn Ile Leu Gly His Lys Leu Glu Tyr
       130                 135                 140
   aac tac aac agc cac aac gtc tat atc atg gcc gac aag cag aag aac    480
   Asn Tyr Asn Ser His Asn Val Tyr Ile Met Ala Asp Lys Gln Lys Asn
   145                 150                 155                 160
   ggc atc aag gtg aac ttc aag atc cgc cac aac atc gag gac ggc agc    528
   Gly Ile Lys Val Asn Phe Lys Ile Arg His Asn Ile Glu Asp Gly Ser
                   165                 170                 175
   gtg cag ctc gcc gac cac tac cag cag aac acc ccc atc ggc gac ggc    576
   Val Gln Leu Ala Asp His Tyr Gln Gln Asn Thr Pro Ile Gly Asp Gly
               180                 185                 190
   ccc gtg ctg ctg ccc gac aac cac tac ctg agc acc cag tcc gcc ctg    624
   Pro Val Leu Leu Pro Asp Asn His Tyr Leu Ser Thr Gln Ser Ala Leu
           195                 200                 205
   agc aaa gac ccc aac gag aag cgc gat cac atg gtc ctg ctg gag ttc    672
   Ser Lys Asp Pro Asn Glu Lys Arg Asp His Met Val Leu Leu Glu Phe
       210                 215                 220
   gtg acc gcc gcc ggg atc act ctc ggc atg gac gag ctg tac aag tcc    720
   Val Thr Ala Ala Gly Ile Thr Leu Gly Met Asp Glu Leu Tyr Lys Ser
   225                 230                 235                 240
   gga ctc aga tct atg tcc aag gga cct gca gtt ggt att gat ctt ggc    768
   Gly Leu Arg Ser Met Ser Lys Gly Pro Ala Val Gly Ile Asp Leu Gly
                   245                 250                 255
   acc acc tac tct tgt gtg ggt gtt ttc cag cac gga aaa gtc gag ata    816
   Thr Thr Tyr Ser Cys Val Gly Val Phe Gln His Gly Lys Val Glu Ile
               260                 265                 270
   att gcc aat gat cag gga aac cga acc act cca agc tat gtc gcc ttt    864
   Ile Ala Asn Asp Gln Gly Asn Arg Thr Thr Pro Ser Tyr Val Ala Phe
           275                 280                 285
   acg gac act gaa cgg ttg atc ggt gat gcc gca aag aat caa gtt gca    912
   Thr Asp Thr Glu Arg Leu Ile Gly Asp Ala Ala Lys Asn Gln Val Ala
       290                 295                 300
   atg aac ccc acc aac aca gtt ttt gat gcc aaa cgt ctg att gga cgc    960
   Met Asn Pro Thr Asn Thr Val Phe Asp Ala Lys Arg Leu Ile Gly Arg
   305                 310                 315                 320
   aga ttt gat gat gct gtt gtc cag tct gat atg aaa cat tgg ccc ttt    1008
   Arg Phe Asp Asp Ala Val Val Gln Ser Asp Met Lys His Trp Pro Phe
                   325                 330                 335
   atg gtg gtg aat gat gct ggc agg ccc aag gtc caa gta gaa tac aag    1056
   Met Val Val Asn Asp Ala Gly Arg Pro Lys Val Gln Val Glu Tyr Lys
               340                 345                 350
   gga gag acc aaa agc ttc tat cca gag gag gtg tct tct atg gtt ctg    1104
   Gly Glu Thr Lys Ser Phe Tyr Pro Glu Glu Val Ser Ser Met Val Leu
           355                 360                 365
   aca aag atg aag gaa att gca gaa gcc tac ctt ggg aag act gtt acc    1152
   Thr Lys Met Lys Glu Ile Ala Glu Ala Tyr Leu Gly Lys Thr Val Thr
       370                 375                 380
   aat gct gtg gtc aca gtg cca gct tac ttt aat gac tct cag cgt cag    1200
   Asn Ala Val Val Thr Val Pro Ala Tyr Phe Asn Asp Ser Gln Arg Gln
   385                 390                 395                 400
   gct acc aaa gat gct gga act att gct ggt ctc aat gta ctt aga att    1248
   Ala Thr Lys Asp Ala Gly Thr Ile Ala Gly Leu Asn Val Leu Arg Ile
                   405                 410                 415
   att aat gag cca act gct gct gct att gct tac ggc tta gac aaa aag    1296
   Ile Asn Glu Pro Thr Ala Ala Ala Ile Ala Tyr Gly Leu Asp Lys Lys
               420                 425                 430
   gtt gga gca gaa aga aac gtg ctc atc ttt gac ctg gga ggt ggc act    1344
   Val Gly Ala Glu Arg Asn Val Leu Ile Phe Asp Leu Gly Gly Gly Thr
           435                 440                 445
   ttt gat gtg tca atc ctc act att gag gat gga atc ttt gag gtc aag    1392
   Phe Asp Val Ser Ile Leu Thr Ile Glu Asp Gly Ile Phe Glu Val Lys
       450                 455                 460
   tct aca gct gga gac acc cac ttg ggt gga gaa gat ttt gac aac cga    1440
   Ser Thr Ala Gly Asp Thr His Leu Gly Gly Glu Asp Phe Asp Asn Arg
   465                 470                 475                 480
   atg gtc aac cat ttt att gct gag ttt aag cgc aag cat aag aag gac    1488
   Met Val Asn His Phe Ile Ala Glu Phe Lys Arg Lys His Lys Lys Asp
                   485                 490                 495
   atc agt gag aac aag aga gct gta aga cgc ctc cgt act gct tgt gaa    1536
   Ile Ser Glu Asn Lys Arg Ala Val Arg Arg Leu Arg Thr Ala Cys Glu
   500 505 510
   cgt gct aag cgt acc ctc tct tcc agc acc cag gcc agt att gag atc 1584
   Arg Ala Lys Arg Thr Leu Ser Ser Ser Thr Gln Ala Ser Ile Glu Ile
   515 520 525
   gat tct ctc tat gaa gga atc gac ttc tat acc tcc att acc cgt gcc 1632
   Asp Ser Leu Tyr Glu Gly Ile Asp Phe Tyr Thr Ser Ile Thr Arg Ala
   530 535 540
   cga ttt gaa gaa ctg aat gct gac ctg ttc cgt ggc acc ctg gac cca 1680
   Arg Phe Glu Glu Leu Asn Ala Asp Leu Phe Arg Gly Thr Leu Asp Pro
   545 550 555 560
   gta gag aaa gcc ctt cga gat gcc aaa cta gac aag tca cag att cat 1728
   Val Glu Lys Ala Leu Arg Asp Ala Lys Leu Asp Lys Ser Gln Ile His
   565 570 575
   gat att gtc ctg gtt ggt ggt tct act cgt atc ccc aag att cag aag 1776
   Asp Ile Val Leu Val Gly Gly Ser Thr Arg Ile Pro Lys Ile Gln Lys
   580 585 590
   ctt ctc caa gac ttc ttc aat gga aaa gaa ctg aat aag agc atc aac 1824
   Leu Leu Gln Asp Phe Phe Asn Gly Lys Glu Leu Asn Lys Ser Ile Asn
   595 600 605
   cct gat gaa gct gtt gct tat ggt gca gct gtc cag gca gcc atc ttg 1872
   Pro Asp Glu Ala Val Ala Tyr Gly Ala Ala Val Gln Ala Ala Ile Leu
   610 615 620
   tct gga gac aag tct gag aat gtt caa gat ttg ctg ctc ttg gat gtc 1920
   Ser Gly Asp Lys Ser Glu Asn Val Gln Asp Leu Leu Leu Leu Asp Val
   625 630 635 640
   act cct ctt tcc ctt ggt att gaa act gct ggt gga gtc atg act gtc 1968
   Thr Pro Leu Ser Leu Gly Ile Glu Thr Ala Gly Gly Val Met Thr Val
   645 650 655
   ctc atc aag cgt aat acc acc att cct acc aag cag aca cag acc ttc 2016
   Leu Ile Lys Arg Asn Thr Thr Ile Pro Thr Lys Gln Thr Gln Thr Phe
   660 665 670
   act acc tat tct gac aac cag cct ggt gtg ctt att cag gtt tat gaa 2064
   Thr Thr Tyr Ser Asp Asn Gln Pro Gly Val Leu Ile Gln Val Tyr Glu
   675 680 685
   ggc gag cgt gcc atg aca aag gat aac aac ctg ctt ggc aag ttt gaa 2112
   Gly Glu Arg Ala Met Thr Lys Asp Asn Asn Leu Leu Gly Lys Phe Glu
   690 695 700
   ctc aca ggc ata cct cct gca ccc cga ggt gtt cct cag att gaa gtc 2160
   Leu Thr Gly Ile Pro Pro Ala Pro Arg Gly Val Pro Gln Ile Glu Val
   705 710 715 720
   act ttt gac att gat gcc aat ggt ata ctc aat gtc tct gct gtg gac 2208
   Thr Phe Asp Ile Asp Ala Asn Gly Ile Leu Asn Val Ser Ala Val Asp
                   725                 730                 735
   aag agt acg gga aaa gag aac aag att act atc act aat gac aag ggc    2256
   Lys Ser Thr Gly Lys Glu Asn Lys Ile Thr Ile Thr Asn Asp Lys Gly
               740                 745                 750
   cgt ttg agc aag gaa gac att gaa cgt atg gtc cag gaa gct gag aag    2304
   Arg Leu Ser Lys Glu Asp Ile Glu Arg Met Val Gln Glu Ala Glu Lys
           755                 760                 765
   tac aaa gct gaa gat gag aag cag agg gac aag gtg tca tcc aag aat    2352
   Tyr Lys Ala Glu Asp Glu Lys Gln Arg Asp Lys Val Ser Ser Lys Asn
       770                 775                 780
   tca ctt gag tcc tat gcc ttc aac atg aaa gca act gtt gaa gat gag    2400
   Ser Leu Glu Ser Tyr Ala Phe Asn Met Lys Ala Thr Val Glu Asp Glu
   785                 790                 795                 800
   aaa ctt caa ggc aag att aac gat gag gac aaa cag aag att ctg gac    2448
   Lys Leu Gln Gly Lys Ile Asn Asp Glu Asp Lys Gln Lys Ile Leu Asp
                   805                 810                 815
   aag tgt aat gaa att atc aac tgg ctt gat aag aat cag act gct gag    2496
   Lys Cys Asn Glu Ile Ile Asn Trp Leu Asp Lys Asn Gln Thr Ala Glu
               820                 825                 830
   aag gaa gaa ttt gaa cat caa cag aaa gag ctg gag aaa gtt tgc aac    2544
   Lys Glu Glu Phe Glu His Gln Gln Lys Glu Leu Glu Lys Val Cys Asn
           835                 840                 845
   ccc atc atc acc aag ctg tac cag agt gca gga ggc atg cca gga gga    2592
   Pro Ile Ile Thr Lys Leu Tyr Gln Ser Ala Gly Gly Met Pro Gly Gly
       850                 855                 860
   atg cct ggg gga ttt cct ggt ggt gga gct cct ccc tct ggt ggt gct    2640
   Met Pro Gly Gly Phe Pro Gly Gly Gly Ala Pro Pro Ser Gly Gly Ala
   865                 870                 875                 880
   tcc tca ggg ccc acc att gaa gag gtt gat taa g                      2674
   Ser Ser Gly Pro Thr Ile Glu Glu Val Asp
                   885                 890
<210> 174
<211> 890
<212> PRT
<213> Artificial Sequence
<220>
<223> Description of Artificial Sequence: GFP-HSC70
<400> 174
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
   35 40 45
   Cys Thr Thr Gly Lys Leu Pro Val Pro Trp Pro Thr Leu Val Thr Thr
   50 55 60
   Leu Thr Tyr Gly Val Gln Cys Phe Ser Arg Tyr Pro Asp His Met Lys
   65 70 75 80
   Gln His Asp Phe Phe Lys Ser Ala Met Pro Glu Gly Tyr Val Gln Glu
   85 90 95
   Arg Thr Ile Phe Phe Lys Asp Asp Gly Asn Tyr Lys Thr Arg Ala Glu
   100 105 110
   Val Lys Phe Glu Gly Asp Thr Leu Val Asn Arg Ile Glu Leu Lys Gly
   115 120 125
   Ile Asp Phe Lys Glu Asp Gly Asn Ile Leu Gly His Lys Leu Glu Tyr
   130 135 140
   Asn Tyr Asn Ser His Asn Val Tyr Ile Met Ala Asp Lys Gln Lys Asn
   145 150 155 160
   Gly Ile Lys Val Asn Phe Lys Ile Arg His Asn Ile Glu Asp Gly Ser
   165 170 175
   Val Gln Leu Ala Asp His Tyr Gln Gln Asn Thr Pro Ile Gly Asp Gly
   180 185 190
   Pro Val Leu Leu Pro Asp Asn His Tyr Leu Ser Thr Gln Ser Ala Leu
   195 200 205
   Ser Lys Asp Pro Asn Glu Lys Arg Asp His Met Val Leu Leu Glu Phe
   210 215 220
   Val Thr Ala Ala Gly Ile Thr Leu Gly Met Asp Glu Leu Tyr Lys Ser
   225 230 235 240
   Gly Leu Arg Ser Met Ser Lys Gly Pro Ala Val Gly Ile Asp Leu Gly
   245 250 255
   Thr Thr Tyr Ser Cys Val Gly Val Phe Gln His Gly Lys Val Glu Ile
   260 265 270
   Ile Ala Asn Asp Gln Gly Asn Arg Thr Thr Pro Ser Tyr Val Ala Phe
   275 280 285
   Thr Asp Thr Glu Arg Leu Ile Gly Asp Ala Ala Lys Asn Gln Val Ala
   290 295 300
   Met Asn Pro Thr Asn Thr Val Phe Asp Ala Lys Arg Leu Ile Gly Arg
   305 310 315 320
   Arg Phe Asp Asp Ala Val Val Gln Ser Asp Met Lys His Trp Pro Phe
   325 330 335
   Met Val Val Asn Asp Ala Gly Arg Pro Lys Val Gln Val Glu Tyr Lys
   340 345 350
   Gly Glu Thr Lys Ser Phe Tyr Pro Glu Glu Val Ser Ser Met Val Leu
   355 360 365
   Thr Lys Met Lys Glu Ile Ala Glu Ala Tyr Leu Gly Lys Thr Val Thr
   370 375 380
   Asn Ala Val Val Thr Val Pro Ala Tyr Phe Asn Asp Ser Gln Arg Gln
   385 390 395 400
   Ala Thr Lys Asp Ala Gly Thr Ile Ala Gly Leu Asn Val Leu Arg Ile
   405 410 415
   Ile Asn Glu Pro Thr Ala Ala Ala Ile Ala Tyr Gly Leu Asp Lys Lys
   420 425 430
   Val Gly Ala Glu Arg Asn Val Leu Ile Phe Asp Leu Gly Gly Gly Thr
   435 440 445
   Phe Asp Val Ser Ile Leu Thr Ile Glu Asp Gly Ile Phe Glu Val Lys
   450 455 460
   Ser Thr Ala Gly Asp Thr His Leu Gly Gly Glu Asp Phe Asp Asn Arg
   465 470 475 480
   Met Val Asn His Phe Ile Ala Glu Phe Lys Arg Lys His Lys Lys Asp
   485 490 495
   Ile Ser Glu Asn Lys Arg Ala Val Arg Arg Leu Arg Thr Ala Cys Glu
   500 505 510
   Arg Ala Lys Arg Thr Leu Ser Ser Ser Thr Gln Ala Ser Ile Glu Ile
   515 520 525
   Asp Ser Leu Tyr Glu Gly Ile Asp Phe Tyr Thr Ser Ile Thr Arg Ala
   530 535 540
   Arg Phe Glu Glu Leu Asn Ala Asp Leu Phe Arg Gly Thr Leu Asp Pro
   545 550 555 560
   Val Glu Lys Ala Leu Arg Asp Ala Lys Leu Asp Lys Ser Gln Ile His
   565 570 575
   Asp Ile Val Leu Val Gly Gly Ser Thr Arg Ile Pro Lys Ile Gln Lys
   580 585 590
   Leu Leu Gln Asp Phe Phe Asn Gly Lys Glu Leu Asn Lys Ser Ile Asn
   595 600 605
   Pro Asp Glu Ala Val Ala Tyr Gly Ala Ala Val Gln Ala Ala Ile Leu
   610 615 620
   Ser Gly Asp Lys Ser Glu Asn Val Gln Asp Leu Leu Leu Leu Asp Val
   625 630 635 640
   Thr Pro Leu Ser Leu Gly Ile Glu Thr Ala Gly Gly Val Met Thr Val
   645 650 655
   Leu Ile Lys Arg Asn Thr Thr Ile Pro Thr Lys Gln Thr Gln Thr Phe
   660 665 670
   Thr Thr Tyr Ser Asp Asn Gln Pro Gly Val Leu Ile Gln Val Tyr Glu
   675 680 685
   Gly Glu Arg Ala Met Thr Lys Asp Asn Asn Leu Leu Gly Lys Phe Glu
   690 695 700
   Leu Thr Gly Ile Pro Pro Ala Pro Arg Gly Val Pro Gln Ile Glu Val
   705 710 715 720
   Thr Phe Asp Ile Asp Ala Asn Gly Ile Leu Asn Val Ser Ala Val Asp
   725 730 735
   Lys Ser Thr Gly Lys Glu Asn Lys Ile Thr Ile Thr Asn Asp Lys Gly
   740 745 750
   Arg Leu Ser Lys Glu Asp Ile Glu Arg Met Val Gln Glu Ala Glu Lys
   755 760 765
   Tyr Lys Ala Glu Asp Glu Lys Gln Arg Asp Lys Val Ser Ser Lys Asn
   770 775 780
   Ser Leu Glu Ser Tyr Ala Phe Asn Met Lys Ala Thr Val Glu Asp Glu
   785 790 795 800
   Lys Leu Gln Gly Lys Ile Asn Asp Glu Asp Lys Gln Lys Ile Leu Asp
   805 810 815
   Lys Cys Asn Glu Ile Ile Asn Trp Leu Asp Lys Asn Gln Thr Ala Glu
   820 825 830
   Lys Glu Glu Phe Glu His Gln Gln Lys Glu Leu Glu Lys Val Cys Asn
   835 840 845
   Pro Ile Ile Thr Lys Leu Tyr Gln Ser Ala Gly Gly Met Pro Gly Gly
   850 855 860
   Met Pro Gly Gly Phe Pro Gly Gly Gly Ala Pro Pro Ser Gly Gly Ala
   865 870 875 880
   Ser Ser Gly Pro Thr Ile Glu Glu Val Asp
   885 890
<210> 175
<211> 2458
<212> DNA
<213> Artificial Sequence
<220>
<223> Description of Artificial Sequence: GFP-HSF1
<220>
<221> CDS
<222> (1) .. (2349)
<400> 175
   atg gtg agc aag ggc gag gag ctg ttc acc ggg gtg gtg ccc atc ctg    48
   gtc gag ctg gac ggc gac gta aac ggc cac aag ttc agc gtg tcc ggc    96
   gag ggc gag ggc gat gcc acc tac ggc aag ctg acc ctg aag ttc atc   144
   tgc acc acc ggc aag ctg ccc gtg ccc tgg ccc acc ctc gtg acc acc   192
   ctg acc tac ggc gtg cag tgc ttc agc cgc tac ccc gac cac atg aag   240
   cag cac gac ttc ttc aag tcc gcc atg ccc gaa ggc tac gtc cag gag   288
   cgc acc atc ttc ttc aag gac gac ggc aac tac aag acc cgc gcc gag   336
   gtg aag ttc gag ggc gac acc ctg gtg aac cgc atc gag ctg aag ggc   384
   atc gac ttc aag gag gac ggc aac atc ctg ggg cac aag ctg gag tac   432
   aac tac aac agc cac aac gtc tat atc atg gcc gac aag cag aag aac   480
   ggc atc aag gtg aac ttc aag atc cgc cac aac atc gag gac ggc agc   528
   gtg cag ctc gcc gac cac tac cag cag aac acc ccc atc ggc gac ggc   576
   ccc gtg ctg ctg ccc gac aac cac tac ctg agc acc cag tcc gcc ctg   624
   agc aaa gac ccc aac gag aag cgc gat cac atg gtc ctg ctg gag ttc     672
   gtg acc gcc gcc ggg atc act ctc ggc atg gac gag ctg tac aag tcc     720
   gga ctc aga tct cga gct caa gct tcg aat tct gca gtc gag atg gat     768
   ctg ccc gtg ggc ccc ggc gcg gcg ggg ccc agc aac gtc ccg gcc ttc     816
   ctg acc aag ctg tgg acc ctc gtg agc gac ccg gac acc gac gcg ctc     864
   atc tgc tgg agc ccg agc ggg aac agc ttc cac gtg ttc gac cag ggc     912
   cag ttt gcc aag gag gtg ctg ccc aag tac ttc aag cac aac aac atg     960
   gcc agc ttc gtg cgg cag ctc aac atg tat ggc ttc cgg aaa gtg gtc    1008
   cac atc gag cag ggc ggc ctg gtc aag cca gag aga gac gac acg gag    1056
   ttc cag cac cca tgc ttc ctg cgt ggc cag gag cag ctc ctt gag aac    1104
   atc aag agg aaa gtg acc agt gtg tcc acc ctg aag agt gaa gac ata    1152
   aag atc cgc cag gac agc gtc acc aag ctg ctg acg gac gtg cag ctg    1200
   atg aag ggg aag cag gag tgc atg gac tcc aag ctc ctg gcc atg aag    1248
   cat gag aat gag gct ctg tgg cgg gag gtg gcc agc ctt cgg cag aag    1296
   cat gcc cag caa cag aaa gtc gtc aac aag ctc att cag ttc ctg atc    1344
   tca ctg gtg cag tca aac cgg atc ctg ggg gtg aag aga aag atc ccc    1392
   ctg atg ctg aac gac agt ggc tca gca cat tcc atg ccc aag tat agc    1440
   cgg cag ttc tcc ctg gag cac gtc cac ggc tcg ggc ccc tac tcg gcc    1488
   ccc tcc cca gcc tac agc agc tcc agc ctc tac gcc cct gat gct gtg    1536
   gcc agc tct gga ccc atc atc tcc gac atc acc gag ctg gct cct gcc    1584
   agc ccc atg gcc tcc ccc ggc ggg agc ata gac gag agg ccc cta tcc    1632
   agc agc ccc ctg gtg cgt gtc aag gag gag ccc ccc agc ccg cct cag    1680
   agc ccc cgg gta gag gag gcg agt ccc ggg cgc cca tct tcc gtg gac    1728
   acc ctc ttg tcc ccg acc gcc ctc att gac tcc atc ctg cgg gag agt    1776
   gaa cct gcc ccc gcc tcc gtc aca gcc ctc acg gac gcc agg ggc cac    1824
   acg gac acc gag ggc cgg cct ccc tcc ccc ccg ccc acc tcc acc cct    1872
   gaa aag tgc ctc agc gta gcc tgc ctg gac aag aat gag ctc agt gac    1920
   cac ttg gat gct atg gac tcc aac ctg gat aac ctg cag acc atg ctg    1968
   agc agc cac ggc ttc agc gtg gac acc agt gcc ctg ctg gac ctg ttc    2016
   agc ccc tcg gtg acc gtg ccc gac atg agc ctg cct gac ctt gac agc    2064
   agc ctg gcc agt atc caa gag ctc ctg tct ccc cag gag ccc ccc agg    2112
   cct ccc gag gca gag aac agc agc ccg gat tca ggg aag cag ctg gtg    2160
   cac tac aca gcg cag ccg ctg ttc ctg ctg gac ccc ggc tcc gtg gac    2208
   acc ggg agc aac gac ctg ccg gtg ctg ttt gag ctg gga gag ggc tcc    2256
   tac ttc tcc gaa ggg gac ggc ttc gcc gag gac ccc acc atc tcc ctg    2304
   ctg aca ggc tcg gag cct ccc aaa gcc aag gac ccc act gtc tcc        2349
   tagaggcccc ggaggagctg ggccagccgc ccacccccac ccccagtgca gggctggtct 2409
   tggggaggca gggcagcctc gcggtcttgg gcactggtgg gtcggccgg              2458
<210> 176
<211> 783
<212> PRT
<213> Artificial Sequence
<220>
<223> Description of Artificial Sequence: GFP-HSF1
<400> 176
<210> 177
<211> 2416
<212> DNA
<213> Artificial Sequence
<220>
<223> Description of Artificial Sequence: GFP-NFKB
<220>
<221> CDS
<222> (1)..(2415)
<400> 177
   atg gtg agc aag ggc gag gag ctg ttc acc ggg gtg gtg ccc atc ctg      48
   gtc gag ctg gac ggc gac gta aac ggc cac aag ttc agc gtg tcc ggc      96
   gag ggc gag ggc gat gcc acc tac ggc aag ctg acc ctg aag ttc atc     144
   tgc acc acc ggc aag ctg ccc gtg ccc tgg ccc acc ctc gtg acc acc     192
   ctg acc tac ggc gtg cag tgc ttc agc cgc tac ccc gac cac atg aag     240
   cag cac gac ttc ttc aag tcc gcc atg ccc gaa ggc tac gtc cag gag     288
   cgc acc atc ttc ttc aag gac gac ggc aac tac aag acc cgc gcc gag     336
   gtg aag ttc gag ggc gac acc ctg gtg aac cgc atc gag ctg aag ggc     384
   atc gac ttc aag gag gac ggc aac atc ctg ggg cac aag ctg gag tac     432
   aac tac aac agc cac aac gtc tat atc atg gcc gac aag cag aag aac     480
   ggc atc aag gtg aac ttc aag atc cgc cac aac atc gag gac ggc agc     528
   gtg cag ctc gcc gac cac tac cag cag aac acc ccc atc ggc gac ggc     576
   ccc gtg ctg ctg ccc gac aac cac tac ctg agc acc cag tcc gcc ctg     624
   agc aaa gac ccc aac gag aag cgc gat cac atg gtc ctg ctg gag ttc     672
   gtg acc gcc gcc ggg atc act ctc ggc atg gac gag ctg tac aag tcc     720
   gga ctc aga tct cga gat ccg ccc ttc atg gac gaa ctg ttc ccc ctc     768
   atc ttc ccg gca gag cca gcc cag gcc tct ggc ccc tat gtg gag atc     816
   att gag cag ccc aag cag cgg ggc atg cgc ttc cgc tac aag tgc gag     864
   ggg cgc tcc gcg ggc agc atc cca ggc gag agg agc aca gat acc acc     912
   aag acc cac ccc acc atc aag atc aat ggc tac aca gga cca ggg aca     960
   gtg cgc atc tcc ctg gtc acc aag gac cct cct cac cgg cct cac ccc    1008
   cac gag ctt gta gga aag gac tgc cgg gat ggc ttc tat gag gct gag    1056
   ctc tgc ccg gac cgc tgc atc cac agt ttc cag aac ctg gga atc cag    1104
   tgt gtg aag aag cgg gac ctg gag cag gct atc agt cag cgc atc cag    1152
   acc aac aac aac ccc ttc caa gtt cct ata gaa gag cag cgt ggg gac    1200
   tac gac ctg aat gct gtg cgg ctc tgc ttc cag gtg aca gtg cgg gac    1248
   cca tca ggc agg ccc ctc cgc ctg ccg cct gtc ctt tct cat ccc atc    1296
   ttt gac aat cgt gcc ccc aac act gcc gag ctc aag atc tgc cga gtg    1344
   aac cga aac tct ggc agc tgc ctc ggt ggg gat gag atc ttc cta ctg    1392
   tgt gac aag gtg cag aaa gag gac att gag gtg tat ttc acg gga cca    1440
   ggc tgg gag gcc cga ggc tcc ttt tcg caa gct gat gtg cac cga caa    1488
   gtg gcc att gtg ttc cgg acc cct ccc tac gca gac ccc agc ctg cag    1536
   gct cct gtg cgt gtc tcc atg cag ctg cgg cgg cct tcc gac cgg gag    1584
   ctc agt gag ccc atg gaa ttc cag tac ctg cca gat aca gac gat cgt    1632
   cac cgg att gag gag aaa cgt aaa agg aca tat gag acc ttc aag agc    1680
   atc atg aag aag agt cct ttc agc gga ccc acc gac ccc cgg cct cca    1728
   cct cga cgc att gct gtg cct tcc cgc agc tca gct tct gtc ccc aag    1776
   cca gca ccc cag ccc tat ccc ttt acg tca tcc ctg agc acc atc aac    1824
   tat gat gag ttt ccc acc atg gtg ttt cct tct ggg cag atc agc cag    1872
   gcc tcg gcc ttg gcc ccg gcc cct ccc caa gtc ctg ccc cag gct cca    1920
   gcc cct gcc cct gct cca gcc atg gta tca gct ctg gcc cag gcc cca    1968
   gcc cct gtc cca gtc cta gcc cca ggc cct cct cag gct gtg gcc cca    2016
   cct gcc ccc aag ccc acc cag gct ggg gaa gga acg ctg tca gag gcc    2064
   ctg ctg cag ctg cag ttt gat gat gaa gac ctg ggg gcc ttg ctt ggc    2112
   aac agc aca gac cca gct gtg ttc aca gac ctg gca tcc gtc gac aac    2160
   tcc gag ttt cag cag ctg ctg aac cag ggc ata cct gtg gcc ccc cac    2208
   aca act gag ccc atg ctg atg gag tac cct gag gct ata act cgc cta    2256
   gtg aca gcc cag agg ccc ccc gac cca gct cct gct cca ctg ggg gcc    2304
   ccg ggg ctc ccc aat ggc ctc ctt tca gga gat gaa gac ttc tcc tcc    2352
   att gcg gac atg gac ttc tca gcc ctg ctg agt cag atc agc tcc aag    2400
   ggc gaa ttc gaa gct t                                               2416
<210> 178
<211> 805
<212> PRT
<213> Artificial Sequence
<220>
<223> Description of Artificial Sequence: GFP-NFKB
<400> 178
<210> 179
<211> 1677
<212> DNA
<213> Artificial Sequence
<220>
<223> Description of Artificial Sequence: GFP-IKB
<220>
<221> CDS
<222> (1)..(1674)
<400> 179
   atg ttc cag gcg gct gag cgc ccc cag gag tgg gcc atg gag ggc ccc      48
   cgc gac ggg ctg aag aag gag cgg cta ctg gac gac cgc cac gac agc      96
   ggc ctg gac tcc atg aaa gac gag gag tac gag cag atg gtc aag gag     144
   ctg cag gag atc cgc ctc gag ccg cag gag gtg ccg cgc ggc tcg gag     192
   ccc tgg aag cag cag ctc acc gag gac ggg gac tcg ttc ctg cac ttg     240
   gcc atc atc cat gaa gaa aag gca ctg acc atg gaa gtg atc cgc cag     288
   gtg aag gga gac ctg gcc ttc ctc aac ctc cag aac aac ctg cag cag     336
   act cca ctc cac ttg gct gtg atc acc aac cag cca gaa att gct gag     384
   gca ctt ctg gga gct ggc tgt gat cct gag ctc cga gac ttt cga gga     432
   aat acc ccc cta cac ctt gcc tgt gag cag ggc tgc ctg gcc agc gtg     480
   gga gtc ctg act cag tcc tgc acc acc ccg cac ctc cac tcc atc ttg    528
   aag gct acc aac tac aat ggc cac acg tgt cta cac tta gcc tct atc    576
   cat ggc tac ctg ggc atc gtg gag ctt ttg gtg tcc ttg ggt gct gat    624
   gtc aat gct cag gag ccc tgt aat ggc cgg act gcc ctt cac ctc gca    672
   gtg gac ctg caa aat cct gac ctg gtg tca ctc ctg ttg aag tgt ggg    720
   gct gat gtc aac aga gtt acc tac cag ggc tat tct ccc tac cag ctc    768
   acc tgg ggc cgc cca agc acc cgg ata cag cag cag ctg ggc cag ctg    816
   aca cta gaa aac ctt cag atg ctg cca gag agt gag gat gag gag agc    864
   tat gac aca gag tca gag ttc acg gag ttc aca gag gac gag ctg ccc    912
   tat gat gac tgt gtg ttt gga ggc cag cgt ctg acg tta acc ggt atg    960
   gct agc aaa gga gaa gaa ctc ttc act gga gtt gtc cca att ctt gtt   1008
   gaa tta gat ggt gat gtt aac ggc cac aag ttc tct gtc agt gga gag   1056
   ggt gaa ggt gat gca aca tac gga aaa ctt acc ctg aag ttc atc tgc   1104
   act act ggc aaa ctg cct gtt cca tgg cca aca cta gtc act act ctg   1152
   tgc tat ggt gtt caa tgc ttt tca aga tac ccg gat cat atg aaa cgg   1200
   cat gac ttt ttc aag agt gcc atg ccc gaa ggt tat gta cag gaa agg 1248
   acc atc ttc ttc aaa gat gac ggc aac tac aag aca cgt gct gaa gtc 1296
   aag ttt gaa ggt gat acc ctt gtt aat aga atc gag tta aaa ggt att 1344
   gac ttc aag gaa gat ggc aac att ctg gga cac aaa ttg gaa tac aac 1392
   tat aac tca cac aat gta tac atc atg gca gac aaa caa aag aat gga 1440
   atc aaa gtg aac ttc aag acc cgc cac aac att gaa gat gga agc gtt 1488
   caa cta gca gac cat tat caa caa aat act cca att ggc gat ggc cct 1536
   gtc ctt tta cca gac aac cat tac ctg tcc aca caa tct gcc ctt tcg 1584
   aaa gat ccc aac gaa aag aga gac cac atg gtc ctt ctt gag ttt gta 1632
   aca gct gct ggg att aca cat ggc atg gat gaa ctg tac aac tag 1677
<210> 180
<211> 558
<212> PRT
<213> Artificial Sequence
<220>
<223> Description of Artificial Sequence: GFP-IKB
<400> 180

## Description

### Cross Reference

This application claims priority to U.S. Provisional Applications for Patent Serial Nos. 60/122,152 (February 26, 1999), 60/123,399 (March 8, 1999), 09/352,141, (July 12, 1999), 60/151,797 (August 31, 1999), 60/168,408 (December 1, 1999); and is a continuation in part of 09/430,656 (October 29, 1999); 09/398,965 filed September 17, 1999 which is a continuation in part of Serial No. 09/031,271 filed February 27, 1998 which is a continuation in part of U.S. Application S/N 08/810983, filed on February 27, 1997.

### Field of The Invention

This invention is in the field of fluorescence-based cell and molecular biochemical assays for drug discovery.

### Background of the Invention

Drug discovery, as currently practiced in the art, is a long, multiple step process involving identification of specific disease targets, development of an assay based on a specific target, validation of the assay, optimization and automation of the assay to produce a screen, high throughput screening of compound libraries using the assay to identify "hits", hit validation and hit compound optimization. The output of this process is a lead compound that goes into pre-clinical and, if validated, eventually into clinical trials. In this process, the screening phase is distinct from the assay development phases, and involves testing compound efficacy in living biological systems.

Historically, drug discovery is a slow and costly process, spanning numerous years and consuming hundreds of millions of dollars per drug created. Developments in the areas of genomics and high throughput screening have resulted in increased capacity and efficiency in the areas of target identification and volume of compounds

Historically, drug discovery is a slow and costly process, spanning numerous years and consuming hundreds of millions of dollars per drug created. Developments in the areas of genomics and high throughput screening have resulted in increased capacity and efficiency in the areas of target identification and volume of compounds screened. Significant advances in automated DNA sequencing, PCR application, positional cloning, hybridization arrays, and bioinformatics have greatly increased the number of genes (and gene fragments) encoding potential drug screening targets. However, the basic scheme for drug screening remians the same.

Validation of genomic targets as points for therapeutic intervention using the existing methods and protocols has become a bottleneck in the drug discovery process due to the slow, manual methods employed, such as *in vivo* functional models, functional analysis of recombinant proteins, and stable cell line expression of candidate genes. Primary DNA sequence data acquired through automated sequencing does not permit identification of gene function, but can provide information about common "motifs" and specific gene homology when compared to known sequence databases. Genomic methods such as subtraction hybridization and RADE (rapid amplification of differential expression) can be used to identify genes that are up or down regulated in a disease state model. However, identification and validation still proceed down the same pathway. Some proteomic methods use protein identification (global expression arrays, 2D electrophoresis, combinatorial libraries) in combination with reverse genetics to identify candidate genes of interest. Such putative "disease associated sequences" or DAS isolated as intact cDNA are a great advantage to these methods, but they are identified by the hundreds without providing any information regarding type, activity, and distribution of the encoded protein. Choosing a subset of DAS as drug screening targets is "random", and thus extremely inefficient, without functional data to provide a mechanistic link with disease. It is necessary, therefore, to provide new technologies to rapidly screen DAS to establish biological function, thereby improving target validation and candidate optimization in drug discovery.

There are three major avenues for improving early drug discovery productivity. First, there is a need for tools that provide increased information handling capability. Bioinformatics has blossomed with the rapid development of DNA sequencing systems and the evolution of the genomics database. Genomics is beginning to play a critical role in the identification of potential new targets. Proteomics has become indispensible in relating structure and function of protein targets in order to predict drug interactions. However, the next level of biological complexity is the cell. Therefore, there is a need to acquire, manage and search multi-dimensional information from cells. Secondly, there is a need for higher throughput tools. Automation is a key to improving productivity as has already been demonstrated in DNA sequencing and high throughput primary screening. The instant invention provides for automated systems that extract multiple parameter information from cells that meet the need for higher throughput tools. The instant invention also provides for miniaturizing the methods, thereby allowing increased throughput, while decreasing the volumes of reagents and test compounds required in each assay.

Radioactivity has been the dominant read-out in early drug discovery assays. However, the need for more information, higher throughput and miniaturization has caused a shift towards using fluorescence detection. Fluorescence-based reagents can yield more powerful, multiple parameter assays that are higher in throughput and information content and require lower volumes of reagents and test compounds. Fluorescence is also safer and less expensive than radioactivity-based methods.

Screening of cells treated with dyes and fluorescent reagents is well known in the art. There is a considerable body of literature related to genetic engineering of cells to produce fluorescent proteins, such as modified green fluorescent protein (GFP), as a reporter molecule. Some properties of wild-type GFP are disclosed by Morise et al. (*Biochemistry* 13 (1974), p. 2656-2662), and Ward et al. (*Photochem. Photobiol*. 31 (1980), p. 611-615). The GFP of the jellyfish *Aequorea victoria* has an excitation maximum at 395 nm and an emission maximum at 510 nm, and does not require an exogenous factor for fluorescence activity. Uses for GFP disclosed in the literature are widespread and include the study of gene expression and protein localization (Chalfie et al., *Science* 263 (1994), p. 12501-12504)), as a tool for visualizing subcellular organelles (Rizzuto et al., *Curr. Biology* 5 (1995), p. 635-642)), visualization of protein transport along the secretory pathway (Kaether and Gerdes, *FEBS Letters* 369 (1995), p. 267-271)), expression in plant cells (Hu and Cheng, *FEBS Letters* 369 (1995), p. 331-334)) and Drosophila embryos (Davis et al., *Dev. Biology* 170 (1995), p. 726-729)), and as a reporter molecule fused to another protein of interest (U. S. Patent 5,491,084). Similarly, WO96/23898 relates to methods of detecting biologically active substances affecting intracellular processes by utilizing a GFP construct having a protein kinase activation site. This patent, and all other patents referenced in this application are incorporated by reference in their entirety

Numerous references are related to GFP proteins in biological systems. For example, WO 96/09598 describes a system for isolating cells of interest utilizing the expression of a GFP like protein. WO 96/27675 describes the expression of GFP in plants. WO 95/21191 describes modified GFP protein expressed in transformed organisms to detect mutagenesis. U. S. Patents 5,401,629 and 5,436,128 describe assays and compositions for detecting and evaluating the intracellular transduction of an extracellular signal using recombinant cells that express cell surface receptors and contain reporter gene constructs that include transcriptional regulatory elements that are responsive to the activity of cell surface receptors.

Performing a screen on many thousands of compounds requires parallel handling and processing of many compounds and assay component reagents. Standard high throughput screens ("HTS") use mixtures of compounds and biological reagents along with some indicator compound loaded into arrays of wells in standard microtiter plates with 96 or 384 wells. The signal measured from each well, either fluorescence emission, optical density, or radioactivity, integrates the signal from all the material in the well giving an overall population average of all the molecules in the well.

Science Applications International Corporation (SAIC) 130 Fifth Avenue, Seattle, WA. 98109) describes an imaging plate reader. This system uses a CCD camera to image the whole area of a 96 well plate. The image is analyzed to calculate the total fluorescence per well for all the material in the well.

Molecular Devices, Inc. (Sunnyvale, CA) describes a system (FLIPR) which uses low angle laser scanning illumination and a mask to selectively excite fluorescence within approximately 200 microns of the bottoms of the wells in standard 96 well plates in order to reduce background when imaging cell monolayers. This system uses a CCD camera to image the whole area of the plate bottom. Although this system measures signals originating from a cell monolayer at the bottom of the well, the signal measured is averaged over the area of the well and is therefore still considered a measurement of the average response of a population of cells. The image is analyzed to calculate the total fluorescence per well for cell-based assays. Fluid delivery devices have also been incorporated into cell based screening systems, such as the FLIPR system, in order to initiate a response, which is then observed as a whole well population average response using a macro-imaging system.

In contrast to high throughput screens, various high-content screens ("HCS") have been developed to address the need for more detailed information about the temporal-spatial dynamics of cell constituents and processes. High-content screens automate the extraction of multicolor fluorescence information derived from specific fluorescence-based reagents incorporated into cells (Giuliano and Taylor (1995), *Curr. Op. Cell Biol.* 7:4; Giuliano et al. (1995) *Ann. Rev. Biophys. Biomol. Struct.* 24:405). Cells are analyzed using an optical system that can measure spatial, as well as temporal dynamics. (Farkas et al. (1993) *Ann. Rev. Physiol.* 55:785; Giuliano et al. (1990) In *Optical Microscopy for Biology.* B. Herman and K. Jacobson (eds.), pp. 543-557. Wiley-Liss, New York; Hahn et al (1992) *Nature* 359:736; Waggoner et al. (1996) *Hum. Pathol.* 27:494). The concept is to treat each cell as a "well" that has spatial and temporal information on the activities of the labeled constituents.

The types of biochemical and molecular information now accessible through fluorescence-based reagents applied to cells include ion concentrations, membrane potential, specific translocations, enzyme activities, gene expression, as well as the presence, amounts and patterns of metabolites, proteins, lipids, carbohydrates, and nucleic acid sequences (DeBiasio et al., (1996) *Mol. Biol. Cell.* 7:1259;Giuliano et al., (1995) *Ann. Rev. Biophys. Biomol. Struct.* 24:405; Heim and Tsien, (1996) *Curr. Biol.* 6:178).

High-content screens can be performed on either fixed cells, using fluorescently labeled antibodies, biological ligands, and/or nucleic acid hybridization probes, or live cells using multicolor fluorescent indicators and "biosensors." The choice of fixed or live cell screens depends on the specific cell-based assay required.

Fixed cell assays are the simplest, since an array of initially living cells in a microtiter plate format can be treated with various compounds and doses being tested, then the cells can be fixed, labeled with specific reagents, and measured. No environmental control of the cells is required after fixation. Spatial information is acquired, but only at one time point. The availability of thousands of antibodies, ligands and nucleic acid hybridization probes that can be applied to cells makes this an attractive approach for many types of cell-based screens. The fixation and labeling steps can be automated, allowing efficient processing of assays.

Live cell assays are more sophisticated and powerful, since an array of living cells containing the desired reagents can be screened over time, as well as space. Environmental control of the cells (temperature, humidity, and carbon dioxide) is required during measurement, since the physiological health of the cells must be maintained for multiple fluorescence measurements over time. There is a growing list of fluorescent physiological indicators and "biosensors" that can report changes in biochemical and molecular activities within cells (Giuliano et al., (1995) *Ann. Rev. Biophys. Biomol. Struct*. 24:405; Hahn et al., (1993) In *Fluorescent and Luminescent Probes for Biological Activity.* W.T. Mason, (ed.), pp. 349-359, Academic Press, San Diego).

The availability and use of fluorescence-based reagents has helped to advance the development of both fixed and live cell high-content screens. Advances in instrumentation to automatically extract multicolor, high-content information has recently made it possible to develop HCS into an automated tool. An article by Taylor, et al. (*American Scientist* 80 (1992), p. 322-335) describes many of these methods and their applications. For example, Proffitt et. al. *(Cytometry* 24: 204-213 (1996)) describe a semi-automated fluorescence digital imaging system for quantifying relative cell numbers in situ in a variety of tissue culture plate formats, especially 96-well microtiter plates. The system consists of an epifluorescence inverted microscope with a motorized stage, video camera, image intensifier, and a microcomputer with a PC-Vision digitizer. Turbo Pascal software controls the stage and scans the plate taking multiple images per well. The software calculates total fluorescence per well, provides for daily calibration, and configures easily for a variety of tissue culture plate formats. Thresholding of digital images and reagents which fluoresce only when taken up by living cells are used to reduce background fluorescence without removing excess fluorescent reagent.

Scanning confocal microscope imaging (Go et al., (1997) *Analytical Biochemistry* 247:210-215; Goldman et al., (1995) *Experimental Cell Research* 221:311-319) and multiphoton microscope imaging (Denk et al., (1990) *Science* 248:73; Gratton et al., (1994) *Proc. of the Microscopical Society of America*, pp. 154-155) are also well established methods for acquiring high resolution images of microscopic samples. The principle advantage of these optical systems is the very shallow depth of focus, which allows features of limited axial extent to be resolved against the background. For example, it is possible to resolve internal cytoplasmic features of adherent cells from the features on the cell surface. Because scanning multiphoton imaging requires very short duration pulsed laser systems to achieve the high photon flux required, fluorescence lifetimes can also be measured in these systems (Lakowicz et al., (1992) *Anal. Biochem.* 202:316-330; Gerrittsen et al. (1997), *J. of Fluorescence* 7:11-15)), providing additional capability for different detection modes. Small, reliable and relatively inexpensive laser systems, such as laser diode pumped lasers, are now available to allow multiphoton confocal microscopy to be applied in a fairly routine fashion.

A combination of the biological heterogeneity of cells in populations (Bright, et al., (1989). *J. Cell. Physiol.* 141:410; Giuliano, (1996) *Cell Motil. Cytoskel.* 35:237)) as well as the high spatial and temporal frequency of chemical and molecular information present within cells, makes it impossible to extract high-content information from populations of cells using existing whole microtiter plate readers. No existing high-content screening platform has been designed for multicolor, fluorescence-based screens using cells that are analyzed individually. Similarly, no method is currently available that combines automated fluid delivery to arrays of cells for the purpose of systematically screening compounds for the ability to induce a cellular response that is identified by HCS analysis, especially from cells grown in microtiter plates. Furthermore, no method exists in the art combining high throughput well-by-well measurements to identify "hits" in one assay followed by a second high content cell-by-cell measurement on the same plate of only those wells identified as hits.

The instant invention provides systems, methods, and screens that combine high throughput screening (HTS) and high content screening (HCS) that significantly improve target validation and candidate optimization by combining many cell screening formats with fluorescence-based molecular reagents and computer-based feature extraction, data analysis, and automation, resulting in increased quantity and speed of data collection, shortened cycle times, and, ultimately, faster evaluation of promising drug candidates. The instant invention also provides for miniaturizing the methods, thereby allowing increased throughput, while decreasing the volumes of reagents and test compounds required in each assay.

### SUMMARY OF THE INVENTION

In one aspect, the present invention relates to a method for analyzing cells comprising providing cells containing fluorescent reporter molecules in an array of locations, treating the cells in the array of locations with one or more reagents, imaging numerous cells in each location with fluorescence optics, converting the optical information into digital data, utilizing the digital data to determine the distribution, environment or activity of the fluorescently labeled reporter molecules in the cells and the distribution of the cells, and interpreting that information in terms of a positive, negative or null effect of the compound being tested on the biological function

In this embodiment, the method rapidly determines the distribution, environment, or activity of fluorescently labeled reporter molecules in cells for the purpose of screening large numbers of compounds for those that specifically affect particular biological functions. The array of locations may be a microtiter plate or a microchip which is a microplate having cells in an array of locations. In a preferred embodiment, the method includes computerized means for acquiring, processing, displaying and storing the data received. In a preferred embodiment, the method further comprises automated fluid delivery to the arrays of cells. In another preferred embodiment, the information obtained from high throughput measurements on the same plate are used to selectively perform high content screening on only a subset of the cell locations on the plate.

In another aspect of the present invention, a cell screening system is provided that comprises:
- a high magnification fluorescence optical system having a microscope objective,
- an XY stage adapted for holding a plate containing an array of cells and having a means for moving the plate for proper alignment and focusing on the cell arrays;
- a digital camera;
- a light source having optical means for directing excitation light to cell arrays and a means for directing fluorescent light emitted from the cells to the digital camera; and
- a computer means for receiving and processing digital data from the digital camera wherein the computer means includes a digital frame grabber for receiving the images from the camera, a display for user interaction and display of assay results, digital storage media for data storage and archiving, and a means for control, acquisition, processing and display of results.

In a preferred embodiment, the cell screening system further comprises a computer screen operatively associated with the computer for displaying data. In another preferred embodiment, the computer means for receiving and processing digital data from the digital camera stores the data in a bioinformatics data base. In a further preferred embodiment, the cell screening system further comprises a reader that measures a signal from many or all the wells in parallel. In another preferred embodiment, the cell screening system further comprises a mechanical-optical means for changing the magnification of the system, to allow changing modes between high throughput and high content screening. In another preferred embodiment, the cell screening system further comprises a chamber and control system to maintain the temperature, CO₂ concentration and humidity surrounding the plate at levels required to keep cells alive. In a further preferred embodiment, the cell screening system utilizes a confocal scanning illumination and detection system.

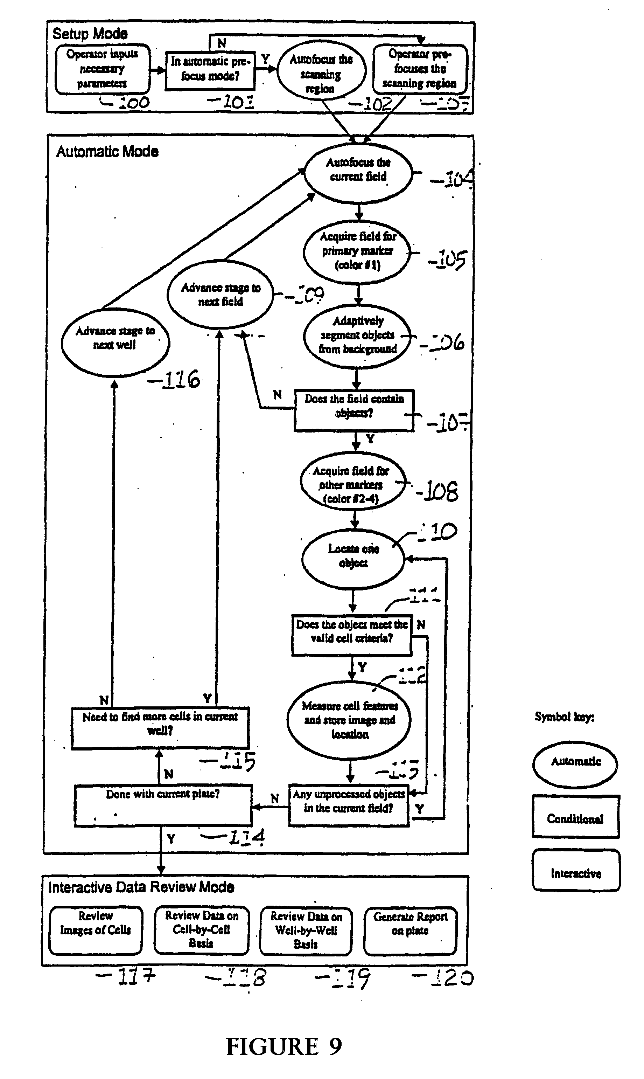

In another aspect of the present invention, a machine readable storage medium comprising a program containing a set of instructions for causing a cell screening system to execute procedures for defining the distribution and activity of specific cellular constituents and processes is provided. In a preferred embodiment, the cell screening system comprises a high magnification fluorescence optical system with a stage adapted for holding cells and a means for moving the stage, a digital camera, a light source for receiving and processing the digital data from the digital camera, and a computer means for receiving and processing the digital data from the digital camera. Preferred embodiments of the machine readable storage medium comprise programs consisting of a set of instructions for causing a cell screening system to execute the procedures set forth in Figures 9, 11, 12, 13, 14 or 15. Another preferred embodiment comprises a program consisting of a set of instructions for causing a cell screening system to execute procedures for detecting the distribution and activity of specific cellular constituents and processes. In most preferred embodiments, the cellular processes include, but are not limited to, nuclear translocation of a protein, cellular hypertrophy, apoptosis, and protease-induced translocation of a protein.

In another preferred embodiment, a variety of automated cell screening methods are provided, including screens to identify compounds that affect transcription factor activity, protein kinase activity, cell morphology, microtubule structure, apoptosis, receptor internalization, and protease-induced translocation of a protein.

In another aspect, the present invention provides recombinant nucleic acids encoding a protease biosensor, comprising:
a. a first nucleic acid sequence that encodes at least one detectable polypeptide signal;
b. a second nucleic acid sequence that encodes at least one protease recognition site, wherein the second nucleic acid sequence is operatively linked to the first nucleic acid sequence that encodes the at least one detectable polypeptide signal; and
c. a third nucleic acid sequence that encodes at least one reactant target sequence, wherein the third nucleic acid sequence is operatively linked to the second nucleic acid sequence that encodes the at least one protease recognition site.

The present invention also provides the recombinant expression vectors capable of expressing the recombinant nucleic acids encoding protease biosensors, as well as genetically modified host cells that are transfected with the expression vectors.

The invention further provides recombinant protease biosensors, comprising
a. a first domain comprising at least one detectable polypeptide signal;
b. a second domain comprising at least one protease recognition site; and
c. a third domain comprising at least one reactant target sequence;
wherein the first domain and the third domain are separated by the second domain.

In a further aspect, the present invention involves assays and reagents for characterizing a sample for the presence of a toxin. The method comprises the use of detector, classifier, and identifier classes of toxin biosensors to provide for various levels of toxin characterization.

### BRIEF DESCRIPTION OF THE DRAWINGS

**Figure 1** shows a diagram of the components of the cell-based scanning system.
**Figure 2** shows a schematic of the microscope subassembly.
**Figure 3** shows the camera subassembly.
**Figure 4** illustrates cell scanning system process.
**Figure 5** illustrates a user interface showing major functions to guide the user.
**Figure 6** is a block diagram of the two platform architecture of the Dual Mode System for Cell Based Screening in which one platform uses a telescope lens to read all wells of a microtiter plate and a second platform that uses a higher magnification lens to read individual cells in a well.
**Figure 7** is a detail of an optical system for a single platform architecture of the Dual Mode System for Cell Based Screening that uses a moveable 'telescope' lens to read all wells of a microtiter plate and a moveable higher magnification lens to read individual cells in a well.
**Figure 8** is an illustration of the fluid delivery system for acquiring kinetic data on the Cell Based Screening System.
**Figure 9** is a flow chart of processing step for the cell-based scanning system.
**Figure 10 A-J** illustrates the strategy of the Nuclear Translocation Assay.
**Figure 11** is a flow chart defining the processing steps in the Dual Mode System for Cell Based Screening combining high throughput and high content screening of microtiter plates.
**Figure 12** is a flow chart defining the processing steps in the High Throughput mode of the System for Cell Based Screening.
**Figure 13** is a flow chart defining the processing steps in the High Content mode of the System for Cell Based Screening.
**Figure 14** is a flow chart defining the processing steps required for acquiring kinetic data in the High Content mode of the System for Cell Based Screening.
**Figure 15** is a flow chart defining the processing steps performed within a well during the acquisition of kinetic data.
**Figure 16** is an example of data from a known inhibitor of translocation.
**Figure 17** is an example of data from a known stimulator of translocation.
**Figure 18** illustrates data presentation on a graphical display.
**Figure 19** is an illustration of the data from the High Throughput mode of the System for Cell Based Screening, an example of the data passed to the High Content mode, the data acquired in the high content mode, and the results of the analysis of that data,
**Figure 20** shows the measurement of a drug-induced cytoplasm to nuclear translocation.
**Figure 21** illustrates a graphical user interface of the measurement shown in Figure 20.
**Figure 22** illustrates a graphical user interface, with data presentation, of the measurement shown in Fig. 20.
**Figure 23** is a graph representing the kinetic data obtained from the measurements depicted in Fig. 20.
**Figure 24** details a high-content screen of drug-induced apoptosis.
**Figure 25.** Graphs depicting changes in morphology upon induction of apoptosis. Staurosporine (A) and paclitaxel (B) induce classic nuclear fragmentation in L929 cells. BHK cells exhibit concentration dependent changes in response to staurosporine (C), but a more classical response to paclitaxel (D). MCF-7 cells exhibit either nuclear condensation (E) or fragmentation (F) in response to staurosporine and paclitaxel, respectively. In all cases, cells were exposed to the compounds for 30 hours.
**Figure 26** illustrates the dose response of cells to staurosporine in terms of both nuclear size and nuclear perimeter convolution.

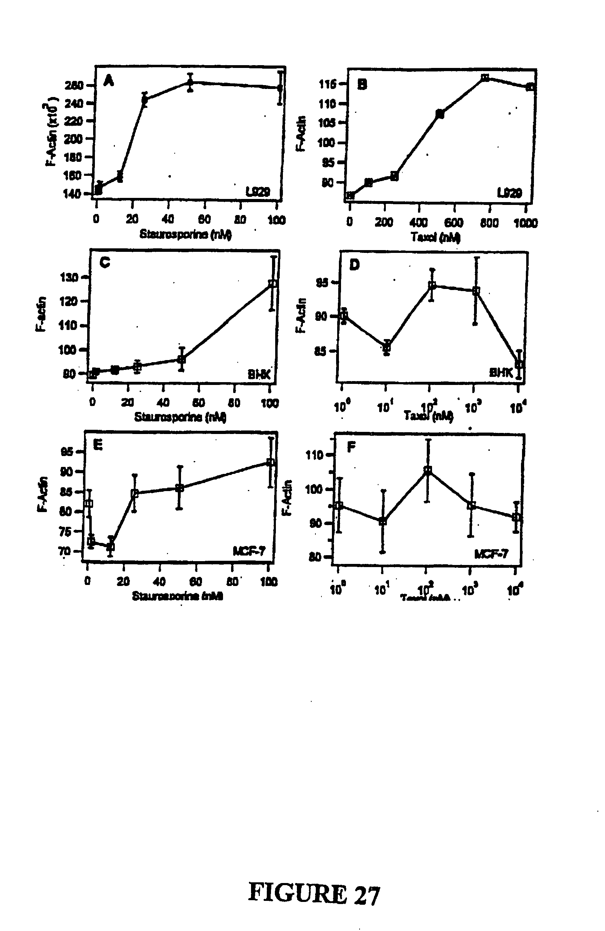

**Figure 27.** Graphs depicting induction of apoptosis by staurosporine and paclitaxel leading to changes in peri-nuclear f-actin content. (A, B) Both apoptotic stimulators induce dose-dependent increases in f-actin content in L929 cells. (C) In BHK cells, staurosporine induces a dose-dependent increase in f-actin, whereas paclitaxel (D) produces results that are more variable. (E) MCF-7 cells exhibit either a decrease or increase depending on the concentration of staurosporine. (F) Paclitaxel induced changes in f-actin content were highly variable and not significant. Cells were exposed to the compounds for 30 hours.
**Figure 28.** Graphs depicting mitochondrial changes in response to induction of apoptosis. L929 (A,B) and BHK (C,D) cells responded to both staurosporine (A,C) and paclitaxel (B,D) with increases in mitochondrial mass. MCF-7 cells exhibit either a decrease in membrane potential (E, staurosporine) or an increase in mitochondrial mass (F, paclitaxel) depending on the stimulus. Cells were exposed to the compounds for 30 hours. 28G is a graph showing the simultaneous measurement of staurosporine effects on mitochondrial mass and mitochondrial potential in BHK cells.
**Figure 29** shows the nucleic acid and amino acid sequence for various types of protesae biosensor domains. (A) Signal sequences. (B) Protease recognition sites. (C) Product/Reactant target sequences
**Figure 30** shows schematically shows some basic organization of domains in the protease biosensors of the invention.
**Figure 31** is a schematic diagram of a specific 3-domain protease biosensor.
**Figure 32** is a photograph showing the effect of stimulation of apoptosis by cis-platin on BHK cells transfected with an expression vector that expresses the caspase biosensor shown in Figure 32.
**Figure 33** is a schematic diagram of a specific 4-domain protease biosensor.
**Figure 34** is a schematic diagram of a specific 4-domain protease biosensor, containing a nucleolar localization signal.
**Figure 35** is a schematic diagram of a specific 5-domain protease biosensor.
**Figure 36** shows the differential response in a dual labeling assay of the p38 MAPK and NF-κB pathways across three model toxins and two different cell types. Treatments marked with an asterisk are different from controls at a 99% confidence level (p < 0.01).

### DETAILED DESCRIPTION OF THE INVENTION

All cited patents, patent applications and other references are hereby incorporated by reference in their entirety.

As used herein, the following terms have the specified meaning:

*Markers of cellular domains.* Luminescent probes that have high affinity for specific cellular constituents including specific organelles or molecules. These probes can either be small luminescent molecules or fluorescently tagged macromolecules used as "labeling reagents", "environmental indicators", or "biosensors."

*Labeling reagents.* Labeling reagents include, but are not limited to, luminescently labeled macromolecules including fluorescent protein analogs and biosensors, luminescent macromolecular chimeras including those formed with the green fluorescent protein and mutants thereof, luminescently labeled primary or secondary antibodies that react with cellular antigens involved in a physiological response, luminescent stains, dyes, and other small molecules.

*Markers of cellular translocations.* Luminescently tagged macromolecules or organelles that move from one cell domain to another during some cellular process or physiological response. Translocation markers can either simply report location relative to the markers of cellular domains or they can also be "biosensors" that report some biochemical or molecular activity as well.

*Biosensors.* Macromolecules consisting of a biological functional domain and a luminescent probe or probes that report the environmental changes that occur either internally or on their surface. A class of luminescently labeled macromolecules designed to sense and report these changes have been termed "fluorescent-protein biosensors". The protein component of the biosensor provides a highly evolved molecular recognition moiety. A fluorescent molecule attached to the protein component in the proximity of an active site transduces environmental changes into fluorescence signals that are detected using a system with an appropriate temporal and spatial resolution such as the cell scanning system of the present invention. Because the modulation of native protein activity within the living cell is reversible, and because fluorescent-protein biosensors can be designed to sense reversible changes in protein activity, these biosensors are essentially reusable.

*Disease associated sequences ("DAS").* This term refers to nucleic acid sequences identified by standard techniques, such as primary DNA sequence data, genomic methods such as subtraction hybridization and RADE, and proteomic methods in combination with reverse genetics, as being of drug candidate compounds. The term does not mean that the sequence is only associated with a disease state.

High content screening (HCS) can be used to measure the effects of drugs on complex molecular events such as signal transduction pathways, as well as cell functions including, but not limited to, apoptosis, cell division, cell adhesion, locomotion, exocytosis, and cell-cell communication. Multicolor fluorescence permits multiple targets and cell processes to be assayed in a single screen. Cross-correlation of cellular responses will yield a wealth of information required for target validation and lead optimization.

In one aspect of the present invention, a cell screening system is provided comprising a high magnification fluorescence optical system having a microscope objective, an XY stage adapted for holding a plate with an array of locations for holding cells and having a means for moving the plate to align the locations with the microscope objective and a means for moving the plate in the direction to effect focusing; a digital camera; a light source having optical means for directing excitation light to cells in the array of locations and a means for directing fluorescent light emitted from the cells to the digital camera; and a computer means for receiving and processing digital data from the digital camera wherein the computer means includes: a digital frame grabber for receiving the images from the camera, a display for user interaction and display of assay results, digital storage media for data storage and archiving, and means for control, acquisition, processing and display of results.

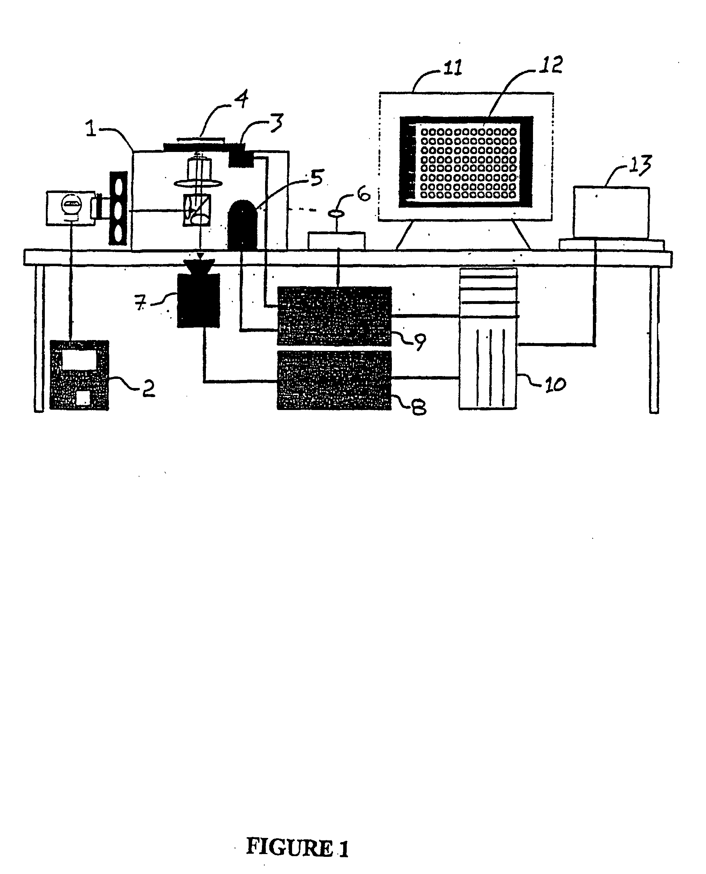

Figure 1 is a schematic diagram of a preferred embodiment of the cell scanning system. An inverted fluorescence microscope is used 1, such as a Zeiss Axiovert inverted fluorescence microscope which uses standard objectives with magnification of 1-100x to the camera, and a white light source (e.g. 100W mercury-arc lamp or 75W xenon lamp) with power supply 2. There is an XY stage 3 to move the plate 4 in the XY direction over the microscope objective. A Z-axis focus drive 5 moves the objective in the Z direction for focusing. A joystick 6 provides for manual movement of the stage in the XYZ direction. A high resolution digital camera 7 acquires images from each well or location on the plate. There is a camera power supply 8, an automation controller 9 and a central processing unit 10. The PC 11 provides a display 12 and has associated software. The printer 13 provides for printing of a hard copy record.

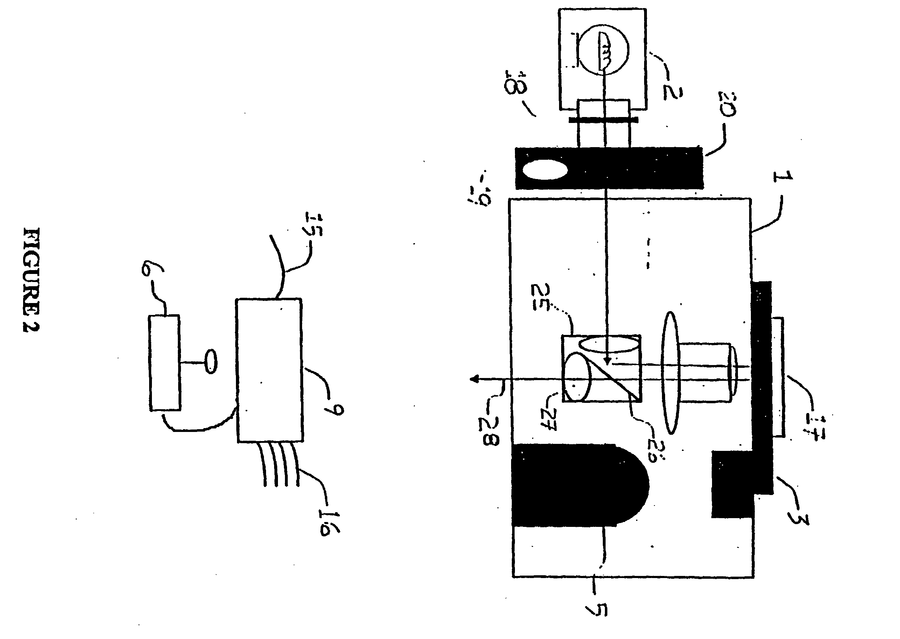

Figure 2 is a schematic of one embodiment of the microscope assembly 1 of the invention, showing in more detail the XY stage 3, Z-axis focus drive 5, joystick 6, light source 2, and automation controller 9. Cables to the computer 15 and microscope 16, respectively, are provided. In addition, Figure 2 shows a 96 well microtiter plate 17 which is moved on the XY stage 3 in the XY direction. Light from the light source 2 passes through the PC controlled shutter 18 to a motorized filter wheel 19 with excitation filters 20. The light passes into filter cube 25 which has a dichroic mirror 26 and an emission filter 27. Excitation light reflects off the dichroic mirror to the wells in the microtiter plate 17 and fluorescent light 28 passes through the dichroic mirror 26 and the emission filter 27 and to the digital camera 7.

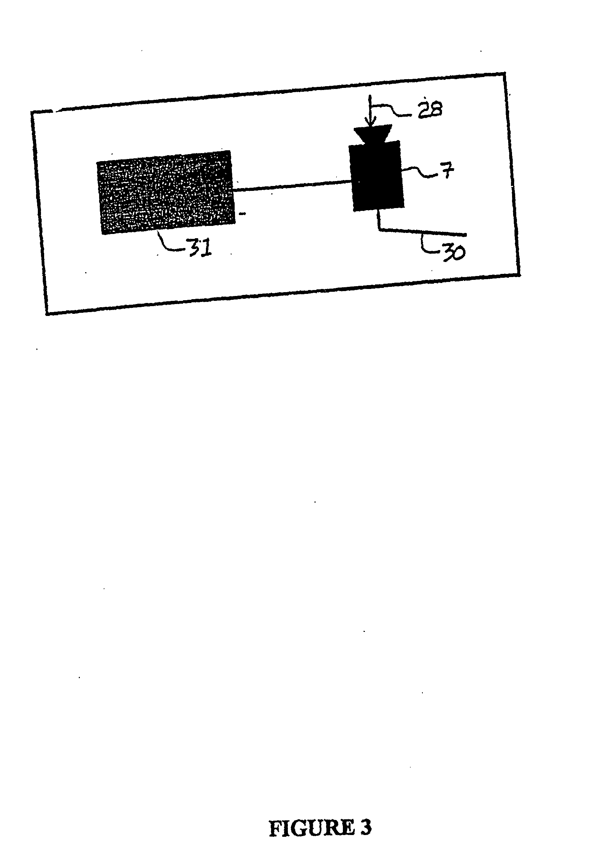

Figure 3 shows a schematic drawing of a preferred camera assembly. The digital camera 7, which contains an automatic shutter for exposure control and a power supply 31, receives fluorescent light 28 from the microscope assembly. A digital cable 30 transports digital signals to the computer.

The standard optical configurations described above use microscope optics to directly produce an enlarged image of the specimen on the camera sensor in order to capture a high resolution image of the specimen. This optical system is commonly referred to as 'wide field' microscopy. Those skilled in the art of microscopy will recognize that a high resolution image of the specimen can be created by a variety of other optical systems, including, but not limited to, standard scanning confocal detection of a focused point or line of illumination scanned over the specimen (Go et al. 1997, *supra*), and multi-photon scanning confocal microscopy (Denk et al., 1990, *supra*), both of which can form images on a CCD detector or by synchronous digitization of the analog output of a photomultiplier tube.

In screening applications, it is often necessary to use a particular cell line, or primary cell culture, to take advantage of particular features of those cells. Those skilled in the art of cell culture will recognize that some cell lines are contact inhibited, meaning that they will stop growing when they become surrounded by other cells, while other cell lines will continue to grow under those conditions and the cells will literally pile up, forming many layers. An example of such a cell line is the HEK 293 (ATCC CRL-1573) line. An optical system that can acquire images of single cell layers in multilayer preparations is required for use with cell lines that tend to form layers. The large depth of field of wide field microscopes produces an image that is a projection through the many layers of cells, making analysis of subcellular spatial distributions extremely difficult in layer-forming cells. Alternatively, the very shallow depth of field that can be achieved on a confocal microscope, (about one micron), allows discrimination of a single cell layer at high resolution, simplifying the determination of the subcellular spatial distribution. Similarly, confocal imaging is preferable when detection modes such as fluorescence lifetime imaging are required.

The output of a standard confocal imaging attachment for a microscope is a digital image that can be converted to the same format as the images produced by the other cell screening system embodiments described above, and can therefore be processed in exactly the same way as those images. The overall control, acquisition and analysis in this embodiment is essentially the same. The optical configuration of the confocal microscope system, is essentially the same as that described above, except for the illuminator and detectors. Illumination and detection systems required for confocal microscopy have been designed as accessories to be attached to standard microscope optical systems such as that of the present invention (Zeiss, Germany). These alternative optical systems therefore can be easily integrated into the system as described above.

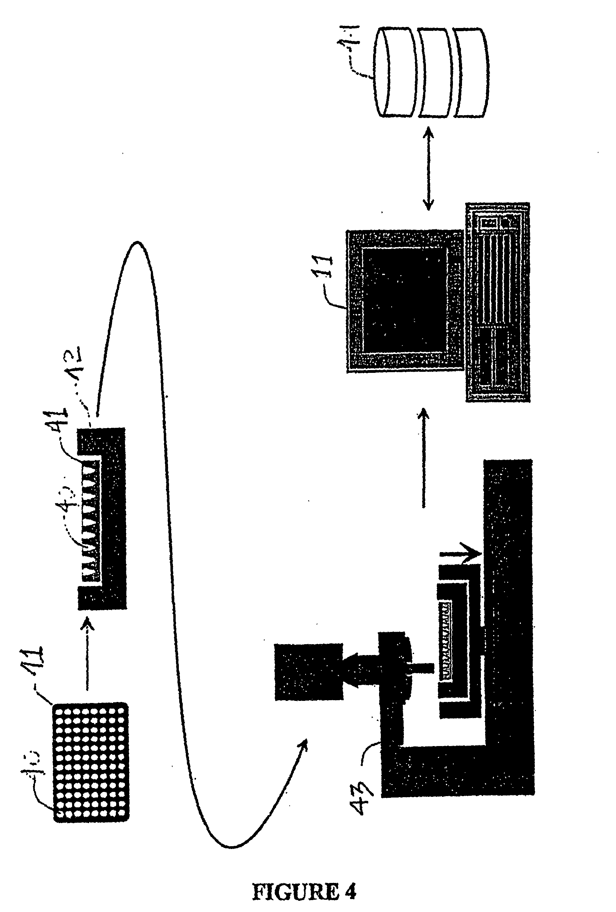

Figure 4 illustrates an alternative embodiment of the invention in which cell arrays are in microwells 40 on a microplate 41, described ion co-pending U.S. Application S/N 08/865,341, incorporated by reference herein in its entirety. Typically the microplate is 20 mm by 30 mm as compared to a standard 96 well microtiter plate which is 86 mm by 129 mm. The higher density array of cells on a microplate allows the microplate to be imaged at a low resolution of a few microns per pixel for high throughput and particular locations on the microplate to be imaged at a higher resolution of less than 0.5 microns per pixel. These two resolution modes help to improve the overall throughput of the system.

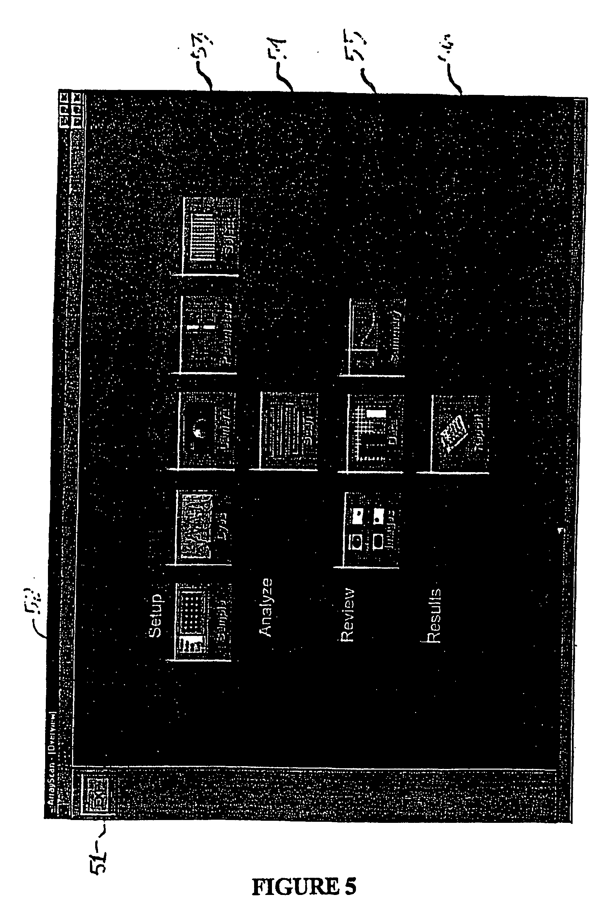

The microplate chamber 42 serves as a microfluidic delivery system for the addition of compounds to cells. The microplate 41 in the microplate chamber 42 is placed in an XY microplate reader 43. Digital data is processed as described above. The small size of this microplate system increases throughput, minimizes reagent volume and allows control of the distribution and placement of cells for fast and precise cell-based analysis. Processed data can be displayed on a PC screen 11 and made part of a bioinformatics data base 44. This data base not only permits storage and retrieval of data obtained through the methods of this invention, but also permits acquisition and storage of external data relating to cells. Figure 5 is a PC display which illustrates the operation of the software.

In an alternative embodiment, a high throughput system (HTS) is directly coupled with the HCS either on the same platform or on two separate platforms connected electronically (e.g. via a local area network). This embodiment of the invention, referred to as a dual mode optical system, has the advantage of increasing the throughput of a HCS by coupling it with a HTS and thereby requiring slower high resolution data acquisition and analysis only on the small subset of wells that show a response in the coupled HTS.

High throughput 'whole plate' reader systems are well known in the art and are commonly used as a component of an HTS system used to screen large numbers of compounds (Beggs (1997), *J. of Biomolec. Screening* 2:71-78; Macaffrey et al., (1996) *J. Biomolec. Screening* 1:187-190).

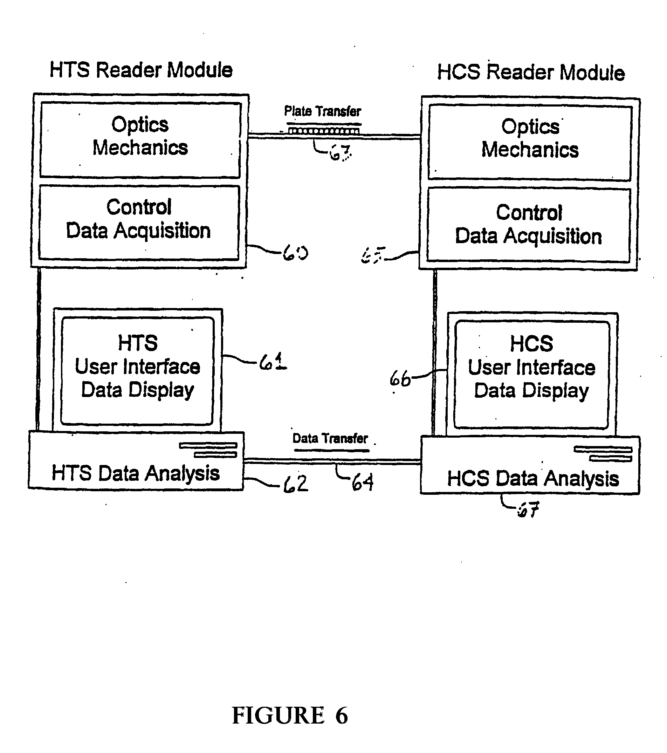

In one embodiment of dual mode cell based screening, a two platform architecture in which high throughput acquisition occurs on one platform and high content acquisition occurs on a second platform is provided (Figure 6). Processing occurs on each platform independently, with results passed over a network interface, or a single controller is used to process the data from both platforms.

As illustrated in Figure 6, an exemplified two platform dual mode optical system consists of two light optical instruments, a high throughput platform 60 and a high content platform 65, which read fluorescent signals emitted from cells cultured in microtiter plates or microwell arrays on a microplate, and communicate with each other via an electronic connection 64. The high throughput platform 60 analyzes all the wells in the whole plate either in parallel or rapid serial fashion. Those skilled in the art of screening will recognize that there are a many such commercially available high throughput reader systems that could be integrated into a dual mode cell based screening system (Topcount (Packard Instruments, Meriden, CT); Spectramax, Lumiskan (Molecular Devices, Sunnyvale, CA); Fluoroscan (Labsystems, Beverly, MA)). The high content platform 65, as described above, scans from well to well and acquires and analyzes high resolution image data collected from individual cells within a well.

The HTS software, residing on the system's computer 62, controls the high throughput instrument, and results are displayed on the monitor 61. The HCS software, residing on it's computer system 67, controls the high content instrument hardware 65, optional devices (e.g. plate loader, environmental chamber, fluid dispenser), analyzes digital image data from the plate, displays results on the monitor 66 and manages data measured in an integrated database. The two systems can also share a single computer, in which case all data would be collected, processed and displayed on that computer, without the need for a local area network to transfer the data. Microtiter plates are transferred from the high throughput system to the high content system 63 either manually or by a robotic plate transfer device, as is well known in the art (Beggs (1997), *supra;* Mcaffrey (1996), *supra).*

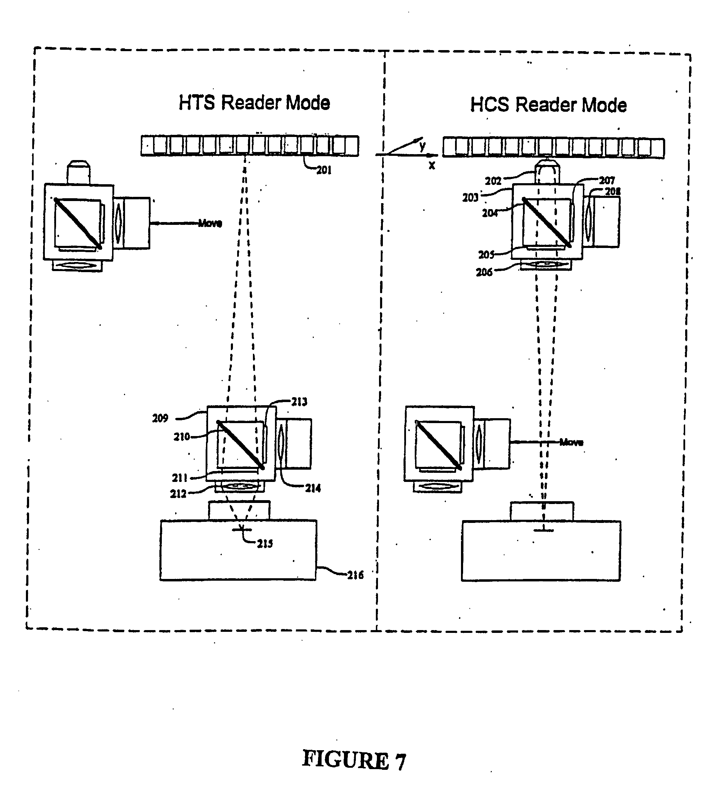

In a preferred embodiment, the dual mode optical system utilizes a single platform system (Figure 7). It consists of two separate optical modules, an HCS module 203 and an HTS module 209 that can be independently or collectively moved so that only one at a time is used to collect data from the microtiter plate 201. The microtiter plate 201 is mounted in a motorized X,Y stage so it can be positioned for imaging in either HTS or HCS mode. After collecting and analyzing the HTS image data as described below, the HTS optical module 209 is moved out of the optical path and the HCS optical module 203 is moved into place.

The optical module for HTS 209 consists of a projection lens 214, excitation wavelength filter 213 and dichroic mirror 210 which are used to illuminate the whole bottom of the plate with a specific wavelength band from a conventional microscope lamp system (not illustrated). The fluorescence emission is collected through the dichroic mirror 210 and emission wavelength filter 211 by a lens 212 which forms an image on the camera 216 with sensor 215.

The optical module for HCS 203 consists of a projection lens 208, excitation wavelength filter 207 and dichroic mirror 204 which are used to illuminate the back aperture of the microscope objective 202, and thereby the field of that objective, from a standard microscope illumination system (not shown). The fluorescence emission is collected by the microscope objective 202, passes through the dichroic mirror 204 and emission wavelength filter 205 and is focused by a tube lens 206 which forms an image on the same camera 216 with sensor 215.

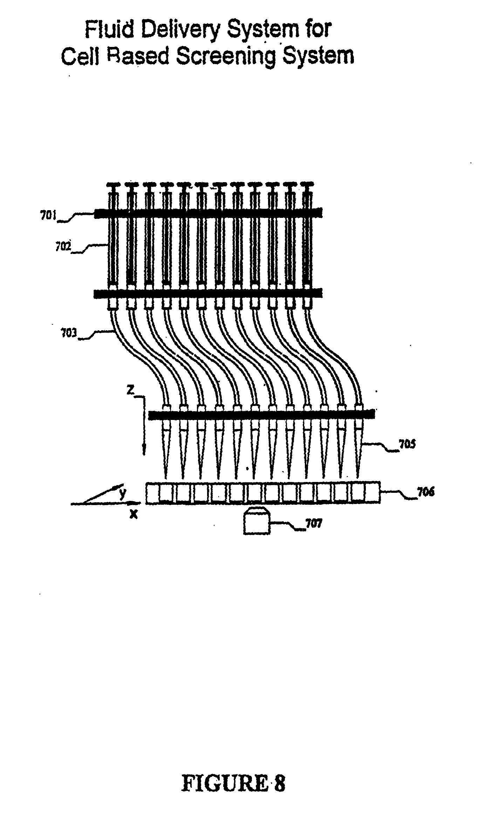

In an alternative embodiment of the present invention, the cell screening system further comprises a fluid delivery device for use with the live cell embodiment of the method of cell screening (see below). Figure 8 exemplifies a fluid delivery device for use with the system of the invention. It consists of a bank of 12 syringe pumps 701 driven by a single motor drive. Each syringe 702 is sized according to the volume to be delivered to each well, typically between 1 and 100 µL. Each syringe is attached via flexible tubing 703 to a similar bank of connectors which accept standard pipette tips 705. The bank of pipette tips are attached to a drive system so they can be lowered and raised relative to the microtiter plate 706 to deliver fluid to each well. The plate is mounted on an X,Y stage, allowing movement relative to the optical system 707 for data collection purposes. This set-up allows one set of pipette tips, or even a single pipette tip, to deliver reagent to all the wells on the plate. The bank of syringe pumps can be used to deliver fluid to 12 wells simultaneously, or to fewer wells by removing some of the tips.

In another aspect, the present invention provides a method for analyzing cells comprising providing an array of locations which contain multiple cells wherein the cells contain one or more fluorescent reporter molecules; scanning multiple cells in each of the locations containing cells to obtain fluorescent signals from the fluorescent reporter molecule in the cells; converting the fluorescent signals into digital data; and utilizing the digital data to determine the distribution, environment or activity of the fluorescent reporter molecule within the cells.

### Cell Arrays

Screening large numbers of compounds for activity with respect to a particular biological function requires preparing arrays of cells for parallel handling of cells and reagents. Standard 96 well microtiter plates which are 86 mm by 129 mm, with 6mm diameter wells on a 9mm pitch, are used for compatibility with current automated loading and robotic handling systems. The microplate is typically 20 mm by 30 mm, with cell locations that are 100-200 microns in dimension on a pitch of about 500 microns. Methods for making microplates are described in U.S. Patent Application Serial No. 08/865,341, incorporated by reference herein in its entirety. Microplates may consist of coplanar layers of materials to which cells adhere, patterned with materials to which cells will not adhere, or etched 3-dimensional surfaces of similarly pattered materials. For the purpose of the following discussion, the terms 'well' and 'microwell' refer to a location in an array of any construction to which cells adhere and within which the cells are imaged. Microplates may also include fluid delivery channels in the spaces between the wells. The smaller format of a microplate increases the overall efficiency of the system by minimizing the quantities of the reagents, storage and handling during preparation and the overall movement required for the scanning operation. In addition, the whole area of the microplate can be imaged more efficiently, allowing a second mode of operation for the microplate reader as described later in this document.

### Fluorescence Reporter Molecules

A major component of the new drug discovery paradigm is a continually growing family of fluorescent and luminescent reagents that are used to measure the temporal and spatial distribution, content, and activity of intracellular ions, metabolites, macromolecules, and organelles. Classes of these reagents include labeling reagents that measure the distribution and amount of molecules in living and fixed cells, environmental indicators to report signal transduction events in time and space, and fluorescent protein biosensors to measure target molecular activities within living cells. A multiparameter approach that combines several reagents in a single cell is a powerful new tool for drug discovery.

The method of the present invention is based on the high affinity of fluorescent or luminescent molecules for specific cellular components. The affinity for specific components is governed by physical forces such as ionic interactions, covalent bonding (which includes chimeric fusion with protein-based chromophores, fluorophores, and lumiphores), as well as hydrophobic interactions, electrical potential, and, in some cases, simple entrapment within a cellular component. The luminescent probes can be small molecules, labeled macromolecules, or genetically engineered proteins, including, but not limited to green fluorescent protein chimeras.

Those skilled in this art will recognize a wide variety of fluorescent reporter molecules that can be used in the present invention, including, but not limited to, fluorescently labeled biomolecules such as proteins, phospholipids and DNA hybridizing probes. Similarly, fluorescent reagents specifically synthesized with particular chemical properties of binding or association have been used as fluorescent reporter molecules (Barak et al., (1997), *J. Biol. Chem.* 272:27497-27500; Southwick et al., (1990), *Cytometry* 11:418-430; Tsien (1989) in *Methods in Cell Biology,* Vol. 29 Taylor and Wang (eds.), pp. 127-156). Fluorescently labeled antibodies are particularly useful reporter molecules due to their high degree of specificity for attaching to a single molecular target in a mixture of molecules as complex as a cell or tissue.

The luminescent probes can be synthesized within the living cell or can be transported into the cell via several non-mechanical modes including diffusion, facilitated or active transport, signal-sequence-mediated transport, and endocytotic or pinocytotic uptake. Mechanical bulk loading methods, which are well known in the art, can also be used to load luminescent probes into living cells (Barber et al. (1996), *Neuroscience Letters* 207:17-20; Bright et al. (1996), *Cytometry* 24:226-233; McNeil (1989) in *Methods in Cell Biology,* Vol. 29, Taylor and Wang (eds.), pp. 153-173). These methods include electroporation and other mechanical methods such as scrape-loading, bead-loading, impact-loading, syringe-loading, hypertonic and hypotonic loading. Additionally, cells can be genetically engineered to express reporter molecules, such as GFP, coupled to a protein of interest as previously described (Chalfie and Prasher U.S. Patent No. 5,491,084; Cubitt et al. (1995), *Trends in Biochemical Science* 20:448-455).

Once in the cell, the luminescent probes accumulate at their target domain as a result of specific and high affinity interactions with the target domain or other modes of molecular targeting such as signal-sequence-mediated transport. Fluorescently labeled reporter molecules are useful for determining the location, amount and chemical environment of the reporter. For example, whether the reporter is in a lipophilic membrane environment or in a more aqueous environment can be determined (Giuliano et al. (1995), *Ann. Rev. of Biophysics and Biomolecular Structure* 24:405-434; Giuliano and Taylor (1995), *Methods in Neuroscience* 27:1-16). The pH environment of the reporter can be determined (Bright et al. (1989), *J. Cell Biology* 104:1019-1033; Giuliano et al. (1987), *Anal. Biochem.* 167:362-371; Thomas et al. (1979), *Biochemistry* 18:2210-2218). It can be determined whether a reporter having a chelating group is bound to an ion, such as Ca++, or not (Bright et al. (1989), In *Methods in Cell Biology,* Vol. 30, Taylor and Wang (eds.), pp. 157-192; Shimoura et al. (1988), *J. of Biochemistry* (Tokyo) 251:405-410; Tsien (1989) In *Methods in Cell Biology,* Vol. 30, Taylor and Wang (eds.), pp. 127-156).

Furthermore, certain cell types within an organism may contain components that can be specifically labeled that may not occur in other cell types. For example, epithelial cells often contain polarized membrane components. That is, these cells asymmetrically distribute macromolecules along their plasma membrane. Connective or supporting tissue cells often contain granules in which are trapped molecules specific to that cell type (e.g., heparin, histamine, serotonin, etc.). Most muscular tissue cells contain a sarcoplasmic reticulum, a specialized organelle whose function is to regulate the concentration of calcium ions within the cell cytoplasm. Many nervous tissue cells contain secretory granules and vesicles in which are trapped neurohormones or neurotransmitters. Therefore, fluorescent molecules can be designed to label not only specific components within specific cells, but also specific cells within a population of mixed cell types.

Those skilled in the art will recognize a wide variety of ways to measure fluorescence. For example, some fluorescent reporter molecules exhibit a change in excitation or emission spectra, some exhibit resonance energy transfer where one fluorescent reporter loses fluorescence, while a second gains in fluorescence, some exhibit a loss (quenching) or appearance of fluorescence, while some report rotational movements (Giuliano et al. (1995), *Ann. Rev. of Biophysics and Biomol. Structure* 24:405-434; Giuliano et al. (1995), *Methods in Neuroscience* 27:1-16).

### Scanning cell arrays

Referring to Figure 9, a preferred embodiment is provided to analyze cells that comprises operator-directed parameters being selected based on the assay being conducted, data acquisition by the cell screening system on the distribution of fluorescent signals within a sample, and interactive data review and analysis. At the start of an automated scan the operator enters information 100 that describes the sample, specifies the filter settings and fluorescent channels to match the biological labels being used and the information sought, and then adjusts the camera settings to match the sample brightness. For flexibility to handle a range of samples, the software allows selection of various parameter settings used to identify nuclei and cytoplasm, and selection of different fluorescent reagents, identification of cells of interest based on morphology or brightness, and cell numbers to be analyzed per well. These parameters are stored in the system's for easy retrieval for each automated run. The system's interactive cell identification mode simplifies the selection of morphological parameter limits such as the range of size, shape, and intensity of cells to be analyzed. The user specifies which wells of the plate the system will scan and how many fields or how many cells to analyze in each well. Depending on the setup mode selected by the user at step 101, the system either automatically pre-focuses the region of the plate to be scanned using an autofocus procedure to "find focus" of the plate 102 or the user interactively pre-focuses 103 the scanning region by selecting three "tag" points which define the rectangular area to be scanned. A least-squares fit "focal plane model" is then calculated from these tag points to estimate the focus of each well during an automated scan. The focus of each well is estimated by interpolating from the focal plane model during a scan.

During an automated scan, the software dynamically displays the scan status, including the number of cells analyzed, the current well being analyzed, images of each independent wavelength as they are acquired, and the result of the screen for each well as it is determined. The plate 4 (Figure 1) is scanned in a serpentine style as the software automatically moves the motorized microscope XY stage 3 from well to well and field to field within each well of a 96-well plate. Those skilled in the programming art will recognize how to adapt software for scanning of other microplate formats such as 24, 48, and 384 well plates. The scan pattern of the entire plate as well as the scan pattern of fields within each well are programmed. The system adjusts sample focus with an autofocus procedure 104 (Figure 9) through the Z axis focus drive 5, controls filter selection via a motorized filter wheel 19, and acquires and analyzes images of up to four different colors ("channels" or "wavelengths"),

The autofocus procedure is called at a user selected frequency, typically for the first field in each well and then once every 4 to 5 fields within each well. The autofocus procedure calculates the starting Z-axis point by interpolating from the pre-calculated plane focal model. Starting a programmable distance above or below this set point, the procedure moves the mechanical Z-axis through a number of different positions, acquires an image at each position, and finds the maximum of a calculated focus score that estimates the contrast of each image. The Z position of the image with the maximum focus score determines the best focus for a particular field. Those skilled in the art will recognize this as a variant of automatic focusing methods as described in Harms et al. in *Cytometry* 5 (1984), 236-243, Groen et al. in *Cytometry* 6 (1985), 81-91, and Firestone et al. in *Cytometry* 12 (1991), 195-206.

For image acquisition, the camera's exposure time is separately adjusted for each dye to ensure a high-quality image from each channel. Software procedures can be called, at the user's option, to correct for registration shifts between wavelengths by accounting for linear (X and Y) shifts between wavelengths before making any further measurements. The electronic shutter 18 is controlled so that sample photo-bleaching is kept to a minimum. Background shading and uneven illumination can be corrected by the software using methods known in the art (Bright et al. (1987), *J. Cell Biol.* 104:1019-1033).

In one channel, images are acquired of a primary marker 105 (Figure 9) (typically cell nuclei counterstained with DAPI or PI fluorescent dyes) which are segmented ("identified") using an adaptive thresholding procedure. The adaptive thresholding procedure 106 is used to dynamically select the threshold of an image for separating cells from the background. The staining of cells with fluorescent dyes can vary to an unknown degree across cells in a microtiter plate sample as well as within images of a field of cells within each well of a microtiter plate. This variation can occur as a result of sample preparation and/or the dynamic nature of cells. A global threshold is calculated for the complete image to separate the cells from background and account for field to field variation. These global adaptive techniques are variants of those described in the art. (Kittler et al. in *Computer Vision, Graphics, and Image Processing* 30 (1985), 125-147, Ridler et al. in *IEEE Trans. Systems, Man, and Cybernetics* (1978), 630-632.)

An alternative adaptive thresholding method utilizes local region thresholding in contrast to global image thresholding. Image analysis of local regions leads to better overall segmentation since staining of cell nuclei (as well as other labeled components) can vary across an image. Using this global/local procedure, a reduced resolution image (reduced in size by a factor of 2 to 4) is first globally segmented (using adaptive thresholding) to find regions of interest in the image. These regions then serve as guides to more fully analyze the same regions at full resolution. A more localized threshold is then calculated (again using adaptive thresholding) for each region of interest.

The output of the segmentation procedure is a binary image wherein the objects are white and the background is black. This binary image, also called a mask in the art, is used to determine if the field contains objects 107. The mask is labeled with a blob labeling method whereby each object (or blob) has a unique number assigned to it. Morphological features, such as area and shape, of the blobs are used to differentiate blobs likely to be cells from those that are considered artifacts. The user pre-sets the morphological selection criteria by either typing in known cell morphological features or by using the interactive training utility. If objects of interest are found in the field, images are acquired for all other active channels 108, otherwise the stage is advanced to the next field 109 in the current well. Each object of interest is located in the image for further analysis 110. The software determines if the object meets the criteria for a valid cell nucleus 111 by measuring its morphological features (size and shape). For each valid cell, the XYZ stage location is recorded, a small image of the cell is stored, and features are measured 112.

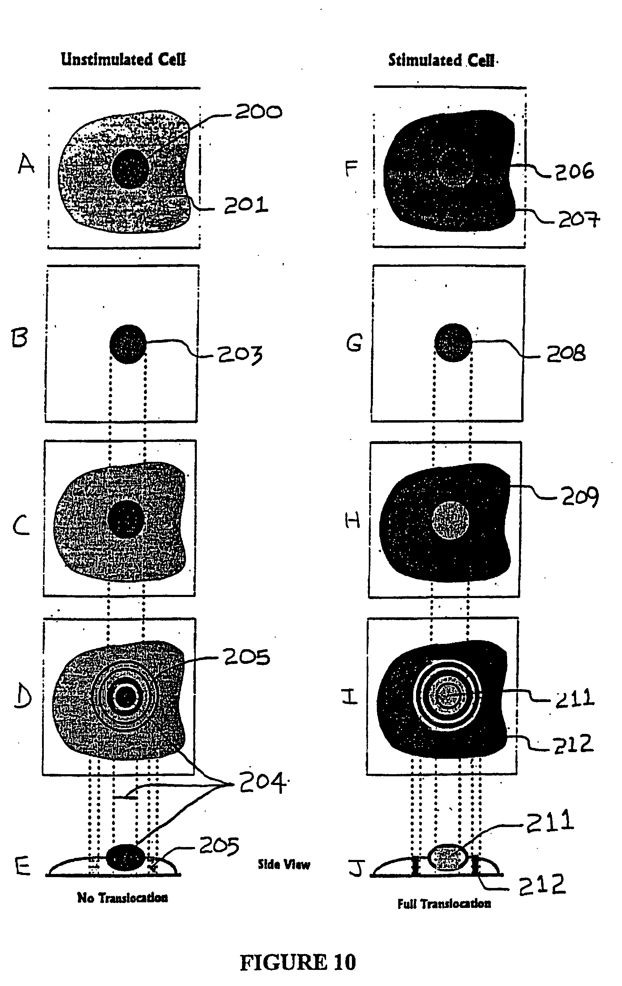

The cell scanning method of the present invention can be used to perform many different assays on cellular samples by applying a number of analytical methods simultaneously to measure features at multiple wavelengths. An example of one such assay provides for the following measurements:
1. The total fluorescent intensity within the cell nucleus for colors 1-4
2. The area of the cell nucleus for color 1 (the primary marker)
3. The shape of the cell nucleus for color 1 is described by three shape features:
   a) perimeter squared area
   b) box area ratio
   c) height width ratio
4. The average fluorescent intensity within the cell nucleus for colors 1-4 (i.e. #1 divided by #2)
5. The total fluorescent intensity of a ring outside the nucleus (see Figure 10) that represents fluorescence of the cell's cytoplasm (cytoplasmic mask) for colors 2-4
6. The area of the cytoplasmic mask
7. The average fluorescent intensity of the cytoplasmic mask for colors 2-4 (i.e. #5 divided by #6)
8. The ratio of the average fluorescent intensity of the cytoplasmic mask to average fluorescent intensity within the cell nucleus for colors 2-4 (i.e. #7 divided by #4)
9. The difference of the average fluorescent intensity of the cytoplasmic mask and the average fluorescent intensity within the cell nucleus for colors 2-4 (i.e. #7 minus #4)
10. The number of fluorescent domains (also call spots, dots, or grains) within the cell nucleus for colors 2-4

Features 1 through 4 are general features of the different cell screening assays of the invention. These steps are commonly used in a variety of image analysis applications and are well known in art (Russ (1992) *The Image Processing Handbook*, CRC Press Inc.; Gonzales et al. (1987), *Digital Image Processing.* Addison-Wesley Publishing Co. pp. 391-448). Features 5-9 have been developed specifically to provide measurements of a cell's fluorescent molecules within the local cytoplasmic region of the cell and the translocation (i.e. movement) of fluorescent molecules from the cytoplasm to the nucleus. These features (steps 5-9) are used for analyzing cells in microplates for the inhibition of nuclear translocation. For example, inhibition of nuclear translocation of transcription factors provides a novel approach to screening intact cells (detailed examples of other types of screens will be provided below). A specific method measures the amount of probe in the nuclear region (feature 4) versus the local cytoplasmic region (feature 7) of each cell. Quantification of the difference between these two sub-cellular compartments provides a measure of cytoplasm-nuclear translocation (feature 9).

Feature 10 describes a screen used for counting of DNA or RNA probes within the nuclear region in colors 2-4. For example, probes are commercially available for identifying chromosome-specific DNA sequences (Life Technologies, Gaithersburg, MD; Genosys, Woodlands, TX; Biotechnologies, Inc., Richmond, CA; Bio 101, Inc., Vista, CA) Cells are three-dimensional in nature and when examined at a high magnification under a microscope one probe may be in-focus while another may be completely out-of-focus. The cell screening method of the present invention provides for detecting three-dimensional probes in nuclei by acquiring images from multiple focal planes. The software moves the Z-axis motor drive 5 (Figure 1) in small steps where the step distance is user selected to account for a wide range of different nuclear diameters. At each of the focal steps, an image is acquired. The maximum gray-level intensity from each pixel in each image is found and stored in a resulting maximum projection image. The maximum projection image is then used to count the probes. The above method works well in counting probes that are not stacked directly above or below another one. To account for probes stacked on top of each other in the Z-direction, users can select an option to analyze probes in each of the focal planes acquired. In this mode, the scanning system performs the maximum plane projection method as discussed above, detects probe regions of interest in this image, then further analyzes these regions in all the focal plane images.

After measuring cell features 112 (Figure 9), the system checks if there are any unprocessed objects in the current field 113. If there are any unprocessed objects, it locates the next object 110 and determines whether it meets the criteria for a valid cell nucleus 111, and measures its features. Once all the objects in the current field are processed, the system determines whether analysis of the current plate is complete 114; if not, it determines the need to find more cells in the current well 115. If the need exists, the system advances the XYZ stage to the next field within the current well 109 or advances the stage to the next well 116 of the plate.

After a plate scan is complete, images and data can be reviewed with the system's image review, data review, and summary review facilities. All images, data, and settings from a scan are archived in the system's database for later review or for interfacing with a network information management system. Data can also be exported to other third-party statistical packages to tabulate results and generate other reports. Users can review the images alone of every cell analyzed by the system with an interactive image review procedure 117. The user can review data on a cell-by-cell basis using a combination of interactive graphs, a data spreadsheet of measured features, and images of all the fluorescence channels of a cell of interest with the interactive cell-by-cell data review procedure 118. Graphical plotting capabilities are provided in which data can be analyzed via interactive graphs such as histograms and scatter plots. Users can review summary data that are accumulated and summarized for all cells within each well of a plate with an interactive well-by-well data review procedure 119. Hard copies of graphs and images can be printed on a wide range of standard printers.

As a final phase of a complete scan, reports can be generated on one or more statistics of the measured features. Users can generate a graphical report of data summarized on a well-by-well basis for the scanned region of the plate using an interactive report generation procedure 120. This report includes a summary of the statistics by well in tabular and graphical format and identification information on the sample. The report window allows the operator to enter comments about the scan for later retrieval. Multiple reports can be generated on many statistics and be printed with the touch of one button. Reports can be previewed for placement and data before being printed.

The above-recited embodiment of the method operates in a single high resolution mode referred to as the high content screening (HCS) mode. The HCS mode provides sufficient spatial resolution within a well (on the order of 1 µm) to define the distribution of material within the well, as well as within individual cells in the well. The high degree of information content accessible in that mode, comes at the expense of speed and complexity of the required signal processing.

In an alternative embodiment, a high throughput system (HTS) is directly coupled with the HCS either on the same platform or on two separate platforms connected electronically (e.g. via a local area network). This embodiment of the invention, referred to as a dual mode optical system, has the advantage of increasing the throughput of an HCS by coupling it with an HTS and thereby requiring slower high resolution data acquisition and analysis only on the small subset of wells that show a response in the coupled HTS.

High throughput 'whole plate' reader systems are well known in the art and are commonly used as a component of an HTS system used to screen large numbers of compounds (Beggs et al. (1997), *supra*; McCaffrey et al. (1996), *supra* ). The HTS of the present invention is carried out on the microtiter plate or microwell array by reading many or all wells in the plate simultaneously with sufficient resolution to make determinations on a well-by-well basis. That is, calculations are made by averaging the total signal output of many or all the cells or the bulk of the material in each well. Wells that exhibit some defined response in the HTS (the 'hits') are flagged by the system. Then on the same microtiter plate or microwell array, each well identified as a hit is measured via HCS as described above. Thus, the dual mode process involves:
1. Rapidly measuring numerous wells of a microtiter plate or microwell array,
2. Interpreting the data to determine the overall activity of fluorescently labeled reporter molecules in the cells on a well-by-well basis to identify "hits" (wells that exhibit a defined response),
3. Imaging numerous cells in each "hit" well, and
4. Interpreting the digital image data to determine the distribution, environment or activity of the fluorescently labeled reporter molecules in the individual cells (i.e. intracellular measurements) and the distribution of the cells to test for specific biological functions

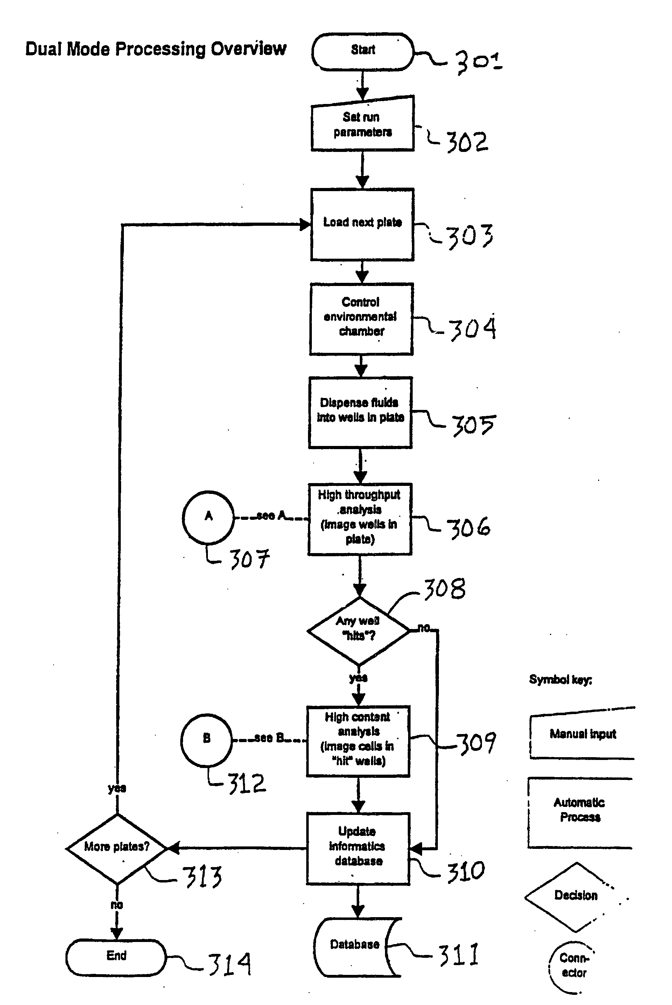

In a preferred embodiment of dual mode processing (Figure 11), at the start of a run 301, the operator enters information 302 that describes the plate and its contents, specifies the filter settings and fluorescent channels to match the biological labels being used, the information sought and the camera settings to match the sample brightness. These parameters are stored in the system's database for easy retrieval for each automated run. The microtiter plate or microwell array is loaded into the cell screening system 303 either manually or automatically by controlling a robotic loading device. An optional environmental chamber 304 is controlled by the system to maintain the temperature, humidity and CO₂ levels in the air surrounding live cells in the microtiter plate or microwell array. An optional fluid delivery device 305 (see Figure 8) is controlled by the system to dispense fluids into the wells during the scan.

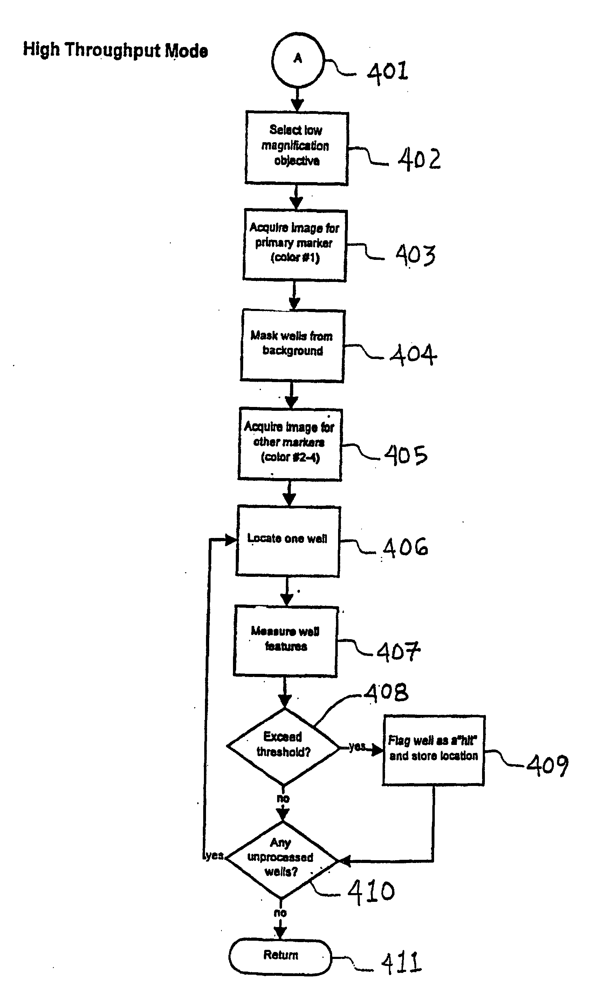

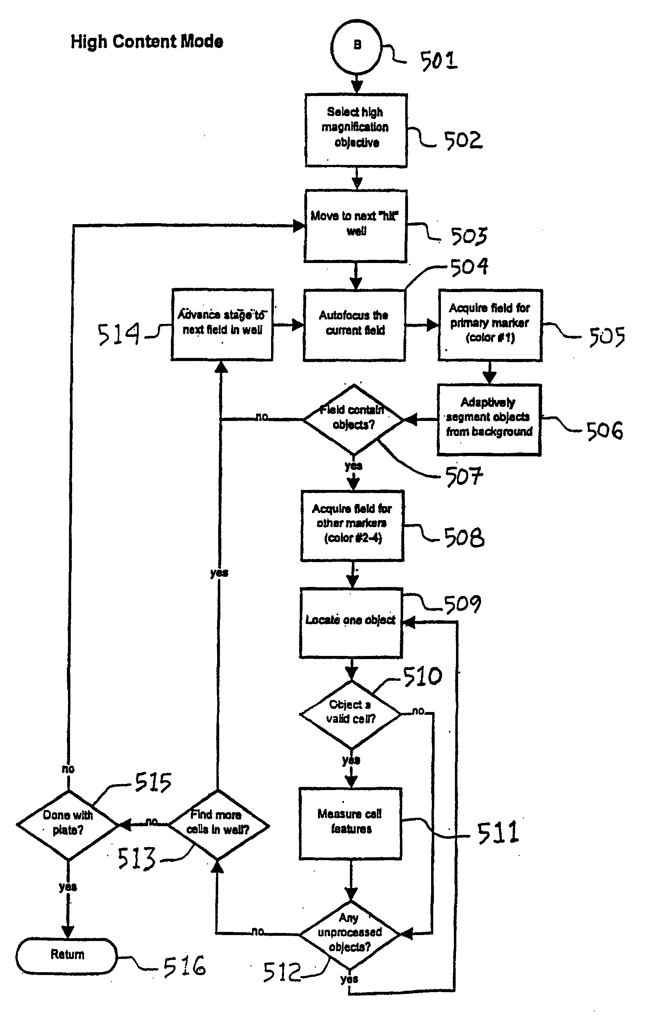

High throughput processing 306 is first performed on the microtiter plate or microwell array by acquiring and analyzing the signal from each of the wells in the plate. The processing performed in high throughput mode 307 is illustrated in Figure 12 and described below. Wells that exhibit some selected intensity response in this high throughput mode ("hits") are identified by the system. The system performs a conditional operation 308 that tests for hits. If hits are found, those specific hit wells are further analyzed in high content (micro level) mode 309. The processing performed in high content mode. 312 is illustrated in Figure 13. The system then updates 310 the informatics database 311 with results of the measurements on the plate. If there are more plates to be analyzed 313 the system loads the next plate 303; otherwise the analysis of the plates terminates 314.

The following discussion describes the high throughput mode illustrated in Figure 12. The preferred embodiment of the system, the single platform dual mode screening system, will be described. Those skilled in the art will recognize that operationally the dual platform system simply involves moving the plate between two optical systems rather than moving the optics. Once the system has been set up and the plate loaded, the system begins the HTS acquisition and analysis 401. The HTS optical module is selected by controlling a motorized optical positioning device 402 on the dual mode system. In one fluorescence channel, data from a primary marker on the plate is acquired 403 and wells are isolated from the plate background using a masking procedure 404. Images are also acquired in other fluorescence channels being used 405. The region in each image corresponding to each well 406 is measured 407. A feature calculated from the measurements for a particular well is compared with a predefined threshold or intensity response 408, and based on the result the well is either flagged as a "hit" 409 or not. The locations of the wells flagged as hits are recorded for subsequent high content mode processing. If there are wells remaining to be processed 410 the program loops back 406 until all the wells have been processed 411 and the system exits high throughput mode.

Following HTS analysis, the system starts the high content mode processing 501 defined in Figure 13. The system selects the HCS optical module 502 by controlling the motorized positioning system. For each "hit" well identified in high throughput mode, the XY stage location of the well is retrieved from memory or disk and the stage is then moved to the selected stage location 503. The autofocus procedure 504 is called for the first field in each hit well and then once every 5 to 8 fields within each well. In one channel, images are acquired of the primary marker 505 (typically cell nuclei counterstained with DAPI, Hoechst or PI fluorescent dye). The images are then segmented (separated into regions of nuclei and non-nuclei) using an adaptive thresholding procedure 506. The output of the segmentation procedure is a binary mask wherein the objects are white and the background is black. This binary image, also called a mask in the art, is used to determine if the field contains objects 507. The mask is labeled with a blob labeling method whereby each object (or blob) has a unique number assigned to it. If objects are found in the field, images are acquired for all other active channels 508, otherwise the stage is advanced to the next field 514 in the current well. Each object is located in the image for further analysis 509. Morphological features, such as area and shape of the objects, are used to select objects likely to be cell nuclei 510, and discard (do no further processing on) those that are considered artifacts. For each valid cell nucleus, the XYZ stage location is recorded, a small image of the cell is stored, and assay specific features are measured 511. The system then performs multiple tests on the cells by applying several analytical methods to measure features at each of several wavelengths. After measuring the cell features, the systems checks if there are any unprocessed objects in the current field 512. If there are any unprocessed objects, it locates the next object 509 and determines whether it meets the criteria for a valid cell nucleus 510, and measures its features. After processing all the objects in the current field, the system deteremines whether it needs to find more cells or fields in the current well 513. If it needs to find more cells or fields in the current well it advances the XYZ stage to the next field within the current well 515. Otherwise, the system checks whether it has any remaining hit wells to measure 515. If so, it advances to the next hit well 503 and proceeds through another cycle of acquisition and analysis, otherwise the HCS mode is finished 516.

In an alternative embodiment of the present invention, a method of kinetic live cell screening is provided. The previously described embodiments of the invention are used to characterize the spatial distribution of cellular components at a specific point in time, the time of chemical fixation. As such, these embodiments have limited utility for implementing kinetic based screens, due to the sequential nature of the image acquisition, and the amount of time required to read all the wells on a plate. For example, since a plate can require 30 - 60 minutes to read through all the wells, only very slow kinetic processes can be measured by simply preparing a plate of live cells and then reading through all the wells more than once. Faster kinetic processes can be measured by taking multiple readings of each well before proceeding to the next well, but the elapsed time between the first and last well would be too long, and fast kinetic processes would likely be complete before reaching the last well.

The kinetic live cell extension of the invention enables the design and use of screens in which a biological process is characterized by its kinetics instead of, or in addition to, its spatial characteristics. In many cases, a response in live cells can be measured by adding a reagent to a specific well and making multiple measurements on that well with the appropriate timing. This dynamic live cell embodiment of the invention therefore includes apparatus for fluid delivery to individual wells of the system in order to deliver reagents to each well at a specific time in advance of reading the well. This embodiment thereby allows kinetic measurements to be made with temporal resolution of seconds to minutes on each well of the plate. To improve the overall efficiency of the dynamic live cell system, the acquisition control program is modified to allow repetitive data collection from sub-regions of the plate, allowing the system to read other wells between the time points required for an individual well.

Figure 8 describes an example of a fluid delivery device for use with the live cell embodiment of the invention and is described above. This set-up allows one set of pipette tips 705, or even a single pipette tip, to deliver reagent to all the wells on the plate. The bank of syringe pumps 701 can be used to deliver fluid to 12 wells simultaneously, or to fewer wells by removing some of the tips 705. The temporal resolution of the system can therefore be adjusted, without sacrificing data collection efficiency, by changing the number of tips and the scan pattern as follows. Typically, the data collection and analysis from a single well takes about 5 seconds. Moving from well to well and focusing in a well requires about 5 seconds, so the overall cycle time for a well is about 10 seconds. Therefore, if a single pipette tip is used to deliver fluid to a single well, and data is collected repetitively from that well, measurements can be made with about 5 seconds temporal resolution. If 6 pipette tips are used to deliver fluids to 6 wells simultaneously, and the system repetitively scans all 6 wells, each scan will require 60 seconds, thereby establishing the temporal resolution. For slower processes which only require data collection every 8 minutes, fluids can be delivered to one half of the plate, by moving the plate during the fluid delivery phase, and then repetitively scanning that half of the plate. Therefore, by adjusting the size of the sub-region being scanned on the plate, the temporal resolution can be adjusted without having to insert wait times between acquisitions. Because the system is continuously scanning and acquiring data, the overall time to collect a kinetic data set from the plate is then simply the time to perform a single scan of the plate, multiplied by the number of time points required. Typically, 1 time point before addition of compounds and 2 or 3 time points following addition should be sufficient for screening purposes.

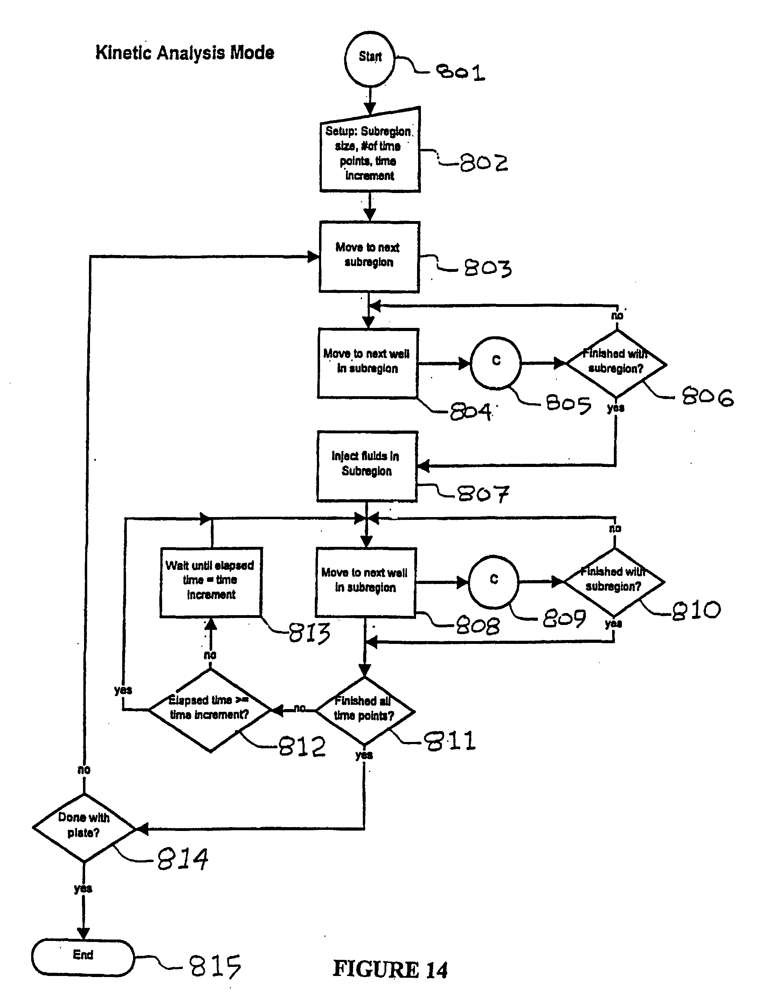

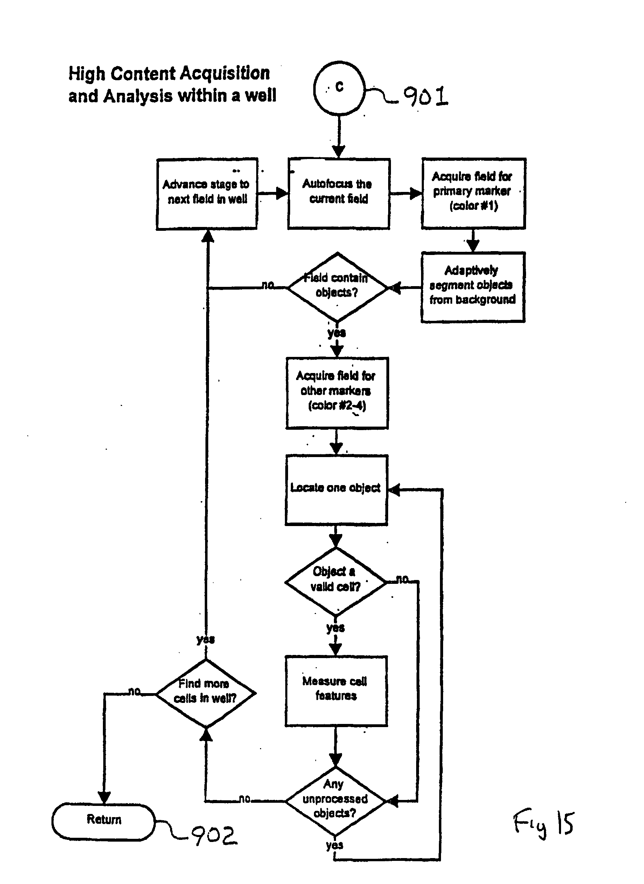

Figure 14 shows the acquisition sequence used for kinetic analysis. The start of processing 801 is configuration of the system, much of which is identical to the standard HCS configuration. In addition, the operator must enter information specific to the kinetic analysis being performed 802, such as the sub-region size, the number of time points required, and the required time increment. A sub-region is a group of wells that will be scanned repetitively in order to accumulate kinetic data. The size of the sub-region is adjusted so that the system can scan a whole sub-region once during a single time increment, thus minimizing wait times. The optimum sub-region size is calculated from the setup parameters, and adjusted if necessary by the operator. The system then moves the plate to the first sub-region 803, and to the first well in that sub-region 804 to acquire the prestimulation (time = 0) time points. The acquisition sequence performed in each well is exactly the same as that required for the specific HCS being run in kinetic mode. Figure 15 details a flow chart for that processing. All of the steps between the start 901 and the return 902 are identical to those described as steps 504 - 514 in Figure 13.

After processing each well in a sub-region, the system checks to see if all the wells in the sub-region have been processed 806 (Figure 14), and cycles through all the wells until the whole region has been processed. The system then moves the plate into position for fluid addition, and controls fluidic system delivery of fluids to the entire sub-region 807. This may require multiple additions for sub-regions which span several rows on the plate, with the system moving the plate on the X,Y stage between additions. Once the fluids have been added, the system moves to the first well in the sub-region 808 to begin acquisition of time points. The data is acquired from each well 809 and as before the system cycles through all the wells in the sub-region 810. After each pass through the sub-region, the system checks whether all the time points have been collected 811 and if not, pauses 813 if necessary 812 to stay synchronized with the requested time increment. Otherwise, the system checks for additional sub-regions on the plate 814 and either moves to the next sub-region 803 or finishes 815. Thus, the kinetic analysis mode comprises operator identification of sub-regions of the microtiter plate or microwells to be screened, based on the kinetic response to be investigated, with data acquisitions within a sub-region prior to data acquisition in subsequent sub-regions.

### Specific Screens

In another aspect of the present invention, cell screening methods and machine readable storage medium comprising a program containing a set of instructions for causing a cell screening system to execute procedures for defining the distribution and activity of specific cellular constituents and processes is provided. In a preferred embodiment, the cell screening system comprises a high magnification fluorescence optical system with a stage adapted for holding cells and a means for moving the stage, a digital camera, a light source for receiving and processing the digital data from the digital camera, and a computer means for receiving and processing the digital data from the digital camera. This aspect of the invention comprises programs that instruct the cell screening system to define the distribution and activity of specific cellular constituents and processes, using the luminescent probes, the optical imaging system, and the pattern recognition software of the invention. Preferred embodiments of the machine readable storage medium comprise programs consisting of a set of instructions for causing a cell screening system to execute the procedures set forth in Figures 9, 11, 12, 13, 14 or 15. Another preferred embodiment comprises a program consisting of a set of instructions for causing a cell screening system to execute procedures for detecting the distribution and activity of specific cellular constituents and processes. In most preferred embodiments, the cellular processes include, but are not limited to, nuclear translocation of a protein, cellular morphology, apoptosis, receptor intemalization, and protease-induced translocation of a protein.

In a preferred embodiment, the cell screening methods are used to identify compounds that modify the various cellular processes. The cells can be contacted with a test compound, and the effect of the test compound on a particular cellular process can be analyzed. Alternatively, the cells can be contacted with a test compound and a known agent that modifies the particular cellular process, to determine whether the test compound can inhibit or enhance the effect of the known agent. Thus, the methods can be used to identify test compounds that increase or decrease a particular cellular response, as well as to identify test compounds that affects the ability of other agents to increase or decrease a particular cellular response.

In another preferred embodiment, the locations containing cells are analyzed using the above methods at low resolution in a high throughput mode, and only a subset of the locations containing cells are analyzed in a high content mode to obtain luminescent signals from the luminescently labeled reporter molecules in subcellular compartments of the cells being analyzed.

The following examples are intended for purposes of illustration only and should not be construed to limit the scope of the invention, as defined in the claims appended hereto.

The various chemical compounds, reagents, dyes, and antibodies that are referred to in the following Examples are commercially available from such sources as Sigma Chemical (St. Louis, MO), Molecular Probes (Eugene, OR), Aldrich Chemical Company (Milwaukee, WI), Accurate Chemical Company (Westbury, NY), Jackson Immunolabs, and Clontech (Palo Alto, CA).

### Example 1 Cytoplasm to Nucleus Translocation Screening:

### a. Transcription Factors

Regulation of transcription of some genes involves activation of a transcription factor in the cytoplasm, resulting in that factor being transported into the nucleus where it can initiate transcription of a particular gene or genes. This change in transcription factor distribution is the basis of a screen for the cell-based screening system to detect compounds that inhibit or induce transcription of a particular gene or group of genes. A general description of the screen is given followed by a specific example.

The distribution of the transcription factor is determined by labeling the nuclei with a DNA specific fluorophore like Hoechst 33423 and the transcription factor with a specific fluorescent antibody. After autofocusing on the Hoechst labeled nuclei, an image of the nuclei is acquired in the cell-based screening system and used to create a mask by one of several optional thresholding methods, as described *supra.* The morphological descriptors of the regions defined by the mask are compared with the user defined parameters and valid nuclear masks are identified and used with the following method to extract transcription factor distributions. Each valid nuclear mask is eroded to define a slightly smaller nuclear region. The original nuclear mask is then dilated in two steps to define a ring shaped region around the nucleus, which represents a cytoplasmic region. The average antibody fluorescence in each of these two regions is determined, and the difference between these averages is defined as the NucCyt Difference. Two examples of determining nuclear translocation are discussed below and illustrated in **Figure 10A-J. Figure 10A** illustrates an unstimulated cell with its nucleus 200 labeled with a blue fluorophore and a transcription factor in the cytoplasm 201 labeled with a green fluorophore. **Figure 10B** illustrates the nuclear mask 202 derived by the cell-based screening system. **Figure 10C** illustrates the cytoplasm 203 of the unstimulated cell imaged at a green wavelength. **Figure 10D** illustrates the nuclear mask 202 is eroded (reduced) once to define a nuclear sampling region 204 with minimal cytoplasmic distribution. The nucleus boundary 202 is dilated (expanded) several times to form a ring that is 2-3 pixels wide that is used to define the cytoplasmic sampling region 205 for the same cell. Figure 10E further illustrates a side view which shows the nuclear sampling region 204 and the cytoplasmic sampling region 205. Using these two sampling regions, data on nuclear translocation can be automatically analyzed by the cell-based screening system on a cell by cell basis. **Figure 10F-J** illustrates the strategy for determining nuclear translocation in a stimulated cell. Figure 10F illustrates a stimulated cell with its nucleus 206 labeled with a blue fluorophore and a transcription factor in the cytoplasm 207 labeled with a green fluorophore. The nuclear mask 208 in **Figure 10G** is derived by the cell based screening system. **Figure 10H** illustrates the cytoplasm 209 of a stimulated cell imaged at a green wavelength. **Figure 10I** illustrates the nuclear sampling region 211 and cytoplasmic sampling region 212 of the stimulated cell. **Figure 10J** further illustrates a side view which shows the nuclear sampling region 211 and the cytoplasmic sampling region 212.

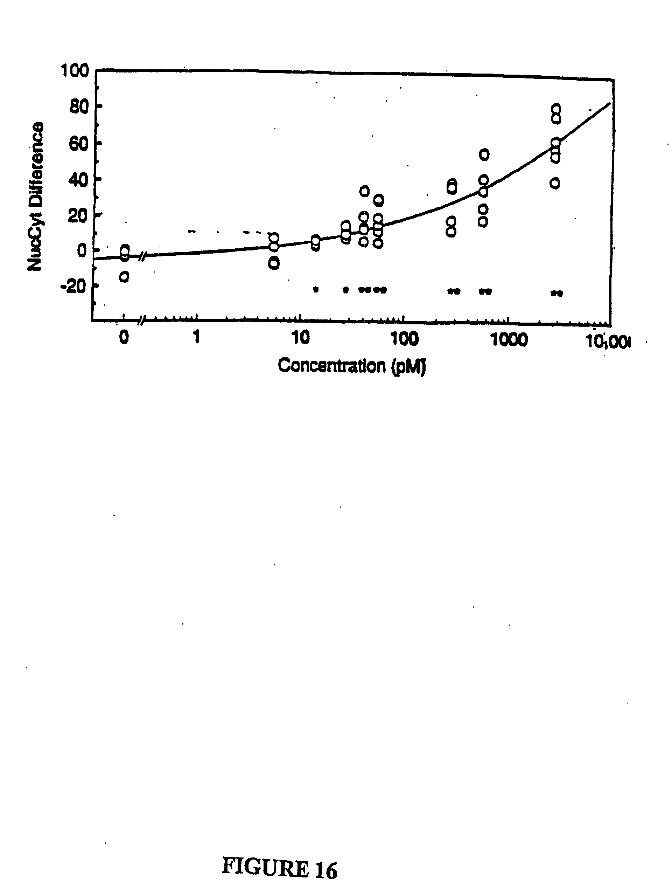

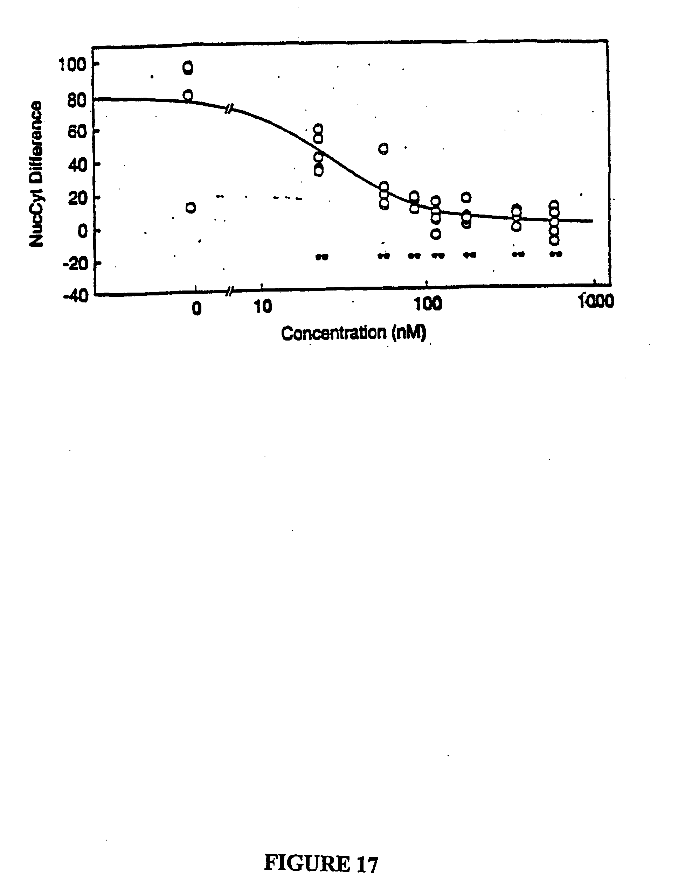

A specific application of this method has been used to validate this method as a screen. A human cell line was plated in 96 well microtiter plates. Some rows of wells were titrated with IL-1, a known inducer of the NF-KB transcription factor. The cells were then fixed and stained by standard methods with a fluorescein labeled antibody to the transcription factor, and Hoechst 33423. The cell-based screening system was used to acquire and analyze images from this plate and the NucCyt Difference was found to be strongly correlated with the amount of agonist added to the wells as illustrated in Figure 16. In a second experiment, an antagonist to the receptor for IL-1, IL-1RA was titrated in the presence of IL-1α, progressively inhibiting the translocation induced by IL-1α. The NucCyt Difference was found to strongly correlate with this inhibition of translocation, as illustrated in **Figure 17**.

Additional experiments have shown that the NucCyt Difference, as well as the NucCyt ratio, gives consistent results over a wide range of cell densities and reagent concentrations, and can therefore be routinely used to screen compound libraries for specific nuclear translocation activity. Furthermore, the same method can be used with antibodies to other transcription factors, or GFP-transcription factor chimeras, or fluorescently labeled transcription factors introduced into living or fixed cells, to screen for effects on the regulation of transcription factor activity.

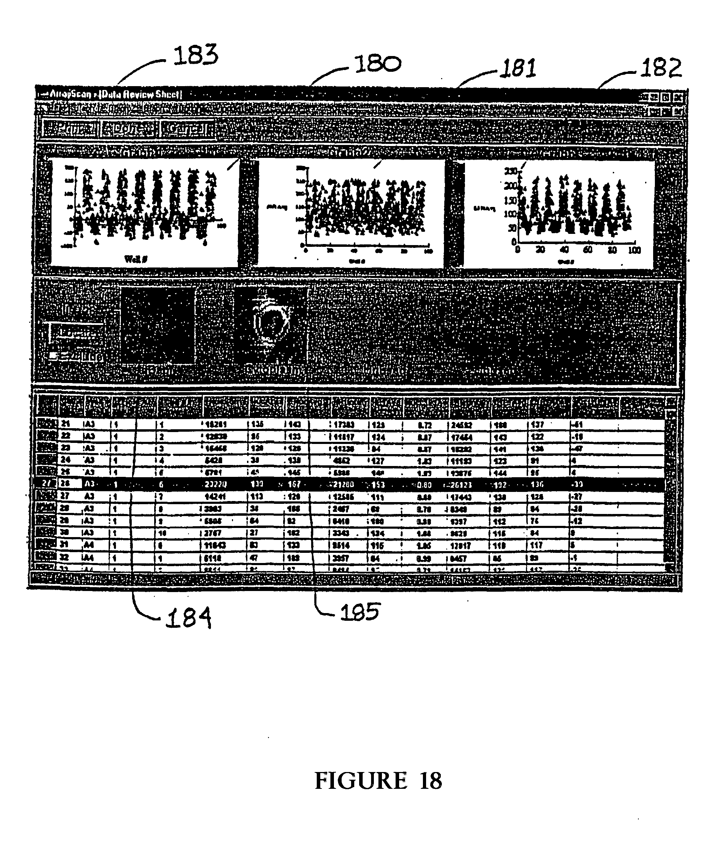

**Figure 18** is a representative display on a PC screen of data which was obtained in accordance with Example 1. Graph 1 180 plots the difference between the average antibody fluorescence in the nuclear sampling region and cytoplasmic sampling region, NucCyt Difference verses Well #. Graph 2 181 plots the average fluorescence of the antibody in the nuclear sampling region, NP1 average, versus the Well #. Graph 3 182 plots the average antibody fluorescence in the cytoplasmic sampling region, LIP1 average, versus Well #. The software permits displaying data from each cell. For example, **Figure 18** shows a screen display 183, the nuclear image 184, and the fluorescent antibody image 185 for cell #26.

NucCyt Difference referred to in graph 1 180 of **Figure 18** is the difference between the average cytoplasmic probe (fluorescent reporter molecule) intensity and the average nuclear probe (fluorescent reporter molecule) intensity. NP1 average referred to in graph 2 181 of **Figure 18** is the average of cytoplasmic probe (fluorescent reporter molecule) intensity within the nuclear sampling region. L1P1 average referred to in graph 3 182 of **Figure 18** is the average probe (fluorescent reporter molecule) intensity within the cytoplasmic sampling region.

It will be understood by one of skill in the art that this aspect of the invention can be performed using other transcription factors that translocate from the cytoplasm to the nucleus upon activation. In another specific example, activation of the c-fos transcription factor was assessed by defining its spatial position within cells. Activated c-fos is found only within the nucleus, while inactivated c-fos resides within the cytoplasm.

3T3 cells were plated at 5000-10000 cells per well in a Polyfiltronics 96-well plate. The cells were allowed to attach and grow overnight. The cells were rinsed twice with 100 µl serum-free medium, incubated for 24-30 hours in serum-free MEM culture medium, and then stimulated with platelet derived growth factor (PDGF-BB) (Sigma Chemical Co., St. Louis, MO) diluted directly into serum free medium at concentrations ranging from 1-50 ng/ml for an average time of 20 minutes.

Following stimulation, cells were fixed for 20 minutes in 3.7% formaldehyde solution in 1X Hanks buffered saline solution (HBSS). After fixation, the cells were washed with HBSS to remove residual fixative, permeabilized for 90 seconds with 0.5% Triton X-100 solution in HBSS, and washed twice with HBSS to remove residual detergent. The cells were then blocked for 15 minutes with a 0.1% solution of BSA in HBSS, and further washed with HBSS prior to addition of diluted primary antibody solution.

c-Fos rabbit polyclonal antibody (Calbiochem, PC05) was diluted 1:50 in HBSS, and 50 µl of the dilution was applied to each well. Cells were incubated in the presence of primary antibody for one hour at room temperature, and then incubated for one hour at room temperature in a light tight container with goat anti-rabbit secondary antibody conjugated to ALEXA™ 488 (Molecular Probes), diluted 1:500 from a 100 µg/ml stock in HBSS. Hoechst DNA dye (Molecular Probes) was then added at a 1:1000 dilution of the manufacturer's stock solution (10 mg/ml). The cells were then washed with HBSS, and the plate was sealed prior to analysis with the cell screening system of the invention. The data from these experiments demonstrated that the methods of the invention could be used to measure transcriptional activation of c-fos by defining its spatial position within cells.

One of skill in the art will recognize that while the following method is applied to detection of c-fos activation, it can be applied to the analysis of any transcription factor that translocates from the cytoplasm to the nucleus upon activation. Examples of such transcription factors include, but are not limited to fos and jun homologs, NF-KB (nuclear factor kappa from B cells), NFAT (nuclear factor of activated T-lymphocytes), and STATs (signal transducer and activator of transcription) factors (For example, see Strehlow, I., and Schindler, C. 1998. *J. Biol. Chem.* 273:28049-28056; Chow, et al. 1997 *Science.* 278:1638-1641; Ding et al. 1998 *J. Biol. Chem.* 273:28897-28905; Baldwin, 1996. *Annu Rev Immunol.* 14:649-83; Kuo, C.T., and J.M. Leiden. 1999. *Annu Rev Immunol.* 17:149-87; Rao, et al. 1997. *Annu Rev Immunol.* 15:707-47; Masuda,et al. 1998. *Cell Signal.* 10:599-611; Hoey, T., and U. Schindler. 1998. *Curr Opin Genet Dev.* 8:582-7; Liu, et al. 1998. *Curr Opin Immunol.* 10:271-8.)

Thus, in this aspect of the invention, indicator cells are treated with test compounds and the distribution of luminescently labeled transcription factor is measured in space and time using a cell screening system, such as the one disclosed above. The luminescently labeled transcription factor may be expressed by or added to the cells either before, together with, or after contacting the cells with a test compound.

For example, the transcription factor may be expressed as a luminescently labeled protein chimera by transfected indicator cells. Alternatively, the luminescently labeled transcription factor may be expressed, isolated, and bulk-loaded into the indicator cells as described above, or the transcription factor may be luminescently labeled after isolation. As a further alternative, the transcription factor is expressed by the indicator cell, which is subsequently contacted with a luminescent label, such as an antibody, that detects the transcription factor.

In a further aspect, kits are provided for analyzing transcription factor activation, comprising an antibody that specifically recognizes a transcription factor of interest, and instructions for using the antibody for carrying out the methods described above. In a preferred embodiment, the transcription factor-specific antibody, or a secondary antibody that detects the transcription factor antibody, is luminescently labeled. In further preferred embodiments, the kit contains cells that express the transcription factor of interest, and/or the kit contains a compound that is known to modify activation of the transcription factor of interest, including but not limited to platelet derived growth factor (PDGF) and serum, which both modify fos activation; and interleukin 1(IL-1) and tumor necrosis factor (TNF), which both modify NF-KB activation.

In another embodiment, the kit comprises a recombinant expression vector comprising a nucleic acid encoding a transcription factor of interest that translocates from the cytoplasm to the nucleus upon activation, and instructions for using the expression vector to identify compounds that modify transcription factor activation in a cell of interest. Alternatively, the kits contain a purified, luminescently labeled transcription factor. In a preferred embodiment, the transcription factor is expressed as a fusion protein with a luminescent protein, including but not limited to green fluorescent protein, luceriferase, or mutants or fragments thereof. In various preferred embodiments, the kit further contains cells that are transfected with the expression vector, an antibody or fragment that specifically bind to the transcription factor of interest, and/or a compound that is known to modify activation of the transcription factor of interest (as above).

### b. Protein Kinases

The cytoplasm to nucleus screening methods can also be used to analyze the activation of any protein kinase that is present in an inactive state in the cytoplasm and is transported to the nucleus upon activation, or that phosphorylates a substrate that translocates from the cytoplasm to the nucleus upon phosphorylation. Examples of appropriate protein kinases include, but are not limited to extracellular signal-regulated protein kinases (ERKs), c-Jun amino-terminal kinases (JNKs), Fos regulating protein kinases (FRKs), p38 mitogen activated protein kinase (p38MAPK), protein kinase A (PKA), and mitogen activated protein kinase kinases (MAPKKs). (For example, see Hall, et al. 1999. *J Biol Chem.* 274:376-83; Han, et al. 1995. *Biochim. Biophys. Acta.* 1265:224-227; Jaaro et al. 1997. *Proc. Natl. Acad. Sci. U.S.A.* 94:3742-3747; Taylor, et al. 1994. *J. Biol. Chem.* 269:308-318; Zhao, Q., and F. S. Lee. 1999. *J Biol Chem.* 274:8355-8; Paolilloet al. 1999. *J Biol Chem.* 274:6546-52; Coso et al. 1995. Cell 81:1137-1146; Tibbles, L.A., and J.R. Woodgett. 1999. *Cell Mol Life Sci.* 55:1230-54; Schaeffer, H.J., and M.J. Weber. 1999. *Mol Cell Biol.* 19:2435-44.)

Alternatively, protein kinase activity is assayed by monitoring translocation of a luminescently labeled protein kinase substrate from the cytoplasm to the nucleus after being phosphorylated by the protein kinase of interest. In this embodiment, the substrate is non-phosphorylated and cytoplasmic prior to phosphorylation, and is translocated to the nucleus upon phosphorylation by the protein kinase. There is no requirement that the protein kinase itself translocates from the cytoplasm to the nucleus in this embodiment. Examples of such substrates (and the corresponding protein kinase) include, but are not limited to c-jun (JNK substrate); fos (FRK substrate), and p38 (p38 MAPK substrate).

Thus, in these embodiments, indicator cells are treated with test compounds and the distribution of luminescently labeled protein kinase or protein kinase substrate is measured in space and time using a cell screening system, such as the one disclosed above. The luminescently labeled protein kinase or protein kinase substrate may be expressed by or added to the cells either before, together with, or after contacting the cells with a test compound. For example, the protein kinase or protein kinase substrate may be expressed as a luminescently labeled protein chimera by transfected indicator cells. Alternatively, the luminescently labeled protein kinase or protein kinase substrate may be expressed, isolated, and bulk-loaded into the indicator cells as described above, or the protein kinase or protein kinase substrate may be luminescently labeled after isolation. As a further alternative, the protein kinase or protein kinase substrate is expressed by the indicator cell, which is subsequently contacted with a luminescent label, such as a labeled antibody, that detects the protein kinase or protein kinase substrate.

In a further embodiment, protein kinase activity is assayed by monitoring the phosphorylation state (ie: phosphorylated or not phosphorylated) of a protein kinase substrate. In this embodiment, there is no requirement that either the protein kinase or the protein kinase substrate translocate from the cytoplasm to the nucleus upon activation. In a preferred embodiment, phosphorylation state is monitored by contacting the cells with an antibody that binds only to the phosphorylated form of the protein kinase substrate of interest (For example, as disclosed in U.S. Patent No. 5,599,681).

In another preferred embodiment, a biosensor of phosphorylation is used. For example, a luminescently labeled protein or fragment thereof can be fused to a protein that has been engineered to contain (a) a phosphorylation site that is recognized by a protein kinase of interest; and (b) a nuclear localization signal that is unmasked by the phosphorylation. Such a biosensor will thus be translocated to the nucleus upon phosphorylation, and its translocation can be used as a measure of protein kinase activation.

In another aspect, kits are provided for analyzing protein kinase activation, comprising a primary antibody that specifically binds to a protein kinase, a protein kinase substrate, or a phosphorylated form of the protein kinase substrate of interest and instructions for using the primary antibody to identify compounds that modify protein kinase activation in a cell of interest. In a preferred embodiment, the primary antibody, or a secondary antibody that detects the primary antibody, is luminescently labeled. In other preferred embodiments, the kit further comprises cells that express the protein kinase of interest, and/or a compound that is known to modify activation of the protein kinase of interest, including but not limited to dibutyryl cAMP (modifies PKA), forskolin (PKA), and anisomycin (p38MAPK).

Alternatively, the kits comprise an expression vector encoding a protein kinase or a protein kinase substrate of interest that translocates from the cytoplasm to the nucleus upon activation and instructions for using the expression vector to identify compounds that modify protein kinase activation in a cell of interest. Alternatively, the kits contain a purified, luminescently labeled protein kinase or protein kinase substrate. In a preferred embodiment, the protein kinase or protein kinase substrate of interest is expressed as a fusion protein with a luminescent protein. In further preferred embodiments, the kit further comprises cells that are transfected with the expression vector, an antibody or fragment thereof that specifically binds to the protein kinase or protein kinase substrate of interest, and/or a compound that is known to modify activation of the protein kinase of interest. (as above)

In another aspect, the present invention comprises a machine readable storage medium comprising a program containing a set of instructions for causing a cell screening system to execute the methods disclosed for analyzing transcription factor or protein kinase activation, wherein the cell screening system comprises an optical system with a stage adapted for holding a plate containing cells, a digital camera, a means for directing fluorescence or luminescence emitted from the cells to the digital camera, and a computer means for receiving and processing the digital data from the digital camera.

### Example 2 Automated Screen for Compounds that Modify Cellular Morphology

Changes in cell size are associated with a number of cellular conditions, such as hypertrophy, cell attachment and spreading, differentiation, growth and division, necrotic and programmed cell death, cell motility, morphogenesis, tube formation, and colony formation.

For example, cellular hypertrophy has been associated with a cascade of alterations in gene expression and can be characterized in cell culture by an alteration in cell size, that is clearly visible in adherent cells growing on a coverslip.

Cell size can also be measured to determine the attachment and spreading of adherent cells. Cell spreading is the result of selective binding of cell surface receptors to substrate ligands and subsequent activation of signaling pathways to the cytoskeleton. Cell attachment and spreading to substrate molecules is an important step for the metastasis of cancer cells, leukocyte activation during the inflammatory response, keratinocyte movement during wound healing, and endothelial cell movement during angiogenesis. Compounds that affect these surface receptors, signaling pathways, or the cytoskeleton will affect cell spreading and can be screened by measuring cell size.

Total cellular area can be monitored by labeling the entire cell body or the cell cytoplasm using cytoskeletal markers, cytosolic volume markers, or cell surface markers, in conjunction with a DNA label. Examples of such labels (many available from Molecular Probes (Eugene, Oregon) and Sigma Chemical Co. (St. Louis, Missouri)) include the following:

| **CELL SIZE AND AREA MARKERS** | |
|---|---|
| **Cytoskeletal Markers** | |
| • | ALEXA™ 488 phalloidin (Molecular Probes, Oregon) |
| • | Tubulin-green fluorescent protein chimeras |
| • | Cytokeratin-green fluorescent protein chimeras |
| • | Antibodies to cytoskeletal proteins |
| | |

| **Cytosolic Volume Markers** | |
|---|---|
| • | Green fluorescent proteins |
| • | Chloromethylfluorescein diacetate (CMFDA) |
| • | Calcein green |
| • | BCECF/AM ester |
| • | Rhodamine dextran |
| | |

| **Cell Surface Markers for Lipid, Protein, or Oligosaccharide** | |
|---|---|
| • | Dihexadecyl tetramethylindocarbocyanine perchlorate (DiIC16) lipid dyes |
| • | Triethylammonium propyl dibutylamino styryl pyridinium (FM 4-64, FM 1-43) lipid dyes |
| • | MITOTRACKER™ Green FM |
| • | Lectins to oligosaccarides such as fluorescein concanavalin A or wheat germ agglutinin |
| • | SYPRO™ Red non-specific protein markers |
| • | Antibodies to various surface proteins such as epidermal growth factor |
| • | Biotin labeling of surface proteins followed by fluorescent strepavidin labeleing |

Protocols for cell staining with these various agents are well known to those skilled in the art. Cells are stained live or after fixation and the cell area can be measured. For example, live cells stained with DiIC16 have homogeneously labeled plasma membranes, and the projected cross-sectional area of the cell is uniformly discriminated from background by fluorescence intensity of the dye. Live cells stained with cytosolic stains such as CMFDA produce a fluorescence intensity that is proportional to cell thickness. Although cell labeling is dimmer in thin regions of the cell, total cell area can be discriminated from background. Fixed cells can be stained with cytoskeletal markers such as ALEXA™ 488 phalloidin that label polymerized actin. Phalloidin does not homogeneously stain the cytoplasm, but still permits discrimination of the total cell area from background.

### Cellular hypertrophy

A screen to analyze cellular hypertrophy is implemented using the following strategy. Primary rat myocytes can be cultured in 96 well plates, treated with various compounds and then fixed and labeled with a fluorescent marker for the cell membrane or cytoplasm, or cytoskeleton, such as an antibody to a cell surface marker or a fluorescent marker for the cytoskeleton like rhodamine-phalloidin, in combination with a DNA label like Hoechst.

After focusing on the Hoechst labeled nuclei, two images are acquired, one of the Hoechst labeled nuclei and one of the fluorescent cytoplasm image. The nuclei are identified by thresholding to create a mask and then comparing the morphological descriptors of the mask with a set of user defined descriptor values. Each non-nucleus image (or "cytoplasmic image") is then processed separately. The original cytoplasm image can be thresholded, creating a cytoplasmic mask image. Local regions containing cells are defined around the nuclei. The limits of the cells in those regions are then defined by a local dynamic threshold operation on the same region in the fluorescent antibody image. A sequence of erosions and dilations is used to separate slightly touching cells and a second set of morphological descriptors is used to identify single cells. The area of the individual cells is tabulated in order to define the distribution of cell sizes for comparison with size data from normal and hypertrophic cells.

Responses from entire 96-well plates (measured as average cytoplasmic area/cell) were analyzed by the above methods, and the results demonstrated that the assay will perform the same on a well-to-well, plate-to-plate, and day-to-day basis (below a 15% cov for maximum signal). The data showed very good correlation for each day, and that there was no variability due to well position in the plate.

The following totals can be computed for the field. The aggregate whole nucleus area is the number of nonzero pixels in the nuclear mask. The average whole nucleus area is the aggregate whole nucleus area divided by the total number of nuclei. For each cytoplasm image several values can be computed. These are the total cytoplasmic area, which is the count of nonzero pixels in the cytoplasmic mask. The aggregate cytoplasm intensity is the sum of the intensities of all pixels in the cytoplasmic mask. The cytoplasmic area per nucleus is the total cytoplasmic area divided by the total nucleus count. The cytoplasmic intensity per nucleus is the aggregate cytoplasm intensity divided by the total nucleus count. The average cytoplasm intensity is the aggregate cytoplasm intensity divided by the cytoplasm area. The cytoplasm nucleus ratio is the total cytoplasm area divided by the total nucleus area.

Additionally, one or more fluorescent antibodies to other cellular proteins, such as the major muscle proteins actin or myosin, can be included. Images of these additional labeled proteins can be acquired and stored with the above images, for later review, to identify anomalies in the distribution and morphology of these proteins in hypertrophic cells. This example of a multi-parametric screen allows for simultaneous analysis of cellular hypertrophy and changes in actin or myosin distribution.

One of skill in the art will recognize that while the example analyzes myocyte hypertrophy, the methods can be applied to analyzing hypertrophy, or general morphological changes in any cell type.

### Cell morphology assays for prostate carcinoma

Cell spreading is a measure of the response of cell surface receptors to substrate attachment ligands. Spreading is proportional to the ligand concentration or to the concentration of compounds that reduce receptor-ligand function. One example of selective cell-substrate attachment is prostate carcinoma cell adhesion to the extracellular matrix protein collagen. Prostate carcinoma cells metastasize to bone via selective adhesion to collagen.

Compounds that interfere with metastasis of prostate carcinoma cells were screened as follows. PC3 human prostate carcinoma cells were cultured in media with appropriate stimulants and are passaged to collagen coated 96 well plates. Ligand concentration can be varied or inhibitors of cell spreading can be added to the wells. Examples of compounds that can affect spreading are receptor antagonists such as integrin- or proteoglycan-blocking antibodies, signaling inhibitors including phosphatidyl inositol-3 kinase inhibitors, and cytoskeletal inhibitors such as cytochalasin D. After two hours, cells were fixed and stained with ALEXA™ 488 phalloidin (Molecular Probes) and Hoechst 33342 as per the protocol for cellular hypertrophy. The size of cells under these various conditions, as measured by cytoplasmic staining, can be distinguished above background levels. The number of cells per field is determined by measuring the number of nuclei stained with the Hoechst DNA dye. The area per cell is found by dividing the cytoplasmic area (phalloidin image) by the cell number (Hoechst image). The size of cells is proportional to the ligand-receptor function. Since the area is determined by ligand concentration and by the resultant function of the cell, drug efficacy, as well as drug potency, can be determined by this cell-based assay. Other measurements can be made as discussed above for cellular hypertrophy.

The methods for analyzing cellular morphology can be used in a combined high throughput-high content screen. In one example, the high throughput mode scans the whole well for an increase in fluorescent phalloidin intensity. A threshold is set above which both nuclei (Hoechst) and cells (phalloidin) are measured in a high content mode. In another example, an environmental biosensor (examples include, but are not limited to, those biosensors that are sensitive to calcium and pH changes) is added to the cells, and the cells are contacted with a compound. The cells are scanned in a high throughput mode, and those wells that exceed a pre-determined threshold for luminescence of the biosensor are scanned in a high content mode.

In a further aspect, kits are provided for analyzing cellular morphology, comprising a luminescent compound that can be used to specifically label the cell cytoplasm, membrane, or cytoskeleton (such as those described above), and instructions for using the luminescent compound to identify test stimuli that induce or inhibit changes in cellular morphology according to the above methods. In a preferred embodiment, the kit further comprises a luminescent marker for cell nuclei. In a further preferred embodiment, the kit comprises at least one compound that is known to modify cellular morphology, including, but not limited to integrin- or proteoglycan-blocking antibodies, signaling inhibitors including phosphatidyl inositol-3 kinase inhibitors, and cytoskeletal inhibitors such as cytochalasin D.

In another aspect, the present invention comprises a machine readable storage medium comprising a program containing a set of instructions for causing a cell screening system to execute the disclosed methods for analyzing cellular morphology, wherein the cell screening system comprises an optical system with a stage adapted for holding a plate containing cells, a digital camera, a means for directing fluorescence or luminescence emitted from the cells to the digital camera, and a computer means for receiving and processing the digital data from the digital camera.

### Example 3 Dual Mode High Throughput and High-Content Screen

The following example is a screen for activation of a G-protein coupled receptor (GPCR) as detected by the translocation of the GPCR from the plasma membrane to a proximal nuclear location. This example illustrates how a high throughput screen can be coupled with a high-content screen in the dual mode System for Cell Based Screening.

G-protein coupled receptors are a large class of 7 trans-membrane domain cell surface receptors. Ligands for these receptors stimulate a cascade of secondary signals in the cell, which may include, but are not limited to, Ca⁺⁺ transients, cyclic AMP production, inositol triphosphate (IP₃) production and phosphorylation. Each of these signals are rapid, occuring in a matter of seconds to minutes, but are also generic. For example, many different GPCRs produce a secondary Ca⁺⁺ signal when activated. Stimulation of a GPCR also results in the transport of that GPCR from the cell surface membrane to an internal, proximal nuclear compartment. This internalization is a much more receptor-specific indicator of activation of a particular receptor than are the secondary signals described above.

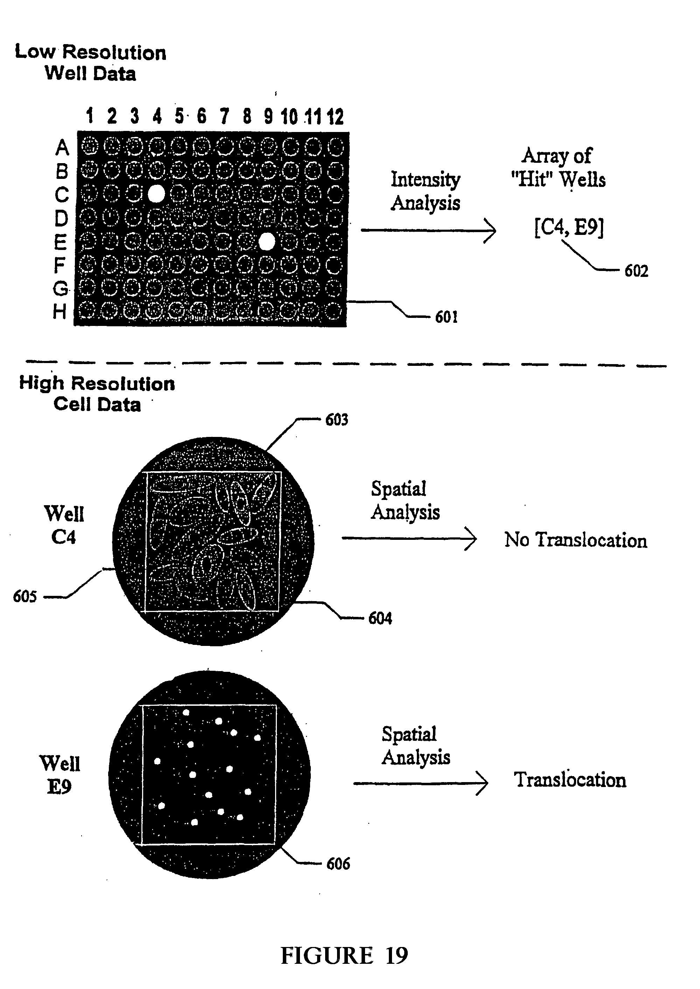

Figure 19 illustrates a dual mode screen for activation of a GPCR. Cells carrying a stable chimera of the GPCR with a blue fluorescent protein (BFP) would be loaded with the acetoxymethylester form of Fluo-3, a cell permeable calcium indicator (green fluorescence) that is trapped in living cells by the hydrolysis of the esters. They would then be deposited into the wells of a microtiter plate 601. The wells would then be treated with an array of test compounds using a fluid delivery system, and a short sequence of Fluo-3 images of the whole microtiter plate would be acquired and analyzed for wells exhibiting a calcium response (i.e., high throughput mode). The images would appear like the illustration of the microtiter plate 601 in Figure 19. A small number of wells, such as wells C4 and E9 in the illustration, would fluoresce more brightly due to the Ca⁺⁺ released upon stimulation of the receptors. The locations of wells containing compounds that induced a response 602, would then be transferred to the HCS program and the optics switched for detailed cell by cell analysis of the blue fluorescence for evidence of GPCR translocation to the perinuclear region. The bottom of Figure 19 illustrates the two possible outcomes of the analysis of the high resolution cell data. The camera images a sub-region 604 of the well area 603, producing images of the fluorescent cells 605. In well C4, the uniform distribution of the fluorescence in the cells indicates that the receptor has not internalized, implying that the Ca⁺⁺ response seen was the result of the stimulation of some other signalling system in the cell. The cells in well E9 606 on the other hand, clearly indicate a concentration of the receptor in the perinuclear region clearly indicating the full activation of the receptor. Because only a few hit wells have to be analyzed with high resolution, the overall throughput of the dual mode system can be quite high, comparable to the high throughput system alone.

### Example 4 Kinetic High Content Screen

The following is an example of a screen to measure the kinetics of internalization of a receptor. As described above, the stimulation of a GPCR, results in the internalization of the receptor, with a time course of about 15 min. Simply detecting the endpoint as internalized or not, may not be sufficient for defining the potency of a compound as a GPCR agonist or antagonist. However, 3 time points at 5 min intervals would provide information not only about potency during the time course of measurement, but would also allow extrapolation of the data to much longer time periods. To perform this assay, the sub-region would be defined as two rows, the sampling interval as 5 minutes and the total number of time points 3. The system would then start by scanning two rows, and then adding reagent to the two rows, establishing the time=0 reference. After reagent addition, the system would again scan the two row sub-region acquiring the first time point data. Since this process would take about 250 seconds, including scanning back to the beginning of the sub-region, the system would wait 50 seconds to begin acquisition of the second time point. Two more cycles would produce the three time points and the system would move on to the second 2 row sub-region. The final two 2-row sub-regions would be scanned to finish all the wells on the plate, resulting in four time points for each well over the whole plate. Although the time points for the wells would be offset slightly relative to time=0, the spacing of the time points would be very close to the required 5 minutes, and the actual acquisition times and results recorded with much greater precision than in a fixed-cell screen.

### Example 5 High-content screen of human glucocorticoid receptor translocation

One class of HCS involves the drug-induced dynamic redistribution of intracellular constituents. The human glucocorticoid receptor (hGR), a single "sensor" in the complex environmental response machinery of the cell, binds steroid molecules that have diffused into the cell. The ligand-receptor complex translocates to the nucleus where transcriptional activation occurs (Htun et al., *Proc. Natl. Acad. Sci.* 93:4845, 1996).

In general, hormone receptors are excellent drug targets because their activity lies at the apex of key intracellular signaling pathways. Therefore, a high-content screen of hGR translocation has distinct advantage over *in vitro* ligand-receptor binding assays. The availability of up to two more channels of fluorescence in the cell screening system of the present invention permits the screen to contain two additional parameters in parallel, such as other receptors, other distinct targets or other cellular processes.

***Plasmid construct.*** A eukaryotic expression plasmid containing a coding sequence for a green fluorescent protein - human glucocorticoid receptor (GFP-hGR) chimera was prepared using GFP mutants (Palm et al., *Nat. Struct. Biol.* 4:361 (1997). The construct was used to transfect a human cervical carcinoma cell line (HeLa).

***Cell preparation and transfection.*** HeLa cells (ATCC CCL-2) were trypsinized and plated using DMEM containing 5% charcoal/dextran-treated fetal bovine serum (FBS) (HyClone) and 1% penicillin-streptomycin (C-DMEM) 12-24 hours prior to transfection and incubated at 37°C and 5% CO₂. Transfections were performed by calcium phosphate co-precipitation (Graham and Van der Eb, *Virology* 52:456, 1973; Sambrook et al., (1989). *Molecular Cloning: A Laboratory Manual,* Second ed. Cold Spring Harbor Laboratory Press, Cold Spring Harbor, 1989) or with Lipofectamine (Life Technologies, Gaithersburg, MD). For the calcium phosphate transfections, the medium was replaced, prior to transfection, with DMEM containing 5% charcoal/dextran-treated FBS. Cells were incubated with the calcium phosphate-DNA precipitate for 4-5 hours at 37°C and 5% CO₂, washed 3-4 times with DMEM to remove the precipitate, followed by the addition of C-DMEM.

Lipofectamine transfections were performed in serum-free DMEM without antibiotics according to the manufacturer's instructions (Life Technologies, Gaithersburg, MD). Following a 2-3 hour incubation with the DNA-liposome complexes, the medium was removed and replaced with C-DMEM. All transfected cells in 96-well microtiter plates were incubated at 33°C and 5% CO₂ for 24-48 hours prior to drug treatment. Experiments were performed with the receptor expressed transiently in HeLa cells.

***Dexamethasone induction of GFP-hGR translocation.*** To obtain receptor-ligand translocation kinetic data, nuclei of transfected cells were first labeled with 5 µg/ml Hoechst 33342 (Molecular Probes) in C-DMEM for 20 minutes at 33°C and 5% CO₂. Cells were washed once in Hank's Balanced Salt Solution (HBSS) followed by the addition of 100 nM dexamethasone in HBSS with 1% charcoal/dextran-treated FBS. To obtain fixed time point dexamethasone titration data, transfected HeLa cells were first washed with DMEM and then incubated at 33°C and 5% CO₂ for 1 h in the presence of 0 - 1000 nM dexamethasone in DMEM containing 1% charcoal/dextran-treated FBS. Cells were analyzed live or they were rinsed with HBSS, fixed for 15 min with 3.7% formaldehyde in HBSS, stained with Hoechst 33342, and washed before analysis. The intracellular GFP-hGR fluorescence signal was not diminished by this fixation procedure.

***Image acquisition and analysis.*** Kinetic data were collected by acquiring fluorescence image pairs (GFP-hGR and Hoechst 33342-labeled nuclei) from fields of living cells at 1 min intervals for 30 min after the addition of dexamethasone. Likewise, image pairs were obtained from each well of the fixed time point screening plates 1 h after the addition of dexamethasone. In both cases, the image pairs obtained at each time point were used to define nuclear and cytoplasmic regions in each cell. Translocation of GFP-hGR was calculated by dividing the integrated fluorescence intensity of GFP-hGR in the nucleus by the integrated fluorescence intensity of the chimera in the cytoplasm or as a nuclear-cytoplasmic difference of GFP fluorescence. In the fixed time point screen this translocation ratio was calculated from data obtained from at least 200 cells at each concentration of dexamethasone tested. Drug-induced translocation of GFP-hGR from the cytoplasm to the nucleus was therefore correlated with an increase in the translocation ratio.

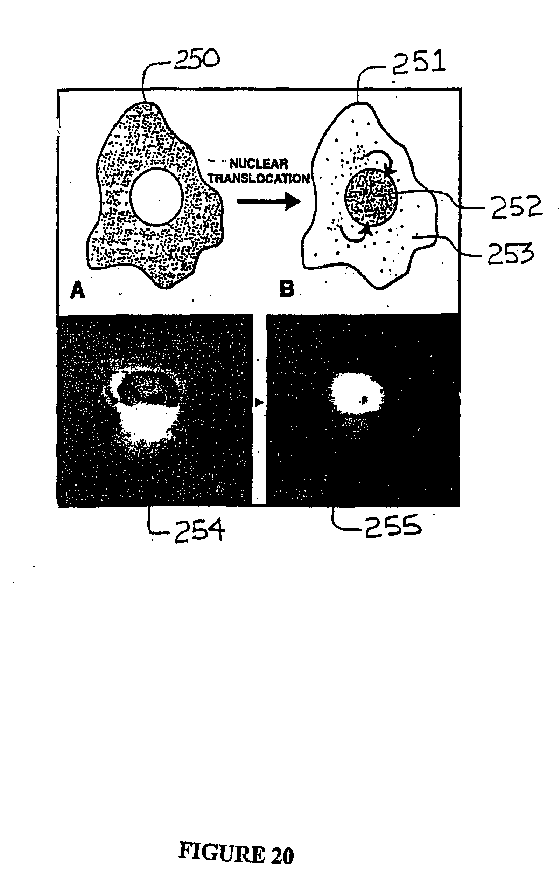

*Results.* Figure 20 schematically displays the drug-induced cytoplasm 253 to nucleus 252 translocation of the human glucocorticoid receptor. The upper pair of schematic diagrams depicts the localization of GFP-hGR within the cell before 250 (A) and after 251 (B) stimulation with dexamethasone. Under these experimental conditions, the drug induces a large portion of the cytoplasmic GFP-hGR to translocate into the nucleus. This redistribution is quantified by determining the integrated intensities ratio of the cytoplasmic and nuclear fluorescence in treated 255 and untreated 254 cells. The lower pair of fluorescence micrographs show the dynamic redistribution of GFP-hGR in a single cell, before 254 and after 255 treatment. The HCS is performed on wells containing hundreds to thousands of transfected cells and the translocation is quantified for each cell in the field exhibiting GFP fluorescence. Although the use of a stably transfected cell line would yield the most consistently labeled cells, the heterogeneous levels of GFP-hGR expression induced by transient transfection did not interfere with analysis by the cell screening system of the present invention.

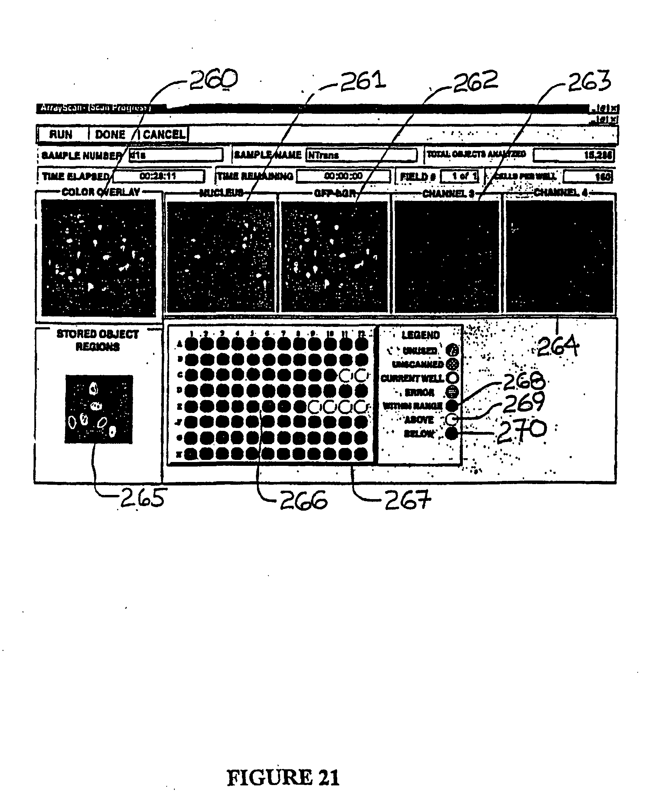

To execute the screen, the cell screening system scans each well of the plate, images a population of cells in each, and analyzes cells individually. Here, two channels of fluorescence are used to define the cytoplasmic and nuclear distribution of the GFP-hGR within each cell. Depicted in Figure 21 is the graphical user interface of the cell screening system near the end of a GFP-hGR screen. The user interface depicts the parallel data collection and analysis capability of the system. The windows labeled "Nucleus" 261 and "GFP-hGR" 262 show the pair of fluorescence images being obtained and analyzed in a single field. The window labeled "Color Overlay" 260 is formed by pseudocoloring the above images and merging them so the user can immediately identify cellular changes. Within the "Stored Object Regions" window 265, an image containing each analyzed cell and its neighbors is presented as it is archived. Furthermore, as the HCS data are being collected, they are analyzed, in this case for GFP-hGR translocation, and translated into an immediate "hit" response. The 96 well plate depicted in the lower window of the screen 267 shows which wells have met a set of user-defined screening criteria. For example, a white-colored well 269 indicates that the drug-induced translocation has exceeded a predetermined threshold value of 50%. On the other hand, a black-colored well 270 indicates that the drug being tested induced less than 10% translocation. Gray-colored wells 268 indicate "hits" where the translocation value fell between 10% and 50%. Row "E" on the 96 well plate being analyzed 266 shows a titration with a drug known to activate GFP-hGR translocation, dexamethasone. This example screen used only two fluorescence channels. Two additional channels (Channels 3 263 and 4 264) are available for parallel analysis of other specific targets, cell processes, or cytotoxicity to create multiple parameter screens.

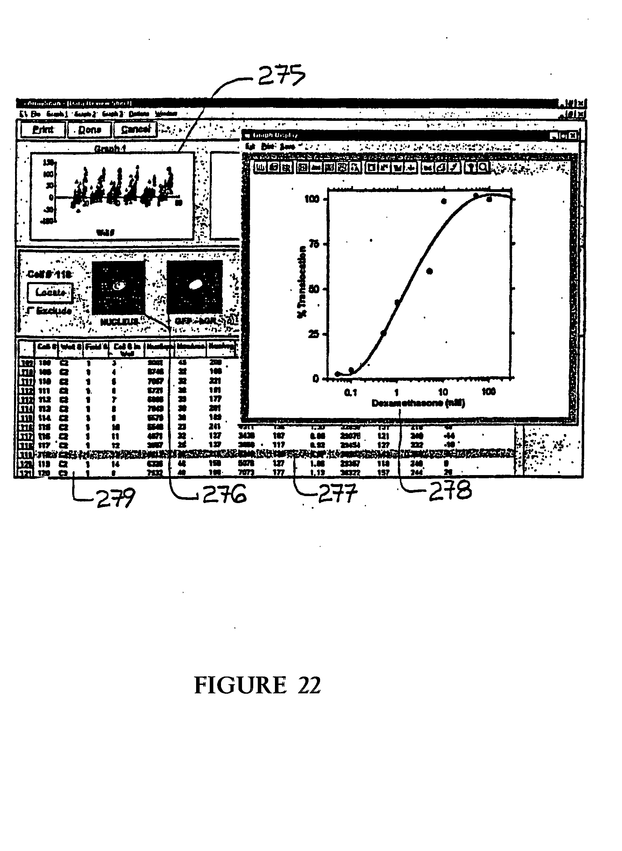

There is a link between the image database and the information database that is a powerful tool during the validation process of new screens. At the completion of a screen, the user has total access to image and calculated data (Figure 22). The comprehensive data analysis package of the cell screening system allows the user to examine HCS data at multiple levels. Images 276 and detailed data in a spread sheet 279 for individual cells can be viewed separately, or summary data can be plotted. For example, the calculated results of a single parameter for each cell in a 96 well plate are shown in the panel labeled Graph 1 275. By selecting a single point in the graph, the user can display the entire data set for a particular cell that is recalled from an existing database. Shown here are the image pair 276 and detailed fluorescence and morphometric data from a single cell (Cell #118, gray line 277). The large graphical insert 278 shows the results of dexamethasone concentration on the translocation of GFP-hGR. Each point is the average of data from at least 200 cells. The calculated EC₅₀ for dexamethasone in this assay is 2 nM.

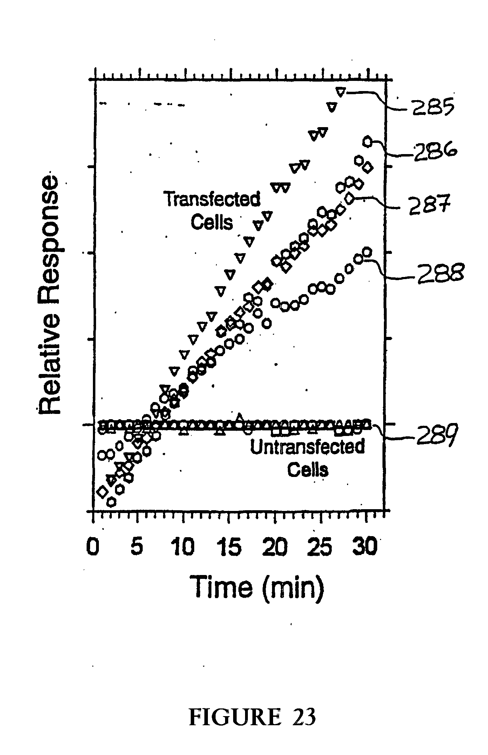

A powerful aspect of HCS with the cell screening system is the capability of kinetic measurements using multicolor fluorescence and morphometric parameters in living cells. Temporal and spatial measurements can be made on single cells within a population of cells in a field. Figure 23 shows kinetic data for the dexamethasone-induced translocation of GFP-hGR in several cells within a single field. Human HeLa cells transfected with GFP-hGR were treated with 100 nM dexamethasone and the translocation of GFP-hGR was measured over time in a population of single cells. The graph shows the response of transfected cells 285, 286, 287, and 288 and non-transfected cells 289. These data also illustrate the ability to analyze cells with different expression levels.

### Example 6 High-content screen of drug-induced apoptosis

Apoptosis is a complex cellular program that involves myriad molecular events and pathways. To understand the mechanisms of drug action on this process, it is essential to measure as many of these events within cells as possible with temporal and spatial resolution. Therefore, an apoptosis screen that requires little cell sample preparation yet provides an automated readout of several apoptosis-related parameters would be ideal. A cell-based assay designed for the cell screening system has been used to simultaneously quantify several of the morphological, organellar, and macromolecular hallmarks of paclitaxel-induced apoptosis.

***Cell preparation.*** The cells chosen for this study were mouse connective tissue fibroblasts (L-929; ATCC CCL-1) and a highly invasive glioblastoma cell line (SNB-19; ATCC CRL-2219) (Welch et al., *In Vitro Cell. Dev. Biol.* 31:610, 1995). The day before treatment with an apoptosis inducing drug, 3500 cells were placed into each well of a 96-well plate and incubated overnight at 37°C in a humidified 5% CO₂ atmosphere. The following day, the culture medium was removed from each well and replaced with fresh medium containing various concentrations of paclitaxel (0 - 50 µM) from a 20 mM stock made in DMSO. The maximal concentration of DMSO used in these experiments was 0.25%. The cells were then incubated for 26 h as above. At the end of the paclitaxel treatment period, each well received fresh medium containing 750 nM MitoTracker Red (Molecular Probes; Eugene, OR) and 3 µg/ml Hoechst 33342 DNA-binding dye (Molecular Probes) and was incubated as above for 20 min. Each well on the plate was then washed with HBSS and fixed with 3.7% formaldehyde in HBSS for 15 min at room temperature. The formaldehyde was washed out with HBSS and the cells were permeabilized for 90 s with 0.5% (v/v) Triton X-100, washed with HBSS, incubated with 2 U ml⁻¹ Bodipy FL phallacidin (Molecular Probes) for 30 min, and washed with HBSS. The wells on the plate were then filled with 200 µl HBSS, sealed, and the plate stored at 4°C if necessary. The fluorescence signals from plates stored this way were stable for at least two weeks after preparation. As in the nuclear translocation assay, fluorescence reagents can be designed to convert this assay into a live cell high-content screen.

***Image acquisition and analysis on the ArrayScan System.*** The fluorescence intensity of intracellular MitoTracker Red, Hoechst 33342, and Bodipy FL phallacidin was measured with the cell screening system as described *supra.* Morphometric data from each pair of images obtained from each well was also obtained to detect each object in the image field (*e.g*., cells and nuclei), and to calculate its size, shape, and integrated intensity.

***Calculations and output.*** A total of 50-250 cells were measured per image field. For each field of cells, the following calculations were performed: (1) The average nuclear area (µm²) was calculated by dividing the total nuclear area in a field by the number of nuclei detected. (2) The average nuclear perimeter (µm) was calculated by dividing the sum of the perimeters of all nuclei in a field by the number of nuclei detected in that field. Highly convoluted apoptotic nuclei had the largest nuclear perimeter values. (3) The average nuclear brightness was calculated by dividing the integrated intensity of the entire field of nuclei by the number of nuclei in that field. An increase in nuclear brightness was correlated with increased DNA content. (4) The average cellular brightness was calculated by dividing the integrated intensity of an entire field of cells stained with MitoTracker dye by the number of nuclei in that field. Because the amount of MitoTracker dye that accumulates within the mitochondria is proportional to the mitochondrial potential, an increase in the average cell brightness is consistent with an increase in mitochondrial potential. (5) The average cellular brightness was also calculated by dividing the integrated intensity of an entire field of cells stained with Bodipy FL phallacidin dye by the number of nuclei in that field. Because the phallotoxins bind with high affinity to the polymerized form of actin, the amount of Bodipy FL phallacidin dye that accumulates within the cell is proportional to actin polymerization state. An increase in the average cell brightness is consistent with an increase in actin polymerization.

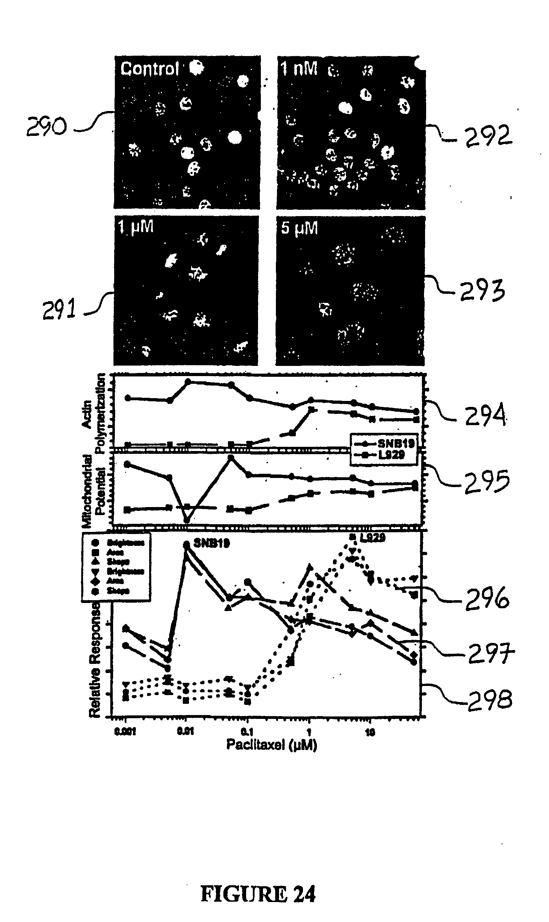

**Results.** Figure 24 (top panels) shows the changes paclitaxel induced in the nuclear morphology of L-929 cells. Increasing amounts of paclitaxel caused nuclei to enlarge and fragment 293, a hallmark of apoptosis. Quantitative analysis of these and other images obtained by the cell screening system is presented in the same figure. Each parameter measured showed that the L-929 cells 296 were less sensitive to low concentrations of paclitaxel than were SNB-19 cells 297. At higher concentrations though, the L-929 cells showed a response for each parameter measured. The multiparameter approach of this assay is useful in dissecting the mechanisms of drug action. For example, the area, brightness, and fragmentation of the nucleus 298 and actin polymerization values 294 reached a maximum value when SNB-19 cells were treated with 10 nM paclitaxel (Figure 24; top and bottom graphs). However, mitochondrial potential 295 was minimal at the same concentration of paclitaxel (Figure 24; middle graph). The fact that all the parameters measured approached control levels at increasing paclitaxel concentrations (>10 nM) suggests that SNB-19 cells have low affinity drug metabolic or clearance pathways that are compensatory at sufficiently high levels of the drug. Contrasting the drug sensitivity of SNB-19 cells 297, L-929 showed a different response to paclitaxel 296. These fibroblastic cells showed a maximal response in many parameters at 5 µM paclitaxel, a 500-fold higher dose than SNB-19 cells. Furthermore, the L-929 cells did not show a sharp decrease in mitochondrial potential 295 at any of the paclitaxel concentrations tested. This result is consistent with the presence of unique apoptosis pathways between a normal and cancer cell line. Therefore, these results indicate that a relatively simple fluorescence labeling protocol can be coupled with the cell screening system of the present invention to produce a high-content screen of key events involved in programmed cell death.

### Background

A key to the mechanism of apoptosis was the discovery that, irrespective of the lethal stimulus, death results in identical apoptotic morphology that includes cell and organelle dismantling and repackaging, DNA cleavage to nucleosome sized fragments, and engulfment of the fragmented cell to avoid an inflammatory response. Apoptosis is therefore distinct from necrosis, which is mediated more by acute trauma to a cell, resulting in spillage of potentially toxic and antigenic cellular components into the intercellular milieu, leading to an inflammatory response.

The criteria for determining whether a cell is undergoing apoptosis (Wyllie et al. 1980. *Int Rev Cytol.* 68:251-306; Thompson, 1995. *Science.* 267:1456-62; Majno and Joris. 1995. *Am J Pathol.* 146:3-15; Allen et al. 1998. *Cell Mol Life Sci.* 54:427-45) include distinct morphological changes in the appearance of the cell, as well as alterations in biochemical and molecular markers. For example, apoptotic cells often undergo cytoplasmic membrane blebbing, their chromosomes rapidly condense and aggregate around the nuclear periphery, the nucleus fragments, and small apoptotic bodies are formed. In many, but not all, apoptotic cells, chromatin becomes a target for specific nucleases that cleave the DNA.

Apoptosis is commonly accompanied by a characteristic change in nuclear morphology (chromatin condensation or fragmentation) and a step-wise fragmentation of DNA culminating in the formation of mono- and/or oligomeric fragments of 200 base pairs. Specific changes in organellar function, such as mitochondrial membrane potential, occur. In addition, specific cysteine proteases (caspases) are activated, which catalyzes a highly selective pattern of protein degradation by proteolytic cleavage after specific aspartic acid residues. In addition, the external surface exposure of phosphatidylserine residues (normally on the inner membrane leaflet) allows for the recognition and elimination of apoptotic cells, before the membrane breaks up and cytosol or organelles spill into the intercellular space and elicit inflammatory reactions. Moreover, cells undergoing apoptosis tend to shrink, while also having a reduced intracellular potassium level.

The general patterns of apoptotic signals are very similar among different cell types and apoptotic inducers. However, the details of the pathways actually vary significantly depending on cell type and inducer. The dependence and independence of various signal transduction pathways involved in apoptosis are currently topics of intense research. We show here that the pathway also varies depending upon the dose of the inducer in specific cell types.

### Nuclear Morphology

Cells undergoing apoptosis generally exhibit two types of nuclear change, fragmentation or condensation ((Majno and Joris, 1995), (Earnshaw, 1995)). The response in a given cell type appears to vary depending on the apoptotic inducer. During nuclear fragmentation, a circular or oval nucleus becomes increasingly lobular. Eventually, the nucleus fragments dramatically into multiple sub-nuclei. Sometimes the density of the chromatin within the lobular nucleus may show spatial variations in distribution (heterochromatization), approximating the margination seen in nuclear condensation.

Nuclear condensation has been reported in some cell types, such as MCF-7 (Saunders et al. 1997. *Int J Cancer.* 70:214-20). Condensation appears to arise as a consequence of the loss of structural integrity of the euchromatin, nuclear matrix and nuclear lamina (Hendzel et al. 1998. *J Biol Chem.* 273:24470-8). During nuclear condensation, the chromatin concentrates near the margin of the nucleus, leading to the overall shrinkage of the nucleus. Thus, the use of nuclear morphology as a measure of apoptosis must take both condensation and fragmentation into account.

### Material and Methods

Cells were plated into 96-well plates at densities of 3 x 10³ to 1 x 10⁴ cells/well. The following day apoptotic inducers were added at indicated concentrations and cells were incubated for indicated time periods (usually 16-30 hours). The next day medium was removed and cells were stained with 5 µg/ml Hoechst (Molecular Probes, Inc.) in fresh medium and incubated for 30 minutes at 37°C. Cells were washed in Hank's Balanced Salt Solution (HBSS) and fixed with 3.7% formaldehyde in HBSS at room temperature. Cells were washed 2X with HBSS at room temperature and the plate was sealed.

Quantitation of changes in nuclear morphology upon induction of apoptosis was accomplished by (1) measuring the effective size of the nuclear region; and (2) measuring the degree of convolution of the perimeter. The size parameter provides the more sensitive measure of nuclear condensation, whereas the perimeter measure provides a more sensitive measure of nuclear fragmentation.

### Results & Discussion

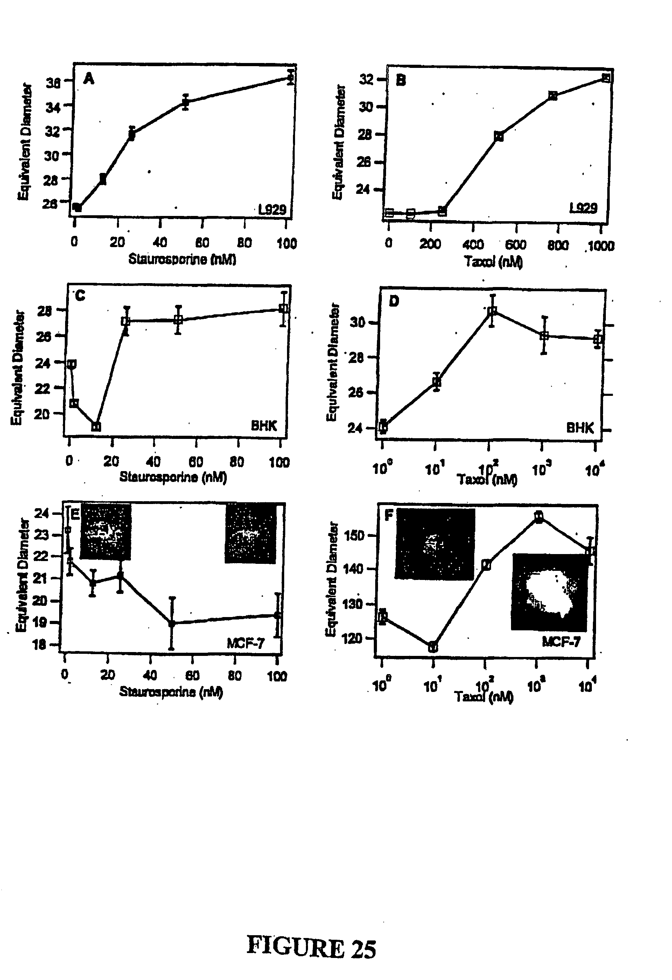

L929 cells responded to both staurosporine (30 hours) and paclitaxel (30 hours) with a dose-dependent change in nuclear morphology (**Fig 25A and 25B**). BHK cells illustrated a slightly more complicated, yet clearly visible response. Staurosporine appeared to stimulate nuclear condensation at lower doses and nuclear fragmentation at higher doses (**Fig 25C and 25D**). In contrast, paclitaxel induced a consistent increase in nuclear fragmentation with increasing concentrations. The response of MCF-7 cells varied dramatically depending upon the apoptotic inducer. Staurosporine appeared to elicit nuclear condensation whereas paclitaxel induced nuclear fragmentation **(Fig 25E and 25F).**

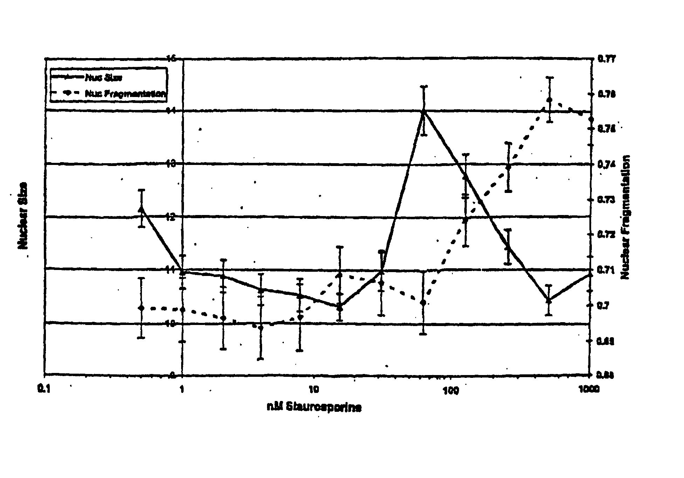

**Figure 26** illustrates the dose response of cells in terms of both nuclear size and nuclear perimeter convolution. There appears to be a swelling of the nuclei that precedes the fragmentation.

**Result of evaluation:** Differential responses by cell lines and by apoptotic inducers were observed in a dose dependent manner, indicating that this assay will be useful for detecting changes in the nucleus characteristic of apoptosis.

### Actin reorganization

We assessed changes in the actin cytoskeleton as a potential parameter related to apoptotic changes. This was based on preliminary observations of an early increase in f-actin content detected with fluorescent phalloidin labeling, an f-actin specific stain (our unpublished data; Levee et al. 1996. *Am J Physiol.* 271:C1981-92; Maekawa et al. 1996. *Clin Exp Immunol.* 105:389-96). Changes in the actin cytoskeleton during apoptosis have not been observed in all cell types. (Endresen et al. 1995. *Cytometry.* 20:162-71, van Engeland et al. 1997. *Exp Cell Res.* 235:421-30).

### Material and Methods

Cells were plated in 96-well plates at densities of 3 x 10³ to 1 x 10⁴ cells/well. The following day apoptotic inducers were added at indicated concentrations. Cells were incubated for the indicated time periods (usually 16-30 hours). The next day the medium was removed and cells were stained with 5 µg/ml Hoechst (Molecular Probes, Inc.) in fresh medium and incubated for 30 minutes at 30°C. Cells were washed in HBSS and fixed with 3.7% formaldehyde in HBSS at room temperature. Plates were washed with HBSS and permeabilized with 0.5% v/v Triton X-100 in HBSS at room temperature. Plates were washed in HBSS and stained with 100 µl of 1U/ml of Alexa 488 Phalloidin stock (100 µl/well, Molecular Probes, Inc.). Cells were washed 2X with HBSS at RT and the plate was sealed.

Quantitation of f-actin content was accomplished by measuring the intensity of phalloidin staining around the nucleus. This was determined to be a reasonable approximation of a full cytoplasmic average of the intensity. The mask used to approximate this cytoplasmic measure was derived from the nuclear mask defined by the Hoechst stain. Derivation was accomplished by combinations of erosions and dilations.

### Results and Discussion

Changes in f-actin content varied based on cell type and apoptotic inducer **(Fig 27**). Staurosporine (30 hours) induced increases in f-actin in L929 (**Fig. 27A**) and BHK (**Fig. 27B**) cells. MCF-7 cells exhibited a concentration-dependent response. At low concentrations **(Fig. 27E)** there appeared to be a decrease in f-actin content. At higher concentrations, f-actin content increased. Paclitaxel (30 hours) treatment led to a wide variety of responses. L929 cells responded with graded increases in f-actin (**Fig. 27B**) whereas both BHK and MCF-7 responses were highly variable (**Figs. 27D & 27F,** respectively).

**Result of Evaluation:** Both increases and decreases in signal intensity were measured for several cell lines and found to exhibit a concentration dependent response. For certain cell line/apoptotic inducer pairs this could be a statistically significant apoptotic indicator.

### Changes in Mitochondrial Mass/Potential

### Introduction

Changes in mitochondria play a central role in apoptosis (Henkart and Grinstein. 1996. *J Exp Med.* 183:1293-5). Mitochondria release apoptogenic factors through the outer membrane and dissipate the electrochemical gradient of the inner membrane. This is thought to occur via formation of the mitochondria permeability transition (MPT), although it is apparently not true in all cases. An obvious manifestation of the formation of the MPT is collapse of the mitochondrial membrane potential. Inhibition of MPT by pharmacological intervention or mitochondrial expression of the anti-apoptotic protein Bc1-2 prevents cell death, suggesting the formation of the MPT may be a rate-limiting event of the death process (For review see: Kroemer et al. 1998. *Annu Rev Physiol.* 60:619-42). It has also been observed that mitochondria can proliferate during stimulation of apoptosis (Mancini et al. 1997. *J Cell Biol.* 138:449-69; Camilleri-Broet et al. 1998. *Exp Cell Res.* 239:277-92).

One approach for measuring apoptosis-induced changes in mitochondria is to measure the mitochondrial membrane potential. Of the methods available, the simplest measure is the redistribution of a cationic dye that distributes within intracellular organelles based on the membrane potential. Such an approach traditionally requires live cells for the measurements. The recent introduction of the MitoTracker dyes (Poot et al. 1997. *Cytometry.* 27:358-64; available from Molecular Probes, Inc., Oregon) provides a means of measuring mitochondrial membrane potential after fixation.

Given the observations of a possible increase in mitochondrial mass during apoptosis, the amount of dye labeling the mitochondria is related to both membrane potential and the number of mitochondria. If the number of mitochondria remains constant then the amount of dye is directly related to the membrane potential. If the number of mitochondria is not constant, then the signal will likely be dominated by the increase in mass (Reipert et al. 1995. *Exp Cell Res.* 221:281-8).

Probes are available that allow a clear separation between changes in mass and potential in HCS assays. Mitochondrial mass is measured directly by labeling with Mitotracker Green FM (Poot and Pierce, 1999, *Cytometry.* 35:311-7; available from Molecular Probes, Inc., Oregon). The labeling is independent of mitochondrial membrane potential but proportional to mitochondrial mass. This also provides a means of normalizing other mitochondrial measures in each cell with respect to mitochondrial mass.

### Material and Methods

Cells were plated into 96-well plates at densities of 3 x 10³ to 1 x 10⁴ cells/well. The following day apoptotic inducers were added at the indicated concentrations and cells were incubated for the indicated time periods (usually 16-30 hours). Cells were stained with 5 µg/ml Hoechst (Molecular Probes, Inc.) and 750 nM MitoTracker Red (CMXRos, Molecular Probes, Inc.) in fresh medium and incubated for 30 minutes at 37°C. Cells were washed in HBSS and fixed with 3.7% formaldehyde in HBSS at room temperature. Plates were washed with HBSS and permeabilized with 0.5% v/v Triton X-100 in HBSS at room temperature. Cells were washed 2X with HBSS at room temperature and the plate was sealed. For dual labeling of mitochondria, cells were treated with 200 nM Mitotracker Green and 200 nM Mitotracker Red for 0.5 hours before fixation.

### Results & Discussion

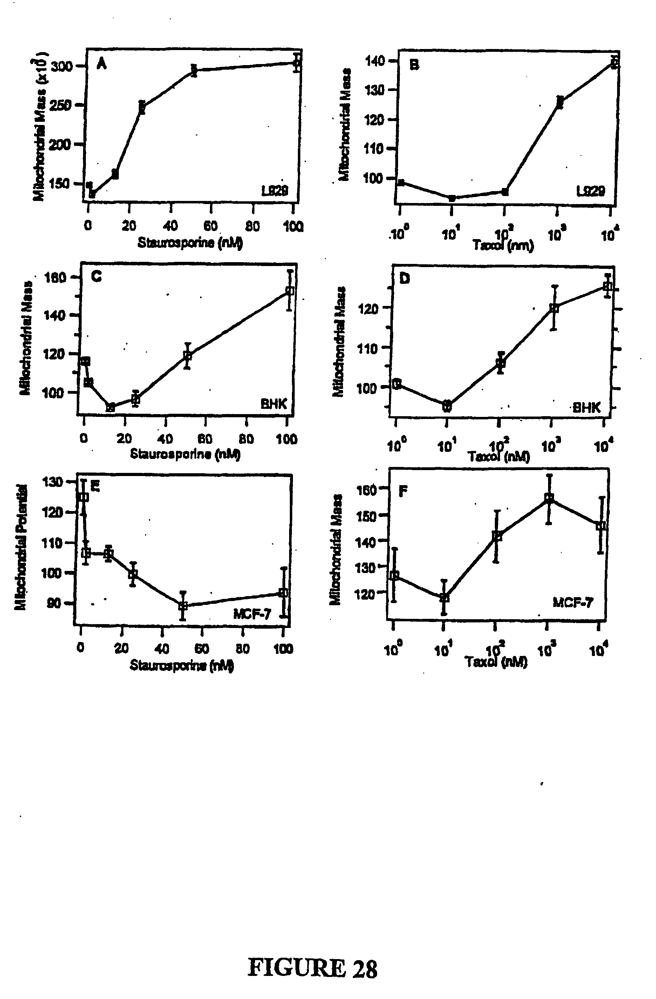

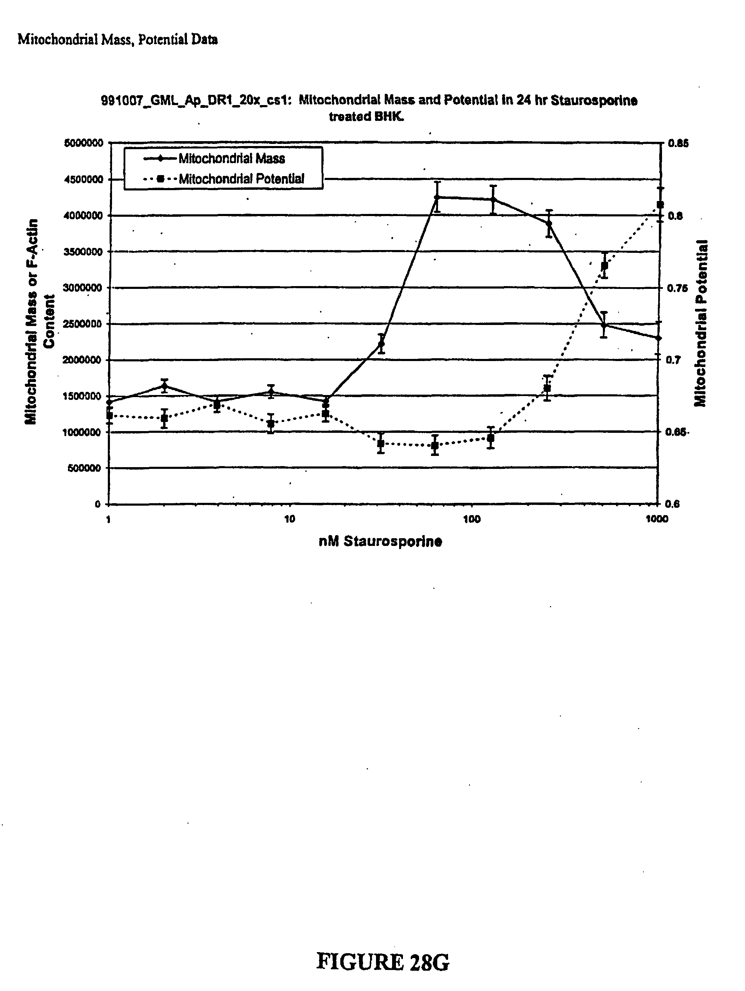

Induction of apoptosis by staurosporine and paclitaxel led to varying mitochondrial changes depending upon the stimulus. L929 cells exhibited a clear increase in mitochondrial mass with increasing staurosporine concentrations (**Fig. 28**). BHK cells exhibited either a decrease in membrane potential at lower concentrations of staurosporine, or an increase in mass at higher concentrations of staurosporine (Fig. 28C). MCF-7 cells responded by a consistent decrease in mitochondrial membrane potential in response to increasing concentrations of staurosporine (**Fig 28E**). Increasing concentrations of paclitaxel caused consistent increases in mitochondrial mass **(Fig 28B, 28D,** and **28F).**

The mitochondrial membrane potential is measured by labeling mitochondria with both Mitotracker Green FM and Mitotracker Red (Molecular Probes, Inc). Mitotracker Red labeling is proportional to both mass and membrane potential. Mitotracker Green FM labeling is proportional to mass. The ratio of Mitotracker Red signal to the Mitotracker Green FM signal provides a measure of mitochondrial membrane potential (Poot and Pierce, 1999). This ratio normalizes the mitochondrial mass with respect to the Mitotracker Red signal. (**See Figure 28G**) Combining the ability to normalize to mitochondrial mass with a measure of the membrane potential allows independent assessment of both parameters.

**Result of Evaluation:** Both decreases in potential and increases in mass were observed depending on the cell line and inducer tested. Dose dependent correlation demonstrates that this is a promising apoptotic indicator.

It is possible to combine multiple measures of apoptosis by exploiting the spectral domain of fluorescence spectroscopy. In fact, all of the nuclear morphology/factin content/mitochondrial mass/mitochondrial potential data shown earlier were collected as multiparameter assays, but were presented individually for clarity.

### Example 7. Protease induced translocation of a signaling enzyme containing a disease-associated sequence from cytoplasm to nucleus.

***Plasmid construct.*** A eukaryotic expression plasmid containing a coding sequence for a green fluorescent protein - caspase (Cohen (1997), *Biochemical J.* 326:1-16; Liang et al. (1997), *J. of Molec. Biol.* 274:291-302) chimera is prepared using GFP mutants. The construct is used to transfect eukaryotic cells.

***Cell preparation and transfection.*** Cells are trypsinized and plated 24 h prior to transfection and incubated at 37°C and 5% CO₂. Transfections are performed by methods including, but not limited to calcium phosphate coprecipitation or lipofection. Cells are incubated with the calcium phosphate-DNA precipitate for 4-5 hours at 37°C and 5% CO₂, washed 3-4 times with DMEM to remove the precipitate, followed by the addition of C-DMEM. Lipofectamine transfections are performed in serum-free DMEM without antibiotics according to the manufacturer's instructions. Following a 2-3 hour incubation with the DNA-liposome complexes, the medium is removed and replaced with C-DMEM.

***Apopototic induction of Caspase-GFP translocation.*** To obtain Caspase-GFP translocation kinetic data, nuclei of transfected cells are first labeled with 5 µg/ml Hoechst 33342 (Molecular Probes) in C-DMEM for 20 minutes at 37°C and 5% CO₂. Cells are washed once in Hank's Balanced Salt Solution (HBSS) followed by the addition of compounds that induce apoptosis. These compounds include, but are not limited to paclitaxel, staurosporine, ceramide, and tumor necrosis factor. To obtain fixed time point titration data, transfected cells are first washed with DMEM and then incubated at 37°C and 5% CO₂ for 1 h in the presence of 0 - 1000 nM compound in DMEM. Cells are analyzed live or they are rinsed with HBSS, fixed for 15 min with 3.7% formaldehyde in HBSS, stained with Hoechst 33342, and washed before analysis.

***Image acquisition and analysis.*** Kinetic data are collected by acquiring fluorescence image pairs (Caspase-GFP and Hoechst 33342-labeled nuclei) from fields of living cells at 1 min intervals for 30 min after the addition of compound. Likewise, image pairs are obtained from each well of the fixed time point screening plates 1 h after the addition of compound. In both cases, the image pairs obtained at each time point are used to define nuclear and cytoplasmic regions in each cell. Translocation of Caspase-GFP is calculated by dividing the integrated fluorescence intensity of Caspase-GFP in the nucleus by the integrated fluorescence intensity of the chimera in the cytoplasm or as a nuclear-cytoplasmic difference of GFP fluorescence. In the fixed time point screen this translocation ratio is calculated from data obtained from at least 200 cells at each concentration of compound tested. Drug-induced translocation of Caspase-GFP from the cytoplasm to the nucleus is therefore correlated with an increase in the translocation ratio. Molecular interaction libraries including, but not limited to those comprising putative activators or inhibitors of apoptosis-activated enzymes are use to screen the indicator cell lines and identify a specific ligand for the DAS, and a pathway activated by compound activity.

### Example 8. Identification of novel steroid receptors from DAS

Two sources of material and/or information are required to make use of this embodiment, which allows assessment of the function of an uncharacterized gene. First, disease associated sequence bank(s) containing cDNA sequences suitable for transfection into mammalian cells can be used. Because every RADE or differential expression experiment generates up to several hundred sequences, it is possible to generate an ample supply of DAS. Second, information from primary sequence database searches can be used to place DAS into broad categories, including, but not limited to, those that contain signal sequences, seven trans-membrane motifs, conserved protease active site domains, or other identifiable motifs. Based on the information acquired from these sources, method types and indicator cell lines to be transfected are selected. A large number of motifs are already well characterized and encoded in the linear sequences contained within the large number genes in existing genomic databases.

In one embodiment, the following steps are taken:
1) Information from the DAS identification experiment (including database searches) is used as the basis for selecting the relevant biological processes. (for example, look at the DAS from a tumor line for cell cycle modulation, apoptosis, metastatic proteases, etc.)
2) Sorting of DNA sequences or DAS by identifiable motifs (ie. signal sequences, 7- transmembrane domains, conserved protease active site domains, etc.) This initial grouping will determine fluorescent tagging strategies, host cell lines, indicator cell lines, and banks of bioactive molecules to be screened, as described *supra.*
3) Using well established molecular biology methods, ligate DAS into an expression vector designed for this purpose. Generalized expression vectors contain promoters, enhancers, and terminators for which to deliver target sequences to the cell for transient expression. Such vectors may also contain antibody tagging sequences, direct association sequences, chromophore fusion sequences like GFP, etc. to facilitate detection when expressed by the host.
4) Transiently transfect cells with DAS containing vectors using standard transfection protocols including: calcium phosphate co-precipitation, liposome mediated, DEAE dextran mediated, polycationic mediated, viral mediated, or electroporation, and plate into microtiter plates or microwell arrays. Alternatively, transfection can be done directly in the microtiter plate itself.
5) Carry out the cell screening methods as described *supra.*

In this embodiment, DAS shown to possess a motif(s) suggestive of transcriptional activation potential (for example, DNA binding domain, amino terminal modulating domain, hinge region, or carboxy terminal ligand binding domain) are utilized to identify novel steroid receptors.

Defining the fluorescent tags for this experiment involves identification of the nucleus through staining, and tagging the DAS by creating a GFP chimera via insertion of DAS into an expression vector, proximally fused to the gene encoding GFP. Alternatively, a single chain antibody fragment with high affinity to some portion of the expressed DAS could be constructed using technology available in the art (Cambridge Antibody Technologies) and linked to a fluorophore (FITC) to tag the putative transcriptional activator/receptor in the cells. This alternative would provide an external tag requiring no DNA transfection and therefore would be useful if distribution data were to be gathered from the original primary cultures used to generate the DAS.

***Plasmid construct.*** A eukaryotic expression plasmid containing a coding sequence for a green fluorescent protein - DAS chimera is prepared using GFP mutants. The construct is used to transfect HeLa cells. The plasmid, when transfected into the host cell, produces a GFP fused to the DAS protein product, designated GFP-DASpp.

***Cell preparation and transfection.*** HeLa cells are trypsinized and plated using DMEM containing 5% charcoal/dextran-treated fetal bovine serum (FBS) (Hyclone) and 1% penicillin-streptomycin (C-DMEM) 12-24 hours prior to transfection and incubated at 37°C and 5% CO₂ . Transfections are performed by calcium phosphate coprecipitation or with Lipofectamine (Life Technologies). For the calcium phosphate transfections, the medium is replaced, prior to transfection, with DMEM containing 5% charcoal/dextran-treated FBS. Cells are incubated with the calcium phosphate-DNA precipitate for 4-5 hours at 37°C and 5% CO₂, and washed 3-4 times with DMEM to remove the precipitate, followed by the addition of C-DMEM. Lipofectamine transfections are performed in serum-free DMEM without antibiotics according to the manufacturer's instructions. Following a 2-3 hour incubation with the DNA-liposome complexes, the medium is removed and replaced with C-DMEM. All transfected cells in 96-well microtiter plates are incubated at 33°C and 5% CO₂ for 24-48 hours prior to drug treatment. Experiments are performed with the receptor expressed transiently in HeLa cells.

***Localization of expressed GFP-DASpp inside cells.*** To obtain cellular distribution data, nuclei of transfected cells are first labeled with 5 µg/ml Hoechst 33342 (Molecular Probes) in C-DMEM for 20 minutes at 33°C and 5% CO₂. Cells are washed once in Hank's Balanced Salt Solution (HBSS). The cells are analyzed live or they are rinsed with HBSS, fixed for 15 min with 3.7% formaldehyde in HBSS, stained with Hoechst 33342, and washed before analysis.

In a preferred embodiment, image acquisition and analysis are performed using the cell screening system of the present invention. The intracellular GFP-DASpp fluorescence signal is collected by acquiring fluorescence image pairs (GFP-DASpp and Hoechst 33342-labeled nuclei) from field cells. The image pairs obtained at each time point are used to define nuclear and cytoplasmic regions in each cell. Data demonstrating dispersed signal in the cytoplasm would be consistent with known steroid receptors that are DNA transcriptional activators.

***Screening for induction of GFP-DASpp translocation.*** Using the above construct, confirmed for appropriate expression of the GFP-DASpp, as an indicator cell line, a screen of various ligands is performed using a series of steroid type ligands including, but not limited to: estrogen, progesterone, retinoids, growth factors, androgens, and many other steroid and steroid based molecules. Image acquisition and analysis are performed using the cell screening system of the invention. The intracellular GFP-DASpp fluorescence signal is collected by acquiring fluorescence image pairs (GFP-DASpp and Hoechst 33342-labeled nuclei) from fields cells. The image pairs obtained at each time point are used to define nuclear and cytoplasmic regions in each cell. Translocation of GFP-DASpp is calculated by dividing the integrated fluorescence intensity of GFP-DASpp in the nucleus by the integrated fluorescence intensity of the chimera in the cytoplasm or as a nuclear-cytoplasmic difference of GFP fluorescence. A translocation from the cytoplasm into the nucleus indicates a ligand binding activation of the DASpp thus identifying the potential receptor class and action. Combining this data with other data obtained in a similar fashion using known inhibitors and modifiers of steroid receptors, would either validate the DASpp as a target, or more data would be generated from various sources.

### Example 9 Additional Screens

### Translocation between the plasma membrane and the cytoplasm:

**Profilactin complex dissociation and binding of profilin to the plasma membrane.** In one embodiment, a fluorescent protein biosensor of profilin membrane binding is prepared by labeling purified profilin (Federov et al.(1994), *J. Molec. Biol.* 241:480-482; Lanbrechts et al. (1995), *Eur. J. Biochem.* 230:281-286) with a probe possessing a fluorescence lifetime in the range of 2-300 ns. The labeled profilin is introduced into living indicator cells using bulk loading methodology and the indicator cells are treated with test compounds. Fluorescence anisotropy imaging microscopy (Gough and Taylor (1993), *J. Cell Biol.* 121:1095-1107) is used to measure test-compound dependent movement of the fluorescent derivative of profilin between the cytoplasm and membrane for a period of time after treatment ranging from 0.1 s to 10 h.

**Rho-RhoGDI complex translocation to the membrane.** In another embodiment, indicator cells are treated with test compounds and then fixed, washed, and permeabilized. The indicator cell plasma membrane, cytoplasm, and nucleus are all labeled with distinctly colored markers followed by immunolocalization of Rho protein (Self et al. (1995), *Methods in Enzymology* 256:3-10; Tanaka et al. (1995), *Methods in Enzymology* 256:41-49) with antibodies labeled with a fourth color. Each of the four labels is imaged separately using the cell screening system, and the images used to calculate the amount of inhibition or activation of translocation effected by the test compound. To do this calculation, the images of the probes used to mark the plasma membrane and cytoplasm are used to mask the image of the immunological probe marking the location of intracellular Rho protein. The integrated brightness per unit area under each mask is used to form a translocation quotient by dividing the plasma membrane integrated brightness/area by the cytoplasmic integrated brightness/area. By comparing the translocation quotient values from control and experimental wells, the percent translocation is calculated for each potential lead compound.

### β-Arrestin translocation to the plasma membrane upon G-protein receptor activation.

In another embodiment of a cytoplasm to membrane translocation high-content screen, the translocation of β-arrestin protein from the cytoplasm to the plasma membrane is measured in response to cell treatment. To measure the translocation, living indicator cells containing luminescent domain markers are treated with test compounds and the movement of the β-arrestin marker is measured in time and space using the cell screening system of the present invention. In a preferred embodiment, the indicator cells contain luminescent markers consisting of a green fluorescent protein β-arrestin (GFP-β-arrestin) protein chimera (Barak et al. (1997), *J. Biol. Chem.* 272:27497-27500; Daaka et al. (1998), *J. Biol. Chem.* 273:685-688) that is expressed by the indicator cells through the use of transient or stable cell transfection and other reporters used to mark cytoplasmic and membrane domains. When the indicator cells are in the resting state, the domain marker molecules partition predominately in the plasma membrane or in the cytoplasm. In the high-content screen, these markers are used to delineate the cell cytoplasm and plasma membrane in distinct channels of fluorescence. When the indicator cells are treated with a test compound, the dynamic redistribution of the GFP-β-arrestin is recorded as a series of images over a time scale ranging from 0.1 s to 10 h. In a preferred embodiment, the time scale is 1 h. Each image is analyzed by a method that quantifies the movement of the GFP-β-arrestin protein chimera between the plasma membrane and the cytoplasm. To do this calculation, the images of the probes used to mark the plasma membrane and cytoplasm are used to mask the image of the GFP-β-arrestin probe marking the location of intracellular GFP-β-arrestin protein. The integrated brightness per unit area under each mask is used to form a translocation quotient by dividing the plasma membrane integrated brightness/area by the cytoplasmic integrated brightness/area. By comparing the translocation quotient values from control and experimental wells, the percent translocation is calculated for each potential lead compound. The output of the high-content screen relates quantitative data describing the magnitude of the translocation within a large number of individual cells that have been treated with test compounds of interest.

### Translocation between the endoplasmic reticulum and the Golgi:

In one embodiment of an endoplasmic reticulum to Golgi translocation high-content screen, the translocation of a VSVG protein from the ts045 mutant strain of vesicular stomatitis virus (Ellenberg et al. (1997), *J. Cell Biol.* 138:1193-1206; Presley et al. (1997) *Nature* 389:81-85) from the endoplasmic reticulum to the Golgi domain is measured in response to cell treatment. To measure the translocation, indicator cells containing luminescent reporters are treated with test compounds and the movement of the reporters is measured in space and time using the cell screening system of the present invention. The indicator cells contain luminescent reporters consisting of a GFP-VSVG protein chimera that is expressed by the indicator cell through the use of transient or stable cell transfection and other domain markers used to measure the localization of the endoplasmic reticulum and Golgi domains. When the indicator cells are in their resting state at 40°C, the GFP-VSVG protein chimera molecules are partitioned predominately in the endoplasmic reticulum. In this high-content screen, domain markers of distinct colors used to delineate the endoplasmic reticulum and the Golgi domains in distinct channels of fluorescence. When the indicator cells are treated with a test compound and the temperature is simultaneously lowered to 32°C, the dynamic redistribution of the GFP-VSVG protein chimera is recorded as a series of images over a time scale ranging from 0.1 s to 10 h. Each image is analyzed by a method that quantifies the movement of the GFP-VSVG protein chimera between the endoplasmic reticulum and the Golgi domains. To do this calculation, the images of the probes used to mark the endoplasmic reticulum and the Golgi domains are used to mask the image of the GFP-VSVG probe marking the location of intracellular GFP-VSVG protein. The integrated brightness per unit area under each mask is used to form a translocation quotient by dividing the endoplasmic reticulum integrated brightness/area by the Golgi integrated brightness/area. By comparing the translocation quotient values from control and experimental wells, the percent translocation is calculated for each potential lead compound. The output of the high-content screen relates quantitative data describing the magnitude of the translocation within a large number of individual cells that have been treated with test compounds of interest at final concentrations ranging from 10⁻¹² M to 10⁻³ M for a period ranging from 1 min to 10 h.

### Induction and inhibition of organellar function:

### Intracellular microtubule stability.

In another aspect of the invention, an automated method for identifying compounds that modify microtubule structure is provided. In this embodiment, indicator cells are treated with test compounds and the distribution of luminescent microtubule-labeling molecules is measured in space and time using a cell screening system, such as the one disclosed above. The luminescent microtubule-labeling molecules may be expressed by or added to the cells either before, together with, or after contacting the cells with a test compound.

In one embodiment of this aspect of the invention, living cells express a luminescently labeled protein biosensor of microtubule dynamics, comprising a protein that labels microtubules fused to a luminescent protein. Appropriate microtubule-labeling proteins for this aspect of the invention include, but are not limited to α and β tubulin isoforms, and MAP4. Preferred embodiments of the luminescent protein include, but are not limited to green fluorescent protein (GFP) and GFP mutants. In a preferred embodiment, the method involves transfecting cells with a microtubule labeling luminescent protein, wherein the microtubule labeling protein can be, but is not limited to, α-tubulin, β-tubulin, or microtubule-associated protein 4 (MAP4). The approach outlined here enables those skilled in the art to make live cell measurements to determine the effect of lead compounds on tubulin activity and microtubule stability *in vivo.*

In a most preferred embodiment, MAP4 is fused to a modified version of the *Aequorea victoria* green fluorescent protein (GFP). A DNA construct has been made which consists of a fusion between the EGFP coding sequence (available from Clontech) and the coding sequence for mouse MAP4. (Olson et al., (1995), J. Cell Biol. 130(3): 639-650). MAP4 is a ubiquitous microtubule-associated protein that is known to interact with microtubules in interphase as well as mitotic cells (Olmsted and Murofushi, (1993), MAP4. In "Guidebook to the Cytoskeleton and Motor Proteins." Oxford University Press. T. Kreis and R. Vale, eds.) Its localization, then, can serve as an indicator of the localization, organization, and integrity of microtubules in living (or fixed) cells at all stages of the cell cycle for cell-based HCS assays. While MAP2 and tau (microtubule associated proteins expressed specifically in neuronal cells) have been used to form GFP chimeras (Kaech *et al.,* (1996) Neuron. 17: 1189-1199; Hall *et al*., (1997), Proc. Nat. Acad. Sci. 94: 4733-4738) their restricted cell type distribution and the tendency of these proteins to bundle microtubules when overexpressed make these proteins less desirable as molecular reagents for analysis in live cells originating from varied tissues and organs. Moderate overexpression of GFP-MAP4 does not disrupt microtubule function or integrity (Olson et al., 1995). Similar constructs can be made using β-tubulin or α-tubulin via standard techniques in the art. These chimeras will provide a means to observe and analyze microtubule activity in living cells during all stages of the cell cycle.

In another embodiment, the luminescently labeled protein biosensor of microtubule dynamics is expressed, isolated, and added to the cells to be analyzed via bulk loading techniques, such as microinjection, scrape loading, and impact-mediated loading. In this embodiment, there is not an issue of overexpression within the cell, and thus α and β tubulin isoforms, MAP4, MAP2 and/or tau can all be used.

In a further embodiment, the protein biosensor is expressed by the cell, and the cell is subsequently contacted with a luminescent label, such as a labeled antibody, that detects the protein biosensor, endogenous levels of a protein antigen, or both. In this embodiment, a luminescent label that detects α and β tubulin isoforms, MAP4, MAP2 and/or tau, can be used.

A variety of GFP mutants are available, all of which would be effective in this invention, including, but not limited to, GFP mutants which are commercially available (Clontech, California).

The MAP4 construct has been introduced into several mammalian cell lines (BHK-21, Swiss 3T3, HeLa, HEK 293, LLCPK) and the organization and localization of tubulin has been visualized in live cells by virtue of the GFP fluorescence as an indicator of MAP4 localization. The construct can be expressed transiently or stable cell lines can be prepared by standard methods. Stable HeLa cell lines expressing the EGFP-MAP4 chimera have been obtained, indicating that expression of the chimera is not toxic and does not interfere with mitosis.

Possible selectable markers for establishment and maintenance of stable cell lines include, but are not limited to the neomycin resistance gene, hygromycin resistance gene, zeocin resistance gene, puromycin resistance gene, bleomycin resistance gene, and blastacidin resistance gene.

The utility of this method for the monitoring of microtubule assembly, disassembly, and rearrangement has been demonstrated by treatment of transiently and stably transfected cells with microtubule drugs such as paclitaxel, nocodazole, vincristine, or vinblastine.

The present method provides high-content and combined high throughput-high content cell-based screens for anti-microtubule drugs, particularly as one parameter in a multi-parametric cancer target screen. The EGFP-MAP4 construct used herein can also be used as one of the components of a high-content screen that measures multiple signaling pathways or physiological events. In a preferred embodiment, a combined high throughput and high content screen is employed, wherein multiple cells in each of the locations containing cells are analyzed in a high throughput mode, and only a subset of the locations containing cells are analyzed in a high content mode. The high throughput screen can be any screen that would be useful to identify those locations containing cells that should be further analyzed, including, but not limited to, identifying locations with increased luminescence intensity, those exhibiting expression of a reporter gene, those undergoing calcium changes, and those undergoing pH changes.

In addition to drug screening applications, the present invention may be applied to clinical diagnostics, the detection of chemical and biological warfare weapons, and the basic research market since fundamental cell processes, such as cell division and motility, are highly dependent upon microtubule dynamics.

### Image Acquisition and Analysis

Image data can be obtained from either fixed or living indicator cells. To extract morphometric data from each of the images obtained the following method of analysis is used:
1. Threshold each nucleus and cytoplasmic image to produce a mask that has value = 0 for each pixel outside a nucleus or cell boundary.
2. Overlay the mask on the original image, detect each object in the field (*i.e.*, nucleus or cell), and calculate its size, shape, and integrated intensity.
3. Overlay the whole cell mask obtained above on the corresponding luminescent microtubule image and apply one or more of the following set of classifiers to determine the micrtotubule morphology and the effect of drugs on microtubule morphology.

Microtubule morphology is defined using a set of classifiers to quantify aspects of microtubule shape, size, aggregation state, and polymerization state. These classifiers can be based on approaches that include co-occurrence matrices, texture measurements, spectral methods, structural methods, wavelet transforms, statistical methods, or combinations thereof. Examples of such classifiers are as follows:
1. A classifier to quantify microtubule length and width using edge detection methods such as that discussed in Kolega et al. ((1993). *BioImaging* 1:136-150), which discloses a non-automated method to determine edge strength in individual cells), to calculate the total edge strength within each cell. To normalize for cell size, the total edge strength can be divided by the cell area to give a "microtubule morphology" value. Large microtubule morphology values are associated with strong edge strength values and are therefore maximal in cells containing distinct microtubule structures. Likewise, small microtubule morphology values are associated with weak edge strength and are minimal in cells with depolymerized microtubules. The physiological range of microtubule morphology values is set by treating cells with either the microtubule stabilizing drug paclitaxel (10 µM) or the microtubule depolymerizing drug nocodazole (10 µg/ml).
2. A classifier to quantify microtubule aggregation into punctate spots or foci using methodology from the receptor internalization methods discussed supra.
3. A classifier to quantify microtubule depolymerization using a measure of image texture.
4. A classifier to quantify apparent interconnectivity, or branching (or both), of the microtubules.
5. Measurement of the kinetics of microtubule reorganization using the above classifiers on a time series of images of cells treated with test compounds.

In a further aspect, kits are provided for analyzing microtubule stability, comprising an expression vector comprising a nucleic acid that encodes a microtubule labeling protein and instructions for using the expression vector for carrying out the methods described above. In a preferred embodiment, the expression vector further comprises a nucleic acid that encodes a luminescent protein, wherein the microtubule binding protein and the luminescent protein thereof are expressed as a fusion protein. Alternatively, the kit may contain an antibody that specifically binds to the microtubule-labeling protein. In a further embodiment, the kit includes cells that express the microtubule labeling protein. In a preferred embodiment, the cells are transfected with the expression vector. In another preferred embodiment, the kits further contain a compound that is known to disrupt microtubule structure, including but not limited to curacin, nocodazole, vincristine, or vinblastine. In another preferred embodiment, the kits further comprise a compound that is known to stabilize microtubule structure, including but not limited to taxol (paclitaxel), and discodermolide.

In another aspect, the present invention comprises a machine readable storage medium comprising a program containing a set of instructions for causing a cell screening system to execute the disclosed methods for analyzing microtubule stability, wherein the cell screening system comprises an optical system with a stage adapted for holding a plate containing cells, a digital camera, a means for directing fluorescence or luminescence emitted from the cells to the digital camera, and a computer means for receiving and processing the digital data from the digital camera.

### High-content screens involving the functional localization of macromolecules

Within this class of high-content screen, the functional localization of macromolecules in response to external stimuli is measured within living cells.

**Glycolytic enzyme activity regulation.** In a preferred embodiment of a cellular enzyme activity high-content screen, the activity of key glycolytic regulatory enzymes are measured in treated cells. To measure enzyme activity, indicator cells containing luminescent labeling reagents are treated with test compounds and the activity of the reporters is measured in space and time using cell screening system of the present invention.

In one embodiment, the reporter of intracellular enzyme activity is fructose-6-phosphate, 2-kinase/fructose-2,6-bisphosphatase (PFK-2), a regulatory enzyme whose phosphorylation state indicates intracellular carbohydrate anabolism or catabolism (Deprez et al. (1997) *J. Biol. Chem.* 272:17269-17275; Kealer et al. (1996) *FEBS Letters* 395:225-227; Lee et al. (1996), *Biochemistry* 35:6010-6019). The indicator cells contain luminescent reporters consisting of a fluorescent protein biosensor of PFK-2 phosphorylation. The fluorescent protein biosensor is constructed by introducing an environmentally sensitive fluorescent dye near to the known phosphorylation site of the enzyme (Deprez et al. (1997), *supra*; Giuliano et al. (1995), *supra).* The dye can be of the ketocyanine class (Kessler and Wolfbeis (1991), *Spectrochimica Acta* 47A:187-192 ) or any class that contains a protein reactive moiety and a fluorochrome whose excitation or emission spectrum is sensitive to solution polarity. The fluorescent protein biosensor is introduced into the indicator cells using bulk loading methodology.

Living indicator cells are treated with test compounds, at final concentrations ranging from 10⁻¹² M to 10⁻³ M for times ranging from 0.1 s to 10 h. In a preferred embodiment, ratio image data are obtained from living treated indicator cells by collecting a spectral pair of fluorescence images at each time point. To extract morphometric data from each time point, a ratio is made between each pair of images by numerically dividing the two spectral images at each time point, pixel by pixel. Each pixel value is then used to calculate the fractional phosphorylation of PFK-2. At small fractional values of phosphorylation, PFK-2 stimulates carbohydrate catabolism. At high fractional values of phosphorylation, PFK-2 stimulates carbohydrate anabolism.

**Protein kinase A activity and localization of subunits.** In another embodiment of a high-content screen, both the domain localization and activity of protein kinase A (PKA) within indicator cells are measured in response to treatment with test compounds.

The indicator cells contain luminescent reporters including a fluorescent protein biosensor of PKA activation. The fluorescent protein biosensor is constructed by introducing an environmentally sensitive fluorescent dye into the catalytic subunit of PKA near the site known to interact with the regulatory subunit of PKA (Harootunian et al. (1993), *Mol. Biol. of the Cell* 4:993-1002; Johnson et al. (1996), *Cell* 85:149-158; Giuliano et al. (1995), *supra).* The dye can be of the ketocyanine class (Kessler, and Wolfbeis (1991), *Spectrochimica Acta* 47A:187-192) or any class that contains a protein reactive moiety and a fluorochrome whose excitation or emission spectrum is sensitive to solution polarity. The fluorescent protein biosensor of PKA activation is introduced into the indicator cells using bulk loading methodology.

In one embodiment, living indicator cells are treated with test compounds, at final concentrations ranging from 10⁻¹² M to 10⁻³ M for times ranging from 0.1 s to 10 h. In a preferred embodiment, ratio image data are obtained from living treated indicator cells. To extract biosensor data from each time point, a ratio is made between each pair of images, and each pixel value is then used to calculate the fractional activation of PKA (*e*.*g*., separation of the catalytic and regulatory subunits after cAMP binding). At high fractional values of activity, PFK-2 stimulates biochemical cascades within the living cell.

To measure the translocation of the catalytic subunit of PKA, indicator cells containing luminescent reporters are treated with test compounds and the movement of the reporters is measured in space and time using the cell screening system. The indicator cells contain luminescent reporters consisting of domain markers used to measure the localization of the cytoplasmic and nuclear domains. When the indicator cells are treated with a test compounds, the dynamic redistribution of a PKA fluorescent protein biosensor is recorded intracellularly as a series of images over a time scale ranging from 0.1 s to 10 h. Each image is analyzed by a method that quantifies the movement of the PKA between the cytoplasmic and nuclear domains. To do this calculation, the images of the probes used to mark the cytoplasmic and nuclear domains are used to mask the image of the PKA fluorescent protein biosensor. The integrated brightness per unit area under each mask is used to form a translocation quotient by dividing the cytoplasmic integrated brightness/area by the nuclear integrated brightness/area. By comparing the translocation quotient values from control and experimental wells, the percent translocation is calculated for each potential lead compound. The output of the high-content screen relates quantitative data describing the magnitude of the translocation within a large number of individual cells that have been treated with test compound in the concentration range of 10⁻¹² M to 10⁻³ M.

### High-content screens involving the induction or inhibition of gene expression

### RNA-based fluorescent biosensors

**Cytoskeletal protein transcription and message localization.** Regulation of the general classes of cell physiological responses including cell-substrate adhesion, cell-cell adhesion, signal transduction, cell-cycle events, intermediary and signaling molecule metabolism, cell locomotion, cell-cell communication, and cell death can involve the alteration of gene expression. High-content screens can also be designed to measure this class of physiological response.

In one embodiment, the reporter of intracellular gene expression is an oligonucleotide that can hybridize with the target mRNA and alter its fluorescence signal. In a preferred embodiment, the oligonucleotide is a molecular beacon (Tyagi and Kramer (1996) *Nat. Biotechnol.* 14:303-308), a luminescence-based reagent whose fluorescence signal is dependent on intermolecular and intramolecular interactions. The fluorescent biosensor is constructed by introducing a fluorescence energy transfer pair of fluorescent dyes such that there is one at each end (5' and 3') of the reagent. The dyes can be of any class that contains a protein reactive moiety and fluorochromes whose excitation and emission spectra overlap sufficiently to provide fluorescence energy transfer between the dyes in the resting state, including, but not limited to, fluorescein and rhodamine (Molecular Probes, Inc.). In a preferred embodiment, a portion of the message coding for β-actin (Kislauskis et al. (1994), *J. Cell Biol.* 127:441-451; McCann et al. (1997), *Proc. Natl. Acad. Sci.* 94:5679-5684; Sutoh (1982), *Biochemistry* 21:3654-3661) is inserted into the loop region of a hairpin-shaped oligonucleotide with the ends tethered together due to intramolecular hybridization. At each end of the biosensor a fluorescence donor (fluorescein) and a fluorescence acceptor (rhodamine) are covalently bound. In the tethered state, the fluorescence energy transfer is maximal and therefore indicative of an unhybridized molecule. When hybridized with the mRNA coding for β-actin, the tether is broken and energy transfer is lost. The complete fluorescent biosensor is introduced into the indicator cells using bulk loading methodology.

In one embodiment, living indicator cells are treated with test compounds, at final concentrations ranging from 10⁻¹² M to 10⁻³ M for times ranging from 0.1 s to 10 h. In a preferred embodiment, ratio image data are obtained from living treated indicator cells. To extract morphometric data from each time point, a ratio is made between each pair of images, and each pixel value is then used to calculate the fractional hybridization of the labeled nucleotide. At small fractional values of hybridization little expression of β-actin is indicated. At high fractional values of hybridization, maximal expression of β-actin is indicated. Furthermore, the distribution of hybridized molecules within the cytoplasm of the indicator cells is also a measure of the physiological response of the indicator cells.

### Cell surface binding of a ligand

**Labeled insulin binding to its cell surface receptor in living cells.** Cells whose plasma membrane domain has been labeled with a labeling reagent of a particular color are incubated with a solution containing insulin molecules (Lee et al. (1997), *Biochemistry* 36:2701-2708; Martinez-Zaguilan et al. (1996), *Am. J. Physiol.* 270:C1438-C1446) that are labeled with a luminescent probe of a different color for an appropriate time under the appropriate conditions. After incubation, unbound insulin molecules are washed away, the cells fixed and the distribution and concentration of the insulin on the plasma membrane is measured. To do this, the cell membrane image is used as a mask for the insulin image. The integrated intensity from the masked insulin image is compared to a set of images containing known amounts of labeled insulin. The amount of insulin bound to the cell is determined from the standards and used in conjunction with the total concentration of insulin incubated with the cell to calculate a dissociation constant or insulin to its cell surface receptor.

### Labeling of cellular compartments

### Whole cell labeling

Whole cell labeling is accomplished by labeling cellular components such that dynamics of cell shape and motility of the cell can be measured over time by analyzing fluorescence images of cells.

In one embodiment, small reactive fluorescent molecules are introduced into living cells. These membrane-permeant molecules both diffuse through and react with protein components in the plasma membrane. Dye molecules react with intracellular molecules to both increase the fluorescence signal emitted from each molecule and to entrap the fluorescent dye within living cells. These molecules include reactive chloromethyl derivatives of aminocoumarins, hydroxycoumarins, eosin diacetate, fluorescein diacetate, some Bodipy dye derivatives, and tetramethylrhodamine. The reactivity of these dyes toward macromolecules includes free primary amino groups and free sulfhydryl groups.

In another embodiment, the cell surface is labeled by allowing the cell to interact with fluorescently labeled antibodies or lectins (Sigma Chemical Company, St. Louis, MO) that react specifically with molecules on the cell surface. Cell surface protein chimeras expressed by the cell of interest that contain a green fluorescent protein, or mutant thereof, component can also be used to fluorescently label the entire cell surface. Once the entire cell is labeled, images of the entire cell or cell array can become a parameter in high content screens, involving the measurement of cell shape, motility, size, and growth and division.

### Plasma membrane labeling

In one embodiment, labeling the whole plasma membrane employs some of the same methodology described above for labeling the entire cells. Luminescent molecules that label the entire cell surface act to delineate the plasma membrane.

In a second embodiment subdomains of the plasma membrane, the extracellular surface, the lipid bilayer, and the intracellular surface can be labeled separately and used as components of high content screens. In the first embodiment, the extracellular surface is labeled using a brief treatment with a reactive fluorescent molecule such as the succinimidyl ester or iodoacetamde derivatives of fluorescent dyes such as the fluoresceins, rhodamines, cyanines, and Bodipys.

In a third embodiment, the extracellular surface is labeled using fluorescently labeled macromolecules with a high affinity for cell surface molecules. These include fluorescently labeled lectins such as the fluorescein, rhodamine, and cyanine derivatives of lectins derived from jack bean (Con A), red kidney bean (erythroagglutinin PHA-E), or wheat germ.

In a fourth embodiment, fluorescently labeled antibodies with a high affinity for cell surface components are used to label the extracellular region of the plasma membrane. Extracellular regions of cell surface receptors and ion channels are examples of proteins that can be labeled with antibodies.

In a fifth embodiment, the lipid bilayer of the plasma membrane is labeled with fluorescent molecules. These molecules include fluorescent dyes attached to long chain hydrophobic molecules that interact strongly with the hydrophobic region in the center of the plasma membrane lipid bilayer. Examples of these dyes include the PKH series of dyes (U.S. 4,783,401, 4,762701, and 4,859,584; available commercially from Sigma Chemical Company, St. Loius, MO), fluorescent phospholipids such as nitrobenzoxadiazole glycerophosphoethanolamine and fluorescein-derivatized dihexadecanoylglycerophosphoetha-nolamine, fluorescent fatty acids such as 5-butyl-4,4-difluoro-4-bora-3a,4a-diaza-s-indacene-3-nonanoic acid and 1-pyrenedecanoic acid (Molecular Probes, Inc.), fluorescent sterols including cholesteryl 4,4-difluoro-5,7-dimethyl-4-bora-3a,4a-diaza-s-indacene-3-dodecanoate and cholesteryl 1-pyrenehexanoate, and fluorescently labeled proteins that interact specifically with lipid bilayer components such as the fluorescein derivative of annexin V (Caltag Antibody Co, Burlingame, CA).

In another embodiment, the intracellular component of the plasma membrane is labeled with fluorescent molecules. Examples of these molecules are the intracellular components of the trimeric G-protein receptor, adenylyl cyclase, and ionic transport proteins. These molecules can be labeled as a result of tight binding to a fluorescently labeled specific antibody or by the incorporation of a fluorescent protein chimera that is comprised of a membrane-associated protein and the green fluorescent protein, and mutants thereof.

### Endosome fluorescence labeling

In one embodiment, ligands that are transported into cells by receptor-mediated endocytosis are used to trace the dynamics of endosomal organelles. Examples of labeled ligands include Bodipy FL-labeled low density lipoprotein complexes, tetramethylrhodamine transferrin analogs, and fluorescently labeled epidermal growth factor (Molecular Probes, Inc.)

In a second embodiment, fluorescently labeled primary or secondary antibodies (Sigma Chemical Co. St. Louis, MO; Molecular Probes, Inc. Eugene, OR; Caltag Antibody Co.) that specifically label endosomal ligands are used to mark the endosomal compartment in cells.

In a third embodiment, endosomes are fluorescently labeled in cells expressing protein chimeras formed by fusing a green fluorescent protein, or mutants thereof, with a receptor whose internalization labels endosomes. Chimeras of the EGF, transferrin, and low density lipoprotein receptors are examples of these molecules.

### Lysosome labeling

In one embodiment, membrane permeant lysosome-specific luminescent reagents are used to label the lysosomal compartment of living and fixed cells. These reagents include the luminescent molecules neutral red, N-(3-((2,4-dinitrophenyl)amino)propyl)-N-(3-aminopropyl)methylamine, and the LysoTracker probes which report intralysosomal pH as well as the dynamic distribution of lysosomes (Molecular Probes, Inc.)

In a second embodiment, antibodies against lysosomal antigens (Sigma Chemical Co.; Molecular Probes, Inc.; Caltag Antibody Co.) are used to label lysosomal components that are localized in specific lysosomal domains. Examples of these components are the degradative enzymes involved in cholesterol ester hydrolysis, membrane protein proteases, and nucleases as well as the ATP-driven lysosomal proton pump.

In a third embodiment, protein chimeras consisting of a lysosomal protein genetically fused to an intrinsically luminescent protein such as the green fluorescent protein, or mutants thereof, are used to label the lysosomal domain. Examples of these components are the degradative enzymes involved in cholesterol ester hydrolysis, membrane protein proteases, and nucleases as well as the ATP-driven lysosomal proton pump.

### Cytoplasmic fluorescence labeling

In one embodiment, cell permeant fluorescent dyes (Molecular Probes, Inc.) with a reactive group are reacted with living cells. Reactive dyes including monobromobimane, 5-chloromethylfluorescein diacetate, carboxy fluorescein diacetate succinimidyl ester, and chloromethyl tetramethylrhodamine are examples of cell permeant fluorescent dyes that are used for long term labeling of the cytoplasm of cells.

In a second embodiment, polar tracer molecules such as Lucifer yellow and cascade blue-based fluorescent dyes (Molecular Probes, Inc.) are introduced into cells using bulk loading methods and are also used for cytoplasmic labeling.

In a third embodiment, antibodies against cytoplasmic components (Sigma Chemical Co.; Molecular Probes, Inc.; Caltag Antibody Co.) are used to fluorescently label the cytoplasm. Examples of cytoplasmic antigens are many of the enzymes involved in intermediary metabolism. Enolase, phosphofructokinase, and acetyl-CoA dehydrogenase are examples of uniformly distributed cytoplasmic antigens.

In a fourth embodiment, protein chimeras consisting of a cytoplasmic protein genetically fused to an intrinsically luminescent protein such as the green fluorescent protein, or mutants thereof, are used to label the cytoplasm. Fluorescent chimeras of uniformly distributed proteins are used to label the entire cytoplasmic domain. Examples of these proteins are many of the proteins involved in intermediary metabolism and include enolase, lactate dehydrogenase, and hexokinase.

In a fifth embodiment, antibodies against cytoplasmic antigens (Sigma Chemical Co.; Molecular Probes, Inc.; Caltag Antibody Co.) are used to label cytoplasmic components that are localized in specific cytoplasmic sub-domains. Examples of these components are the cytoskeletal proteins actin, tubulin, and cytokeratin. A population of these proteins within cells is assembled into discrete structures, which in this case, are fibrous. Fluorescence labeling of these proteins with antibody-based reagents therefore labels a specific sub-domain of the cytoplasm.

In a sixth embodiment, non-antibody-based fluorescently labeled molecules that interact strongly with cytoplasmic proteins are used to label specific cytoplasmic components. One example is a fluorescent analog of the enzyme DNAse I (Molecular Probes, Inc.) Fluorescent analogs of this enzyme bind tightly and specifically to cytoplasmic actin, thus labeling a sub-domain of the cytoplasm. In another example, fluorescent analogs of the mushroom toxin phalloidin or the drug paclitaxel (Molecular Probes, Inc.) are used to label components of the actin- and microtubule-cytoskeletons, respectively.

In a seventh embodiment, protein chimeras consisting of a cytoplasmic protein genetically fused to an intrinsically luminescent protein such as the green fluorescent protein, or mutants thereof, are used to label specific domains of the cytoplasm. Fluorescent chimeras of highly localized proteins are used to label cytoplasmic subdomains. Examples of these proteins are many of the proteins involved in regulating the cytoskeleton. They include the structural proteins actin, tubulin, and cytokeratin as well as the regulatory proteins microtubule associated protein 4 and α-actinin.

### Nuclear labeling

In one embodiment, membrane permeant nucleic-acid-specific luminescent reagents (Molecular Probes, Inc.) are used to label the nucleus of living and fixed cells. These reagents include cyanine-based dyes (*e.g.*, TOTO®, YOYO®, and BOBO™), phenanthidines and acridines (*e.g.,* ethidium bromide, propidium iodide, and acridine orange), indoles and imidazoles (*e.g.*, Hoechst 33258, Hoechst 33342, and 4',6-diamidino-2-phenylindole), and other similar reagents (*e*.*g*., 7-aminoactinomycin D, hydroxystilbamidine, and the psoralens).

In a second embodiment, antibodies against nuclear antigens (Sigma Chemical Co.; Molecular Probes, Inc.; Caltag Antibody Co.) are used to label nuclear components that are localized in specific nuclear domains. Examples of these components are the macromolecules involved in maintaining DNA structure and function. DNA, RNA, histones, DNA polymerase, RNA polymerase, lamins, and nuclear variants of cytoplasmic proteins such as actin are examples of nuclear antigens.

In a third embodiment, protein chimeras consisting of a nuclear protein genetically fused to an intrinsically luminescent protein such as the green fluorescent protein, or mutants thereof, are used to label the nuclear domain. Examples of these proteins are many of the proteins involved in maintaining DNA structure and function. Histones, DNA polymerase, RNA polymerase, lamins, and nuclear variants of cytoplasmic proteins such as actin are examples of nuclear proteins.

### Mitochondrial labeling

In one embodiment, membrane permeant mitochondrial-specific luminescent reagents (Molecular Probes, Inc.) are used to label the mitochondria of living and fixed cells. These reagents include rhodamine 123, tetramethyl rosamine, JC-1, and the MitoTracker reactive dyes.

In a second embodiment, antibodies against mitochondrial antigens (Sigma Chemical Co.; Molecular Probes, Inc.; Caltag Antibody Co.) are used to label mitochondrial components that are localized in specific mitochondrial domains. Examples of these components are the macromolecules involved in maintaining mitochondrial DNA structure and function. DNA, RNA, histones, DNA polymerase, RNA polymerase, and mitochondrial variants of cytoplasmic macromolecules such as mitochondrial tRNA and rRNA are examples mitochondrial antigens. Other examples of mitochondrial antigens are the components of the oxidative phosphorylation system found in the mitochondria (*e.g.*, cytochrome c, cytochrome c oxidase, and succinate dehydrogenase).

In a third embodiment, protein chimeras consisting of a mitochondrial protein genetically fused to an intrinsically luminescent protein such as the green fluorescent protein, or mutants thereof, are used to label the mitochondrial domain. Examples of these components are the macromolecules involved in maintaining mitochondrial DNA structure and function. Examples include histones, DNA polymerase, RNA polymerase, and the components of the oxidative phosphorylation system found in the mitochondria (*e.g*., cytochrome c, cytochrome c oxidase, and succinate dehydrogenase).

### Endoplasmic reticulum labeling

In one embodiment, membrane permeant endoplasmic reticulum-specific luminescent reagents (Molecular Probes, Inc.) are used to label the endoplasmic reticulum of living and fixed cells. These reagents include short chain carbocyanine dyes (*e.g*., DiOC₆ and DiOC₃), long chain carbocyanine dyes *(e.g.,* DiIC₁₆ and DiIC₁₈), and luminescently labeled lectins such as concanavalin A.

In a second embodiment, antibodies against endoplasmic reticulum antigens (Sigma Chemical Co.; Molecular Probes, Inc.; Caltag Antibody Co.) are used to label endoplasmic reticulum components that are localized in specific endoplasmic reticulum domains. Examples of these components are the macromolecules involved in the fatty acid elongation systems, glucose-6-phosphatase, and HMG CoA-reductase.

In a third embodiment, protein chimeras consisting of a endoplasmic reticulum protein genetically fused to an intrinsically luminescent protein such as the green fluorescent protein, or mutants thereof, are used to label the endoplasmic reticulum domain. Examples of these components are the macromolecules involved in the fatty acid elongation systems, glucose-6-phosphatase, and HMG CoA-reductase.

### Golgi labeling

In one embodiment, membrane permeant Golgi-specific luminescent reagents (Molecular Probes, Inc.) are used to label the Golgi of living and fixed cells. These reagents include luminescently labeled macromolecules such as wheat germ agglutinin and Brefeldin A as well as luminescently labeled ceramide.

In a second embodiment, antibodies against Golgi antigens (Sigma Chemical Co.; Molecular Probes, Inc.; Caltag Antibody Co.) are used to label Golgi components that are localized in specific Golgi domains. Examples of these components are N-acetylglucosamine phosphotransferase, Golgi-specific phosphodiesterase, and mannose-6-phosphate receptor protein.

In a third embodiment, protein chimeras consisting of a Golgi protein genetically fused to an intrinsically luminescent protein such as the green fluorescent protein, or mutants thereof, are used to label the Golgi domain. Examples of these components are N-acetylglucosamine phosphotransferase, Golgi-specific phosphodiesterase, and mannose-6-phosphate receptor protein.

While many of the examples presented involve the measurement of single cellular processes, this is again is intended for purposes of illustration only. Multiple parameter high-content screens can be produced by combining several single parameter screens into a multiparameter high-content screen or by adding cellular parameters to any existing high-content screen. Furthermore, while each example is described as being based on either live or fixed cells, each high-content screen can be designed to be used with both live and fixed cells.

Those skilled in the art will recognize a wide variety of distinct screens that can be developed based on the disclosure provided herein. There is a large and growing list of known biochemical and molecular processes in cells that involve translocations or reorganizations of specific components within cells. The signaling pathway from the cell surface to target sites within the cell involves the translocation of plasma membrane-associated proteins to the cytoplasm. For example, it is known that one of the src family of protein tyrosine kinases, pp60c-src (Walker et al (1993), *J. Biol. Chem.* 268:19552-19558) translocates from the plasma membrane to the cytoplasm upon stimulation of fibroblasts with platelet-derived growth factor (PDGF). Additionally, the targets for screening can themselves be converted into fluorescence-based reagents that report molecular changes including ligand-binding and post-translocational modifications.

### Example 10. Protease Biosensors

### (1) Background

As used herein, the following terms are defined as follows:
- Reactant - the parent biosensor that interacts with the proteolytic enzyme.
- Product - the signal-containing proteolytic fragment(s) generated by the interaction of the reactant with the enzyme.
- Reactant Target Sequence - an amino acid sequence that imparts a restriction on the cellular distribution of the reactant to a particular subcellular domain of the cell.
- Product Target Sequence - an amino acid sequence that imparts a restriction on the cellular distribution of the signal-containing product(s) of the targeted enzymatic reaction to a particular subcellular domain of the cell. If the product is initially localized within a membrane bound compartment, then the Product Target Sequence must incorporate the ability to export the product out of the membrane-bound compartment. A bi-functional sequence can be used, which first moves the product out of the membrane-bound compartment, and then targets the product to the final compartment. In general, the same amino acid sequences can act as either or both reactant target sequences and product target sequences. Exceptions to this include amino acid sequences which target the nuclear envelope, Golgi apparatus, endoplasmic reticuulum, and which are involved in farnesylation, which are more suitable as reactant target sequences.
- Protease Recognition Site - an amino acid sequence that imparts specificity by mimicking the substrate, providing a specific binding and cleavage site for a protease. Although typically a short sequence of amino acids representing the minimal cleavage site for a protease (e.g. DEVD for caspase-3, Villa, P., S.H. Kaufmann, and W.C. Earnshaw. 1997. Caspases and caspase inhibitors. *Trends Biochem Sci.* 22:388-93), greater specificity may be established by using a longer sequence from an established substrate.
- Compartment - any cellular sub-structure or macromolecular component of the cell, whether it is made of protein, lipid, carbohydrate, or nucleic acid. It could be a macromolecular assembly or an organelle (a membrane delimited cellular component). Compartments include, but are not limited to, cytoplasm, nucleus, nucleolus, inner and outer surface of nuclear envelope, cytoskeleton, peroxisome, endosome, lysosome, inner leaflet of plasma membrane, outer leaflet of plasma membrane, outer leaflet of mitochondrial membrane, inner leaflet of mitochondrial membrane, Golgi, endoplasmic reticulum, or extracellular space.
   Signal - an amino acid sequence that can be detected. This includes, but is not limited to inherently fluorescent proteins (e.g. Green Fluorescent Protein), cofactor-requiring fluorescent or luminescent proteins (e.g. phycobiliproteins or luciferases), and epitopes recognizable by specific antibodies or other specific natural or unnatural binding probes, including but not limited to dyes, enzyme cofactors and engineered binding molecules, which are fluorescently or luminescently labeled. Also included are site-specifically labeled proteins that contain a luminescent dye. Methodology for site-specific labeling of proteins includes, but is not limited to, engineered dye-reactive amino acids (Post, et al., *J. Biol. Chem.* 269:12880-12887 (1994)), enzyme-based incorporation of luminescent substrates into proteins (Buckler, et al., *Analyt. Biochem.* 209:20-31 (1993); Takashi, *Biochemistry.* 27:938-943 (1988)), and the incorporation of unnatural labeled amino acids into proteins (Noren, et al., *Science.* 244:182-188 (1989)).
- Detection - a means for recording the presence, position, or amount of the signal. The approach may be direct, if the signal is inherently fluorescent, or indirect, if, for example, the signal is an epitope that must be subsequently detected with a labeled antibody. Modes of detection include, but are not limited to, the spatial position of fluorescence, luminescence, or phosphorescence: (1) intensity; (2) polarization; (3) lifetime; (4) wavelength; (5) energy transfer; and (6) recovery after photobleaching.

The basic principle of the protease biosensors of the present invention is to spatially separate the reactants from the products generated during a proteolytic reaction. The separation of products from reactants occurs upon proteolytic cleavage of the protease recognition site within the biosensor, allowing the products to bind to, diffuse into, or be imported into compartments of the cell different from those of the reactant. This spatial separation provides a means of quantitating a proteolytic process directly in living or fixed cells. Some designs of the biosensor provide a means of restricting the reactant (uncleaved biosensor) to a particular compartment by a protein sequence ("reactant target sequence") that binds to or imports the biosensor into a compartment of the cell. These compartments include, but are not limited to any cellular substructure, macromolecular cellular component, membrane-limited organelles, or the extracellular space. Given that the characteristics of the proteolytic reaction are related to product concentration divided by the reactant concentration, the spatial separation of products and reactants provides a means of uniquely quantitating products and reactants in single cells, allowing a more direct measure of proteolytic activity.

The molecular-based biosensors may be introduced into cells via transfection and the expressed chimeric proteins analyzed in transient cell populations or stable cell lines. They may also be pre-formed, for example by production in a prokaryotic or eukaryotic expression system, and the purified protein introduced into the cell via a number of physical mechanisms including, but not limited to, micro-injection, scrape loading, electroporation, signal-sequence mediated loading, etc.

Measurement modes may include, but are not limited to, the ratio or difference in fluorescence, luminescence, or phosphorescence: (a) intensity; (b) polarization; or (c) lifetime between reactant and product. These latter modes require appropriate spectroscopic differences between products and reactants. For example, cleaving a reactant containing a limited-mobile signal into a very small translocating component and a relatively large non-translocating component may be detected by polarization. Alternatively, significantly different emission lifetimes between reactants and products allow detection in imaging and non-imaging modes.

One example of a family of enzymes for which this biosensor can be constructed to report activity is the caspases. Caspases are a class of proteins that catalyze proteolytic cleavage of a wide variety of targets during apoptosis. Following initiation of apoptosis, the Class II "downstream" caspases are activated and are the point of no return in the pathway leading to cell death, resulting in cleavage of downstream target proteins. In specific examples, the biosensors described here were engineered to use nuclear translocation of cleaved GFP as a measurable indicator of caspase activation. Additionally, the use of specific recognition sequences that incorporate surrounding amino acids involved in secondary structure formation in naturally occurring proteins may increase the specificity and sensitivity of this class of biosensor.

Another example of a protease class for which this biosensor can be constructed to report activity is zinc metalloproteases. Two specific examples of this class are the biological toxins derived from *Clostridial* species (*C. botulinum* and *C. tetani*) and *Bacillus anthracis.* (Herreros et al. *In* The Comprehensive Sourcebook of Bacterial Protein Toxins. J.E. Alouf and J.H. Freer, Eds. 2^{nd} edition, San Diego, Academic Press, 1999; pp 202-228.) These bacteria express and secrete zinc metalloproteases that enter eukaryotic cells and specifically cleave distinct target proteins. For example, the anthrax protease from *Bacillus anthracis* is delivered into the cytoplasm of target cells via an accessory pore-forming protein, where its proteolytic activity inactivates the MAP-kinase signaling cascade through cleavage of mitogen activated protein kinase kinases 1 or 2 (MEK1 or MEK2). (Leppla, S.A. *In* The Comprehensive Sourcebook of Bacterial Protein Toxins. J.E. Alouf and J.H. Freer, Eds. 2^{nd} edition, San Diego, Academic Press, 1999; pp243-263.) The toxin biosensors described here take advantage of the natural subcellular localization of these and other target proteins to achieve reactant targeting. Upon cleavage, the signal (with or without a product target sequence) is separated from the reactant to create a high-content biosensor.

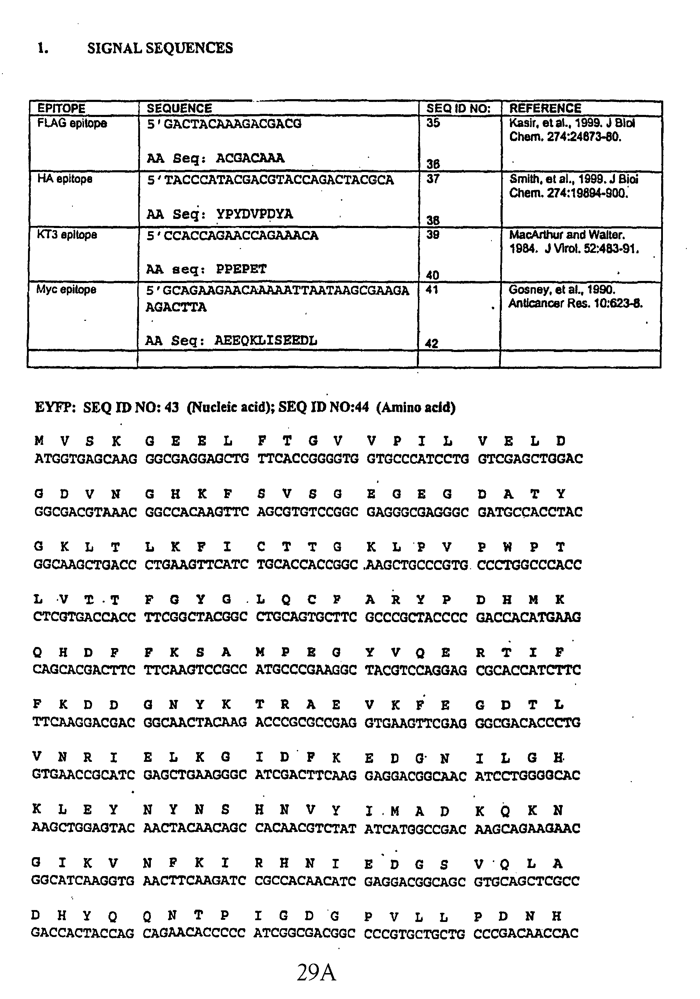

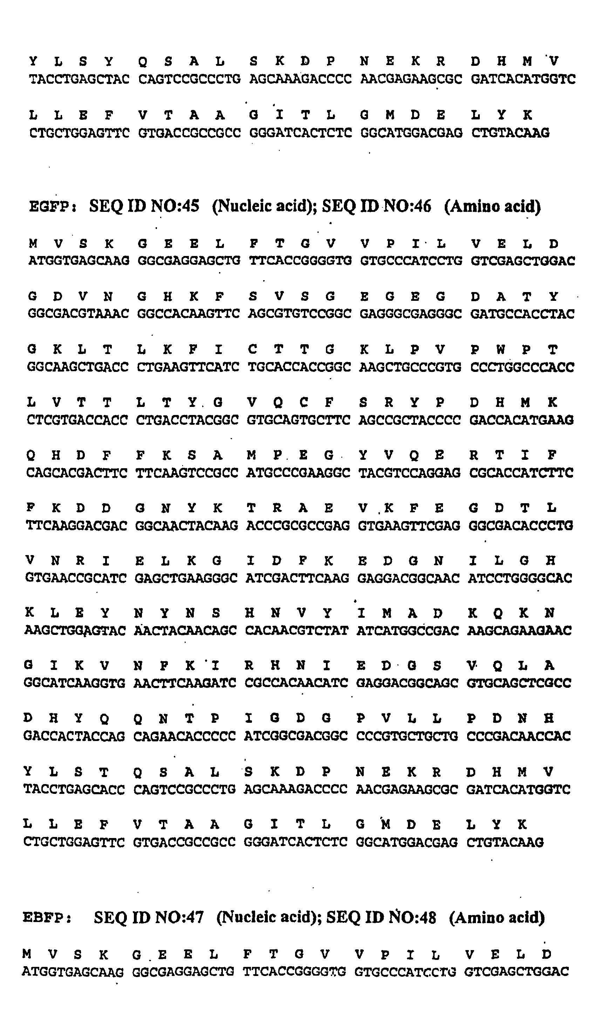

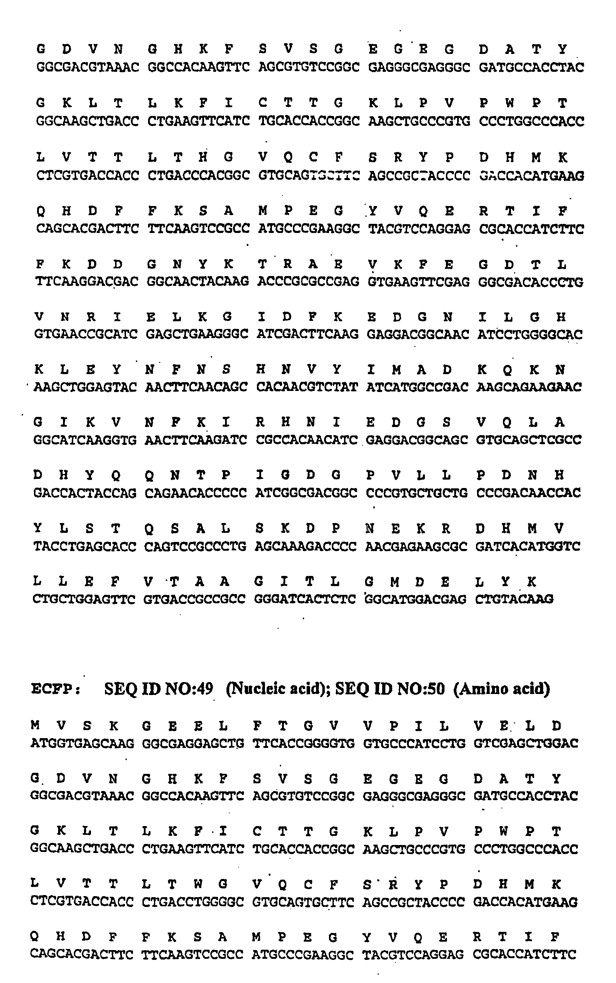

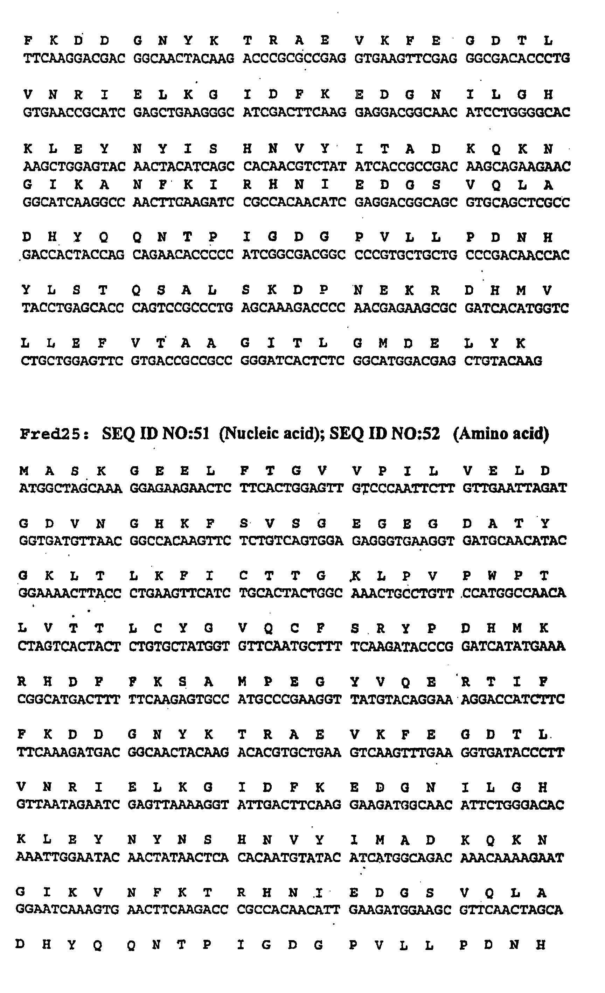

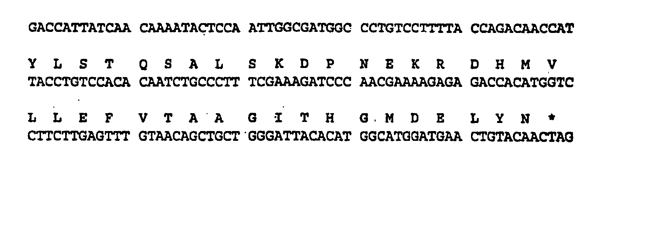

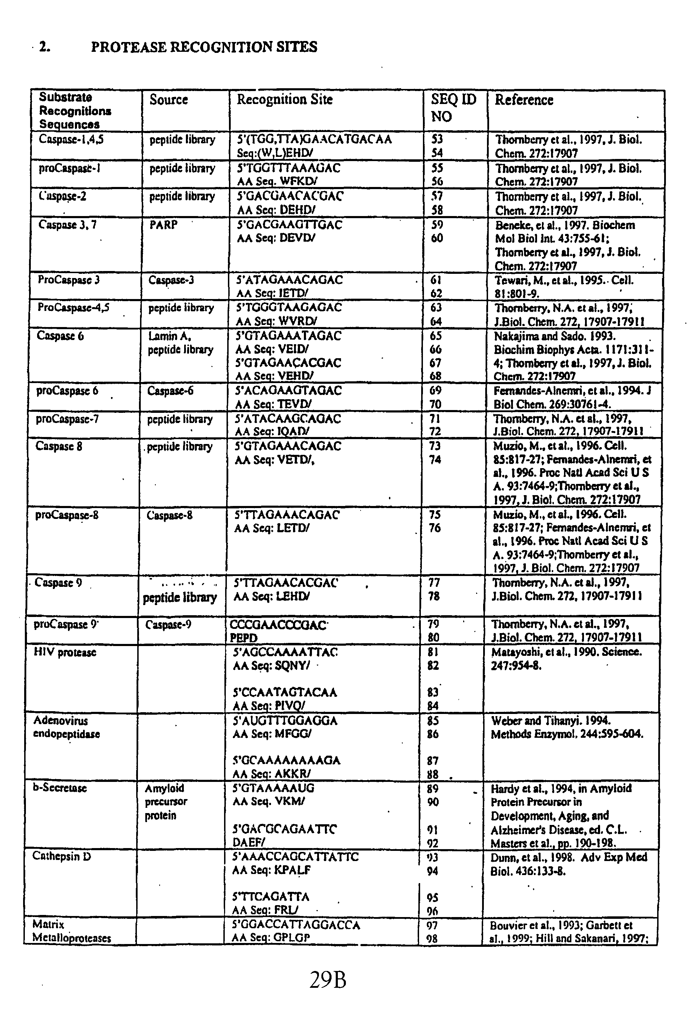

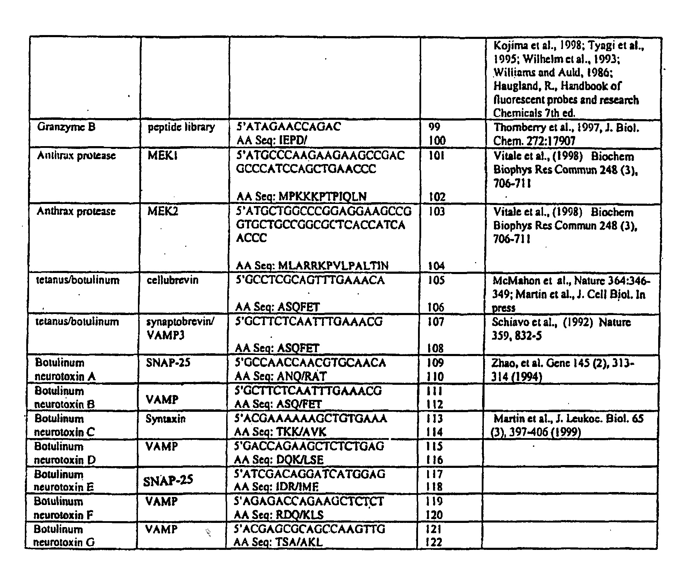

One of skill in the art will recognize that the protein biosensors of this aspect of the invention can be adapted to report the activity of any member of the caspase family of proteases, as well as any other protease, by a substitution of the appropriate protease recognition site in any of the constructs (**see Figure 29B**). These biosensors can be used in high-content screens to detect in vivo activation of enzymatic activity and to identify specific activity based on cleavage of a known recognition motif. This screen can be used for both live cell and fixed end-point assays, and can be combined with additional measurements to provide a multi-parameter assay.

Thus, in another aspect the present invention provides recombinant nucleic acids encoding a protease biosensor, comprising:
a. a first nucleic acid sequence that encodes at least one detectable polypeptide signal;
b. a second nucleic acid sequence that encodes at least one protease recognition site, wherein the second nucleic acid sequence is operatively linked to the first nucleic acid sequence that encodes the at least one detectable polypeptide signal; and
c. a third nucleic acid sequence that encodes at least one reactant target sequence, wherein the third nucleic acid sequence is operatively linked to the second nucleic acid sequence that encodes the at least one protease recognition site.

In this aspect, the first and third nucleic acid sequences are separated by the second nucleic acid sequence, which encodes the protease recognition site.

In a further embodiment, the recombinant nucleic acid encoding a protease biosensor comprises a fourth nucleic acid sequence that encodes at least one product target sequence, wherein the fourth nucleic acid sequence is operatively linked to the first nucleic acid sequence that encodes the at least one detectable polypeptide signal.

In a further embodiment, the recombinant nucleic acid encoding a protease biosensor comprises a fifth nucleic acid sequence that encodes at least one detectable polypeptide signal, wherein the fifth nucleic acid sequence is operatively linked to the third nucleic acid sequence that encodes the reactant target sequence.

In a preferred embodiment, the detectable polypeptide signal is selected from the group consisting of fluorescent proteins, luminescent proteins, and sequence epitopes. In a most preferred embodiment, the first nucleic acid encoding a polypeptide sequence comprises a sequence selected from the group consisting of SEQ ID NOS: 35, 37, 39, 41, 43, 45, 47, 49, and 51.

In another preferred embodiment, the second nucleic acid encoding a protease recognition site comprises a sequence selected from the group consisting of SEQ ID NOS: 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, and 121. In another preferred embodiment, the third nucleic acid encoding a reactant target sequence comprises a sequence selected from the group consisting of SEQ ID NOS: 123, 125, 127, 129, 131, 133, 135, 137, 139, 141, 143, 145, 147, 149, and 151.

In a most preferred embodiment, the recombinant nucleic acid encoding a protease biosensor comprises a sequence substantially similar to sequences selected from the group consisting of SEQ ID NOS:1, 3, 5, 7, 9, 11, 13, 15, 17, 19, 21, 23, 25, 27, 29, 31, and 33.

In another aspect, the present invention provides a recombinant expression vector comprising nucleic acid control sequences operatively linked to the above-described recombinant nucleic acids. In a still further aspect, the present invention provides genetically engineered host cells that have been transfected with the recombinant expression vectors of the invention.

In another aspect, the present invention provides recombinant protease biosensors comprising
a. a first domain comprising at least one detectable polypeptide signal;
b. a second domain comprising at least one protease recognition site; and
c. a third domain comprising at least one reactant target sequence;
wherein the first domain and the third domain are separated by the second domain.

Inherent in this embodiment is the concept that the reactant target sequence restricts the cellular distribution of the reactant, with redistribution of the product occurring after activation (ie: protease cleavage). This redistribution does not require a complete sequestration of products and reactants, as the product distribution can partially overlap the reactant distribution in the absence of a product targeting signal (see below).

In a preferred embodiment, the recombinant protease biosensor further comprises a fourth domain comprising at least one product target sequence, wherein the fourth domain and the first domain are operatively linked and are separated from the third domain by the second domain. In another embodiment, the recombinant protease biosensor further comprises a fifth domain comprising at least one detectable polypeptide signal, wherein the fifth domain and the third domain are operatively linked and are separated from the first domain by the second domain.

In a preferred embodiment, the detectable polypeptide signal domain (first or fifth domain) is selected from the group consisting of fluorescent proteins, luminescent proteins, and sequence epitopes. In a most preferred embodiment, the detectable polypeptide signal domain comprises a sequence selected from the group consisting of SEQ ID NOS:36, 38, 40, 42, 44, 46, 48, 50, and 52.

In another preferred embodiment, the second domain comprising a protease recognition site comprises a sequence selected from the group consisting of SEQ ID NOS:54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, and 122. In another preferred embodiment, the reactant and/or target sequence domains comprise a sequence selected from the group consisting of SEQ ID NOS:124, 126, 128, 130, 132, 134, 136, 138, 140, 142, 144, 146, 148, 150, and 152.

In a most preferred embodiment, the recombinant protease biosensor comprises a sequence substantially similar to sequences selected from the group consisting of SEQ ID NO:2, 4, 6, 8, 10, 12, 14, 16, 18, 20, 22, 24, 26, 28, 30, 32, and 34.

In a still further embodiment, the present invention provides methods and kits for automated analysis of cells, comprising using cells that possess the protease biosensors of the invention to identify compounds that affect protease activity. The method can be combined with the other methods of the invention in a variety of possible multi-parametric assays.

In these various embodiments, the basic protease biosensor is composed of multiple domains, including at least a first detectable polypeptide signal domain, at least one reactant target domain, and at least one protease recognition domain, wherein the detectable signal domain and the reactant target domain are separated by the protease recognition domain. Thus, the exact order of the domains in the molecule is not generally critical, so long as the protease recognition domain separates the reactant target and first detectable signal domain. For each domain, one or more one of the specified recognition sequences is present.

In some cases, the order of the domains in the biosensor may be critical for appropriate targeting of product(s) and/or reactant to the appropriate cellular compartment(s). For example, the targeting of products or reactants to the peroxisome requires that the peroxisomal targeting domain comprise the last three amino acids of the protein. Determination of those biosensor in which the relative placement of targeting domains within the biosensor is critical can be determined by one of skill in the art through routine experimentation.

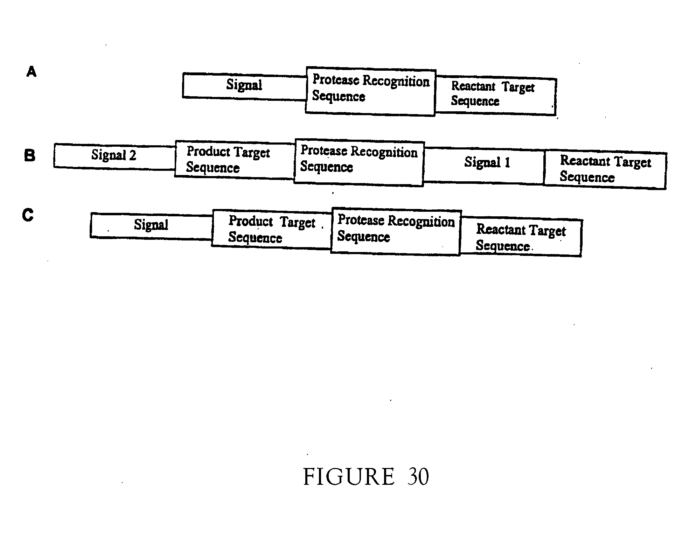

Some examples of the basic organization of domains within the protease biosensor are shown in **Figure 30**. One of skill in the art will recognize that any one of a wide variety of protease recognition sites, product target sequences, polypeptide signals, and/or product target sequences can be used in various combinations in the protein biosensor of the present invention, by substituting the appropriate coding sequences into the multi-domain construct. Non-limiting examples of such alternative sequences are shown in **Figure 29A-29C**. Similarly, one of skill in the art will recognize that modifications, substitutions, and deletions can be made to the coding sequences and the amino acid sequence of each individual domain within the biosensor, while retaining the function of the domain. Such various combinations of domains and modifications, substitutions and deletions to individual domains are within the scope of the invention.

As used herein, the term "coding sequence" or a sequence which "encodes" a particular polypeptide sequence, refers to a nucleic acid sequence which is transcribed (in the case of DNA) and translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and synthetic DNA sequences. A transcription termination sequence will usually be located 3' to the coding sequence.

As used herein, the term DNA "control sequences" refers collectively to promoter sequences, ribosome binding sites, polyadenylation signals, transcription termination sequences, upstream regulatory domains, enhancers, and the like, which collectively provide for the transcription and translation of a coding sequence in a host cell. Not all of these control sequences need always be present in a recombinant vector so long as the DNA sequence of interest is capable of being transcribed and translated appropriately.

As used herein, the term "operatively linked" refers to an arrangement of elements wherein the components so described are configured so as to perform their usual function. Thus, control sequences operatively linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operatively linked" to the coding sequence.

Furthermore, a nucleic acid coding sequence is operatively linked to another nucleic acid coding sequences when the coding region for both nucleic acid molecules are capable of expression in the same reading frame. The nucleic acid sequences need not be contiguous, so long as they are capable of expression in the same reading frame. Thus, for example, intervening coding regions can be present between the specified nucleic acid coding sequences, and the specified nucleic acid coding regions can still be considered "operatively linked".

The intervening coding sequences between the various domains of the biosensors can be of any length so long as the function of each domain is retained. Generally, this requires that the two-dimensional and three-dimensional structure of the intervening protein sequence does not preclude the binding or interaction requirements of the domains of the biosensor, such as product or reactant targeting, binding of the protease of interest to the biosensor, fluorescence or luminescence of the detectable polypeptide signal, or binding of fluorescently labeled epitope-specific antibodies.

One case where the distance between domains of the protease biosensor is important is where the goal is to create a fluorescence resonance energy transfer pair. In this case, the FRET signal will only exist if the distance between the donor and acceptor is sufficiently small as to allow energy transfer (Tsien, Heim and Cubbit, WO 97/28261). The average distance between the donor and acceptor moieties should be between 1 nm and 10 nm with a preference of between 1 nm and 6 nm. This is the physical distance between donor and acceptor. The intervening sequence length can vary considerably since the three dimensional structure of the peptide will determine the physical distance between donor and acceptor.

"Recombinant expression vector" includes vectors that operatively link a nucleic acid coding region or gene to any promoter capable of effecting expression of the gene product. The promoter sequence used to drive expression of the protease biosensor may be constitutive (driven by any of a variety of promoters, including but not limited to, CMV, SV40, RSV, actin, EF) or inducible (driven by any of a number of inducible promoters including, but not limited to, tetracycline, ecdysone, steroid-responsive). The expression vector must be replicable in the host organisms either as an episome or by integration into host chromosomal DNA. In a preferred embodiment, the expression vector comprises a plasmid. However, the invention is intended to include any other suitable expression vectors, such as viral vectors.

The phrase "substantially similar " is used herein in reference to the nucleotide sequence of DNA, or the amino acid sequence of protein, having one or more conservative or non-conservative variations from the protease biosensor sequences disclosed herein, including but not limited to deletions, additions, or substitutions wherein the resulting nucleic acid and/or amino acid sequence is functionally equivalent to the sequences disclosed and claimed herein. Functionally equivalent sequences will function in substantially the same manner to produce substantially the same protease biosensor as the nucleic acid and amino acid compositions disclosed and claimed herein. For example, functionally equivalent DNAs encode protease biosensors that are the same as those disclosed herein or that have one or more conservative amino acid variations, such as substitutions of non-polar residues for other non-polar residues or charged residues for similarly charged residues, or addition to/deletion from regions of the protease biosensor not critical for functionality. These changes include those recognized by those of skill in the art as substitutions, deletions, and/or additions that do not substantially alter the tertiary structure of the protein.

As used herein, substantially similar sequences of nucleotides or amino acids share at least about 70%-75% identity, more preferably 80-85% identity, and most preferably 90-95% identity. It is recognized, however, that proteins (and DNA or mRNA encoding such proteins) containing less than the above-described level of homology (due to the degeneracy of the genetic code) or that are modified by conservative amino acid substitutions (or substitution of degenerate codons) are contemplated to be within the scope of the present invention.

The term "heterologous" as it relates to nucleic acid sequences such as coding sequences and control sequences, denotes sequences that are not normally associated with a region of a recombinant construct, and/or are not normally associated with a particular cell. Thus, a "heterologous" region of a nucleic acid construct is an identifiable segment of nucleic acid within or attached to another nucleic acid molecule that is not found in association with the other molecule in nature. For example, a heterologous region of a construct could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a host cell transformed with a construct which is not normally present in the host cell would be considered heterologous for purposes of this invention.

Within this application, unless otherwise stated, the techniques utilized may be found in any of several well-known references such as: *Molecular Cloning: A Laboratory Manual* (Sambrook, et al., 1989, Cold Spring Harbor Laboratory Press), *Gene Expression Technology* (Methods in Enzymology, Vol. 185, edited by D. Goeddel, 1991. Academic Press, San Diego, CA), "Guide to Protein Purification" in *Methods in Enzymology* (M.P. Deutshcer, ed., (1990) Academic Press, Inc.); *PCR* *Protocols: A Guide to Methods and Applications* (Innis, et al. 1990. Academic Press, San Diego, CA), *Culture of Animal Cells: A Manual of Basic Technique, 2*^{*nd*} *Ed.* (R.I. Freshney. 1987. Liss, Inc. New York, NY), *Gene Transfer and Expression Protocols,* pp. 109-128, ed. E.J. Murray, The Humana Press Inc., Clifton, N.J.), and the Ambion 1998 Catalog (Ambion, Austin, TX).

The biosensors of the present invention are constructed and used to transfect host cells using standard techniques in the molecular biological arts. Any number of such techniques, all of which are within the scope of this invention, can be used to generate protease biosensor-encoding DNA constructs and genetically transfected host cells expressing the biosensors. The non-limiting examples that follow demonstrate one such technique for constructing the biosensors of the invention.

### EXAMPLE OF PROTEASE BIOSENSOR CONSTRUCTION AND USE:

In the following examples, caspase-specific biosensors with specific product target sequences have been constructed using sets of 4 primers (2 sense and 2 antisense). These primers have overlap regions at their termini, and are used for PCR via a primer walking technique. (Sambrook, J., Fritsch, E.F. and Maniatis, T. (1989 ) Molecular Cloning: A Laboratory Manual. Cold Spring Harbor Laboratory Press, Cold Spring Harbor, New York) The two sense primers were chosen to start from the 5' polylinker (BspI) of the GFP-containing vector (Clontech, California) to the middle of the designed biosensor sequence. The two antisense primers start from a 3' GFP vector site (Bam HI), and overlap with the sense primers by 12 nucleotides in the middle.

PCR conditions were as follows: 94°C for 30 seconds for denaturation, 55°C for 30 seconds for annealing, and 72°C for 30 seconds for extension for 15 cycles. The primers have restriction endonuclease sites at both ends, facilitating subsequent cloning of the resulting PCR product.

The resulting PCR product was gel purified, cleaved at BspE1 and BamH1 restriction sites present in the primers, and the resulting fragment was gel purified. Similarly, the GFP vector (Clontech, San Francisco, CA) was digested at BspE1 and BamH1 sites in the polylinker. Ligation of the GFP vector and the PCR product was performed using standard techniques at 16°C overnight. *E. coli* cells were transfected with the ligation mixtures using standard techniques. Transformed cells were selected on LB-agar with an appropriate antibiotic.

**Cells and transfections.** For DNA transfection, BHK cells and MCF-7 cells were cultured to 50-70% confluence in 6 well plates containing 3 ml of minimal Eagle's medium (MEM) with 10% fetal calf serum, 1 mM L-glutamine, 50 µg/ml streptomycin, 50 µg/ml penicillin, 0.1 mM non-essential amino acids, 1 mM sodium pyruvate and 10 µg/ml of bovine insulin (for MCF-7 cell only) at 37 °C in a 5% CO₂ incubator for about 36 hours. The cells were washed with serum free MEM media and incubated for 5 hours with 1 ml of transfection mixture containing 1 µg of the appropriate plasmid and 4 µg of lipofectimine (BRL) in the serum free MEM media. Subsequently, the transfection medium was removed and replaced with 3 ml of normal culture media. The transfected cells were maintained in growth medium for at least 16 hours before performing selection of the stable cells based on standard molecular biology methods (Ausubel. et al 1995).

**Apoptosis assay.** For apoptosis assays, the cells (BHK, MCF-7) stably transfected with the appropriate protease biosensor expression vector were plated on tissue culture treated 96-well plates at 50-60% confluence and cultured overnight at 37°C, 5% CO₂. Varying concentrations of cis-platin, staurosporine, or paclitaxel in normal culture media were freshly prepared from stock and added to cell culture dishes to replace the old culture media. The cells were then observed with the cell screening system of the present invention at the indicated time points either as live cell experiments or as fixed end-point experiments.

### 1. Construction of 3-domain protease biosensors

### a. Caspase-3 biosensor with an annexin II reactant targeting domain (pljkGFP).

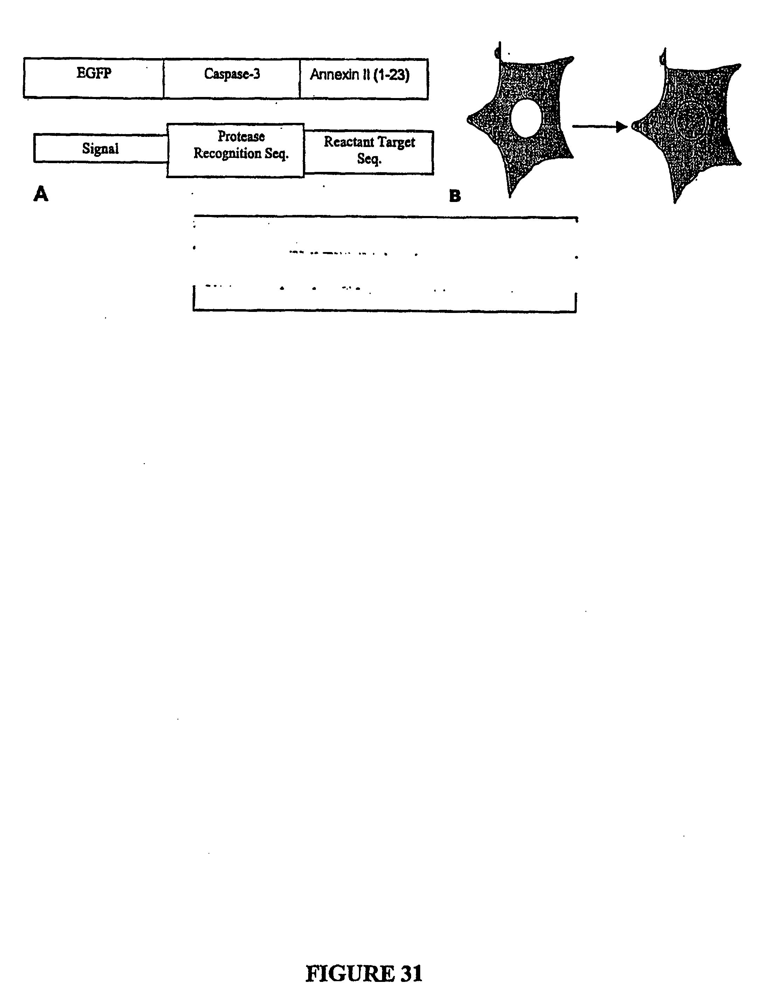

The design of this biosensor is outlined in **Figure 31,** and its sequence is shown in **SEQ ID NO:1 and 2.**

### Primers for Caspase 3, Product target sequence = none (CP3GFP-CYTO):

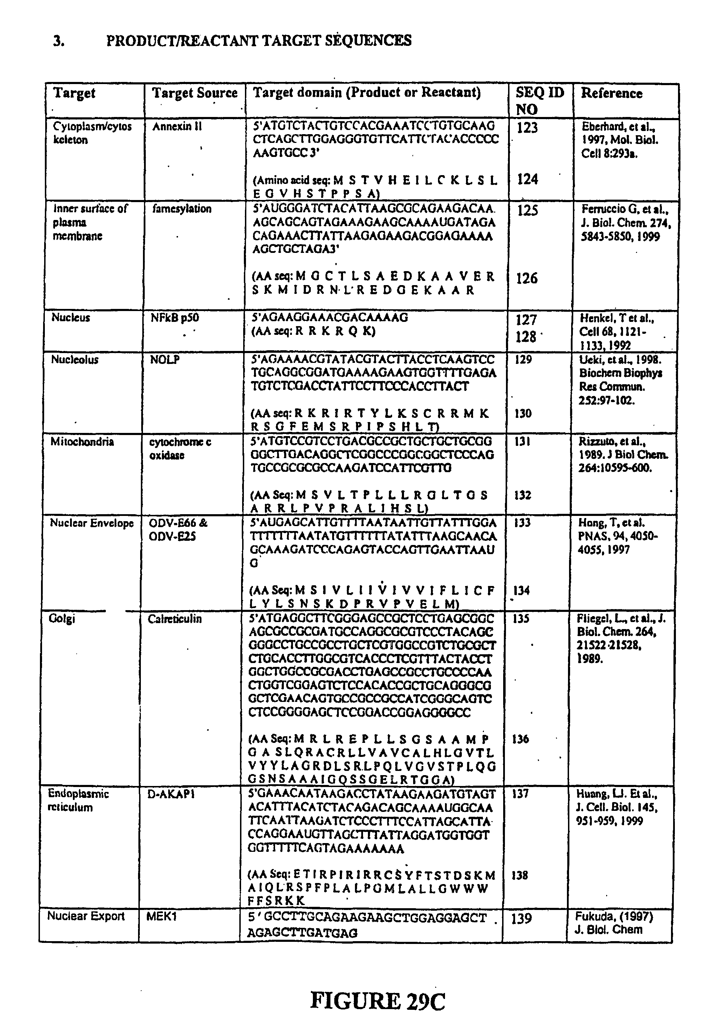

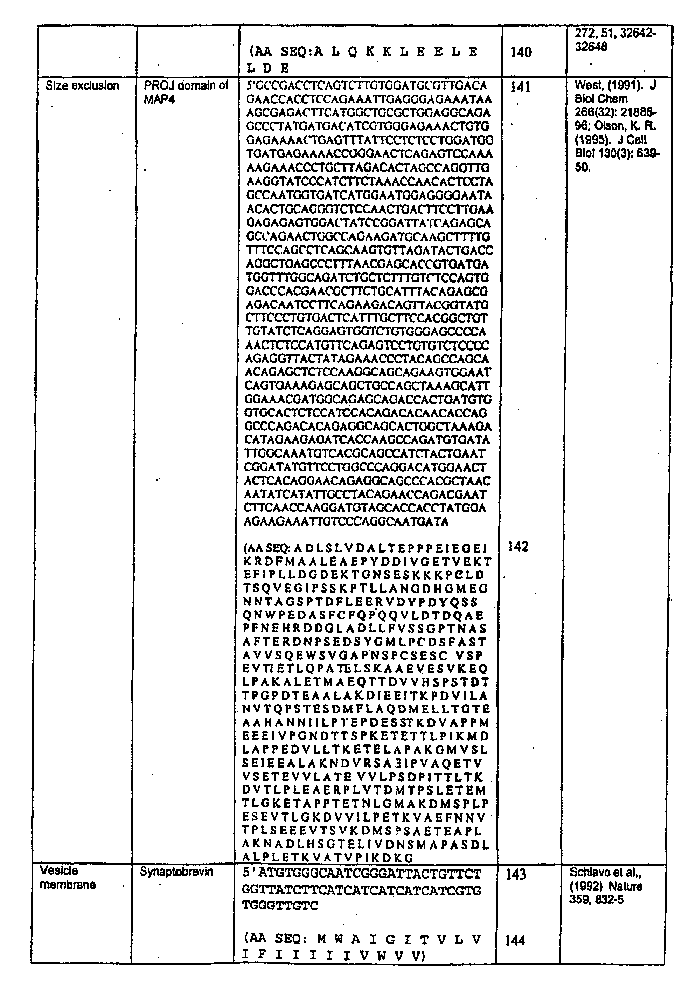

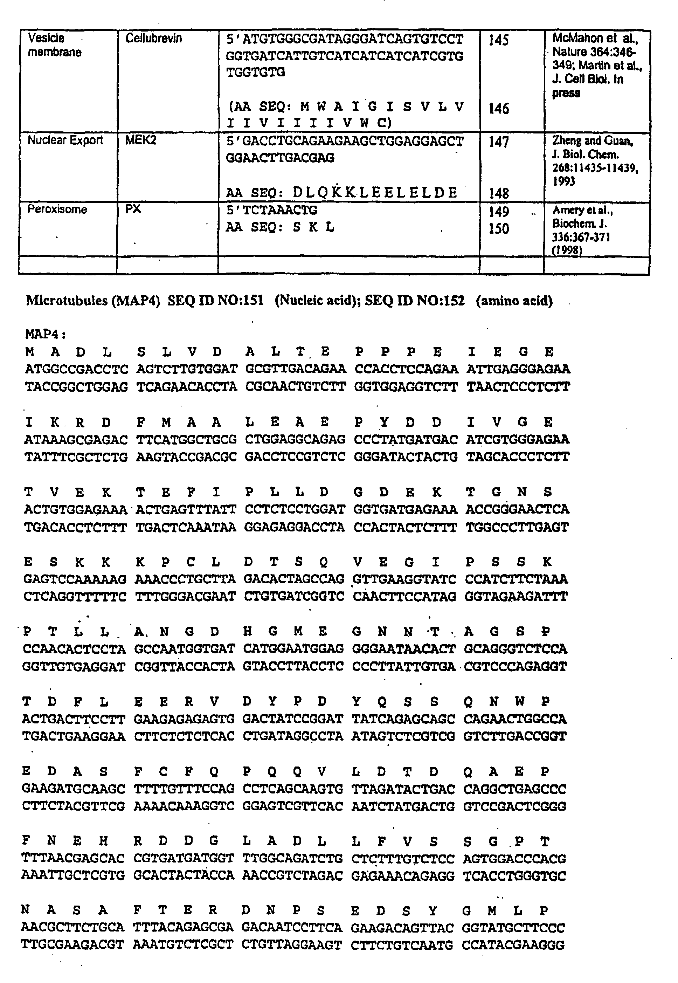

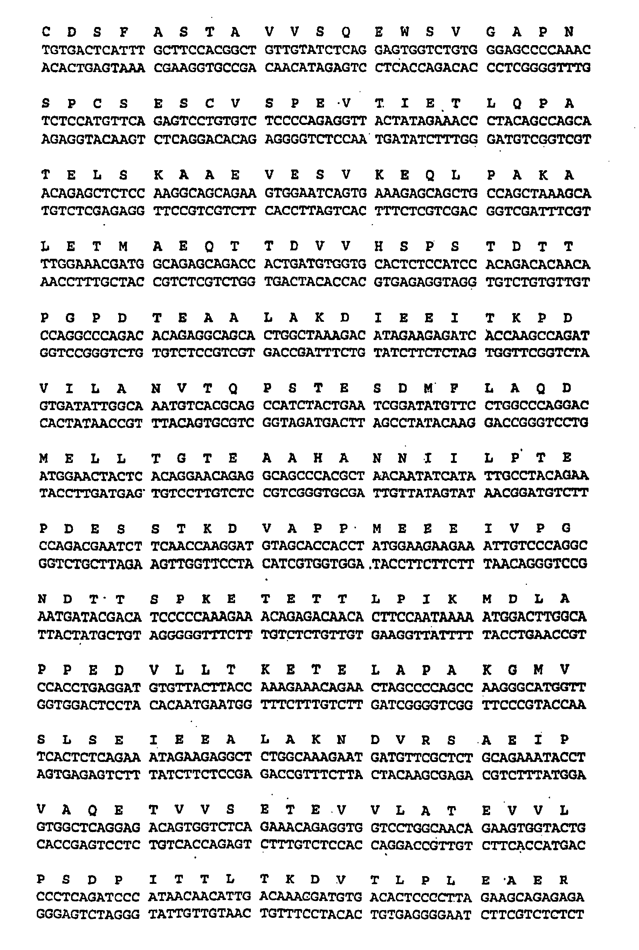

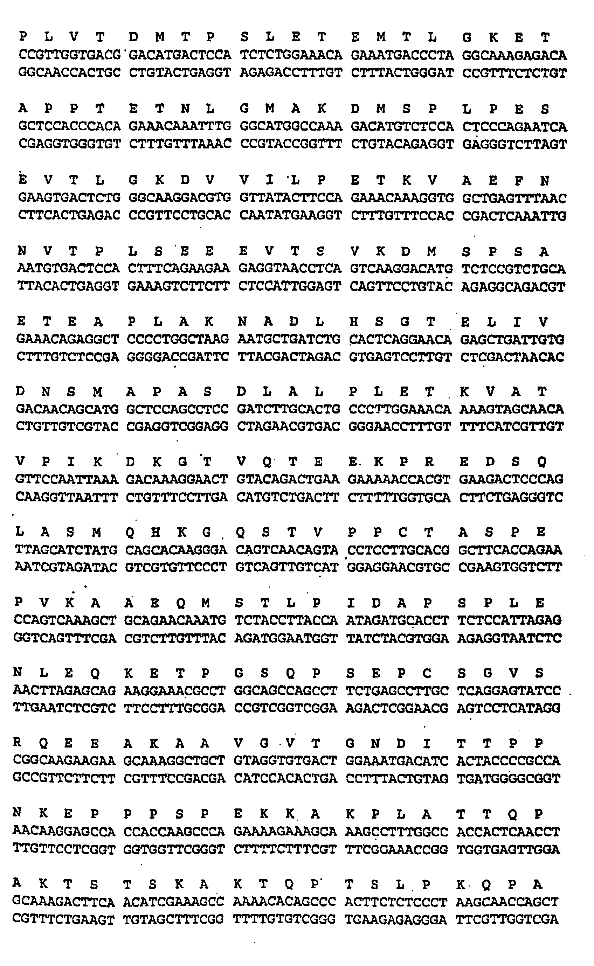

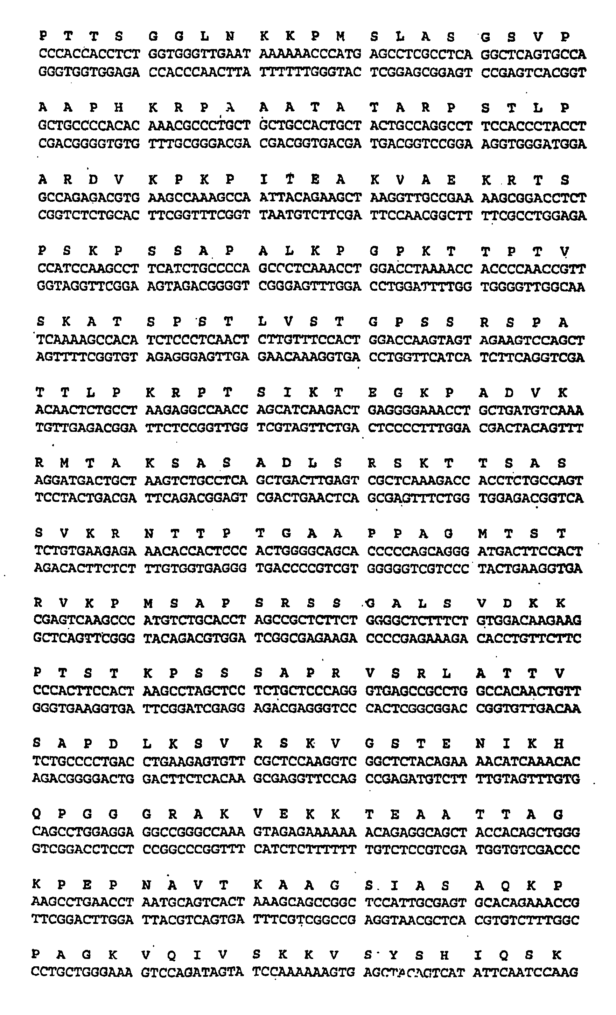

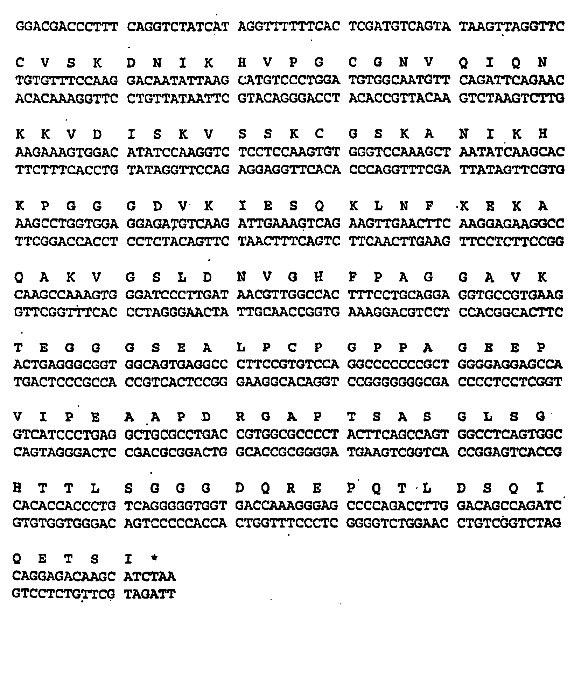

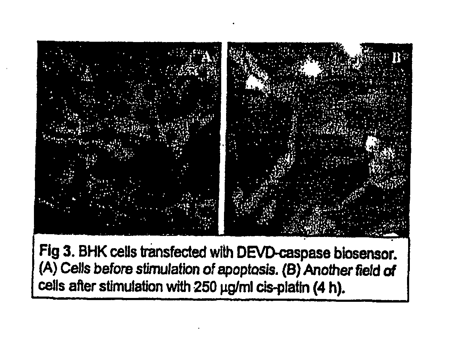

This biosensor is restricted to the cytoplasm by the reactant target sequence. The reactant target sequence is the annexin II cytoskeletal binding domain (MSTVHEILCKLSLEGVHSTPPSA) **(SEQ ID NO:124) (Figure 29C)** (Eberhard et al. 1997. *Mol. Biol. Cell* 8:293a). The enzyme recognition site corresponds to two copies of the amino acid sequence DEVD **(SEQ ID NO:60) (Figure 29B),** which serves as the recognition site of caspase-3. Other examples with different numbers of protease recognition sites and/or additional amino acids from a naturally occurring protease recognition site are shown below. The signal domain is EGFP (**SEQ ID NO:46) (Figure 29A)** (Clontech, California). The parent biosensor (the reactant) is restricted to the cytoplasm by binding of the annexin II domain to the cytoskeleton, and is therefore excluded from the nucleus. Upon cleavage of the protease recognition site by caspase 3, the signal domain (EGFP) is released from the reactant targeting domain (annexin II), and is distributed throughout the whole volume of the cell, because it lacks any specific targeting sequence and is small enough to enter the nucleus passively. **(Fig 32)**

The biosensor response is measured by quantitating the effective cytoplasm-to-nuclear translocation of the signal (see above). Measurement of the response is by one of several modes, including integrated or average nuclear region intensity, the ratio or difference of the integrated or average cytoplasm intensity to integrated or average nuclear intensity. The nucleus is defined using a DNA-specific dye, such as Hoechst 33342.

This biosensor provides a measure of the proteolytic activity around the annexin II cytoskeleton binding sites within the cell. Given the dispersed nature of the cytoskeleton and the effectively diffuse state of cytosolic enzymes, this provides an effective measure of the cytoplasm in general.

### Results & Discussion:

**Fig 32** illustrates images before and after stimulation of apoptosis by cis-platin in BHK cells, transfected with the caspase 3 biosensor. The images clearly illustrate accumulation of fluorescence in the nucleus. Generation of the spatial change in fluorescence is non-reversible and thus the timing of the assay is flexible. Controls for this biosensor include using a version in which the caspase-3-specific site has been omitted. In addition, disruption of the cytoskeleton with subsequent cell rounding did not produce the change in fluorescence distribution. Our experiments demonstrate the correlation of nuclear condensation with activation of caspase activity. We have also tested this biosensor in MCF-7 cells. A recent report measured a peak response in caspase-3 activity 6 h after stimulation of MCF-7 cells with etoposide accompanied by cleavage of PARP (Benjamin et al. 1998.*Mol Pharmacol.* 53:446-50). However, another recent report found that MCF-7 cells do not possess caspase-3 activity and, in fact, the caspase-3 gene is functionally deleted (Janicke et al. 1998. *J Biol Chem.* 273:9357-60). Caspase-3 activity was not detected with the caspase biosensor in MCF-7 cells after a 15 h treatment with 100 µM etoposide.

Janicke et al., (1998) also indicated that many of the conventional substrates of caspase-3 were cleaved in MCF-7 cells upon treatment with staurosporine. Our experiments demonstrate that caspase activity can be measured using the biosensor in MCF-7 cells when treated with staurosporine. The maximum magnitude of the activation by staurosporine was approximately one-half that demonstrated with cis-platin in BHK cells. This also implies that the current biosensor, although designed to be caspase-3-specific, is indeed specific for a class of caspases rather than uniquely specific for caspase-3. The most likely candidate is caspase-7 (Janicke et al., 1998). These experiments also demonstrated that the biosensor can be used in multiparameter experiments, with the correlation of decreases in mitochondrial membrane potential, nuclear condensation, and caspase activation.

We have specifically tested the effects of paclitaxel on caspase activation using the biosensor. Caspase activity in BHK and MCF-7 cells was stimulated by paclitaxel. It also appears that caspase activation occurred after nuclear morphology changes. One caveat is that, based on the above discussions, the caspase activity reported by the biosensor in this assay is likely to be due to the combination of caspase-3 and, at least, caspase-7 activity.

Consistent with the above results using staurosporine stimulation on MCF-7 cells, paclitaxel also stimulated the activation of caspase activity. The magnitude was similar to that of staurosporine. This experiment used a much narrower range of paclitaxel than previous experiments where nuclear condensation appears to dominate the response.

### b. Caspase biosensor with the microtubule associated protein 4 (MAP4) projection domain (CP8GFPNLS-SIZEPROJ)

Another approach for restricting the reactant to the cytoplasm is to make the biosensor too large to penetrate the nuclear pores Cleavage of such a biosensor liberates a product capable of diffusing into the nucleus.

The additional size required for this biosensor is provided by using the projection domain of MAP4 (**SEQ ID NO:142**) **(Figure 29C)** (CP8GFPNLS-SIZEPROJ). The projection domain of MAP4 does not interact with microtubules on its own, and, when expressed, is diffusely distributed throughout the cytoplasm, but is excluded from the nucleus due to its size (∼120 kD). Thus, this biosensor is distinct from the one using the full length MAP4 sequence. (see below) One of skill in the art will recognize that many other such domains could be substituted for the MAP4 projection domain, including but not limited to multiple copies of any GFP or one or more copies of any other protein that lacks an active NLS and exceeds the maximum size for diffusion into the nucleus (approximately 60 kD; Alberts, B., Bray, D., Raff, M., Roberts, K., Watson, J.D. (Eds.) Molecular Biology of the Cell, third edition, New York: Garland publishing, 1994. pp 561-563). The complete sequence of the resulting biosensor is shown in **SEQ ID NO: 3-4**. A similar biosensor with a different protease recognition domain is shown in **SEQ ID NO:5-6**.

### c. Caspase biosensor with a nuclear export signal

Another approach for restricting the reactant to the cytoplasm is to actively restrict the reactant from the nucleus by using a nuclear export signal. Cleavage of such a biosensor liberates a product capable of diffusing into the nucleus.

The Bacillus anthracis bacterium expresses a zinc metalloprotease protein complex called anthrax protease. Human mitogen activated protein kinase kinase 1 (MEK 1) (Seger et al., J. Biol. Chem. 267:25628-25631, 1992) possesses an anthrax protease recognition site (amino acids 1-13) **(SEQ ID NO:102) (Figure 29B)** that is cleaved after amino acid 8, as well as a nuclear export signal at amino acids **32-44 (SEQ ID NO:140) (Figure 29C).** Human MEK 2 (Zheng and Guan, J. Biol. Chem. 268:11435-11439, 1993) possesses an anthrax protease recognition site comprising amino acid residues 1-16 **(SEQ ID NO:104) (Figure 29B)** and a nuclear export signal at amino acids 36-48. (**SEQ ID NO:148**) **(Figure 29C).**

The anthrax protease biosensor comprises Fret25 (**SEQ ID NO:48) (Figure 29A)** as the signal, the anthrax protease recognition site, and the nuclear export signal from MEK 1 or MEK2. **(SEQ ID NOS: 7-8 (MEK1); 9-10 (MEK2))** The intact biosensor will be retained in the cytoplasm by virture of this nuclear export signal (eg., the reactant target site). Upon cleavage of the fusion protein by anthrax protease, the NES will be separated from the GFP allowing the GFP to diffuse into the nucleus.

### 2. Construction of 4- and 5-domain biosensors

For all of the examples presented above for 3-domain protease biosensors, a product targeting sequence, including but not limited to those in **Figure 29C**, such as a nuclear localization sequence (NLS), can be operatively linked to the signal sequence, and thus cause the signal sequence to segregate from the reactant target domain after proteolytic cleavage. Addition of a second detectable signal domain, including but not limited to those in **Figure 29A**, operatively linked with the reactant target domain is also useful in allowing measurement of the reaction by multiple means. Specific examples of such biosensors are presented below.

### a. 4 domain biosensors

### 1. Caspase biosensors with nuclear localization sequences (pcas3nlsGFP; CP3GFPNLS-CYTO):

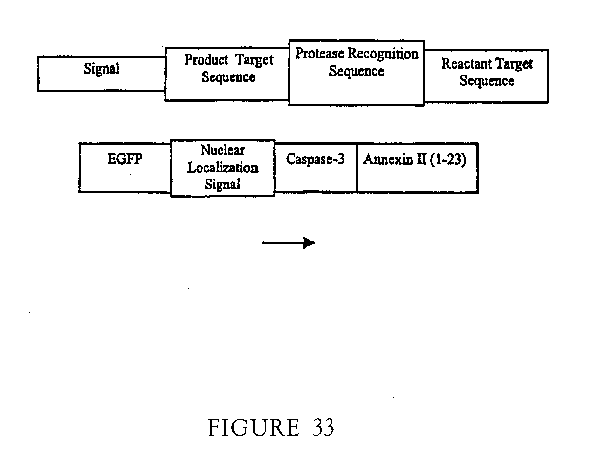

The design of the biosensor is outlined in **Figure 33**, and its sequence is shown in **SEQ ID NO:11-12.** PCR and cloning procedures were performed as described above, except that the following oligonucleotides were used:
**Primers for Caspase 3, Product target sequence = NLS (CP3GFPNLS-CYTO):**

This biosensor is similar to that shown in **SEQ ID NO:2** except upon recognition and cleavage of the protease recognition site, the product is released and the signal accumulates specifically in the nucleus due to the presence of a nuclear localization sequence, RRKRQK **(SEQ ID NO:128) (Figure 29C)**(Briggs et al., J. Biol. Chem. 273:22745, 1998) attached to the signal. A specific benefit of this construct is that the products are clearly separated from the reactants. The reactants remain in the cytoplasm, while the product of the enzymatic reaction is restricted to the nuclear compartment. The response is measured by quantitating the effective cytoplasm-to-nuclear translocation of the signal, as described above.

With the presence of both product and reactant targeting sequences in the parent biosensor, the reactant target sequence should be dominant prior to activation (e.g., protease cleavage) of the biosensor. One way to accomplish this is by masking the product targeting sequence in the parent biosensor until after protease cleavage. In one such example, the product target sequence is functional only when relatively near the end of a polypeptide chain (ie: after protease cleavage). Alternatively, the biosensor may be designed so that its tertiary structure masks the function of the target sequence until after protease cleavage. Both of these approaches include comparing targeting sequences with different relative strengths for targeting Using the example of the nuclear localization sequence (NLS) and annexin II sequences, different strengths of NLS have been tried with clone selection based on cytoplasmic restriction of the parent biosensor. Upon activation, the product targeting sequence will naturally dominate the localization of its associated detectable sequence domain because it is then separated from the reactant targeting sequence.

An added benefit of using this biosensor is that the product is targeted, and thus concentrated, into a smaller region of the cell. Thus, smaller amounts of product are detectable due to the increased concentration of the product. This concentration effect is relatively insensitive to the cellular concentration of the reactant. The signal-to-noise ratio (SNR) of such a measurement is improved over the more dispersed distribution of biosensor #1.

Similar biosensors that incorporate either the caspase 6 **(SEQ ID NO:66) (Figure 29B)** or the caspase 8 protease recognition sequence **(SEQ ID NO:74) (Figure 29B)** can be made using the methods described above, but using the following primer sets:

### Primers for Caspase 6, Product target sequence = NLS (CP6GFPNLS-CYTO)

### Primers for Caspase 8, Product target sequence = NLS (CP8GFPNLS-CYTO)

The sequence of the resulting biosensors is shown in **SEQ ID NO:13-14** (Caspase 6) and **SEQ ID NO: 15-16** (Caspase 8). Furthermore, multiple copies of the protease recognition sites can be inserted into the biosensor, yielding the biosensors shown in **SEQ ID NO: 17-18 (Caspase 3)** and **SEQ ID NO:19-20 (Caspase 8).**

### 2. Caspase 3 biosensor with a second signal domain

An alternative embodiment employs a second signal domain operatively linked to the reactant target domain. In this example, full length MAP4 serves as the reactant target sequence. Upon recognition and cleavage, one product of the reaction, containing the reactant target sequence, remains bound to microtubules in the cytoplasm with its own unique signal, while the other product, containing the product target sequence, diffuses into the nucleus. This biosensor provides a means to measure two activities at once: caspase 3 activity using a translocation of GFP into the nucleus and microtubule cytoskeleton integrity in response to signaling cascades initiated during apoptosis, monitored by the MAP4 reactant target sequence.

The basic premise for this biosensor is that the reactant is tethered to the microtubule cytoskeleton by virtue of the reactant target sequence comprising the full length microtubule associated protein MAP4 **(SEQ ID NO:152) (Figure 29C)** In this case, a DEVD **(SEQ ID NO:60) (Figure 29B)** recognition motif is located between the EYFP signal **(SEQ ID NO:44) (Figure 29A)** operatively linked to the reactant target sequence, as well as the EBFP signal **(SEQ ID NO:48) (Figure 29A)** operatively linked to the C-terminus of MAP4. The resulting biosensor is shown in **SEQ ID NO:21-22.**

This biosensor can also include a product targeting domain, such as an NLS, operatively linked to the signal domain.

With this biosensor, caspase-3 cleavage still releases the N-terminal GFP, which undergoes translocation to the nucleus (directed there by the NLS). Also, the MAP4 fragment, which is still intact following proteolysis by caspase-3, continues to report on the integrity of the microtubule cytoskeleton during the process of apoptosis via the second GFP molecule fused to the C-terminus of the biosensor. Therefore, this single chimeric protein allows simultaneous analysis of caspase-3 activity and the polymerization state of the microtubule cytoskeleton during apoptosis induced by a variety of agents. This biosensor is also useful for analysis of potential drug candidates that specifically target the microtubule cytoskeleton, since one can determine whether a particular drug induced apoptosis in addition to affecting microtubules.

This biosensor potentially combines a unique signal for the reactant, fluorescence resonance energy transfer (FRET) from signal 2 to signal 1, and a unique signal localization for the product, nuclear accumulation of signal 1. The amount of product generated will also be indicated by the magnitude of the loss in FRET, but this will be a smaller SNR than the combination of FRET detection of reactant and spatial localization of the product.

FRET can occur when the emission spectrum of a donor overlaps significantly the absorption spectrum of an acceptor molecule. (dos Remedios, C.G., and P.D. Moens. 1995. Fluorescence resonance energy transfer spectroscopy is a reliable "ruler" for measuring structural changes in proteins. Dispelling the problem of the unknown orientation factor. *J Struct Biol.* 115:175-85; Emmanouilidou, E., A.G. Teschemacher, A.E. Pouli, L.I. Nicholls, E.P. Seward, and G.A. Rutter. 1999. Imaging Ca(2+) concentration changes at the secretory vesicle surface with a recombinant targeted cameleon. *Curr Biol.* 9:915-918.) The average physical distance between the donor and acceptor molecules should be between 1 nm and 10 nm with a preference of between 1 nm and 6 nm. The intervening sequence length can vary considerably since the three dimensional structure of the peptide will determine the physical distance between donor and acceptor. This FRET signal can be measured as (1) the amount of quenching of the donor in the presence of the acceptor, (2) the amount of acceptor emission when exciting the donor, and/or (3) the ratio between the donor and acceptor emission. Alternatively, fluorescent lifetimes of donor and acceptor could be measured.

This case adds value to the above FRET biosensor by nature of the existence of the reactant targeting sequence. This sequence allows the placement of the biosensor into specific compartments of the cell for a more direct readout of activity in those compartments such as the inner surface of the plasma membrane.

The cytoplasmic second signal represents both original reactant plus one part of the product. The nuclear first signal represents another product of the reaction. Thus the enzymatic reaction has the added flexibility in that it can be represented as (1) nuclear intensity; (2) the nucleus /cytoplasm ratio; (3) the nucleus /cytoplasm FRET ratio; (4) cytoplasmic /cytoplasmic FRET ratio.

The present FRET biosensor design differs from previous FRET-based biosensors (see WO 97/28261; W09837226) in that it signal measurement is based on spatial position rather than intensity. The products of the reaction are segregated from the reactants. It is this change in spatial position that is measured. The FRET-based biosensor is based on the separation, but not to another compartment, of a donor and acceptor pair. The intensity change is due to the physical separation of the donor and acceptor upon proteolytic cleavage. The disadvantages of FRET-based biosensors are (1) the SNR is rather low and difficult to measure, (2) the signal is not fixable. It must be recorded using living cells. Chemical fixation, for example with formaldehyde, cannot preserve both the parent and resultant signal; (3) the range of wavelengths are limiting and cover a larger range of the spectrum due to the presence of two fluorophores or a fluorophore and chromophore; (4) the construction has greater limitations in that the donor and acceptor must be precisely arranged to ensure that the distance falls within 1-10 nm.

Benefits of the positional biosensor includes: (1) ability to concentrate the signal in order to achieve a higher SNR. (2) ability to be used with either living or fixed cells; (3) only a single fluorescent signal is needed; (4) the arrangement of the domains of the biosensor is more flexible. The only limiting factor in the application of the positional biosensor is the need to define the spatial position of the signal which requires an imaging method with sufficient spatial resolution to resolve the difference between the reactant compartment and the product compartment.

One of skill in the art will recognize that this approach can be adapted to report any desired combination of activities by simply making the appropriate substitutions for the protease recognition sequence and the reactant target sequence, including but not limited to those sequences shown in **Figure 29A-C**.

### 3. Caspase 8 biosensor with a nucleolar localization domain (CP8GFPNUC-CYTO)

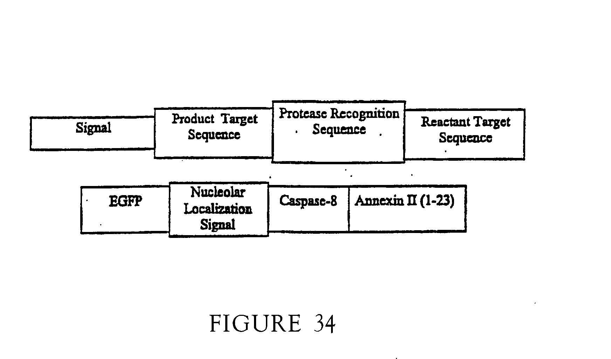

This approach (diagrammed in **Figure 34**) utilizes a biosensor for the detection of caspase-8 activity. In this biosensor, a nucleolar localization signal (RKRIRTYLKSCRRMKRSGFEMSRPIPSHLT) **(SEQ ID NO:130) (Figure 29C)** (Ueki et al., Biochem. Biophys. Res. Comm. 252:97-100, 1998) was used as the product target sequence, and made by PCR using the primers described below. The PCR product was digested with BspE1 and Pvu1 and gel purified. The vector and the PCR product were ligated as described above.

### Primers for Caspase 8, Nucleolar localization signal (CP8GFPNUC-CYTO):

The sequence of the resulting biosensor is shown in **SEQ ID NO: 23-24**. This biosensor includes the protease recognition site for caspase-8 **(SEQ ID NO:74) (Figure 29B)**. A similar biosensor utilizes the protease recognition site for caspase-3. **(SEQ ID NO:25-26)**

These biosensors could be used with other biosensors that possess the same product signal color that are targeted to separate compartments, such as CP3GFPNLS-CYTO. The products of each biosensor reaction can be uniquely measured due to separation of the products based on the product targeting sequences. Both products from CP8GFPNUC-CYTO and CP3GFPNLS-CYTO are separable due to the different spatial positions, nucleus vs. nucleolus, even though the colors of the products are exactly the same. Assessing the non-nucleolar, nuclear region in order to avoid the spatial overlap of the two signals would perform the measurement of CP3GFPNLS in the presence of CP8GFPNUC. The loss of the nucleolar region from the nuclear signal is insignificant and does not significantly affect the SNR. The principle of assessing multiple parameters using the same product color significantly expands the number of parameters that can be assessed simultaneously in living cells. This concept can be extended to other non-overlapping product target compartments.

Measurement of translocation to the nucleolar compartment is performed by (1) defining a mask corresponding to the nucleolus based on a nucleolus-specific marker, including but not limited to an antibody to nucleolin (Lischwe et al., 1981. *Exp. Cell Res.* 136:101-109); (2) defining a mask for the reactant target compartment, and (3) determining the relative distribution of the signal between these two compartments. This relative distribution could be represented by the difference in the two intensities or, preferably, the ratio of the intensities between compartments.

The combination of multiple positional biosensors can be complicated if the reactant compartments are overlapping. Although each signal could be measured by simply determining the amount of signal in each product target compartment, higher SNR will be possible if each reactant is uniquely identified and quantitated. This higher SNR can be maximized by adding a second signal domain of contrasting fluorescent property. This second signal may be produced by a signal domain operatively linked to the product targeting sequence, or by FRET (see above), or by a reactant targeting sequence uniquely identifying it within the reactant compartment based on color, spatial position, or fluorescent property including but not limited to polarization or lifetime. Alternatively, for large compartments, such as the cytoplasm, it is possible to place different, same colored biosensors in different parts of the same compartment.

### 4. Protease biosensors with multiple copies of a second signal domain serving as a reactant target domain

In another example, (CP8YFPNLS-SIZECFPn) increasing the size of the reactant is accomplished by using multiple inserts of a second signal sequence, for example, ECFP (**SEQ ID NO:50) (Figure 29A)** (Tsien, R.Y. 1998. Annu Rev Biochem. 67:509-44). Thus, the multiple copies of the second signal sequence serve as the reactant target domain by excluding the ability of the biosensor to diffuse into the nucleus. This type of biosensor provides the added benefit of additional signal being available per biosensor molecule. Aggregation of multiple fluorescent probes also can result in unique signals being manifested, such as FRET, self quenching, eximer formation, etc. This could provide a unique signal to the reactants.

### 5. Tetanus/botulinum biosensor with trans-membrane targeting domain

In an alternative embodiment, a trans-membrane targeting sequence is used to tether the reactant to cytoplasmic vesicles, and an alternative protease recognition site is used. The tetanus/botulinum biosensor **(SEQ ID NOS:27-28** (cellubrevin); **29-30** (synaptobrevin) consists of an NLS **(SEQ ID NO:128) (Figure 29C),** Fret25 signal domain **(SEQ ID NO:52) (Figure 29A),** a tetanus or botulinum zinc metalloprotease recognition site from cellubrevin **(SEQ ID NO:106) (Figure 29B)** (McMahon et al., Nature 364:346-349, 1993; Martin et al., J. Cell Biol., in press) or synaptobrevin **(SEQ ID NO:108) (Figure 29B)** (GenBank Accession #U64520), and a trans-membrane sequence from cellubrevin **(SEQ ID NO:146) (Figure 29C)** or synaptobrevin **(SEQ ID NO:144) (Figure 29C)** at the 3'-end which tethers the biosensor to cellular vesicles. The N-terminus of each protein is oriented towards the cytoplasm. In the intact biosensor, GFP is tethered to the vesicles. Upon cleavage by the tetanus or botulinum zinc metalloprotease, GFP will no longer be associated with the vesicle and is free to diffuse throughout the cytoplasm and the nucleus.

### b. 5-domain biosensors

### 1. Caspase 3 biosensor with a nuclear localization domain and a second signal domain operatively linked to an annexin II domain

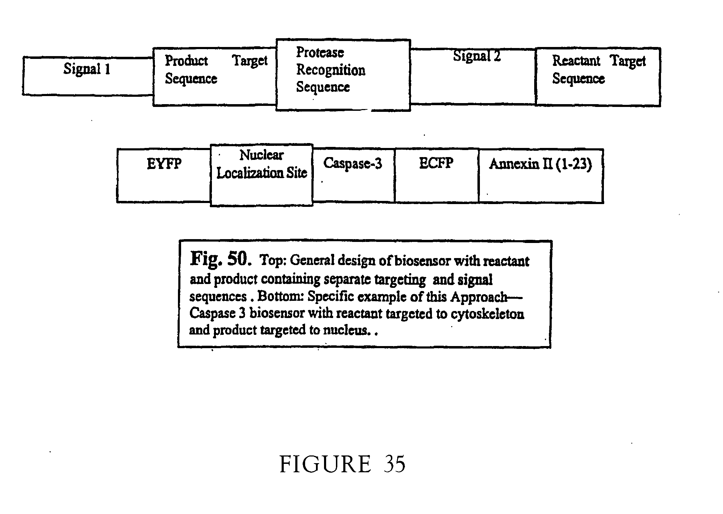

The design of this biosensor is outlined in **Figure 35,** and the sequence is shown in **SEQ ID NO:33-34.** This biosensor differs from **SEQ ID NO 11-12** by including a second detectable signal, ECFP (**SEQ ID NO:50**) **(Figure 29A)** (signal 2) operatively linked to the reactant target sequence.

### 2. Caspase 3 biosensor with a nuclear localization sequence and a second signal domain operatively linked to a MAP4 projection domain (CP3YFPNLS-CFPCYTO)

In this biosensor (**SEQ ID NO:31-32),** an NLS product targeting domain **(SEQ ID NO:128) (Figure 29C)** is present upstream of an EYFP signal domain **(SEQ ID NO:44) (Figure 29A).** A DEVD protease recognition domain (**SEQ ID NO:60**) **(Figure 29B)** is between after the EYFP signal domain and before the MAP4 projection domain **(SEQ ID NO:142) (Figure 29C).**

### Example 11. Fluorescent Biosensor Toxin Characterization

As used herein, "toxin" refers to any organism, macromolecule, or organic or inorganic molecule or ion that alters normal physiological processes found within a cell, or any organism, macromolecule, or organic or inorganic molecule or ion that alters the physiological response to modulators of known physiological processes. Thus, a toxin can mimic a normal cell stimulus, or can alter a response to a normal cell stimulus.

Living cells are the targets of toxic agents that can comprise organisms, macromolecules, or organic or inorganic molecules. A cell-based approach to toxin detection, classification, and identification would exploit the sensitive and specific molecular detection and amplification system developed by cells to sense minute changes in their external milieu. By combining the evolved sensing capability of cells with the luminescent reporter molecules and assays described herein, intracellular molecular and chemical events caused by toxic agents can be converted into detectable spatial and temporal luminescent signals.

When a toxin interacts with a cell, whether it is at the cell surface or within a specific intracellular compartment, the toxin invariably undermines one or more components of the molecular pathways active within the cell. Because the cell is comprised of complex networks of interconnected molecular pathways, the effects of a toxin will likely be transmitted throughout many cellular pathways. Therefore, our strategy is to use molecular markers within key pathways likely to be affected by toxins, including but not limited to cell stress pathways, metabolic pathways, signaling pathways, and growth and division pathways.

We have developed and characterized three classes of cell based luminescent reporter molecules to serve as reporters of toxic threat agents. These 3 classes are as follows:
(1) *Detectors*: general cell stress detection of a toxin;
(2) *Classifiers*: perturbation of key molecular pathway(s) for detection and classification of a toxin; and
(3) *Identifiers*: activity mediated detection and identification of a toxin or a group of toxins.

Thus, in another aspect of the present invention, living cells are used as biosensors to interrogate the environment for the presence of toxic agents. In one embodiment of this aspect, an automated method for cell based toxin characterization is disclosed that comprises providing an array of locations containing cells to be treated with a test substance, wherein the cells possess at least a first luminescent reporter molecule comprising a detector and a second luminescent reporter molecule selected from the group consisting of a classifier or an identifier; contacting the cells with the test substance either before or after possession of the first and second luminescent reporter molecules by the cells; imaging or scanning multiple cells in each of the locations containing multiple cells to obtain luminescent signals from the detector; converting the luminescent signals from the detector into digital data to automatically measure changes in the localization, distribution, or activity of the detector on or in the cell, which indicates the presence of a toxin in the test substance; selectively imaging or scanning the locations containing cells that were contacted with test sample indicated to have a toxin in it to obtain luminescent signals from the second reporter molecule; converting the luminescent signals from the second luminescent reporter molecule into digital data to automatically measure changes in the localization, distribution, or activity of the classifier or identifier on or in the cell, wherein a change in the localization, distribution, structure or activity of the classifier identifies a cell pathway that is perturbed by the toxin present in the test substance, or wherein a change in the localization, distribution, structure or activity of the identifier identifies the specific toxin that is present in the test substance. In a preferred embodiment, the cells possess at least a detector, a classifier, and an identifier. In a further preferred embodiment, the digital data derived from the classifier is used to determine which identifier(s) to employ for identifying the specific toxin or group of toxins.

As used herein, the phrase "the cells possess one or more luminescent reporter molecules" means that the luminescent reporter molecule may be expressed as a luminescent reporter molecule by the cells, added to the cells as a luminescent reporter molecule, or luminescently labeled by contacting the cell with a luminescently labeled molecule that binds to the reporter molecule, such as a dye or antibody, that binds to the reporter molecule. The luminescent reporter molecule can be expressed or added to the cell either before or after treatment with the test substance.

The luminescent reporters comprising detectors, classifiers, and identifiers may also be distributed separately into single or multiple cell types. For example, one cell type may contain a toxin detector, which, when activated by toxic activity, implies to the user that the same toxin sample should be screened with reporters of the classifier or identifier type in yet another population of cells identical to or different from the cells containing the toxin detector.

The detector, classifier, and identifier can comprise the same reporter molecule, or they can comprise different reporters.

Screening for changes in the localization, distribution, structure or activity of the detectors, classifiers, and/or identifiers can be carried out in either a high throughput or a high content mode. In general, a high-content assay can be converted to a high-throughput assay if the spatial information rendered by the high-content assay can be recoded in such a way as to no longer require optical spatial resolution on the cellular or subcellular levels. For example, a high-content assay for microtubule reorganization can be carried out by optically resolving luminescently labeled cellular microtubules and measuring their morphology (*e.g.*, bundled vs. non-bundled or normal). A high-throughput version of a microtubule reorganization assay would involve only a measurement of total microtubule polymer mass after cellular extraction with a detergent. That is, destabilized microtubules, being more easily extracted, would result in a lower total microtubule mass luminescence signal than unperturbed or drug-stabilized luminescently labeled microtubules in another treated cell population. The luminescent signal emanating from a domain containing one or more cells will therefore be proportional to the total microtubule mass remaining in the cells after toxin treatment and detergent extraction.

The methods for detecting, classifying, and identifying toxins can utilize the same screening methods described throughout the instant application, including but not limited to detecting changes in cytoplasm to nucleus translocation, nucleus or nucleolus to cytoplasm translocation, receptor internalization, mitochondrial membrane potential, signal intensity, the spectral response of the reporter molecule, phosphorylation, intracellular free ion concentration, cell size, cell shape, cytoskeleton organization, metabolic processes, cell motility, cell substrate attachment, cell cycle events, and organellar structure and function.

In all of these embodiments, the methods can be operated in both toxin-mimetic and toxin-inhibitory modes.

Such techniques to assess the presence of toxins are useful for methods including, but not limited to, monitoring the presence of environmental toxins in test samples and for toxins utilized in chemical and biological weapons; and for detecting the presence and characteristics of toxins during environmental remediation, drug discovery, clinical applications, and during the normal development and manufacturing process by virtually any type of industry, including but not limited to agriculture, food processing, automobile, electronic, textile, medical device, and petroleum industries.

We have developed and characterized examples of luminescent cell-based reporters, distributed across the 3 sensor classes. The methods disclosed herein can be utilized in conjunction with computer databases, and data management, mining, retrieval, and display methods to extract meaningful patterns from the enormous data set generated by each individual reporter or a combinatorial of reporters in response to toxic agents. Such databases and bioinformatics methods include, but are not limited to, those disclosed in U.S. Patent Application Nos. 09/437,976, filed November 10, 1999; 60/145,770 filed July 27, 1999 and U.S. Patent Application Serial No. to be assigned, filed February 19, 2000. (98,068-C)

Any cell type can be used to carry out this aspect of the invention, including prokaryotes such as bacteria and archaebacteria, and eukaryotes, such as single celled fungi (for example, yeast), molds (for example, Dictyostelium), and protozoa (for example, Euglena). Higher eukaryotes, including, but not limited to, avian, amphibian, insect, and mammalian cells can also be used.

### Examples of Biosensors

| Number | Name | Class | Cell Types | Response to model toxins | |
|---|---|---|---|---|---|
| | | | | Positive | Negative |
| 1 | Mitochondrial Potential [Donnan Equilibrium Dye] | D | • LLCPK (pig epithelia) | Valinomycin (10 nM-100 µM) FCCP (10 nM-100 µM) | Oligomycin (10 nM) |
| | | | • Rat primary hepatocytes | | |
| 2 | Heat Shock Protein (Hsp 27, Hsp 70) GFP-chimera | D | • HeLa | Cadmium (10mM) | TNF-α (100ng/ml) |
| | | | • 3T3 | | |
| 3 | Tubulincytoskeleton [β-tubulin-GFP chimera] | C | • BHK | Paclitaxel (10 nM-20µM) Curacin-A (5 nM-10µM) Nocadazole (7 nM-12µM) Colchicine (5 nM-10µM) Vinblastine (5 nM-10µM) | Staurosporine (1 nM-1 µM) |
| | | | • HeLa | | |
| | | | • LLCPK | | |
| 4 | pp38 MAPK- stress signaling [antibody and GFP-chimera] | C | • 3T3 | Anisomycin (100 µM) Cadmium (10 µM) | TNF-α (100 ng/ml) |
| | | | • LLCPK | | |
| 5 | NF-κB- stress signaling [antibody and GFP-chimera] | C | • HeLa | TNF-α (100ng/ml-0.38pg/ml) IL-1 (4ng/ml-.095pg/ml) Nisin (2-1000 µg/ml) Streptolysin (10 µg/ml) Anisomycin (100 µM) | Anisomycin (10 nM-10 µM) Cadmium (1-10 µM) Penitrem A (10 µM) Valinomycin (1 µM) |
| | | | • 3T3 | | |
| | | | • BHK | | |
| | | | • SNB19 | | |
| | | | • HepG2 | | |
| | | | • LLCPK | | |
| 6 | IκB [complement to NFκB] | C | In many cell types | | |
| 7 | Tetanus Toxin [Protease activity-based sensor] | I | In many cell types | | |
| 8 | Anthrax LF [Protease activity-based sensor] | I | In many cell types | | |
| Sensor Class: D= Detector of toxins; C= Classifier of toxins; I= Identifier of toxin or group of toxins The model toxins can generally be purchased from Sigma Chemical Company (St. Louis, MO) | | | | | |

***Examples of Detectors:*** This class of sensors provides a first line signal that indicates the presence of a toxic agent. This class of sensors provides detection of general cellular stress that requires resolution limited only to the domain over which the measurement is being made, and they are amenable to high content screens as well. Thus, either high throughput or high content screening modes may be used, including but not limited to translocation of heat shock factors from the cytoplasm to the nucleus, and changes in mitochondrial membrane potential, intracellular free ion concentration detection (for example, Ca²⁺; H⁺), general metabolic status, cell cycle timing events, and organellar structure and function.

### 1. Mitochondrial Potential

A key to maintenance of cellular homeostasis is a constant ATP energy charge. The cycling of ATP and its metabolites ADP, AMP, inorganic phosphate, and solution-phase protons is continuously adjusted to meet the catabolic and anabolic needs of the cell. Mitochondria are primarily responsible for maintaining a constant energy charge throughout the entire cell. To produce ATP from its constituents, mitochondria must maintain a constant membrane potential within the organelle itself. Therefore, measurement of this electrical potential with specific luminescent probes provides a sensitive and rapid readout of cellular stress.

We have utilized a positively charged cyanine dye, JC-1 (Molecular Probes, Eugene, OR), which diffuses into the cell and readily partitions into the mitochondrial membrane, for measurement of mitochondrial potential. The photophysics of JC-1 are such that when the probe partitions into the mitochondrial membrane and it experiences an electrical potential >140 mV, the probe aggregates and its spectral response is shifted to the red. At membrane potential values <140 mV, JC-1 is primarily monomeric and its spectral response is shifted toward the blue. Therefore, the ratio of two emission wavelengths (645 nm and 530 nm) of JC-1 partitioned into mitochondria provides a sensitive and continuous measure of mitochondrial membrane potential.

We have been making live cell measurements in a high throughput mode as the basis of a generalized indicator of toxic stress. The goal of our initial experiments was to determine the ratio of J-aggregates of JC-1 dye to its monomeric form both before and after toxic stress.

### Procedure

1. Cells were plated and cultured up to overnight.
2. Cells were stained with JC-1 (10 µg/ml) for 30 minutes at 37° C in a CO₂ incubator.
3. Cells were then washed quickly with HBSS at 37°C (2 times, 150 µl/well), the toxins were added if required, and the entire plate was scanned in a plate reader. The JC-1 monomer was measured optimally with a 485 nm excitation/530 nm emission wavelength filter set, and the aggregates were best measured with a 590 nm excitation/645 nm emission wavelength set.

### Results

The mitochondrial potential within several types of living cells, and the effects of toxins on the potential were measured using the fluorescence ratio Em 645 (590)/Em 530 (485) (excitation wavelengths in parentheses). For example, we measured the effect of 10 µM valinomycin on the mitochondrial potential within LLCPK cells (pig epithelia). Within seconds of treatment, the toxin induced a more rapid and higher magnitude decrease (an approximately 50% reduction) in mitochondrial potential than that found in untreated cells. Hepatocytes were also determined to be sensitive to valinomycin, and the changes in mitochondrial potential were nearly complete within seconds to minutes after addition of various concentrations of the toxin.

These results are consistent with mitochondrial potential being a model intracellular detector of cell stress. Because these measurements require no spatial resolution within individual cells, mitochondrial potential measurements can be made rapidly on an entire cell array (e.g. high throughput). This means, for example, that complex arrays of many cell types can be probed simultaneously and continuously as a generalized toxic response. Such an indicator can provide a first line signal to indicate that a general toxic stress is present in a sample. Further assays can then be conducted to more specifically identify the toxin using cells classifier or identifier type reporter molecules.

### 2. Heat Shock Proteins

Most mammalian cells will respond to a variety of environmental stimuli with the induction of a family of proteins called stress proteins. Anoxia, amino acid analogues, sulfhydryl-reacting reagents, transition metal ions, decouplers of oxidative phosphorylation, viral infections, ethanol, antibiotics, ionophores, non-steroidal antiinflammatory drugs, thermal stress and metal chelators are all inducers of cell stress protein synthesis, function, or both. Upon induction, cell stress proteins play a role in folding and unfolding proteins, stabilizing proteins in abnormal configurations, and repairing DNA damage.

There is evidence that at least four heat shock proteins translocate from the cytoplasm to the nucleus upon stress activation of the cell. These proteins include the heat shock proteins HSP27 and HSP70, the heat shock cognate HSC70, and the heat shock transcription factor HSF1. Therefore, measurement of cytoplasm to nuclear translocation of these proteins (and other stress proteins that translocate from the cytoplasm to the nucleus upon a cell stress) will provide a rapid readout of cellular stress.

We have tested the response of an HSP27-GFP biosensor (SEQ ID 169-170) in two cell lines (BHK and HeLa) using a library of heavy metal chemical compounds as biological toxin stimulants to stress the cells. Briefly, cells expressing the HSP27-GFP biosensor are plated into 96-well microplates, and allowed to attach. The cells are then treated with a panel of cell stress-inducing compounds. Exclusively cytoplasmic localization of the fusion protein was found in unstimulated cells.

Other similar heat shock protein biosensors (HSP-70, HSC70, and HSF1 fused to GFP) can be used as detectors, and are shown in SEQ ID NO: 171-176.

### Examples of Classifiers:

This class of sensors detects the presence of, and further classifies toxins by identifying the cellular pathway(s) perturbed by the toxin. As such, this suite of sensors can detect and/or classify toxins into broad categories, including but not limited to "toxins affecting signal transduction," "toxins affecting the cytoskeleton," and "toxins affecting protein synthesis". Either high throughput or high content screening modes may be used. Classifiers can comprise compounds including but not limited to tubulin, microtubule-associated proteins, actin, actin-binding proteins including but not limited to vinculin, α-actinin, actin depolymerizing factor/cofilin, profilin, and myosin; NF-κB, IκB, GTP-binding proteins including but not limited to rac, rho, and cdc42, and stress-activated protein kinases including but not limited to p38 mitogen-activated protein kinase.

### 1. Tubulin-cytoskeleton

The cell cytoskeleton plays a major role in cellular functions and processes, such as endo- and exocytosis, vesicle transport, and mitosis. Cytoskeleton-affecting toxins, of proteinaceous and non-proteinaceous form, such as C2 toxin, and several classes of enterotoxins, act either directly on the cytoskeleton, or indirectly via regulatory components controlling the organization of the cytoskeleton. Therefore, measurement of structural changes in the cytoskeleton can provide classification of the toxin into a class of cytoskeleton-affecting toxins. This assay can be conducted in a high content mode, as described previously, or in a high throughput mode. For high throughput as discussed previously.

Such measurements will be valuable for identification of toxins including, but not limited to anti-microtubule agents, agents that generally affect cell cycle progression and cell proliferation, intracellular signal transduction, and metabolic processes.

For microtubule disruption assays, LLCPK cells stably transfected with a tubulin-GFP biosensor plasmid were plated on 96 well cell culture dishes at 50-60% confluence and cultured overnight at 37 °C, 5% CO₂. A series of concentrations (10-500 nM) of 5 compounds (paclitaxel, curacin A, nocodazole, vinblastine, and colchicine) in normal culture media were freshly prepared from stock, and were added to cell culture dishes to replace the old culture media. The cells were then observed with the cell screening system described above, at a 12 hour time point.

Our data indicate that the tubulin chimera localizes to and assembles into microtubules throughout the cell. The microtubule arrays in cells expressing the chimera respond as follows to a variety of anti-microtubule compounds:

| Drug | Response |
|---|---|
| Vinblastine | Destabilization |
| Nocodazole | Destabilization |
| Paclitaxel | Stabilization |
| Colchicine | Destabilization |
| Curacin A | Destabilization |

Similar data were obtained using cells expressing the tubulin biosensor that were patterned onto cell arrays (such as those described in U.S. Patent Application Serial No. 08/865,341 filed May 29, 1997, incorporated by reference herein in its entirety) and dosed as above.

### 2. NF-κB

NF-κB is cytoplasmic at basal levels of stimulation, but upon insult translocates to the nucleus where it binds specific DNA response elements and activates transcription of a number of genes. Translocation occurs when IkB is degraded by the proteosome in response to specific phosphorylation and ubiquitination events. IkB normally retains NF-κB in the cytoplasm via direct interaction with the protein, and masking of the NLS sequence of NF-κB. Therefore, although not the initial or defining event of the whole signal cascade, NF-κB translocation to the nucleus can serve as an indicator of cell stress.

We have generated an NF-κB-GFP chimera for analysis in live cells. This was accomplished using standard polymerase chain reaction techniques using a characterized NF-κB p65 cDNA purchased from Invitrogen (Carlsbad, CA) fused to an EYFP PCR amplimer that was obtained from Clontech Laboratories (Palo Alto, CA). The resulting chimera is shown in **SEQ ID NO:177-178.** The two PCR products were ligated into an eukaryotic expression vector designed to produce the chimeric protein at high levels using the ubiquitous CMV promoter.

### NF-κB immunolocalization

For further studies, we characterized endogenous NF-κB activation by immunolocalization in toxin treated cells. The NF-κB antibodies used in this study were purchased from Santa Cruz Biotechnology, Inc. (Santa Cruz, CA), and secondary antibodies are from Molecular Probes (Eugene, OR).

For the 3T3 and SNB19 cell types, we determined the effective concentrations that yield response levels of 50% of the maximum (EC50), expressed in units of mass per volume (ng/ml) and units of molarity. Based on molecular weights of 17 kD for both TNFα and IL-1α, the EC50 levels for these two compounds with 3T3 and SNB19 cell types are given in units of molarity in **Table 1**. Our results demonstrated reproducibility of the relative responses from zero to maximum dose, but from sample to sample there have been occasional shifts in the baseline intensities of the response at zero concentration.

For these experiments, either 10 or 100 TNFα-treated 3T3 or SNB19 cells/well were tested. On the basis of the standard deviations measured for these samples, and by taking t-values for the student's t-test, we have estimated the minimum detectable doses for each case of cell type, compound, number of cells per well, and for different choices of how many wells are sampled per condition. The latter factor determines the number of degrees of freedom that are provided in the sample of data. Increasing the number of wells from 4 to 16, and increasing the number of cells per well from 10 to 100, improves the minimum detectable doses considerably. For 3T3 cells, which show lower minimum detectable doses than the SNB19 cells, and for the case of 1% false negative and 1% false positive rates, we estimate that 100 cells per well and a sampling of 12 or 16 wells are sufficient to detect a dose approximately equal to the EC50 value of 0.15 ng/ml. If the false positive rate is relaxed to 20%, a concentration of approximately half that value can be detected (0.83 ng/ml). One hundred cells can conveniently be sampled over a cell culture surface area of less than 1 mm².

**Table 1.**

| EC50 levels for TNFα and IL-1α (based on molecular weights of 17 kD for both) | | |
|---|---|---|
| Compound | Cell Type | EC50 (10⁻¹² moles/liter) |
| | | |
| TNFα | 3T3 | 8.8 |
| | SNB19 | 5.9 |
| | | |
| IL-1α | 3T3 | 0.24 |
| | SNB19 | 59 |

### 3. Phospho-p38 Mitogen Activated Protein Kinase (pp38MAPK)

MAPKs play a role in not only cell growth and division, but as mediators of cellular stress responses. One MAPK, p38, is activated by chemical stress inducers such as hyper-osmolar sorbitol, hydrogen peroxide, arsenite, cadmium ions, anisomycin, sodium salicylate, and LPS. Activation of p38 is also accompanied by its translocation into the nucleus from the cytoplasm.

MAPK p38 lies in a pathway that is a cascade of kinases. Thus, p38 is a substrate of one or more kinases, and it acts to phosphorylate one or more substrates in time and space within the living cell.

The assay we present here measures, as one of its parameters, p38 activation using immunolocalization of the phosphorylated form of p38 in toxin-treated cells. The assay was developed to be flexible enough to include the simultaneous measurement of other parameters within the same individual cells. Because the signal transduction pathway mediated by the transcription factor NF-κB is also known to be involved in the cell stress response, we included the activation of NF-κB as a second parameter in the same assay.

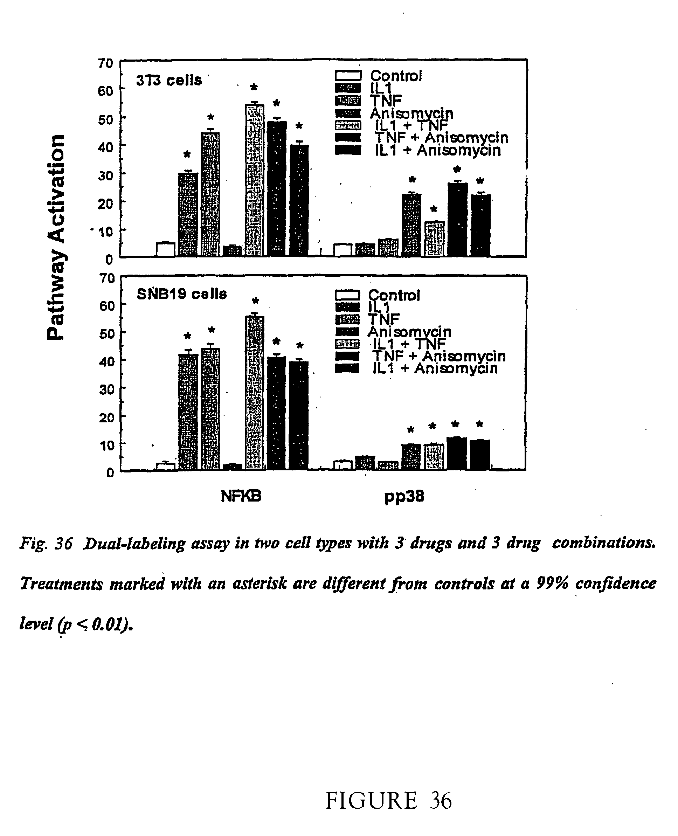

Our experiments demonstrate an immunofluorescence approach can be used to measure p38 MAPK activation either alone or in combination with NF-κB activation in the same cells. Multiple cell types, model toxins, and antibodies were tested, and significant stimulation of both pathways was measured in a high-content mode. The phospho-p38 antibodies used in this study were purchased from Sigma Chemical Company (St. Louis, MO). We report that at least two cell stress signaling pathways can not only be measured simultaneously, but are differentially responsive to classes of model toxins. **Figure 36** shows the differential response of the p38 MAPK and NF-κB pathways across three model toxins and two different cell types. Note that when added alone, three of the model toxins (IL1α, TNFα and Anisomycin) can be differentiated by the two assays as activators of specific pathways.

### IκB chimera

IkB degradation is the key event leading to nuclear translocation of NF-kB and activation of the NFkB-mediated stress response. We have chosen this sensor to complement the NF-kB sensor as a *classifier* in a high-throughput mode: the measurement of loss of signal due to degradation of the IκB-GFP fusion protein requires no spatial resolution within individual cells, and as such we envision IkB degradation measurements being made rapidly on an entire cell substrate.

This biosensor is based on fusion of the first 60 amino acids of IkB to the Fred25 variant of GFP. **SEQ ID 179-180** This region of IkB contains all the regulatory sequences, including phosphorylation sites and ubiquitination sites, necessary to confer proteosome degradation upon the biosensor. Knowing this, stimulation of any pathway that would typically lead to NFkB translocation results in degradation of this biosensor. Monitoring the fluorescence intensity of cells expressing IkB-GFP identifies the degradation process.

### Examples of Identifiers:

In our toxin identification strategy, the first two levels of characterization ensure a rapid readout of toxin class without sacrificing the ability to detect many new mutant toxins or dissect several complex mixtures of known toxins. The third level of biosensors are identifiers, which can identify a specific toxin or group of toxins. In one embodiment, an identifier comprises a protease biosensor that responds to the activity of a specific toxin. Other identifiers are produced with reporters/biosensors specific to their activities. These include, but are not limited to post-translational modifications such as phosphorylation or ADP-ribosylation, translocation between cellular organelles or compartments, effects on specific organelles or cellular components (for example, membrane permeabilization, cytoskeleton rearrangement, etc.)

ADP-ribosylating toxins - These toxins include Pseudomonas toxin A, diptheria toxin, botulinum toxin, pertussis toxin, and cholera toxin. For example, C. botulinum C2 toxin induces the ADP-ribosylation of Arg177 in the cytoskeletal protein actin, thus altering its assembly properties. Besides the construction of a classifier assay to measure actin-cytoskeleton regulation, an identifier assay can be constructed to detect the specific actin ADP-ribosylation. Because the ADP-ribosylation induces a conformational change that no longer permits the modified actin to polymerize, this conformational change can be detected intracellularly in several possible ways using luminescent reagents. For example, actin can be luminescently labeled using a fluorescent reagent with an appropriate excited state lifetime that allows for the measurement of the rotational diffusion of the intracellular actin using steady state fluorescence anisotropy. That is, toxin-modified actin will no longer be able to assemble into rigid filaments and will therefore produce only luminescent signals with relatively low anisotropy, which can be readily measured with an imaging system. In another embodiment, actin can be labeled with a polarity-sensitive fluorescent reagent that reports changes in actin-conformation through spectral shifts of the attached reagent. That is, toxin-treatment will induce a conformational change in intracellular actin such that a ratio of two fluorescence wavelengths will provide a measure of actin ADP-ribosylation.

Cytotoxic phospholipases - Several gram-positive bacterial species produce cytotoxic phospholipases. For example, Clostridium perfringens produces a phospholipase C specific for the cleavage of phosphoinositides. These phosphoinositides (e.g., inositol 1,4,5-trisphosphate) induce the release of calcium ions from intracellular organelles. An assay that can be conducted as either high-content or high-throughput can be constructed to measure the release of calcium ions using fluorescent reagents that have altered spectral properties when complexed with the metal ion. Therefore, a direct consequence of the action of a phospholipase C based toxin can be measured as a change in cellular calcium ion concentration.

Exfoliative toxins - These toxins are produced by several Staphylococcal species and can consist of several serotypes. A specific identifier for these toxins can be constructed by measuring the morphological changes in their target organelle, the desmosome, which occur at the junctions between cells. The exfoliative toxins are known to change the morphology of the desmosomes into two smaller components called hemidesmosomes. In the high-content assay for exfoliative toxins, epithelial cells whose desmosomes are luminescently labeled are subjected to image analysis. An method that detects the morphological change between desmosomes and hemidesmosomes is used to quantify the activity of the toxins on the cells.

Most of these identifiers can be used in high throughput assays requiring no spatial resolution, as well as in high content assays.

Several biological threat agents act as specific proteases, and thus we have focused on the development of fluorescent protein biosensors that report the proteolytic cleavage of specific amino acid sequences found within the target proteins.

A number of such protease biosensors (including FRET biosensors) are disclosed above, such as the caspase biosensors, anthrax, tetanus, Botulinum, and the zinc metalloproteases. FRET is a powerful technique in that small changes in protein conformation, many of which are associated with toxin activity, can not only be measured with high precision in time and space within living cells, but can be measured in a high-throughput mode, as discussed above.

As described above, one of skill in the art will recognize that the protease biosensors of this aspect of the invention can be adapted to report the activity of any protease, by a substitution of the appropriate protease recognition site in any of the constructs **(see Figure 29B)**. As disclosed above, these biosensors can be used in high-content or high throughput screens to detect in vivo activation of enzymatic activity by toxins, and to identify specific activity based on cleavage of a known recognition motif. These biosensors can be used in both live cell and fixed end-point assays, and can be combined with additional measurements to provide a multi-parameter assay.

### Anthrax LF

Anthrax is a well-known agent of biological warfare and is an excellent target for development of a biosensor in the *identifier* class. Lethal factor (LF) is one of the protein components that confer toxicity to anthrax, and recently two of its targets within cells were identified. LF is a metalloprotease that specifically cleaves Mek1 and Mek2 proteins, kinases that are part of the MAP-kinase signaling pathway. Construction of lethal factor protease biosensors are described above. **(SEQ ID NO:7-8; 9-10)** Green fluorescent protein (GFP) is fused in-frame at the amino terminus of either Mek1 or Mek2 (or both), resulting in a chimeric protein that is retained in the cytoplasm due to the presence of a nuclear export sequence (NES) present in both of the target molecules. Upon cleavage by active lethal factor, GFP is released from the chimera and is free to diffuse into the nucleus. Therefore, measuring the accumulation of GFP in the nucleus provides a direct measure of LF activity on its natural target, the living cell.

While a preferred form of the invention has been shown in the drawings and described, since variations in the preferred form will be apparent to those skilled in the art, the invention should not be construed as limited to the specific form shown and described, but instead is as set forth in the claims.

## Claims

1. An automated method for cell based toxin characterization comprising
- providing an array of locations containing cells to be treated with a test substance, wherein the cells possess at least a first luminescent reporter molecule comprising a detector and a second luminescent reporter molecule selected from the group consisting of a classifier or an identifier;
- contacting the cells with the test substance either before or after possession of the first and second luminescent reporter molecules by the cells; wherein the localization, distribution, structure, or activity of the first and second luminescent reporter molecule is modified when the cell is contacted with the toxin,
- imaging or scanning multiple cells in each of the locations containing multiple cells to obtain luminescent signals from the detector;
- converting the luminescent signals from the detector into digital data;
- utilizing the digital data from the detector to automatically measure the localization, distributions, structure or activity of the detector on or in the cell, wherein a change in the localization, distribution, structure or activity of the detector indicates the presence of a toxin in the test substance;
- selectively imaging or scanning the locations containing cells that were contacted with test sample indicated to have a toxin in it to obtain luminescent signals from the second reporter molecule;
- converting the luminescent signals from the second luminescent reporter molecule into digital data;
- utilizing the digital data from the second luminescent reporter molecule to automatically measure the localization, distribution, structure or activity of the classifier or identifier on or in the cell, wherein a change in the localization, distribution, structure or activity of the classifier identifies a cell pathway that is perturbed by the toxin present in the test substance, or wherein a change in the localization, distribution, structure or activity of the identifier identifies the specific toxin or group of toxins that are present in the test substance.

2. The method of claim 1 wherein the second luminescent reporter molecule is a classifier, and the digital data derived from the classifier is used to select an appropriate identifier for identification of the specific toxin or group of toxins.

3. The method of claim 1, wherein the second luminescent reporter molecule is a classifier, and the cells further comprise a third luminescent reporter molecule, wherein the third luminescent reporter molecule comprises an identifier;
wherein the cells are contacted with the test substance either before or after possession of the third luminescent reporter molecule by the cells; wherein the localization, distribution, structure, or activity of the third luminescent reporter molecule is modified when the cell is contacted with the toxin, and wherein the method further comprises:
-- selectively imaging or scanning the locations containing cells that were contacted with test sample indicated to have a toxin in it to obtain luminescent signals from the identifier;
- converting the luminescent signals from the identifier into digital data; and
- utilizing the digital data from the identifier to automatically measure the localization, distribution structure, or activity of the identifier on or in the cell, wherein a change in the localization, distribution, structure or activity of the identifier identifies the specific toxin or group of toxins that is present in the test substance.

4. The method of claim 3 wherein the digital data derived from the classifier is used to select an appropriate identifier for identification of the specific toxin or group of toxins.

5. The method of any one of claim 1-4 wherein the detector comprises a molecule selected from the group consisting of heat shock proteins and compounds that respond to changes in mitochondrial membrane potential, intracellular free ion concentration, cytoskeletal organization, general metabolic status, cell cycle timing events, and organellar structure and function.

6. The method of any one of claim 1-5 wherein the classifier comprises a molecule selected from the group consisting of tubulin, microtubule-associated proteins, actin, actin-binding proteins, NF-κB, IκB, and stress-activated kinases.

7. The method of any one of claim 1-6 wherein the cell pathway is selected from the group consisting of cell stress pathways, cell metabolic pathways, cell signaling pathways, cell growth pathways, and cell division pathways.

8. The method of claim 1, wherein the second luminescent reporter molecule is an identifier, and the identifier identifies a toxin or group of toxins selected from the group consisting of proteases, ADP-ribosylating toxins, cytotoxic phospholipases, and exfoliative toxins.

9. The method of any one of claim 3-7, wherein the identifier identifies a toxin or group of toxins selected from the group consisting of proteases, ADP-ribosylating toxins, cytotoxic phospholipases, and exfoliative toxins.

10. The method of any of claims 1-9 wherein the change in the localization, distribution, structure or activity of the first, second, or third luminescent reporter molecules is selected from the group consisting of cytoplasm to nucleus translocation, nucleus or nucleolus to cytoplasm translocation, receptor internalization, mitochondrial membrane potential, loss of signal, the spectral response of the reporter molecule, phosphorylation, intracellular free ion concentration, cell size, cell shape, cytoskeleton organization, metabolic processes, cell motility, cell substrate attachment, cell cycle events, and organellar structure and function.

11. The method of any one of claims 1-10, wherein the imaging or scanning multiple cells in each of the locations containing multiple cells to obtain luminescent signals from the detector is carried out in a high throughput mode.

12. The method of any one of claims 1-10, wherein the imaging or scanning multiple cells in each of the locations containing multiple cells to obtain luminescent signals from the detector is carried out in a high content mode.

13. The method of claim 1-10 wherein the selective imaging or scanning of the locations containing cells that were contacted with test sample indicated to have a toxin in it to obtain luminescent signals from the second or third reporter molecule is carried out in a high throughput mode.

14. The method of claim 1-10 wherein the selective imaging or scanning of the locations containing cells that were contacted with test sample indicated to have a toxin in it to obtain luminescent signals from the second or third reporter molecule is carried out in a high content mode.

15. The method of any one of claims 1-14 further comprising providing a digital storage media for data storage and archiving.

16. The method of claim 15 further comprising a means for automated control, acquisition, processing and display of results.

17. A computer readable storage medium comprising a program containing a set of instructions for causing a cell screening system to execute the method of any one of claims 1-16, wherein the cell screening system comprises an optical system with a stage adapted for holding a plate containing cells, a means for moving the stage or the optical system, a digital camera, a means for directing light emitted from the cells to the digital camera, and a computer means for receiving and processing the digital data from the digital camera.

18. An automated method for cell based toxin characterization comprising
- providing a first array of locations containing cells to be treated with a test substance, wherein the cells possess at least a first luminescent reporter molecule comprising a reporter molecule selected from the group consisting of detectors and classifiers;
- contacting the cells with the test substance either before or after possession of the first luminescent reporter molecule by the cells; wherein the localization, distribution, structure, or activity of the first luminescent reporter molecule is modified when the cell is contacted with the toxin,
- imaging or scanning multiple cells in each of the locations containing multiple cells to obtain luminescent signals from the at least first luminescent reporter molecule;
- converting the luminescent signals from the at least first luminescent reporter molecule into digital data;
- utilizing the digital data from the at least first luminescent reporter molecule to automatically measure the localization, distribution, structure or activity of the at least first luminescent reporter molecule on or in the cell, wherein a change in the localization, distribution, structure or activity of the at least first luminescent reporter molecule indicates the presence of a toxin in the test substance,
- providing a second array of locations containing cells to be treated with the test substance, wherein the cells possess a least a second luminescent reporter molecule comprising a reporter molecule selected from the group consisting of classifiers and identifiers, and wherein the second array of locations containing cells can comprise either the same or a different cell type as the first array of locations containing cells;
- contacting the second array of locations containing cells with the test substance either before or after possession of the second luminescent reporter molecule by the cells; wherein the localization, distribution, structure, or activity of the second luminescent reporter molecule is modified when the cell is contacted with the toxin;
- utilizing the digital data from the second luminescent reporter molecule to automatically measure the localization, distribution, structure or activity of the classifier or identifier on or in the cell, wherein a change in the localization, distribution, structure or activity of the classifier identifies a cell pathway that is perturbed by the toxin present in the test substance, or wherein a change in the localization, distribution, structure or activity of the identifier identifies the specific toxin or group of toxins that are present in the test substance.

## Patentansprüche

1. Ein automatisiertes Verfahren für Zell-basierte Toxin-Charakterisierung, umfassend
- Bereitstellung einer Anordnung ("Array") von Stellen, die Zellen enthalten, die mit einer Testsubstanz behandelt werden sollen, wobei die Zellen wenigstens ein erstes lumineszierendes Reportermolekül, das einen Detektor umfasst und ein zweites lumineszierendes Reportermolekül besitzen, ausgewählt aus der Gruppe bestehend aus einem Klassifikator ("classifier") oder einem Identifikator ("identifier");
- In Kontakt bringen der Zellen mit der Testsubstanz entweder vor oder nachdem die Zellen die ersten und zweiten lumineszierenden Reportermoleküle enthalten; wobei die Lokalisation, Verteilung, Struktur oder Aktivität des ersten und zweiten lumineszierenden Reportermoleküls modifiziert wird, wenn die Zelle mit dem Toxin in Kontakt gebracht wird,
- Aufnahme oder Scanning multipler Zellen an allen Stellen, die multiple Zellen enthalten, um lumineszierende Signale vom Detektor zu erhalten;
- Konvertierung der lumineszierenden Signale vom Detektor in digitale Daten:
- Verwendung der digitalen Daten vom Detektor zur automatischen Messung der Lokalisation, Verteilung, Struktur oder Aktivität des Detektors auf oder in der Zelle, wobei eine Veränderung der Lokalisation, Verteilung, Struktur oder Aktivität des Detektors die Anwesenheit eines Toxins in der Testsubstanz zeigt;
- selektive Aufnahme oder Scanning der Stellen, die Zellen enthalten, die mit einer Testprobe in Kontakt gebracht wurden, die als Toxin-enthaltend **gekennzeichnet** ist, zum Erhalt von lumineszierenden Signalen vom zweiten Reportermolekül;
- Konvertierung der lumineszierenden Signale vom zweiten lumineszierenden Reportermolekül in digitale Daten;
- Verwendung der digitalen Daten vom zweiten lumineszierenden Reportermolekül zur automatischen Messung der Lokalisation, Verteilung, Struktur oder Aktivität des Klassifikators oder Identifikators auf oder in der Zelle, wobei eine Veränderung der Lokalisation, Verteilung, Struktur oder Aktivität des Klassifikators einen zellulären Weg identifiziert, der durch das in der Testsubstanz anwesende Toxin gestört wird, oder wobei eine Veränderung der Lokalisation, Verteilung, Struktur oder Aktivität des Identifikators das spezifische Toxin oder die Gruppe von Toxinen identifiziert, die in der Testsubstanz anwesend sind.

2. Verfahren gemäß Anspruch 1, wobei das zweite lumineszierende Reportermolekül ein Klassifikator ist und die von dem Klassifikator abgeleiteten digitalen Daten dazu verwendet werden, einen geeigneten Identifikator für die Identifizierung des spezifischen Toxins oder von Gruppen von Toxinen auszuwählen.

3. Verfahren gemäß Anspruch 1, wobei das zweite lumineszierende Reportermolekül ein Klassifikator ist und die Zellen weiterhin ein drittes lumineszierendes Reportermolekül umfassen, wobei das dritte lumineszierende Reportermolekül einen Identifikator umfasst; wobei die Zellen entweder vor oder nachdem sie das dritte lumineszierende Reportermolekül enthalten mit der Testsubstanz in Kontakt gebracht werden; wobei die Lokalisation, Verteilung, Struktur oder Aktivität des dritten lumineszierenden Reportermolekül modifiziert wird, wenn die Zelle mit dem Toxin in Kontakt gebracht wird, wobei das Verfahren weiter umfasst:
- selektive Aufnahme oder Scanning der Stellen, die Zellen enthalten, die mit einer Testprobe in Kontakt gebracht wurden, die als Toxin-enthaltend **gekennzeichnet** ist, um lumineszierende Signale vom Identifikator zu erhalten;
- Konvertierung der lumineszierenden Signale vom Identifikator in digitale Daten; und
- Verwendung der digitalen Daten vom Identifikator zur automatischen Messung der Lokalisation, Verteilung, Struktur oder Aktivität des Identifikators auf oder in der Zelle, wobei eine Veränderung der Lokalisation, Verteilung, Struktur oder Aktivität des Identifikators das spezifische Toxin oder die Gruppe von Toxinen identifiziert, die in der Testsubstanz anwesend ist.

4. Verfahren gemäß Anspruch 3, wobei die digitalen Daten, die vom Klassifikator abgeleitet sind, dazu verwendet werden, einen geeigneten Identifikator für die Identifizierung eines spezifischen Toxins oder einer Gruppe von Toxinen auszuwählen.

5. Verfahren gemäß einem der Ansprüche 1-4, wobei der Detektor ein Molekül umfasst, ausgewählt aus der Gruppe bestehend aus Hitzeschockproteinen und Verbindungen, die auf Veränderungen des mitochondrialen Membranpotentials, der intrazellulären freien Ionenkonzentration, der Organisation des Zytoskeletts, des allgemeinen metabolischen Status, der zeitlich regulierten Ereignisse im Zellzyklus und der Struktur und Funktion von Organellen reagieren.

6. Verfahren gemäß einem der Ansprüche 1-5, wobei der Klassifikator ein Molekül umfasst, ausgewählt aus der Gruppe bestehend aus Tubulin, Microtubuli-assoziierten Proteinen, Actin, Actin-bindenden Proteinen, NF-κB, IκB und stress-aktivierten Kinasen.

7. Verfahren gemäß einem der Ansprüche 1-6, wobei der zelluläre Weg ausgewählt ist aus der Gruppe bestehend aus Zell-Stresswegen, Zell-Metabolismuswegen, Zell-Signalübertragungswegen, Zellwachstumswegen und Zellteilungswegen.

8. Verfahren gemäß Anspruch 1, wobei das zweite lumineszierende Reportermolekül ein Identifikator ist, und der Identifikator identifiziert ein Toxin oder eine Gruppe von Toxinen, ausgewählt aus der Gruppe bestehend aus Proteasen, ADP-ribosylierenden Toxinen, cytotoxischen Phospholipasen und entblätternden Toxinen.

9. Verfahren gemäß einem der Ansprüche 3-7, wobei der Identifikator ein Toxin oder eine Gruppe von Toxinen identifiziert, ausgewählt aus der Gruppe bestehend aus Proteasen, ADP-ribosylierenden Toxinen, cytotoxischen Phospholipasen und entblätternden Toxinen.

10. Verfahren gemäß einem der Ansprüche 1-9, wobei die Veränderung der Lokalisation, Verteilung, Struktur oder Aktivität der ersten, zweiten oder dritten lumineszierenden Reportermoleküle ausgewählt ist aus der Gruppe bestehend aus Translokation vom Cytoplasma in den Nukleus, Translokation vom Nukleus oder Nukleolus ins Cytoplasma, Rezeptorinternalisation, mitochondriales Membranpotential, Signalverlust, spektrale Antwort des Reportermoleküls, Phosphorylierung, intrazelluläre Konzentration freier lonen, Zellgröße, Zellform, Organisation des Zytoskeletts, metabolische Vorgänge, Zellmotilität, Zell-Substratanheftung, Zellzyklusereignisse und Struktur und Funktion von Organellen.

11. Verfahren gemäß einem der Ansprüche 1-10, wobei die Aufnahme oder das Scanning multipler Zellen an allen Stellen, die multiple Zellen enthalten, zum Erhalt lumineszierender Signale vom Detektor in einem Modus mit hohem Durchsatz durchgeführt werden.

12. Verfahren gemäß einem der Ansprüche 1-10, wobei die Aufnahme oder das Scanning multipler Zellen an allen Stellen, die multiple Zellen enthalten, zum Erhalt lumineszierender Signale vom Detektor in einem Modus mit hohem Informationsgehalt ("high content mode") durchgeführt werden.

13. Verfahren gemäß Anspruch 1-10, wobei die selektive Aufnahme oder das Scanning der Stellen, die Zellen enthalten, die mit einer Testprobe in Kontakt gebracht wurden, die als Toxin-enthaltend **gekennzeichnet** ist, zum Erhalt lumineszierender Signale vom zweiten oder dritten Reportermolekül in einem Modus mit hohem Durchsatz durchgeführt werden.

14. Verfahren gemäß Anspruch 1-10, wobei die selektive Aufnahme oder das Scanning der Stellen, die Zellen enthalten, die mit einer Testprobe in Kontakt gebracht wurden, die als Toxin-enthaltend **gekennzeichnet** ist, zum Erhalt lumineszierender Signale vom zweiten oder dritten Reportermolekül in einem Modus mit hohem Informationsgehalt durchgeführt werden.

15. Verfahren gemäß einem der Ansprüche 1-14, weiter umfassend die Bereitstellung eines digitalen Speichermediums für die Datenspeicherung und zur Archivierung.

16. Verfahren gemäß Anspruch 15, weiter umfassend ein Hilfsmittel für die automatisierte Kontrolle, Erfassung, Verarbeitung und Darstellung von Daten.

17. Ein computerlesbares Speichermedium, umfassend ein Programm, das einen Satz von Instruktionen enthält, damit ein Zellscreeningsystem das Verfahren gemäß einem der Ansprüche 1-16 ausführt, wobei das Zellscreening-System ein optisches System mit einem Gestell umfasst, das dazu geeignet ist, eine Platte, die Zellen enthält, zu halten, ein Hilfsmittel zur Bewegung des Gestells oder des optischen Systems, eine Digitalkamera, ein Hilfsmittel zur Ausrichtung des von den Zellen emittierten Lichtes zur Digitalkamera, und einen Computer zum Erhalt und zur Verarbeitung der digitalen Daten von der digitalen Kamera.

18. Ein automatisiertes Verfahren für Zell-basierte Toxin-Charakterisierung, umfassend
- Bereitstellung einer ersten Anordnung ("Array") von Stellen, die Zellen enthalten, die mit einer Testsubstanz behandelt werden sollen, wobei die Zellen wenigstens ein erstes lumineszierendes Reportermolekül, umfassend ein Reportermolekül, ausgewählt aus der Gruppe bestehend aus Detektoren und Klassifikatoren, besitzen;
- In Kontakt bringen der Zellen mit der Testsubstanz entweder vor oder nachdem die Zellen das erste Reportermolekül enthalten; wobei die Lokalisation, Verteilung, Struktur, oder Aktivität des ersten lumineszierenden Reportermoleküls modifiziert wird, wenn die Zelle mit dem Toxin in Kontakt gebracht wird,
- Aufnahme oder Scanning multipler Zellen an allen Stellen, die multiple Zellen enthalten, um lumineszierende Signale vom wenigstens ersten lumineszierenden Reportermolekül zu erhalten;
- Konvertierung der lumineszierenden Signale vom wenigstens ersten lumineszierenden Reportermolekül in digitale Daten:
- Verwendung der digitalen Daten vom wenigstens ersten lumineszierenden Reportermolekül zur automatischen Messung der Lokalisation, Verteilung, Struktur oder Aktivität des wenigstens ersten lumineszierenden Reportermoleküls auf oder in der Zelle, wobei eine Veränderung der Lokalisation, Verteilung, Struktur oder Aktivität des wenigstens ersten lumineszierenden Reportermolekül die Anwesenheit eines Toxins in der Testsubstanz zeigt;
- Bereitstellung einer zweiten Anordnung von Stellen, die Zellen enthalten, die mit einer Testsubstanz behandelt werden sollen, wobei die Zellen wenigstens ein zweites lumineszierendes Reportermolekül, umfassend ein Reportermolekül ausgewählt aus der Gruppe bestehend aus Klassifikatoren und Identifikatoren besitzen, und wobei die zweite Anordnung von Stellen, die Zellen enthalten, entweder den gleichen oder einen anderen Zelltyp als die erste Anordnung von Stellen, die Zellen enthält, umfassen kann;
- In Kontakt bringen der zweiten Anordnung von Stellen mit der Testsubstanz entweder vor oder nachdem die Zellen das zweite lumineszierende Reportermolekül enthalten; wobei die Lokalisation, Verteilung, Struktur oder Aktivität des zweiten lumineszierenden Reportermoleküls modifiziert wird, wenn die Zelle mit dem Toxin in Kontakt gebracht wird,
- Verwendung der digitalen Daten vom zweiten lumineszierenden Reportermolekül zur automatischen Messung der Lokalisation, Verteilung, Struktur oder Aktivität des Klassifikators oder Identifikators auf oder in der Zelle, wobei eine Veränderung der Lokalisation, Verteilung, Struktur oder Aktivität des Klassifikators einen zellulären Weg identifiziert, der durch das in der Testsubstanz anwesende Toxin gestört wird, oder wobei eine Veränderung der Lokalisation, Verteilung, Struktur oder Aktivität des Identifikators das spezifische Toxin oder die Gruppe von Toxinen identifiziert, die in der Testsubstanz anwesend sind.

## Revendications

1. Procédé automatisé pour la caractérisation de toxines à base de cellules, comprenant
- la fourniture d'un arrangement d'emplacements contenant des cellules à traiter avec une substance d'essai, dans lequel les cellules possèdent au moins une première molécule reporter luminescente comprenant un détecteur et une deuxième molécule reporter luminescente choisie dans le groupe consistant en un classificateur et un identificateur;
- la mise en contact des cellules avec la substance d'essai soit avant soit après possession des première et deuxième molécules reporters luminescentes par les cellules; tandis que la localisation, la distribution, la structure ou l'activité de la première et de la deuxième molécule reporter luminescente est modifiée quand la cellule est mise en contact avec la toxine,
- la visualisation ou l'exploration de cellules multiples dans chacune des localisations contenant des cellules multiples pour obtenir des signaux luminescents provenant du détecteur;
- la conversion des signaux luminescents provenant du détecteur en données numériques;
- l'utilisation des données numériques provenant du détecteur pour la mesure automatique de la localisation, la distribution, la structure ou l'activité du détecteur sur ou dans la cellule, tandis qu'une modification de la localisation, la distribution, la structure ou l'activité du détecteur indique la présence d'une toxine dans la substance d'essai;
- la visualisation ou l'exploration sélective des localisations contenant des cellules qui ont été mises en contact avec l'échantillon d'essai indiqué comme ayant une toxine en lui, pour l'obtention de signaux luminescents provenant de la deuxième molécule reporter;
- la conversion des signaux luminescents provenant de la deuxième molécule reporter luminescente en des données numériques;
- l'utilisation des données numériques provenant de la deuxième molécule reporter luminescente pour la mesure automatique de la localisation, la distribution, la structure ou l'activité du classificateur ou de l'identificateur sur ou dans la cellule, tandis qu'une modification de la localisation, la distribution, la structure ou l'activité du classificateur identifie une voie cellulaire qui est perturbée par la toxine présente dans la substance d'essai, ou tandis qu'une modification dans la localisation, la distribution, la structure ou l'activité de l'identificateur identifie la toxine spécifique ou le groupe de toxines qui sont présentes dans la substance d'essai.

2. Procédé selon la revendication 1, dans lequel la deuxième molécule reporter luminescente est un classificateur et les données numériques dérivées du classificateur sont utilisées pour choisir un identificateur approprié pour l'identification de la toxine spécifique ou du groupe de toxines.

3. Procédé selon la revendication 1, dans lequel la deuxième molécule reporter luminescente est un classificateur et les cellules comprennent en outre une troisième molécule reporter luminescente, tandis que la troisième molécule reporter luminescente comprend un identificateur; tandis que les cellules sont mises en contact avec la substance d'essai soit avant soit après possession de la troisième molécule reporter luminescente par les cellules; tandis que la localisation, la distribution, la structure ou l'activité de la troisième molécule reporter luminescente est modifiée quand la cellule est mise en contact avec la toxine, et tandis que le procédé comprend en outre:
- la visualisation ou l'exploration sélective des emplacements contenant des cellules qui ont été mises en contact avec un échantillon d'essai indiqué comme comportant une toxine, pour l'obtention de signaux luminescents à partir de l'identificateur;
- la conversion des signaux luminescents provenant de l'identificateur en données numériques, et
- l'utilisation des données numériques provenant de l'identificateur pour mesurer automatiquement la localisation, la distribution, la structure ou l'activité de l'identificateur sur ou dans la cellule, tandis qu'une modification de la localisation, la distribution, la structure ou l'activité de l'identificateur identifie la toxine spécifique ou le groupe de toxines qui est présent dans la substance d'essai.

4. Procédé selon la revendication 3, dans lequel la donnée numérique dérivée du classificateur est utilisée pour la sélection d'un identificateur approprié pour l'identification de la toxine spécifique ou du groupe de toxines.

5. Procédé selon l'une quelconque des revendications 1-4, dans lequel le détecteur comprend une molécule choisie dans le groupe consistant en des protéines du choc thermique et des composés qui répondent à des modifications dans le potentiel de membrane mytochondriale, la concentration en ions libres intracellulaires, l'organisation du cytosquelette, l'état métabolique général, les événements temporels du cycle cellulaire, et la structure et la fonction des organites.

6. Procédé selon l'une quelconque des revendications 1-5, dans lequel le classificateur comprend une molécule choisie dans le groupe consistant en la tubuline, des protéines associées aux microtubules, l'actine, des protéines de liaison à l'actine, NF-κB, IκB, et des kinases activées par un stress.

7. Procédé selon l'une quelconque des revendications 1-6, dans lequel la voie cellulaire est choisie dans le groupe consistant en les voies de stress cellulaires, les voies métaboliques cellulaires, les voies de signalement cellulaire, les voies de croissance cellulaire et les voies de division cellulaire.

8. Procédé selon la revendication 1, dans lequel la deuxième molécule reporter luminescente est un identificateur, et l'identificateur identifie une toxine ou un groupe de toxines choisies dans le groupe consistant en des protéases, des toxines de ribosylation d'ADP, des phospholipases cytotoxiques, et des toxines exfoliatives.

9. Procédé selon l'une quelconque des revendications 3-7, dans lequel l'identificateur identifie une toxine ou un groupe de toxines choisies dans le groupe consistant en des protéases, des toxines de ribosylation d'ADP, des phospholipases cytotoxiques, et des toxines exfoliatives.

10. Procédé selon l'une quelconque des revendications 1-9, dans lequel la modification dans la localisation, la distribution, la structure ou l'activité des première, deuxième ou troisième molécules reporters luminescentes est choisie dans le groupe consistant en une translocation du cytoplasme au noyau, une translocation du noyau ou du nucléole au cytoplasme, une internalisation du récepteur, un potentiel de membrane mitochondriale, une perte de signal, la réponse spectrale de la molécule reporter, une phosphorylation, une concentration d'ions libres intracellulaires, la taille des cellules, la forme des cellules, l'organisation du cytosquelette, des procédés métaboliques, la mobilité des cellules, la fixation du substrat des cellules, des événements du cycle cellulaire, et la structure et la fonction des organites.

11. Procédé selon l'une quelconque des revendications 1-10, dans lequel la visualisation ou l'exploration de cellules multiples dans chacun des emplacements contenant des cellules multiples pour obtenir des signaux luminescents à partir du détecteur est effectuée dans un mode à haut débit.

12. Procédé selon l'une quelconque des revendications 1-10, dans lequel la visualisation ou l'exploration de cellules multiples dans chacun des emplacements contenant des cellules multiples pour obtenir des signaux luminescents à partir du détecteur est effectuée dans un mode à haute teneur.

13. Procédé selon la revendication 1-10, dans lequel la visualisation ou l'exploration sélective des emplacements contenant des cellules qui ont été mises en contact avec un échantillon d'essai indiqué comme comportant une toxine pour obtenir des signaux luminescents à partir de la deuxième ou troisième molécule reporter est effectuée dans un mode à haut débit.

14. Procédé selon la revendication 1-10, dans lequel la visualisation ou l'exploration sélective des emplacements contenant des cellules qui ont été mises en contact avec un échantillon d'essai indiqué comme comportant une toxine pour obtenir des signaux luminescents à partir de la deuxième ou troisième molécule reporter est effectuée dans un mode à haute teneur.

15. Procédé selon l'une quelconque des revendications 1-14, comprenant en outre la fourniture d'un moyen de stockage numérique pour le stockage et l'archivage des données.

16. Procédé selon la revendication 15, comprenant en outre un moyen pour le contrôle, l'acquisition, le traitement et l'affichage automatisés des résultats.

17. Moyen de stockage lisible par ordinateur, comprenant un programme contenant un ensemble d'instructions pour provoquer une exécution par un système de criblage cellulaire du procédé selon l'une quelconque des revendications 1-16, dans lequel le système de criblage cellulaire comprend un système optique ayant une platine adaptée pour le maintien d'une plaque contenant des cellules, un moyen pour le déplacement de la platine ou du système optique, une caméra numérique, un moyen pour diriger la lumière émise par les cellules sur la caméra numérique, et un moyen informatique pour recevoir et traiter les données numériques provenant de la caméra numérique.

18. Procédé automatisé pour la caractérisation de toxines à base de cellules, comprenant
- la fourniture d'un premier arrangement d'emplacements contenant des cellules à traiter avec une substance d'essai, tandis que les cellules possèdent au moins une première molécule reporter luminescente comprenant une molécule reporter choisie dans le groupe consistant en des détecteurs et des classificateurs;
- la mise en contact des cellules avec la substance d'essai soit avant soit après possession de la première molécule reporter luminescente par les cellules; tandis que la localisation, la distribution, la structure ou l'activité de la première molécule reporter luminescente est modifiée lorsque la cellule est mise en contact avec la toxine,
- la visualisation ou l'exploration de cellules multiples dans chacun des emplacements contenant des cellules multiples pour obtenir des signaux luminescents provenant de la au moins première molécule reporter luminescente;
- la conversion des signaux luminescents provenant de la au moins première molécule reporter luminescente en données numériques;
- l'utilisation des données numériques provenant de la au moins première molécule reporter luminescente pour mesurer automatiquement la localisation, la distribution, la structure ou l'activité de la au moins première molécule reporter luminescente sur ou dans la cellule, tandis qu'une modification dans la localisation, la distribution, la structure ou l'activité de la au moins première molécule reporter luminescente indique la présence d'une toxine dans la substance d'essai,
- la fourniture d'un deuxième arrangement d'emplacements contenant des cellules à traiter avec la substance d'essai, tandis que les cellules possèdent au moins une deuxième molécule reporter luminescente comprenant une molécule reporter choisie dans le groupe consistant en des classificateurs et des identificateurs, et tandis que le deuxième arrangement d'emplacements contenant des cellules peut comprendre un type de cellules soit identique, soit différent du premier arrangement d'emplacements contenant des cellules;
- la mise en contact du deuxième arrangement d'emplacements contenant des cellules avec la substance d'essai soit avant soit après possession de la deuxième molécule reporter luminescente par les cellules; tandis que la localisation, la distribution, la structure ou l'activité de la deuxième molécule reporter luminescente est modifiée lorsque la cellule est mise en contact avec la toxine;
- l'utilisation des données numériques provenant de la deuxième molécule reporter luminescente pour mesurer automatiquement la localisation, la distribution, la structure ou l'activité du classificateur ou de l'identificateur sur ou dans la cellule, tandis qu'une modification dans la localisation, la distribution, la structure ou l'activité du classificateur identifie une voie cellulaire qui est perturbée par la toxine présente dans la substance d'essai, ou tandis qu'une modification dans la localisation, la distribution, la structure ou l'activité de l'identificateur identifie la toxine spécifique ou le groupe de toxines qui sont présentes dans la substance d'essai.
